# EUROPEAN PATENT APPLICATION

(11) **EP 2 067 483 A1**
(43) Date of publication of application: **10.06.2009**
(21) Application number: 09156363.5
(22) Date of filing: 17.02.2004
(51) Int. Cl.: A61K 38/26, C07K 14/605

(54) **Analogues of GLP-1**

(30) Priority: 19.02.2003 US 449203 P
(62) Divisional of application: 04711811.2
(71) Applicant: IPSEN PHARMA, 92100 Boulogne-Billancourt (FR)
(72) Inventor: Dong, Zheng Xin, Holliston, MA 01746 (US)
(74) Representative: Harrison Goddard Foote

(57) **Abstract**

The present invention is directed to peptide analogues of glucagon-like peptide-1, the pharmaceutically-acceptable salts thereof, to methods of using such analogues to treat mammals, and to pharmaceutical compositions useful therefor comprising said analogues.

## Description

### Sequence Listing

The instant application contains a "lengthy" Sequence Listing which has been submitted via four CD-R in lieu of a printed paper copy, and is hereby incorporated by reference in its entirety. Said CD-R, recorded on February, 10, 2004, are labeled "CRF", "Copy 1", "Copy 2" and "Copy 3" respectively, and each contains only one identical 456 Kb file (129PPCT2.APP).

### Background of the Invention

The present invention is directed to peptide analogues of glucagon-like peptide-1, the pharmaceutically-acceptable salts thereof, to methods of using such analogues to treat mammals and to pharmaceutical compositions useful therefore comprising said analogues.

Glucagon-like peptide-1 (7-36) amide (GLP-1)(SEQ ID NO: 775) is synthesized in the intestinal L-cells by tissue-specific post-translational processing of the glucagon precursor preproglucagon (Vamdell, J.M., et al., J. Histochem Cytochem, 1985:33:1080-6) and is released into the circulation in response to a meal. The plasma concentration of GLP-1 rises from a fasting level of approximately 15 pmol/L to a peak postprandial level of 40 pmol/L. It has been demonstrated that, for a given rise in plasma glucose concentration, the increase in plasma insulin is approximately threefold greater when glucose is administered orally compared with intravenously (Kreymann, B., et al., Lancet 1987:2, 1300-4). This alimentary enhancement of insulin release, known as the incretin effect, is primarily humoral and GLP-1 is now thought to be the most potent physiological incretin in humans. In addition to the insulinotropic effect, GLP-1 suppresses glucagon secretion, delays gastric emptying (Wettergren A., et al., Dig Dis Sci 1993:38:665-73) and may enhance peripheral glucose disposal (D'Alessio, D.A. et al., J. Clin Invest 1994:93:2293-6).

In 1994, the therapeutic potential of GLP-1 was suggested following the observation that a single subcutaneous (s/c) dose of GLP-1 could completely normalize postprandial glucose levels in patients with non-insulin-dependent diabetes mellitus (NIDDM) (Gutniak, M.K., et al., Diabetes Care 1994:17:1039-44). This effect was thought to be mediated both by increased insulin release and by a reduction in glucagon secretion. Furthermore, an intravenous infusion of GLP-1 has been shown to delay postprandial gastric emptying in patients with NIDDM (Williams, B., et al., J. Clin Endo Metab 1996:81:327-32). Unlike sulphonylureas, the insulinotropic action of GLP-1 is dependent on plasma glucose concentration (Holz, G.G. 4th, et al., Nature 1993:361:362-5). Thus, the loss of GLP-1-mediated insulin release at low plasma glucose concentration protects against severe hypoglycemia. This combination of actions gives GLP-1 unique potential therapeutic advantages over other agents currently used to treat NIDDM.

Numerous studies have shown that when given to healthy subjects, GLP-1 potently influences glycemic levels as well as insulin and glucagon concentrations (Orskov, C, Diabetologia 35:701-711, 1992; Holst, J.J., et al., Potential of GLP-1 in diabetes management in Glucagon III, Handbook of Experimental Pharmacology, Lefevbre PJ, Ed. Berlin, Springer Verlag, 1996, p. 311-326), effects which are glucose dependent (Kreymann, B., et al., Lancet ii: 1300-1304, 1987; Weir, G.C., et al., Diabetes 38:338-342, 1989). Moreover, it is also effective in patients with diabetes (Gutniak, M., N. Engl J Med 226:1316-1322, 1992; Nathan, D.M., et al., Diabetes Care 15:270-276, 1992), normalizing blood glucose levels in type 2 diabetic subjects (Nauck, M.A., et al., Diagbetologia 36:741-744, 1993), and improving glycemic control in type 1 patients (Creutzfeldt, W.O., et al., Diabetes Care 19:580-586, 1996), raising the possibility of its use as a therapeutic agent.

GLP-1 is, however, metabolically unstable, having a plasma half-life (t_{1/2}) of only 1-2 min *in vivo*. Exogenously administered GLP-1 is also rapidly degraded (Deacon, C.F., et al., Diabetes 44:1126-1731, 1995). This metabolic instability limits the therapeutic potential of native GLP-1. Hence, there is a need for GLP-1 analogues that are more active or are more metabolically stable than native GLP-1.

### Summary of the Invention

In one aspect, the present invention is directed to a compound of formula (I),

(R²R³)-A⁷⁻A⁸-A⁹-A¹⁰-A¹¹-A¹²-A¹³-A¹⁴-A¹⁵-A¹⁶-A¹⁷⁻A¹⁸-A¹⁹-A²⁰-A²¹-A²²-A²³-A²⁴-A²⁵-A²⁶-A²⁷⁻A²⁸-A²⁹-A³⁰-A³¹-A³²-A³³-A³⁴-A³⁵-A³⁶-A³⁷⁻A³⁸-A³⁹-R¹, (I)

wherein:

A⁷ is L-His, Ura, Paa, Pta, Amp, Tma-His, des-amino-His, or deleted;

A⁸ is Abu, β-Ala, Ser, Gly or Val;

A⁹ is Glu, N-Me-Glu, N-Me-Asp or Asp;

A¹⁰ is Gly, Acc, β-Ala or Aib;

A¹¹ is Thr or Ser;

A¹² is Phe, Acc, Aic, Aib, 3-Pal, 4-Pal, 1-Nal, 2-Nal, Cha, Trp or X¹-Phe;

A¹³ is Thr or Ser;

A¹⁴ is Ser or Aib;

A¹⁵ is Asp or Glu;

A¹⁶ is Val, Acc, Aib, Leu, Ile, Tle, Nle, Abu, Ala or Cha;

A¹⁷ is Alb, Ser or Thr;

A¹⁸ is Lys, Ser or Thr;

A¹⁹ is Tyr, Cha, Phe, 3-Pal, 4-Pal, Acc, 1-Nal, 2-Nal, or X¹-Phe;

A²⁰ is Leu, Acc, Aib, Nle, Ile, Cha, Tle, Val, Phe or X¹-Phe;

A²¹ is Glu or Asp;

A²² is Gly, Acc, β-Ala, Glu or Aib;

A²³ is Gln, Asp, Asn or Glu;

A²⁴ is Ala, Aib, Val, Abu, Tle or Acc;

A²⁵ is Ala, Aib, Val, Abu, Tie, Acc, Lys, Arg, hArg, Orn, HN-CH((CH₂)ₙ-N(R¹⁰R¹¹))-C(O) or HN-CH((CH₂)ₑ-X³)-C(O);

A²⁶ is Lys, Arg, hArg, Orn, HN-CH((CH₂)ₙ-N(R¹⁰R¹¹))-C(O) or HN-CH((CH₂)ₑ-X³)-C(O);

A²⁷ is Glu, Asp, Leu, Aib or Lys;

A²⁸ is Phe, 3-Pal, 4-Pal, 1-Nal, 2-Nal, X¹-Phe, Aic, Acc, Aib, Cha or Trp;

A²⁹ is Ile, Acc, Aib, Leu, Nle, Cha, Tle, Val, Abu, Ala or Phe;

A³⁰ is Ala, Aib or Acc;

A³¹ is Trp,-1-Nal, 2-Nal, 3-Pal, 4-Pal, Phe, Acc, Aib or Cha;

A³² is Leu, Acc, Aib, Nle, Ile, Cha, Tle, Phe, X¹-Phe or Ala;

A³³ is Val, Acc, Aib, Leu, Ile, Tie, Nle, Cha, Ala, Phe, Abu, Lys or X¹-Phe;

A³⁴ is Lys, Arg, hArg, Orn, HN-CH((CH₂)ₙ-N(R¹⁰R¹¹))-C(O) or HN-CH((CH₂)ₑ-X³)-C(O);

A³⁵ is Gly, β-Ala, D-Ala, Gaba, Ava, HN-(CH₂)ₘ-C(O), Aib, Acc or a D-amino acid;

A³⁶ is L- or D-Arg, D- or L-Lys, D- or L-hArg, D- or L-Orn, HN-CH((CH₂)ₙ-N(R¹⁰R¹¹))-C(O), HN-CH((CH₂)ₑ-X³)-C(O) or deleted;

A³⁷ is Gly, β-Ala, Gaba, Ava, Aib, Acc, Ado, Arg, Asp, Aun, Aec, HN-(CH₂)ₘ-C(O), HN-CH((CH₂)ₙ-N(R¹⁰R¹¹))-C(O), HN-CH((CH₂)ₙ-O(R¹⁰))-C(O),a D-amino acid, or deleted;

A³⁸ is D- or L-Lys, D- or L-Arg, D- or L-hArg, D- or L-Orn, HN-CH((CH₂)ₙ-N(R¹⁰R¹¹))-C(O), HN-CH((CH₂)ₑ-X³)-C(O) Ava, Ado, Aec or deleted;

A³⁹ is D- or L-Lys, D- or L-Arg, HN-CH((CH₂)ₙ-N(R¹⁰R¹¹))-C(O), Ava, Ado, Aec, or Aun, or is deleted;

X¹ is, independently for each occurrence, selected from the group consisting of (C₁-C₆)alkyl, OH and halo;

R¹ is OH, NH₂, (C₁-C₃₀)alkoxy, or NH-X²-CH₂-Z⁰, wherein X² is a (C₁-C₁₂)hydrocarbon moiety, and Z⁰ is H, OH, CO₂H or CONH₂;

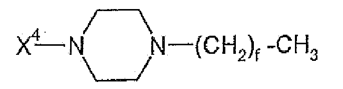

X³ is 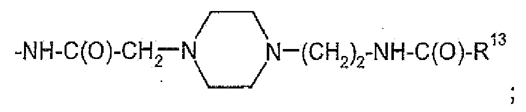 or -C(O)-NHR¹², wherein X⁴ is, independently for each occurrence, -C(O)-, -NH-C(O)- or -CH₂-, and wherein f is, independently for each occurrence, an integer from 1 to 29 inclusive;

each of R² and R³ is independently selected from the group consisting of H, (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, and hydroxynaphthyl(C₁-C₃₀)alkyl; or one of R² and R³ is 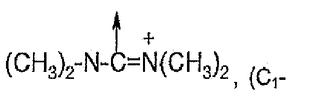 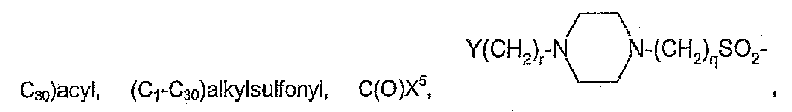 or 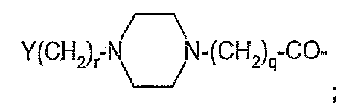 wherein Y is H, OH or NH₂; r is 0 to 4; q is 0 to 4;
and X⁵ is (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl or hydroxynaphthyl(C₁-C₃₀)alkyl;

e is, independently for each occurrence, an integer from 1 to 4 inclusive;

m is, independently for each occurrence, an integer from 5 to 24 inclusive;

n is, independently for each occurrence, an integer from 1 to 5, inclusive;

each of R¹⁰ and R¹¹ is, independently for each occurrence, H, (C₁-C₃₀)alkyl, (C₁-C₃₀)acyl, (C₁-C₃₀)alkylsulfonyl, -C((NH)(NH₂)) or 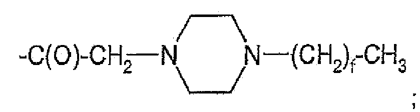 and

R¹² and R¹³ each is, independently for each occurrence, (C₁-C₃₀)alkyl;

provided that:

(i) said compound is not:

(Se⁸, β-Ala³⁵)hGLP-1(7-36)NH₂(SEQ ID NO.779);

(Gly⁸, Aib³⁵)hGLP-1(7-36)NH₂(SEQ ID NO.780); or

(Gly⁸, β-Ala³⁵)hGLP-1(7-36)NH₂(SEQ ID NO.781);

(ii) when A⁷ is Ura, Paa or Pta, then R² and R³ are deleted; and

(iii) when R¹⁰ is (C₁-C₃₀)acyl, (C₁-C₃₀)alkylsulfonyl, -C((NH)(NH₂)) or 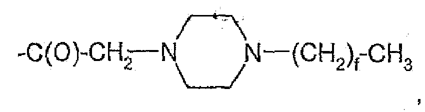 then R¹¹ is H or (C₁-C₃₀)alkyl;

or a pharmaceutical acceptable salt thereof.

A preferred group of compounds of formula (I) is where A¹¹ is Thr; A¹³ is Thr; A¹⁵ is Asp; A¹⁷ is Ser; A¹⁸ is Ser; A²¹ is Glu; A²³ is Gln or Glu; A²⁷ is Glu; and A³¹ is Trp; or a pharmaceutically acceptable salt thereof.

A preferred group of compounds of the immediately foregoing group of compounds is where A⁹ is Glu, N-Me-Glu or N-Me-Asp; A¹² is Phe, Acc or Aic; A¹⁶ is Val, Acc or Aib; A¹⁹ is Tyr; A²⁰ is Leu, Acc or Cha; A²⁴ is Ala, Aib or Acc; A²⁵ is Ala, Aib, Acc, Lys, Arg, hArg, Orn, HN-CH((CH₂)ₙ-N(R¹⁰R¹¹))-C(O) or HN-CH((CH₂)ₑ-X³)-C(O); A²⁸ is Phe; A²⁹ is Ile or Acc; A³⁰ is Ala or Aib; A³² is Leu, Acc or Cha; and A³³ is Val or Acc; or a pharmaceutically acceptable salt thereof.

A preferred group of compounds of the immediately foregoing group of compounds is where A¹⁰ is Gly; A¹² is Phe, A6c or A5c; A¹⁶ is Val, A6c or A5c; A²⁰ is Leu, A6c, A5c or Cha; A²² is Gly, β-Ala or Aib; A²⁴ is Ala or Aib; A²⁹ is Ile, A6c or A5c; A³² is Leu, A6c, A5c or Cha; A³³ is Val, A6c or A5c; A³⁵ is Aib, β-Ala, Ado, A6c, A5c or Gly; and A³⁷ is Gly, Aib, β-Ala, Ado, D-Ala or deleted; or a pharmaceutically acceptable salt thereof.

A preferred group of compounds of the immediately foregoing group of compounds is where X⁴ for each occurrence is -C(O)-; e is, independently for each occurrence, 1 or 2; and R¹ is OH or NH₂; or a pharmaceutically acceptable salt thereof.

A preferred group of compounds according to the immediately foregoing group of compounds is where R² is H and R³ is (C₁-C₃₀)alkyl, (C₂-C₃₀)alkenyl, (C₁-C₃₀)acyl, (C₁-C₃₀)alkylsulfonyl, 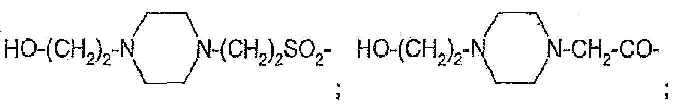 or 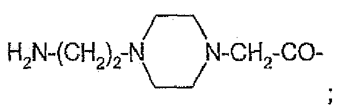 or a pharmaceutically acceptable salt thereof.

Another preferred group of compounds from among the compounds according to paragraph [063] is where R¹⁰ is (C₁-C₃₀)acyl, (C₁-C₃₀)alkylsulfonyl or 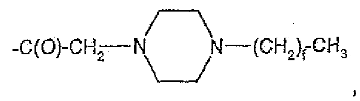 and R¹¹ is H; or a pharmaceutical acceptable salt thereof.

A preferred group of compounds of the immediately foregoing group of compounds is where R¹⁰ is (C₄-C₂₀)acyl, (C₄-C₂₀)alkylsulfonyl or 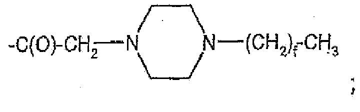 or a pharmaceutically acceptable salt thereof

Another preferred group of compounds from among the compounds according to paragraph [063], is where A⁸ is Gly, Ser, or Val; or a pharmaceutically acceptable salt thereof.

A preferred group of compounds of the immediately foregoing group of compounds is where A⁸ is Gly or Ser; or a pharmaceutically acceptable salt thereof.

A preferred compound of the immediately foregoing group of compounds is a compound according to the formula:

((N^{α}-Me-His)⁷, Gly⁸, Aib³⁵)hGLP-1(7-36)NH₂; (SEQ ID NO.1)

((N^{α}-Me-Nis)⁷, Gly⁸, β-Ala³⁵)hGLP-1(7-36)NH₂; (SEQ ID NO.2)

((N^{α}-Me-His)⁷, Gly⁸, Aib³⁵, Arg^{26,34})hGLP-1(7-36)NH₂; (SEQ ID NO.3)

((N^{α}-Me-His)⁷, Gly⁸, Arg^{26,34}, β-Ala³⁵)hGLP-1(7-36)NH₂; (SEQ ID NO.4)

(Gly⁸, A6c³⁵)hGLP-1(7-36)NH₂; (SEQ ID NO.5)

(Gly⁸, A5c³⁵)hGLP-1(7-36)NH₂; (SEQ ID NO.6)

(Gly⁸, D-Ala³⁵)hGLP-1(7-36)NH₂; (SEQ ID NO.7)

(Gly⁸, Aib³⁵, A6c³²)hGLP-1(7-36)NH₂; (SEQ ID NO.8)

(Gly⁸, Aib³⁵, A5c³²)hGLP-1(7-36)NH₂; (SEQ ID NO.9)

(Gly⁸, Aib³⁵, Glu²³)hGLP-1(7-36)NH₂; (SEQ ID NO.10)

(Gly⁸, Aib^{24,35})hGLP-1(7-36)NH₂; (SEQ ID NO.11)

(Gly⁸, Aib^{30,35})hGLP-1(7-36)NH₂; (SEQ ID NO.12)

(Gly⁸, Aib^{25,35})hGLP-1(7-36)NH₂; (SEQ ID NO.13)

(Gly⁸, Aib³⁵, A6c^{16,20})hGLP-1(7-36)NH₂; (SEQ ID NO.14)

(Gly⁸, Aib³⁵, A6c^{16,29,32})hGLP-1(7-36)NH₂; (SEQ ID NO.15)

(Gly⁸, Aib³⁵, A6c^{20,32})hGLP-1(7-36)NH₂; (SEQ ID NO.16)

(Gly⁸, Aib³⁵, A6c²⁰)hGLP-1(7-36)NH₂; (SEQ ID NO.17)

(Gly⁸, Aib³⁵, Lys²⁵)hGLP-1(7-36)NH₂ ; (SEQ ID NO.18)

(Gly⁸, Aib^{24,35}, A6c²⁰)hGLP-1(7-36)NH₂; (SEQ ID NO.19)

(Gly⁸, Aib³⁵, A6c^{29,32})hGLP-1(7-36)NH₂; (SEQ ID NO.20)

(Gly⁸, Aib^{24,35}, A6c^{29,32})hGLP-1(7-36)NH₂, (SEQ ID NO.21)

(Gly⁸, Aib³⁵, A6c¹²)hGLP-1(7-36)NH₂; (SEQ ID NO.22)

(Gly⁸, Aib³⁵, Cha²⁰)hGLP-1(7-36)NH₂; (SEQ ID NO.23)

(Gly⁸, Aib³⁵, A6c³³)hGLP-1(7-36)NH₂; (SEQ ID NO.24)

(Gly⁸, A6c^{16,20}, β-Ala³⁵)hGLP-1(7-36)NH₂; (SEQ ID NO.25)

(Gly⁸, Aib³⁵, β-Ala²²)hGLP-1(7-36)NH₂; (SEQ ID NO.26)

(Gly⁸, Aib^{22,35})hGLP-1(7-36)NH₂; (SEQ ID NO.27)

(Gly⁸, Aib³⁵, Glu²³, A6c³²)hGLP-1(7-36)NH₂; (SEQ ID NO.28)

(Gly⁸, Aib^{24,35}, Glu²³, A6c³²)hGLP-1(7-36)NH₂; (SEQ ID NO.29)

(Gly⁸, Aib^{24,25,35}, Glu²³, A6c³²)hGLP-1(7-36)NH₂; (SEQ ID NO.30)

(Gly⁸, Aib^{24,25,35}, A6c^{16,20,32}, Glu²³)hGLP-1(7-36)NH₂; (SEQ ID NO.31)

(Gly⁸, A6c³², β-Ala³⁵)hGLP-1(7-36)NH₂; (SEQ ID NO.32)

(Gly⁸, A5c³², β-Ala³⁵)hGLP-1(7-36)NH₂; (SEQ ID NO.33)

(Gly⁸, Glu²³, β-Ala³⁵)hGLP-1(7-36)NH₂; (SEQ ID NO.34)

(Gly⁸, Aib²⁴, β-Ala³⁵)hGLP-1(7-36)NH₂; (SEQ ID NO.35)

(Gly⁸, Aib³⁰, β-Ala³⁵)hGLP-1(7-36)NH₂; (SEQ ID NO.36)

(Gly⁸, Aib²⁵, β-Ala³⁵)hGLP-1(7-36)NH₂; (SEQ ID NO.37)

(Gly⁸, A6c^{16,29,32}, β-Ala³⁵)hGLP-1(7-36)NH₂; (SEQ ID NO.38)

(Gly⁸, A6c^{20,32}, β-Ala³⁵)hGLP-1(7-36)NH₂; (SEQ ID NO.39)

(Gly⁸, A6c²⁰, β-Ala³⁵)hGLP-1(7-36)NH₂; (SEQ ID NO.40)

(Gly⁸, Lys²⁵, β-Ala³⁵)hGLP-1(7-36)NH₂; (SEQ ID NO.41)

(Gly⁸, Aib²⁴, A6c²⁰, β-Ala³⁵)hGLP-1(7-36)NH₂; (SEQ ID NO.42)

(Gly⁸, A6c^{29,32}, β-Ala³⁵)hGLP-1(7-36)NH₂; (SEQ ID NO.43)

(Gly⁸, Aib²⁴, A6c^{29,32}, β-Ala³⁵)hGLP-1(7-36)NH₂; (SEQ ID NO.44)

(Gly⁸, A6c¹², β-Ala³⁵)hGLP-1(7-36)NH₂; (SEQ ID NO.45)

(Gly⁸, Cha²⁰, β-Ala³⁵)hGLP-1(7-36)NH₂; (SEQ ID NO.46)

(Gly⁸, A6c³³, β-Ala³⁵)hGLP-1(7-36)NH₂; (SEQ ID NO.47)

(Gly⁸, β-Ala^{22,35})hGLP-1(7-36)NH₂; (SEQ ID NO.48)

(Gly⁸, Aib²², β-Ala³⁵)hGLP-1(7-36)NH₂; (SEQ ID NO.49)

(Gly⁸, Glu²³, A6c³², β-Ala³⁵)hGLP-1(7-36)NH₂; (SEQ ID NO.50)

(Gly⁸, Aib²⁴, Glu²³, A6c³², β-Ala³⁵)hGLP-1(7-36)NH₂; (SEQ ID NO.51)

(Gly⁸, Aib²⁴, Glu²³, A6c³², Lys³⁴(N^{e}-octanoyl), β -Ala³⁵)hGLP-1(7-36)NH₂; (SEQ ID NO.52)

(Gly⁸, Aib^{24,25}, Glu²³, A6c³², β-Ala³⁵)hGLP-1(7-36)NH₂; (SEQ ID NO.53)

(Gly⁸, Aib^{24,25}, A6c^{16,20,32}, Glu²³, β-Ala³⁵)hGLP-1(7-36)NH₂; (SEQ ID NO.54)

(Gly⁸, Aib³⁵, D-Arg³⁶)hGLP-1(7-36)NH₂; (SEQ ID NO.55)

(Gly⁸, Aib³⁵, D-Lys³⁶)hGLP-1(7-36)NH₂; (SEQ ID NO.56)

(Gly⁸, β-Ala³⁵, D-Arg³⁶)hGLP-1(7-36)NH₂; (SEQ ID NO.57)

(Gly⁸, β-Ala³⁵, D-Lys³⁶)hGLP-1(7-36)NH₂; (SEQ ID NO.58)

(Gly⁸, Aib³⁵, Arg^{26,34})hGLP-1(7-36)NH₂; (SEQ ID NO.59)

(Gly⁸, Arg^{26,34}, β-Ala³⁵)hGLP-1(7-36)NH₂; (SEQ ID NO.60)

(Gly⁸, Aib³⁵, Arg^{25,26,34})hGLP-1(7-36)NH₂; (SEQ ID NO.61)

(Gly⁸, Arg^{25,26,34}, β-Ala³⁵)hGLP-1(7-36)NH₂; (SEQ ID NO.62)

(Gly⁸, Arg^{26,34}, β-Ala³⁵, Lys³⁶(N^{ε}-tetradecanoyl))hGLP-1(7-36)OH; (SEQ ID NO.63)

(Gly⁸, Aib³⁵, Arg^{26,34}, Lys³⁶(N^{ε}-tetradecanoy))hGLP-1(7-37)OH; (SEQ ID NO.64)

(Gly⁸, Aib^{35,37}, Arg^{26,34}, Lys³⁶(N^{ε}-tetradecanoyl))hGLP-1(7-37)OH; (SEQ ID NO.65)

(Gly⁸, Aib³⁵, Arg^{26,34}, Lys³⁶(N^{ε}-tetradecanoyl), D-Ala³⁷)hGLP-1(7-37)OH; (SEQ ID NO.66)

(Gly⁸, Aib^{35,37}, Arg^{26,34}, Lys³⁸(N^{ε}-tetradecanoyl))hGLP-1(7-38)OH; (SEQ ID NO.67)

(Gly⁸, Aib³⁵, Arg^{26,34}, β-Ala³⁷, Lys³⁸(N^{ε}-tetradecanoyl))hGLP-1(7-38)OH; (SEQ ID NO.68)

(Gly⁸, Aib³⁵, Arg^{26,34}, Lys³⁸(N^{ε}-tetradecanoyl))hGLP-1(7-38)OH; (SEQ ID NO.69)

(Gly⁸, Arg^{26,34}, Lys³⁶(N^{ε}-tetradecanoyl), β-Ala³⁷)hGLP-1(7-37)OH; (SEQ ID NO.70)

(Gly⁸, Aib³⁷, Arg^{26,34}, Lys³⁶(N^{ε}-tetradecanoyl))hGLP-1(7-37)OH; (SEQ ID NO.71)

(Gly⁸, Aib³⁵, Arg^{26,34}, Ado³⁷)hGLP-1(7-37)OH; (SEQ ID NO.72)

(Gly⁸, Aib³⁵, Arg^{26,34}, Ado³⁷)hGLP-1(7-37)NH₂; (SEQ ID NO.73)

(Gly⁸, Arg^{26,34}, Lys³⁶(N^{ε}-tetradecanoyl), D-Ala³⁷)hGLP-1(7-37)OH; (SEQ ID NO.74)

(Gly⁸, Aib³⁷, Arg^{26,34}, Lys³⁸(N^{ε}-tetradecanoyl))hGLP-1(7-38)OH; (SEQ ID NO.75)

(Gly⁸, Arg^{26,34}, β-Ara³⁷, Lys³⁸(N^{ε}-tefradecanoyl))hGLP-1(7-38)OH; (SEQ ID NO.76)

(Gly⁸, Aib³⁵, Lys²⁶(N^{ε}-octanoyl))hGLP-1(7-36)NH₂; (SEQ ID NO.77)

(Gly⁸, Aib³⁵, Lys²⁶(N^{ε}-tetradecanoyl))hGLP-1(7-36)NH₂; (SEQ ID NO.78)

(Gly⁸, Aib³⁵, Lys²⁶(N^{ε}-hexadecanoyl))hGLP-1(7-36)NH₂; (SEQ ID NO.79)

(Gly⁸, Lys²⁶(N^{ε}-octanoyl), β-Ala³⁵)hGLP-1(7-36)NH₂; (SEQ ID NO.80)

(Gly⁸, Lys²⁶(N^{ε}-tetradecanoyl), β-Ala³⁵)hGLP-1(7-36)NH₂; (SEQ ID NO.81)

(Gly⁸, Lys²⁶(N^{ε}-hexadecanoyl), β-Ala³⁵)hGLP-1(7-36)NH₂; (SEQ ID NO.82)

(Gly⁸, Aib³⁵, Lys²⁶(N^{ε}-octanoyl), Arg³⁴)hGLP-1(7-36)NH₂; (SEQ ID NO.83)

(Gly⁸, Aib³⁵, Lys²⁶(N^{ε}-tetradecanoyl), Arg³⁴)hGLP-1(7-36)NH₂; (SEQ ID NO.84)

(Gly⁸, Aib³⁵, Lys²⁶(N^{ε}-hexadecanoyl), Arg³⁴)hGLP-1(7-36)NH₂; (SEQ ID NO.85)

(Gly⁸, Aib³⁵, Lys²⁶(N^{ε}-decanoyl), Arg³⁴)hGLP-1(7-36)NH₂; (SEQ ID NO.86)

(Gly⁸, Aib³⁵, Lys²⁵, Lys²⁶(N^{ε}-octanoyl) Arg³⁴)hGLP-1(7-36)NH₂; (SEQ ID NO.87)

(Gly⁸, Aib³⁵, Lys²⁵, Lys²⁶(N^{ε}-tetradecanoyl), Arg³⁴)hGLP-1(7-36)NH₂; (SEQ ID NO.88)

(Gly⁸, Aib³⁵, Lys²⁵, Lys²⁶(N^{ε}-hexadecanoyl), Arg³⁴)hGLP-1(7-36)NH₂; (SEQ ID NO.89)

(Gly⁸, Aib³⁵, Arg^{25,34}, Lys²⁶(N^{ε}-octanoyl))hGLP-1(7-36)NH₂; (SEQ ID NO.90)

(Gly⁸, Aib³⁵, Arg^{25,34}, Lys²⁶(N^{ε}-tetradecanoyl))hGLP-1(7-36)NH₂; (SEQ ID NO,91)

(Gly⁸, Aib³⁵, Arg^{25,34}, Lys²⁶(N^{ε}-hexadecanoyl))hGLP-1(7-36)NH₂; (SEQ ID NO.92)

(Gly⁸, Aib³⁵, Arg^{25,34}, Lys²⁶(N^{ε}-decanoyl))hGLP-1(7-36)NH₂; (SEQ ID NO.93)

(Gly⁸, Lys²⁶(N^{ε}-octanoyl), Arg³⁴, β-Ala³⁵)hGLP-1(7-36)NH₂; (SEQ ID NO.94)

(Gly⁸, Lys²⁶(N^{ε}-tetradecanoyl), Arg³⁴, β-Ala³⁵)hGLP-1(7-36)NH₂; (SEQ ID NO.95)

(Gly⁸, Lys²⁶(N^{ε}-hexadecanoyl), Arg³⁴, β-Ala³⁵)hGLP-1(7-36)NH₂; (SEQ ID NO.96)

(Gly⁸, Lys²⁶(N^{ε}-decanoyl), Arg³⁴, β-Ala³⁵)hGLP-1(7-36)NH₂; (SEQ ID NO.97)

(Gly⁸, Aib³⁵, Lys³⁴(N^{ε}-octanoyl))hGLP-1(7-36)NH₂; (SEQ ID NO.98)

(Gly⁸, Aib³⁵, Lys³⁴(N^{ε}-tetradecartoyl))hGLP-1(7-36)NH₂; (SEQ ID NO.99)

(Gly⁸, Aib³⁵, Lys³⁴(N^{ε}-hexadecanoyl))hGLP-1(7-36)NH₂; (SEQ ID NO.100)

(Gly⁸, Aib³⁵, Arg²⁶, Lys³⁴(N^{ε}-octanoyl))hGLP-1(7-36)NH₂; (SEQ ID NO.101)

(Gly⁸, Aib³⁵, Arg²⁶, Lys³⁴(N^{ε}-hexadecanoyl))hGLP-1(7-36)NH₂; (SEQ ID NO.102)

(Gly⁸, Aib³⁵, Arg²⁶, Lys³⁴(N^{ε}-decanoyl))hGLP-1(7-36)NH₂; (SEQ ID NO.103)

(Gly⁸, Aib³⁵, Arg^{25,26}, Lys³⁴(N^{ε}-octanoyl))hGLP-1(7-36)NH₂; (SEQ ID NO.104)

(Gly⁸, Aib³⁵, Arg^{25,26}, Lys³⁴(N^{ε}-tetradecanoyl))hGLP-1(7-36)NH₂; (SEQ ID NO.105)

(Gly⁸, Aib³⁵, Arg^{25,26}, Lys³⁴(N^{ε}-hexadecanoyl))hGLP-1(7-36)NH₂; (SEQ ID NO.106)

(Gly⁸, Aib³⁵, Arg^{25,26}, Lys³⁴(N^{ε}-decanoyl))hGLP-1(7-36)NH₂; (SEQ ID NO.107)

(Gly⁸, Aib³⁵, Lys²⁵, Arg²⁶, Lys³⁴(N^{ε}-octanoyl))hGLP-1(7-36)NH₂; (SEQ ID NO.708)

(Gly⁸, Aib³⁵, Lys²⁵, Arg²⁶, Lys³⁴(N^{ε}-tetradecancyl))hGLP-1(7-36)NH₂; (SEQ ID NO.109)

(Gly⁸, Aib³⁵, Lys²⁵, Arg²⁶, Lys³⁴(N^{ε}-hexadecanoyl))hGLP-1(7-36)NH₂; (SEQ ID NO.110)

(Gly⁸, Aib³⁵, Lys³⁶(N^{ε}-octanoyl))hGLP-1(7-36)NH₂; (SEQ ID NO.111)

(Gly⁸, Aib³⁵, Lys³⁶(N^{ε}-tetradecanoyl))hGLP-1(7-36)NH₂; (SEQ ID NO.112)

(Gly⁸, Aib³⁵, Lys³⁶(N^{ε}-hexadecanoyl))hGLP-1(7-36)NH₂; (SEQ ID NO.113)

(Gly⁸, Aib³⁵, Arg²⁶, Lys³⁶(N^{ε}-octanoyl))hGLP-1(7-36)NH₂; (SEQ ID NO.114)

(Gly⁸, Aib³⁵, Arg²⁶, Lys³⁶(N^{ε}-tetradecanoyl))hGLP-1(7-36)NH₂; (SEQ ID NO.115)

(Gly⁸, Aib³⁵, Arg²⁶, Lys³⁶(N^{ε}-hexadecanoyl))hGLP-1(7-36)NH₂; (SEQ ID NO.116)

(Gly⁸, Aib³⁵, Arg^{26,34}, Lys³⁶(N^{ε}-octanoyl))hGLP-1(7-36)NH₂; (SEQ ID NO.117)

(Gly⁸, Aib³⁵, Arg^{26,34}, Lys³⁶(N^{ε}-hexadecanoyl))hGLP-1(7-36)NH₂; (SEQ ID NO.118)

(Gly⁸, Aib³⁵, Arg^{26,34}, Lys³⁸(N^{ε}-octanoyl))hGLP-1(7-38)NH₂; (SEQ ID NO.199)

(Gly⁸, Aib³⁵, Arg^{26,34}, Lys³⁸(N^{ε}-decanoyl))hGLP-1(7-38)NH₂; (SEQ ID NO.120)

(Gly⁸, Aib³⁵, Arg^{26,34}, Lys³⁸(N^{ε}-tetradecanoyl)hGLP-1(7-38)NH₂; (SEQ ID NO.121)

(Gly⁸, Aib³⁵, Arg^{26,34}, Lys³⁸(N^{ε}-hexadecanoyl))hGLP-1(7-38)NH₂; (SEQ ID NO.122)

(Gly⁸, Aib^{35,37}, Arg^{25,26,34}, Lys³⁸(N^{ε}-octanoyl))hGLP-1(7-38)NH₂; (SEQ ID NO.123)

(Gly⁸, Aib^{35,37}, Arg^{25,26,34}, Lys³⁸(N^{ε}-decanoyl))hGLP-1(7-38)NH₂; (SEQ ID NO.124)

(Gly⁸, Aib^{35,37}, Arg^{25,26,34}, Lys³⁸(N^{ε}-tetradecanoyl))hGLP-1(7-38)NH₂; (SEQ ID NO.125)

(Gly⁸, Aib^{35,37}, Arg^{25,26,34}, Lys³⁸(N^{ε}-hexadecanoyl))hGLP-1(7-38)NH₂; (SEQ ID NO.126)

(Gly⁸, Aib^{35,37}, Arg^{26,34}, Lys³⁸(N^{ε}-octanoyl))hGLP-1(7-38)NH₂; (SEQ ID NO.127)

(Gly⁸, Aib^{35,37}, Arg^{26,34}, Lys³⁸(N^{ε}-decanoyl))hGLP-1(7-38)NH₂; (SEQ ID NO.128)

(Gly⁸, Aib^{35,37}, Arg^{26,34}, Lys³⁸(N^{ε}-hexadecanoyl))hGLP-1(7-38)NH₂; (SEQ ID NO.129)

(Gly⁸, Aib³⁵, Lys²⁵, Arg^{26,34}, Lys³⁶(N^{ε}-octanoyl))hGLP-1(7-36)NH₂; (SEQ ID NO.130)

(Gly⁸, Aib³⁵, Lys²⁵, Arg^{26,34}, Lys³⁶(N^{ε}-tetradecanoyl))hGLP-1(7-36)NH₂; (SEQ ID NO.131)

(Gly⁸, Aib³⁵, Lys²⁵, Arg^{26,34}, Lys³⁶(N^{ε}-hexadecanoyl))hGLP-1(7-36)NH₂; (SEQ ID NO.132)

(Gly⁸, Aib³⁵, Arg^{25,26,34}, Lys³⁶(N^{ε}-octanoyl))hGLP-1(7-36)NH₂; (SEQ ID NO.133)

(Gly⁸, Aib³⁵, Arg^{25,26,34}, Lys³⁶(N^{ε}-tetrdecanoyl))hGLP-1(7-36)NH₂; (SEQ ID NO.134)

(Gly⁸, Aib³⁵, Arg^{25,26,34}, Lys³⁶(N^{ε}-hexadecanoyl))hGLP-1(7-36)NH₂; (SEQ ID NO.135)

(Gly⁸, Aib³⁵, Arg^{25,26,34}, Lys³⁶(N^{ε}-decanoyl))hGLP-1(7-36)NH₂; (SEQ ID NO.136)

(Gly⁸, Lys³⁴(N^{ε}-octanoyl), β-Ala³⁵)hGLP-1(7-36)NH₂; (SEQ ID NO.137)

(Gly⁸, Lys³⁴(N^{ε}-tetradecanoyl), β-Ala³⁵)hGLP-1(7-36)NH₂; (SEQ ID NO.138)

(Gly⁸, Lys³⁴(N^{ε}-hexadecanoyl), β-Ala³⁵)hGLP-1(7-36)NH₂; (SEQ ID NO.139)

(Gly⁸, A6c³², Lys³⁴(N^{e}-octanoyl), β-Ala³⁵)hGLP-1(7-36)NH₂; (SEQ ID NO.140)

(Gly⁸, Glu²³, Lys³⁴(N^{e}-octanoyl), β-Ala³⁵)hGLP-1(7-36)NH₂; (SEQ ID NO.141)

(Gly⁸, Glu²³, A6c³², Lys³⁴(N^{e}-octanoyl), β-Ala³⁵)hGLP-1(7-36)NH₂; (SEQ ID NO.142)

(Gly⁸, Arg²⁶, Lys³⁴(N^{ε}-octanoyl), β-Ala³⁵)hGLP-1(7-36)NH₂; (SEQ ID NO.143)

(Gly⁸, Arg²⁶, Lys³⁴(N^{ε}-tetradecanoyl), β-Ala³⁵)hGLP-1(7-36)NH₂; (SEQ ID NO.144)

(Gly⁸, Arg²⁶, Lys³⁴(N^{ε}-hexadecanoyl), β-Ala³⁵)hGLP-1(7-36)NH₂; (SEQ ID NO.145)

(Gly⁸, Arg²⁶, Lys³⁴(N^{ε}-decanoyl), β-Ala³⁵)hGLP-1(7-36)NH₂; (SEQ ID NO.146)

(Gly⁸, Arg^{25,26}, Lys³⁴(N^{ε}-octanoyl), β-Ala³⁵)hGLP-1(7-36)NH₂; (SEQ ID NO.147)

(Gly⁸, Arg^{25,26}, Lys³⁴(N^{ε}-tetradecanoyl), β-Ala³⁵)hGLP-1(7-36)NH₂; (SEQ ID NO.148)

(Gly⁸, Arg^{25,26}, Lys³⁴(N^{ε}-hexadecanoyl), β-Ala³⁵)hGLP-1(7-36)NH₂; (SEQ ID NO.149)

(Gly⁸, Arg^{25,26}, Lys³⁴(N^{ε}-decanoyl), β-Ala³⁵)hGLP-1(7-36)NH₂; (SEQ ID NO.156)

(Gly⁸, Lys²⁵, Arg²⁶, Lys³⁴(N^{ε}-octanoyl), β-Ala³⁵)hGLP-1(7-36)NH₂; (SEQ ID NO.151)

(Gly⁸, Lys²⁵, Arg²⁶, Lys³⁴(N^{ε}-tetradecanoyl), β-Ala³⁵)hGLP-1(7-36)NH₂; (SEQ ID NO.15.2)

(Gly⁸, Lys²⁵, Arg²⁶, Lys³⁴(N^{ε}-hexadecanoyl), β-Ala³⁵)hGLP-1(7-36)NH₂; (SEQ ID NO.153)

(Gly⁸, β-Ala³⁵, Lys³⁶(N^{ε}-octanoyl))hGLP-1(7-36)NH₂; (SEQ ID NO.164)

(Gly⁸, β-Ala³⁵, Lys³⁶(N^{ε}-tetradecanoyl))hGLP-1(7-36)NH₂; (SEQ ID NO.155)

(Gly⁸, β-Ala³⁵, Lys³⁶(N^{ε}-hexadecanoyl))hGLP-1(7-36)NH₂; (SEQ ID NO.156)

(Gly⁸, Arg²⁶, β-Ala³⁶, Lys³⁵(N^{ε}-octanoyl))hGLP-1(7-36)NH₂; (SEQ ID NO.157)

(Gly⁸, Arg²⁶, β-Ala³⁶, Lys³⁵(N^{ε}-tetradecanoyl))hGLP-1(7-36)NH₂; (SEQ ID NO.168)

(Gly⁸, Arg²⁶, β-Ala³⁵, Lys³⁶(N^{ε}-hexadecanoyl))hGLP-1(7-36)NH₂; (SEQ ID NO.159)

(Gly⁸, Arg^{26,34}, β-Ala³⁵, Lys³⁶(N^{ε}-octanoyl))hGLP-1(7-36)NH₂; (SEQ ID NO.166)

(Gly⁸, Arg^{26,34}, β-Ala³⁵, Lys³⁶(N^{ε}-tetradecanoyl))hGLP-1(7-36)NH₂; (SEQ ID NO.161)

(Gly⁸, Arg^{26,34}, β-Ala³⁵, Lys³⁶(N^{ε}-hexadecanoyl))hGLP-1(7-36)NH₂; (SEQ ID NO.162)

(Gly⁸, Arg^{26,34}, β-Ala³⁵, Lys³⁶(N^{ε}-decanoyl))hGLP-1(7-36)NH₂; (SEQ ID NO.163)

(Gly⁸, Lys²⁵, Arg^{26,34}, β-Ala³⁵, Lys³⁶(N^{ε}-octanoyl))hGLP-1(7-36)NH₂; (SEQ ID NO.164)

(Gly⁸, Lys²⁵, Arg^{26,34}, Lys³⁶(N^{ε}-tetradecanoyl), β-Ala³⁵)hGLP-1(7-36)NH₂; (SEQ ID NO.165)

(Gly⁸, Lys²⁵, Arg^{26,34}, β-Ala³⁵, Lys³⁶(N^{ε}-hexadecanoyl))hGLP-1(7-36)NH₂; (SEQ ID NO.166)

(Gly⁸, Arg^{25,2634}, β-Ala³⁵, Lys³⁶(N^{ε}-octanoyl)hGLP-1(7-36)NH₂; (SEQ ID NO.167)

(Gly⁸, Arg^{25,26,34}, β-Ala³⁵, Lys³⁶(N^{ε}-tetradecanoyl))hGLP-1(7-38)NH₂; (SEQ ID NO.168)

(Gly⁸, Arg^{25,26,34}, β-Ala³⁵, Lys³⁶(N^{ε}-hexadecanoyl))hGLP-1(7-36)NH₂; (SEQ ID NO.169)

(Gly⁸, Arg^{25,26,34}, β-Ala³⁵, Lys³⁶(N^{ε}-decanoyl))hGLP-1(7-36)NH₂; (SEQ ID NO.170)

(Gly⁸, Aib³⁵, Lys²⁶(N^{ε}-octanoyl), A6c³², Arg³⁴)hGLP-1(7-36)NH₂; (SEQ ID NO.171)

(Gly⁸, Aib³⁵, Lys²⁶(N^{ε}-tetradecanoyl), A6c³², Arg³⁴)hGLP-1(7-36)NH₂; (SEQ ID NO.172)

(Gly⁸, Aib³⁵, Lys²⁶(N^{ε}-hexadecanoyl), A6c³², Arg³⁴)hGLP-1(7-36) NH₂; (SEQ ID NO.173)

(Gly⁸, Aib³⁵, A6c³², Lys³⁴(N^{ε}-octanoyl))hGLP-1(7-36)NH₂; (SEQ ID NO.174)

(Gly⁸, Aib³⁵, A6c³², Lys³⁴(N^{ε}-tetradecanoyl))hGLP-1(7-36)NH₂; (SEQ ID NO.175)

(Gly⁸, Aib³⁵, A6c³², Lys³⁴(N^{ε}-hexadecanoyl))hGLP-1(7-36)NH₂; (SEQ ID NO.176)

(Gly⁸, Aib³⁵, Arg²⁶, A6c³², Lys³⁴(N^{ε}-octanoyl))hGLP-1(7-36)NH₂; (SEQ ID NO.177)

(Gly⁸, Aib³⁵, Arg²⁶, A6c³², Lys³⁴(N^{ε}-tetradecanoyl)hGLP-1(7-36)NH₂; (SEQ ID NO.178)

(Gly⁸, Aib³⁵, A6c³², Lys³⁶(N^{ε}-octanoyl))hGLP-1(7-36)NH₂; (SEQ ID NO.179)

(Gly⁸, Aib³⁵, A6c³², Lys³⁶(N^{ε}-tetradecanoyl))hGLP-1(7-36)NH₂; (SEQ ID NO.180)

(Gly⁸, Aib³⁵, A6c³², Lys³⁶(N^{ε}-hexadecanoy)))hGLP-1(7-36)NH₂; (SEQ ID NO.187)

(Gly⁸, Aib³⁵, Arg²⁶, A6c³², Lys³⁶(N^{ε}-octanoyl))hGLP-1(7-36)NH₂; (SEQ ID NO.182)

(Gly⁸, Aib³⁵, Arg²⁶, A6c³², Lys³⁶(N^{ε}-tetradecanoyl))hGLP-1(7-36)NH₂; (SEQ ID NO.183)

(Gly⁸, Aib³⁵, Arg²⁶, A6c³², Lys³⁶(N^{ε}-hexadecanoyl))hGLP-1(7-36)NH₂; (SEQ ID NO.184)

(Gly⁸, Aib³⁵, Arg^{26,34}, A6c³², Lys³⁶(N^{ε}-octanoyl))hGLP-1(7-36)NH₂; (SEQ ID NO.185)

(Gly⁸, Aib³⁵, Arg^{26,34}, A6c³², Lys³⁶(N^{ε}-decanoyl))hGLP-1(7-36)NH₂; (SEQ ID NO.186)

(Gly⁸, Aib³⁵, Arg^{26,34}, A6c³², Lys³⁶(N^{ε}-tetradecanoyl))hGLP-1(7-36)NH₂; (SEQ ID NO.187)

(Gly⁸, Aib³⁵, Arg^{26,34}, A6c³², Lys³⁶(N^{ε}-hexadecanoyl))hGLP-1(7-36)NH₂; (SEQ ID NO.188)

(Gly⁸, Aib^{24,35}, Lys²⁶(N^{ε}-octanoyl), Arg³⁴)hGLP-1(7-36)NH₂; (SEQ ID NO.189)

(Gly⁸, Aib^{24,35}, Lys²⁶(N^{ε}-tetradecanoyl), Arg³⁴)hGLP-1(7-36)NH₂; (SEQ ID NO.190)

(Gly⁸, Aib^{24,35}, Lys²⁶(N^{ε}-hexadecanoyl), Arg³⁴)hGLP-1(7-36)NH₂; (SEQ ID NO.191)

(Gly⁸, Aib^{24,35}, Arg²⁶, Lys³⁴(N-octanoyl))hGLP-1(7-36)NH₂; (SEQ ID NO. 192)

(Gly⁸, Aib^{24,35}, Arg²⁶, Lys³⁴(N^{ε}-tetradecanoyl))hGLP-1(7-36)NH₂; (SEQ ID NO.193)

(Gly⁸, Aib^{24,35}, Arg²⁶, Lys³⁴(N^{ε}-hexadecanoyl))hGLP-1(7-36)NH₂; (SEQ ID NO.194)

(Gly⁸, Aib^{24,35}, Arg^{26,34}, Lys³⁶(N^{ε}-octanoyl))hGLP-1(7-36)NH₂; (SEQ ID NO.195)

(Gly⁸, Aib^{24,35}, Arg^{26,34}, Lys³⁶(N^{ε}-tetradecanoyl))hGLP-1(7-36)NH₂; (SEQ ID NO.196)

(Gly⁸, Aib^{24,35}, Arg^{26,34}, Lys³⁶(N^{ε}-hexadecanoyl))hGLP-1(7-36)NH₂; (SEQ ID NO.197)

(Gly⁸, Aib^{24,35}, Glu²³, A6c³², Lys³⁴(N^{ε}-octanoyl))hGLP-1(7-36)NH₂; (SEQ ID NO.198)

(Gly⁸, Aib³⁵, Glu²³, Lys²⁶(N^{ε}-octanoyl), Arg³⁴)hGLP-1(7-36)NH₂; (SEQ ID NO.199)

(Gly⁸, Aib³⁵, Glu²³, Lys²⁶(N^{ε}-tetradecanoyl), Arg³⁴)hGLP-1(7-36)NH₂; (SEQ ID NO.200)

(Gly⁸, Aib³⁵, Glu²³, Lys²⁶(N^{ε}-hexadecanoyl), Arg³⁴)hGLP-1(7-36)NH₂; (SEQ ID NO.201)

(Gly⁸, Aib³⁵, Glu²³, Lys³⁴(N^{e}-octanoyl))hGLP-1(7-36)NH₂; (SEQ ID NO.202)

(Gly⁸, Aib³⁵, Glu²³, A6c³², Lys³⁴(N^{e}-octanyl))hGLP-1(7-36)NH₂; (SEQ ID NO.203)

(Gly⁸, Aib³⁵, Glu²³, Arg²⁶, Lys³⁴(N^{ε}-octanoyl))hGLP-1(7-36)NH₂; (SEQ ID NO.204)

(Gly⁸, Aib³⁵, Glu²³, Arg²⁶, Lys³⁴(N^{ε}-tetradecanoyl))hGLP-1(7-36)NH₂; (SEQ ID NO.205)

(Gly⁸, Aib³⁵, Glu²³, Arg²⁶, Lys³⁴(N^{ε}-hexadecanoyl))hGLP-1(7-36)NH₂; (SEQ ID NO.206)

(Gly⁸, Aib³⁵, Glu²³, Lys³⁶(N^{ε}-octanoyl))hGLP-1(7-36)NH₂; (SEQ ID NO.207)

(Gly⁸, Aib³⁵, Glu²³, Lys³⁶(N^{ε}-tetradecanoyl))hGLP-1(7-36)NH₂; (SEQ ID NO.208)

(Gly⁸, Aib³⁵, Glu²³, Lys³⁶(N^{ε}-hexadecanoyl))hGLP-1(7-36)NH₂; (SEQ ID NO.209)

(Gly⁸, Aib³⁵, Glu²³, Arg^{26,34}, Lys³⁶(N^{ε}-octanoyl))hGLP-1(7-36)NH₂; (SEQ ID NO.210)

(Gly⁸, Aib³⁵, Glu²³, Arg^{26,34}, Lys³⁶(N^{ε}-tetradecanoyl))hGLP-1(7-36)NH₂; (SEQ ID NO.211)

(Gly⁸, Aib³⁵, Glu²³, Arg^{26,34}, Lys³⁶(N^{ε}-hexadecanoyl))hGLP-1(7-36)NH₂; (SEQ ID NO.212)

(Gly⁸, Aib^{30,35}, Lys²⁶(N^{ε}-octanoyl), Arg³⁴)hGLP-1(7-36)NH₂; (SEQ ID NO.213)

(Gly⁸, Aib^{30,35}, Lys²⁶(N^{ε}-tetradecanoyl), Arg³⁴)hGLP-1(7-36)NH₂; (SEQ ID NO.214)

(Gly⁸, Aib^{30,35}, Lys²⁶(N^{ε}-hexadecanoyl), Arg³⁴)hGLP-1(7-36)NH₂; (SEQ ID NO.215)

(Gly⁸, Aib^{30,35}, Arg²⁶, Lys³⁴(N^{ε}-octanoyl))hGLP-1(7-36)NH₂; (SEQ ID NO.216)

(Gly⁸, Aib^{30,35}, Arg²⁶, Lys³⁴(N^{ε}-tetradecanoyl))hGLP-1(7-36)NH₂; (SEQ ID NO.217)

(Gly⁸, Aib^{30,35}, Arg²⁶, Lys³⁴(N^{ε}-hexadecanoyl))hGLP-1(7-36)NH₂; (SEQ ID NO.218)

(Gly⁸, Aib^{30,35}, Arg^{26,34}, Lys³⁶(N^{ε}-octanoyl))hGLP-1(7-36)NH₂; (SEQ ID NO.219)

(Gly⁸, Aib^{30,35}, Arg^{26,34}, Lys³⁶(N^{ε}-tetradecanoyl))hGLP-1(7-36)NH₂; (SEQ ID NO.220)

(Gly⁸, Aib^{30,35}, Arg^{26,34}, Lys³⁶(N^{ε}-hexadecanoyl))hGLP-1(7-36)NH₂; (SEQ ID NO.221)

(Gly⁸, Aib³⁵, Glu²³, A6c³², Lys³⁶(N^{ε}-octanoyl))hGLP-1(7-36)NH₂; (SEQ ID NO.222)

(Gly⁸, Aib³⁵, Glu²³, A6c³², Lys³⁶(N^{ε}-tetradecanoyl))hGLP-1(7-36)NH₂; (SEQ ID NO.223)

(Gly⁸, Aib³⁵, Glu²³, A6c³², Lys³⁶(N^{ε}-hexadecanoyl))hGLP-1(7-36)NH₂; (SEQ ID NO.224)

(Gly⁸, Aib³⁵, Glu²³, Arg^{26,34}, A6c³², Lys³⁶(N^{ε}-octanoyl))hGLP-1(7-36)NH₂; (SEQ ID NO.225)

(Gly⁸, Aib³⁵, Glu²³, Arg^{26,34}, A6c³², Lys³⁶(N^{ε}-tetradecanoyl)hGLP-1(7-36)NH₂; (SEQ ID NO.226)

(Gly⁸, Aib³⁵, Glu²³, Arg^{26,34}, A6c³², Lys³⁶(N^{ε}-hexadecanoyl))hGLP-1(7-36)NH₂; (SEQ ID NO.227)

(Gly⁸, Aib^{24,35}, Glu²³, Arg^{26,34}, A6c³², Lys³⁶(N^{ε}-octanoyl))hGLP-1(7-36)NH₂; (SEQ ID NO.228)

(Gly⁸, Aib^{24,35}, Glu²³, Arg^{26,34}, A6c³², Lys³⁶(N^{ε}-tetradecanoyl))hGLP-1(7-36)NH₂; (SEQ ID NO.229)

(Gly⁸, Aib^{24,35}, Glu²³, Arg^{26,34}, A6c³², Lys³⁶(N^{ε}-hexadecanoyl))hGLP-1(7-36)NH₂; (SEQ ID NO.230)

(Gly⁸, Aib^{24,30,35}, Glu²³, Arg^{26,34}, A6c³², Lys³⁶(N^{ε}-octanoyl))hGLP-1(7-36)NH₂; (SEQ ID NO.231)

(Gly⁸, Aib^{24,30,35}, Glu²³, Arg^{26,34}, A6c³², Lys³⁶(N^{ε}-tetradecanoyl))hGLP-1(7-36)NH₂; (SEQ ID NO.232)

(Gly⁸, Aib^{24,30,35}, Glu²³, Arg^{26,34}, A6c³², Lys³⁶(N^{ε}-hexadecancyl))hGLP-1(7-36)NH₂; (SEQ ID NO.233)

((N^{α}-HEPES-His)⁷, Gly⁸, β-Ala³⁵)hGLP-1(7-36)NH₂; (SEQ ID NO.234)

((N^{α}-HEPA-His)⁷, Gly⁸, β-Ala³⁵)hGLP-1(7-36)NH₂; (SEQ ID NO.235)

((N^{α}-tetradecanoyl-His)⁷, Gly⁸, Aib³⁵)hGLP-1(7-36)NH₂; (SEQ ID NO.236)

((N^{α}-tetradecanoyl-His)⁷, Gly⁸, β-Ala³⁵)hGLP-1(7-36)NH₂; (SEQ ID NO.237)

((N^{α}-tetradecanoyl-His)⁷, Gly⁸, Aib³⁵, Arg^{26,34})hGLP-1(7-36)NH₂; (SEQ ID NO.238)

((N^{α}-tetradecanoyl-His)⁷, Gly⁸, Arg^{26,34}, β-Ala³⁵)hGLP-1(7-36)NH₂; (SEQ ID NO.239)

((N^{α}-tetradecanoyl-His)⁷, Gly⁸, Aib³⁵, Arg^{25,26,34})hGLP-1(7-36)NH₂; (SEQ ID NO.240)

((N^{α}-tetradecanoyl-His)⁷, Gly⁸, Arg^{25,26,34}, β-Ala³⁵)hGLP-1(7-36)NH₂; (SEQ ID NO.241)

(Gly⁸, Aib³⁵, Lys²⁶(N^{ε}-octanesulfonyl), Arg³⁴)hGLP-1(7-38)NH₂; (SEQ ID NO.242)

(Gly⁸, Aib³⁵, Lys²⁶(N^{ε}-dodecanesulfonyl), Arg³⁴)hGLP-1(7-36)NH₂; (SEQ ID NO.243)

(Gly⁸, Aib³⁵, Lys²⁶(N^{ε}-hexadecanesulfonyl), Arg³⁴)hGLP-1(7-36)NH₂; (SEQ ID NO.244)

(Gly⁸, Aib³⁵, Arg²⁶, Lys³⁴(N^{ε}-octanesulfonyl))hGLP-1(7-36)NH₂; (SEQ ID NO.245)

(Gly⁸, Aib³⁵, Arg²⁶, Lys³⁴(N^{ε}-dodecanesulfonyl))hGLP-1(7-36)NH₂; (SEQ ID NO.246)

(Gly⁸, Aib³⁵, Arg²⁶, Lys³⁴(N^{ε}-hexadecanesulfonyl))hGLP-1(7-36)NH₂; (SEQ ID NO.247)

(Gly⁸, Aib³⁵, Arg^{26,34}, Lys³⁶(N^{ε}-cctanesulfonyl))hGLP-1(7-36)NH₂; (SEQ ID NO.248)

(Gry⁸, Aib³⁵, Arg^{26,34}, Lys³⁶(N^{ε}-hexadecanesulfonyl))hGLP-1(7-36)NH₂; (SEQ ID NO.249)

(Gly⁸, Aib³⁵, Asp²⁶(1-(4-decylpiperazine)), Arg³⁴)hGLP-1(7-36)NH₂; (SEQ ID NO.250)

(Gly⁸, Aib³⁵, Asp²⁶(1-(4-dodecylpiperazine)), Arg³⁴)hGLP-1(7-36)NH₂; (SEQ ID NO.251)

(Gly⁸, Aib³⁵, ASp²⁶(1-(4-tetradecylpiperazine)), Arg³⁴)hGLP-1(7-36)NH₂; (SEQ ID NO.252)

(Gly⁸, Aib³⁵, Asp²⁶(1-(4-hexadecylpiperazine)), Arg³⁴)hGLP-1(7-36)NH₂; (SEQ ID NO.253)

(Gly⁸, Aib³⁵, Arg²⁶, Asp³⁴(1-(4-decylpiperazine)))hGLP-1(7-36)NH₂; (SEQ ID NO.254)

(Gly⁸, Aib³⁵, Arg²⁶, Asp³⁴(1-(4-dodecylpiperazine)))hGLP-1(7-36)NH₂; (SEQ ID NO.255)

(Gly⁸, Aib³⁵, Arg²⁶, Asp³⁴(1-(4-tetradecylpiperazine)))hGLP-1(7-36)NH₂; (SEQ ID NO.256)

(Gly⁸, Aib³⁵, Arg²⁶, Asp³⁴(1-(4-hexadecylpiperazine)))hGLP-1(7-36)NH₂; (SEQ ID NO.257)

(Gly⁸, Aib³⁵, Arg^{26,34}, Asp³⁶(1-(4-decylpiperazine)))hLGP-1(7-36)NH₂; (SEQ ID NO.258)

(Gly⁸, Aib³⁵, Arg^{26,34}, Asp³⁶(1-(4-dodecylpiperazine)))hGLP-1(7-36)NH₂; (SEQ ID NO.259)

(Gly⁸, Aib³⁵, Arg^{26,34}, Asp³⁶(1-(4-hexadecylpiperazine)))hGLP-1(7-36)NH₂; (SEQ ID NO.260)

(Gly⁸, Aib³⁵, Arg^{26,34}, Asp³⁸(1-(4-decylpiperazine)))hGLP-1(7-38)NH₂; (SEQ ID NO.261)

(Gly⁸, Aib³⁵, Arg^{26,34}, Asp³⁸(1-(4-dodecylpiperazine)))hGLP-1(7-38)NH₂; (SEQ ID NO.262)

(Gly⁸, Aib³⁵, Arg^{26,34}, Asp³⁸(1-(4-tetradecylpiperazine)))hGLP-1(7-38)NH₂; (SEQ ID NO.263)

(Gly⁸, Aib³⁵, Arg^{26,34}, Asp³⁸(1-(4-hexadecylpiperazine)))hGLP-1(7-38)NH₂; (SEQ ID NO.264)

(Gly⁸, Aib^{35,37}, Arg^{26,34}, Asp³⁸(1-(4-decylpiperazine)))hGLP-1(7-38)NH₂; (SEQ ID NO.265)

(Gly⁸, Aib^{35,37}, Arg^{26,34}, Asp³⁸(1-(4-dodecylpiperazine)))hGLP-1(7-38)NH₂; (SEQ ID NO.266)

(Gly⁸, Aib^{35,37}, Arg^{26,34}, Asp³⁸(1-(4-tetradecylpiperazine)))hGLP-1(7-33)NH₂; (SEQ ID NO.267)

(Gly⁸, Aib^{35,37}, Arg^{26,34}, Asp³⁸(1-(4-hexadecylpiperazine)))hGLP-1(7-38)NH₂; (SEQ ID NO.268)

(Gly⁸, Aib³⁵, Arg^{25,34}, Asp²⁶(1-(4-decylpiperazine)))hGLP-1(7-36)NH₂; (SEQ ID NO.269)

(Gly⁸, Aib³⁵, Arg^{25,34}, Asp²⁶(1-(4-dodecylpiperazine)))hGLP-1(7-36)NH₂; (SEQ ID NO.270)

(Gly⁸, Aib³⁵, Arg^{25,34}, Asp²⁶(1-(4-tetradecylpiperazine)))hGLP-1(7-36)NH₂; (SEQ ID NO.271)

(Gly⁸, Aib³⁵, Arg^{25,34}, Asp²⁶(1-(4-hexadecylpiperazine)))hGLP-1(7-36)NH₂; (SEQ ID NO.272)

(Gly⁸, Aib³⁵, Arg^{25,26}, Asp³⁴(1-(4-decylpiperazine)))hGLP-1(7-36)NH₂; (SEQ ID NO.273)

(Gly⁸, Aib³⁵, Arg^{25,26}, Asp³⁴(1-(4-dodecylpiperazine)))hGLP-1(7-36)NH₂; (SEQ ID NO.274)

(Gly⁸, Aib³⁵, Arg^{25,26}, Asp³⁴(1-(4-tetradecylpiperazine)))hGLP-1(7-36)NH₂; (SEQ ID NO.275)

(Gly⁸, Aib³⁵, Arg^{25,26}, Asp³⁴(1-(4-hexadecylpiperazine)))hGLP-1(7-36)NH₂; (SEQ ID NO.276)

(Gly⁸, Aib³⁵, Arg^{25,26,34}, Asp³⁶(1-(4-decylpiperazine)))hGLP-1(7-36)NH₂; (SEQ ID NO.277)

(Gly⁸, Aib³⁵, Arg^{25,26,34}, Asp³⁶(1-(4-dodecylpiperazine)))hGLP-1(7-36)NH₂; (SEQ ID NO.278)

(Gly⁸, Aib³⁵, Arg^{25,26,34}, Asp³⁶(1-(4-tetradecylpiperazine)))hGLP-1(7-36)NH₂; (SEQ ID NO.279)

(Gly⁸, Aib³⁵, Arg^{25,26,34}, Asp³⁶(1-(4-hexadecylpiperazine)))hGLP-1(7-36)NH₂; (SEQ ID NO.280)

(Gly⁸, Aib³⁵, Arg^{25,26,34}, Asp³⁸(1-(4-decylpiperazine)))hGLP-1(7-38)NH₂; (SEQ ID NO.281)

(Gly⁸, Aib³⁵, Arg^{25,26,34}, Asp³⁸(1-(4-dodecylpiperazine)))hGLP-1(7-38)NH₂; (SEQ ID NO.282)

(Gly⁸, Aib³⁶, Arg^{25,26,34}, Asp³⁸(1-(4-tetradecylpiperazine)))hGLP-1(7-38)NH₂; (SEQ ID NO.283)

(Gly⁸, Aib³⁵, Arg^{25,26,34}, Asp³⁸(1-(4-hexadecylpiperazine)))hGLP-1(7-38)NH₂; (SEQ ID NO.284)

(Gly⁸, Aib^{35,37}, Arg^{25,26,34}, Asp³⁸(1-(4-decylpiperazine)))hGLP-1(7-38)NH₂; (SEQ ID NO.285)

(Gly⁸, Aib^{35,37}, Arg^{25,26,34}, Asp³⁸(1-(4-dodecylpiperazine)))hGLP-1(7-38)NH₂; (SEQ ID NO.286)

(Gly⁸, Aib^{35,37}, Arg^{25,26,34}, Asp³⁸(1-(4-tetradecylpiperazine)))hGLP-1(7-38)NH₂; (SEQ ID NO.287)

(Gly⁸, Aib^{35,37}, Arg^{25,26,34}, Asp³⁸(1-(4-hexadecylpiperazine)))hGLP-1(7-38)NH₂; (SEQ ID NO.288)

(Gly⁸, Aib³⁵, Arg^{26,34}, Glu³⁶(1-dodecylamino))hGLP-1(7-36)NH₂; (SEQ ID NO.289)

(Gly⁸, Aib³⁵, Glu²⁶(1-dodecylamino), Arg³⁴)hGLP-1(7-36)NH₂; (SEQ ID NO.290)

(Gly⁸, Aib³⁵, Arg²⁶, Glu³⁴(1-dodecylamino))hGLP-1(7-36)NH₂; (SEQ ID NO.291)

(Gly⁸, Aib^{35,37}, Arg^{26,34}, Glu³⁸(1-dodecylamino))hGLP-1(7-38)NH₂; (SEQ ID NO.292)

(Gly⁸, Aib³⁵, Arg³⁴, Lys²⁶(N^{ε}-(2-(4-decyl-1-piperazine)-acetyl)))hGLP-1(7-36)NH₂; (SEQ ID NO.293)

(Gly⁸, Aib³⁵, Arg³⁴, Lys²⁶(N^{ε}-(2-(4-dodecyl-1-piperazine)-acetyl)))hGLP-1(7-36)NH₂; (SEQ ID NO.294)

(Gly⁸, Aib³⁵, Arg³⁴, Lys²⁶(N^{ε}-(2-(4-tetradecyl-1-piperazine)-acetyl)))hGLP-1(7-36)NH₂; (SEQ ID NO.295)

(Gly⁸, Aib³⁵, Arg³⁴, Lys²⁶(N^{ε}-(2-(4-hexadecyl-1-piperazine)-acetyl)))hGLP-1(7-36)NH₂; (SEQ ID NO.296)

(Gly⁸, Aib³⁵, Arg²⁶, Lys³⁴(N^{ε}-(2-(4-decyl-1-piperazine)-acetyl)))hGLP-1(7-36)NH₂; (SEQ ID NO.297)

(Gly⁸, Aib³⁵, Arg²⁶, Lys³⁴(N^{ε}-(2-(4-dodecyl-1-piperazine)-acetyl))hGLP-1(7-36)NH₂; (SEQ ID NO.298)

(Gly⁸, Aib³⁵, Arg²⁶, Lys³⁴(N^{ε}-(2-(4-tetradecyl-1-piperazine)-acetyl)))hGLP-1(7-36)NH₂; (SEQ ID NO.299)

(Gly⁸, Aib³⁵, Arg²⁶, Lys³⁴(N^{ε}-(2-(4-hexadecyl-piperazine)-acetyl)))hGLP-1(7-36)NH₂; (SEQ ID NO.300)

(Gly⁸, Aib³⁵, Arg^{26,34}, Lys³⁶(N^{ε}-(2-(4-decyl-1-piperazine)-acetyl)))hGLP-1(7-36)NH₂; (SEQ ID NO.301)

(Gly⁸, Aib³⁵, Arg^{26,34}, Lys³⁶(N^{ε}-(2-(4-dodecyl-1-piperazine)-acetyl)))hGLP-1(7-36)NH₂; (SEQ ID NO.302)

(Gly⁸, Aib³⁵, Arg^{26,34}, Lys³⁶(N^{ε}-(2-(4-hexedecyl-1-piperazine)-acetyl)))hGLP-1(7-36)NH₂; (SEQ ID NO.303)

(Gly⁸, Aib³⁵, Arg^{26,34}, Lys³⁸(N^{ε}-(2-(4-decyl-1-piperazine)-acetyl)))hGLP-1(7-38)NH₂; (SEQ ID NO.304)

(Gly⁸, Aib³⁵, Arg^{26,34}, Lys³⁸(N^{ε}-(2-(4-dodecyl-1-piperazine)-acetyl)))hGLP-1(7-38)NH₂; (SEQ ID NO.305)

(Gly⁸, Aib³⁵, Arg^{25,34}, Lys³⁸(N^{ε}-(2-(4-tetradecyl-1-piperazine)-acetyl)))hGLP-1(7-38)NH₂; ((SEQ ID NO.306)

(Gly⁸, Aib³⁵, Arg^{26,34}, Lys³⁸(N^{ε}-(2-(4-hexadecyl-1-piperazine)-acetyl)))hGLP-1(7-38)NH₂; (SEQ ID NO.307)

(Gly⁸, Aib^{35,37}, Arg^{26,34}, Lys³⁸(N^{ε}-(2-(4-decyl-1-piperazine)-acetyl)))hGLP-1(7-38)NH₂; (SEQ ID NO.308)

(Gly⁸, Aib^{35,37}, Arg^{26,34}, Lys³⁸(N^{ε}-(2-(4-dodecyl-1-piperazine)-acetyl)))hGLP-1(7-38)NH₂; (SEQ ID NO.309)

(Gly⁸, Aib^{35,37}, Arg^{26,34}, Lys³⁸(N^{ε}-(2-(4-tetradecyl-1-piperazine)-acetyl)))hGLP-1(7-38)NH₂; (SEQ ID NO.310)

(Gly⁸, Aib^{35,37}, Arg^{26,34}, Lys³⁸(N^{ε}-(2-(4-hexadecyl-1-piperazine)-acetyl)))hGLP-1(7-38)NH₂; (SEQ ID NO.311)

(Gly⁸, Aib³⁵, Arg^{25,34}, Lys²⁶(N^{ε}-(2-(4-decyl-1-piperazine)-acetyl)))hGLP-1(7-36)NH₂; (SEQ ID NO.312)

(Gly⁸, Aib³⁵, Arg^{25,34}, Lys²⁶(N^{ε}-(2-(4-dodecyl-1-piperazine)-acetyl)))hGLP-1(7-36)NH₂; (SEQ ID NO.313)

(Gly⁸, Aib³⁵, Arg^{25,34}, Lys²⁶(N^{ε}-(2-(4-tetradecyl-piperazine)-acetyl)))hGLP-1(7-36)NH₂; (SEQ ID NO.314)

(Gly⁸, Aib³⁵, Arg^{25,34}, Lys²⁶(N^{ε}-(2-(4-hexadecyl-1-piperazine)-acetyl)))hGLP-1(7-36)NH₂; (SEQ ID NO.315)

(Gly⁸, Aib³⁵, Arg^{25,26}, Lys³⁴(N^{ε}(2-(4-decyl-1-piperazine)-acetyl)))hGLP-1(7-36)NH₂; (SEQ ID NO.316)

(Gly⁸, Aib³⁵, Arg^{25,26}, Lys³⁴(N^{ε}-(2-(4-dodecyl-1-pipeazine)-acetyl)))hGLP-1(7-36)NH₂; (SEQ ID NO.317)

(Gly⁸, Aib³⁵, Arg^{25,26}, Lys³⁴(N^{ε}-(2-(4-tetradecyl-1-piperazine)-acetyl)))hGLP-1(7-36)NH₂; (SEQ ID NO.318)

(Gly⁸, Aib³⁵, Arg^{25,26}, Lys³⁴(N^{ε}-(2-(4-hexadecyl-1-piperazine)-acetyl)))hGLP-1(7-36)NH₂; (SEQ ID NO.319)

(Gly⁸, Aib³⁵, Arg^{25,26,34}, Lys³⁶(N^{ε}-(2-(4-decyl-1-piperazine)-acetyl)))hGLP-1(7-36)NH₂; (SEQ ID NO.320)

(Gly⁸, Aib³⁵, Arg^{25,26,34}, Lys³⁶(N^{ε}-(2-(4-dodecyl-1-piperazine)-acetyl)))hGLP-1(7-36)NH₂; (SEQ ID NO.321)

(Gly⁸, Aib³⁵, Arg^{25,26,34}, Lys³⁶(N^{ε}-(2-(4-tetradecyl-1-piperazine)-acetyl)))hGLP-1(7-36)NH₂; (SEQ ID NO.322)

(Gly⁸, Aib³⁵, Arg^{25,26,34}, Lys³⁶(N^{ε}-(2-(4-hexadecyl-1-piperazine)-acetyl)))hGLP-1(7-36)NH₂; (SEQ ID NO.323)

(Gly⁸, Aib³⁵, Arg^{25,26,34}, Lys³⁸(N^{ε}-(2-(4-decyl-1-piperazine)-acetyl)))hGLP-1(7-38)NH₂; (SEQ ID NO.324)

(Gly⁸, Aib³⁵, Arg^{25,26,34}, Lys³⁸(N^{ε}-(2-(4-dodecyl-1-piperazine)-acetyl)))hGLP-1(7-38)NH₂; (SEQ ID NO.325)

(Gly⁸, Aib³⁵, Arg^{25,26,34}, Lys³⁸(N^{ε}-(2-(4-tetradecyl-1-piperazine)-acetyl)))hGLP-1(7-38)NH₂; (SEQ ID NO.326)

(Gly⁸, Aib³⁵, Arg^{25,26,34}, Lys³⁸(N^{ε}-(2-(4-hexadecyl-1-piperazine)-acetyl)))hGLP-1(7-38)NH₂; (SEQ ID NO.327)

(Gly⁸, Aib^{35,37}, Arg^{25,26,34}, Lys³⁸(N^{ε}-(2-(4-decyl-1-piperazine)-acetyl)))hGLP-1(7-38)NH₂; (SEQ ID NO.328)

(Gly⁸, Aib^{35,37}, Arg^{25,26,34}, Lys³⁸(N^{ε}-(2-(4-dodecy[-1-piperazine)-acetyl)))hGLP-1(7-38)NH₂; (SEQ ID NO.329)

(Gly⁸, Aib^{35,37}, Arg^{25,26,34}, Lys³⁸(N^{ε}-(2-(4-tetradecy)-1-piperazine)- (SEQ ID NO.330) acetyl)))hGLP-1(7-38)NH₂;

(Gly⁸, Aib^{35,37}, Arg^{25,26,34}, Lys³⁸(N^{ε}-(2-(4-hexadecyl-1-piperazine)-acetyl)))hGLP-1(7-38)NH₂; (SEQ ID NO.331)

(Gly⁸, Aib³⁵, Arg^{26,34}, Lys³⁶(N^{ε}-decanoy)))hGLP-1(7-36)OH; (SEQ ID NO.332)

(Gly⁸, Aib³⁵, Lys²⁵, Arg^{26,34}, Lys³⁶(N^{ε}-decanoyl))hGLP-1(7-36)OH; (SEQ ID NO.333)

(Gly⁸, Aib³⁵,Arg^{26,34}, Ava³⁷, Ado³⁸)hGLP-1(7-38)NH₂; (SEQ ID NO.334)

(Gly⁸, Aib³⁵,Arg^{26,34}, Asp³⁷, Ava³⁸, Ado³⁹)hGLP-1(7-39)NH₂; (SEQ ID NO.335)

(Gly⁸, Aib³⁵,Arg^{26,34}, Aun³⁷)hGLP-1(7-37)NH₂; (SEQ ID NO.336)

(Gly⁸,Aib^{17,35})hGLP-1(7-36)NH₂; (SEQ ID NO.337)

(Gly⁸ ,Arg^{26,34}, β-Ala³⁵, D-Asp³⁷, Ava³⁸, Aun³⁹)hGLP-1(7-39)NH₂; (SEQ ID NO.338)

(Gly⁸, Glu^{22,23}, β-Ala³⁵)hGLP-1(7-36)NH₂; (SEQ ID NO.339)

(Gly⁸, Lys¹⁸, β-Ala³⁵)hGLP-1(7-36)NH₂; (SEQ ID NO.340)

(Gly⁸, Leu²⁷, β-Ala³⁵)hGLP-1(7-36)NH₂; (SEQ ID NO.341)

(Gly⁸; Lys³³, β-Ala³⁵)hGLP-1(7-36)NH₂; (SEQ ID NO.342)

(Gly⁸, Lys¹⁸, Leu²⁷, β-Ala³⁵)hGLP-1(7-36)NH₂; (SEQ ID NO.343)

(Gly⁸, D-Arg³⁶)hGLP-1(7-36)NH₂; (SEQ ID NO.344)

(Gly⁸, β-Ala³⁵, D-Arg³⁷)hGLP-1(7-37)NH₂; (SEQ ID NO.345)

(Gly⁸, Aib²⁷, β-Ala³⁵)hGLP-1(7-36)NH₂; (SEQ ID NO.346)

(Gly⁸, Aib²⁷, β-Ala^{35,37}, Arg³⁸)hGLP-1(7-38)NH₂; (SEQ ID NO.347)

(Gly⁸, Aib²⁷, β-Ala^{35,37}, Arg^{38,39})hGLP-1(7-39)NH₂; (SEQ ID NO.348)

(Gly⁸, Lys^{18,27}, β-Ala³⁵)hGLP-1(7-36)NH₂; (SEQ ID NO.349)

(Gly⁸, Lys²⁷, β-Ala³⁵)hGLP-1(7-36)NH₂; (SEQ ID NO.350)

(Gly⁸, β-Ala³⁵, Arg³⁸)hGLP-1(7-38)NH₂; (SEQ ID NO.351)

(Gly⁸, Arg^{26,34}, β-Ala³⁵,)hGLP-1(7-36)NH₂; (SEQ ID NO.352)

(Gly⁸, D-Arg³⁵)hGLP-1(7-36)NH₂; (SEQ ID NO.353)

(Gly⁸, β-Ala³⁵, Arg³⁷)hGLP-1(7-37)NH₂; (SEQ ID NO.354)

(Gly⁸, Phe³¹, β-Ala³⁵)hGLP-1(7-36)NH₂; (SEQ ID NO.355)

(Gly⁸, Aib³⁵, Phe³¹)hGLP-1(7-36)NH₂; (SEQ ID NO.356)

(Gly⁸, Aib³⁵, 2-Nal³¹)hGLP-1(7-36)NH₂; (SEQ ID NO.357)

(Gly⁸, Aib³⁵, 2-Nal^{28,31})hGLP-1(7-36)NH₂; (SEQ ID NO.358)

(Gly⁸, Aib³⁵, Arg^{26,34}, 2-Nal³¹)hGLP-1(7-36)NH₂; (SEQ ID NO.359)

(Gly⁸, Aib³⁵, Arg^{26,34}, Phe³¹)hGLP-1(7-36)NH₂; (SEQ ID NO.360)

(Gly⁸, Aib³⁵, 2-Nal^{19,31})hGLP-1(7-36)NH₂; (SEQ ID NO.361)

(Gly⁸, Aib³⁵, 2-Nal^{12,31})hGLP-1(7-36)NH₂; (SEQ ID NO.362)

(Gly⁸, Aib³⁵, Lys³⁶(N^{ε}-decsnoyl))hGLP-1(7-36)NH₂; (SEQ ID NO.363)

(Gly⁸, Aib³⁵, Arg³⁴, Lys²⁶(N^{ε}-decanoyl))hGLP-1(7-36)NH₂; (SEQ ID NO.364)

(Gly⁸, Aib³⁵, Arg^{26,34}, Lys³⁶(N^{ε}-dodecanoyl))hGLP-1(7-36)NH₂; (SEQ ID NO.365)

(Gly⁸, β-Ala³⁵,Ser³⁷(O-decanoyl))hGLP1(7-37)-NH₂; (SEQ ID NO,366)

(Gly⁸, Aib²⁷, β-Ala^{35,37}, Arg³⁸, Lys³⁹(N^{ε}-octanoyl))hGLP-1(7-39)NH₂; (SEQ ID NO.367)

(Gly⁸, Arg^{26,34}, β-Ala³⁵, Lys³⁷(N^{ε}-octanoyl))hGLP-1(7-37)NH₂; (SEQ ID NO.368)

(Gly⁸, Arg^{26,34}, β-Ala³⁵, Lys³⁷(N^{ε}-decahoyl))hGLP-1(7-37)NH₂; (SEQ ID NO.369)

(Gly⁸, Arg^{26,34}, β-Ala³⁵, Ly²⁷(N^{ε}-tetradecanoyl))hGLP-1(7-37)NH₂; (SEQ ID NO.370)

(Gly⁸, Arg^{26,34}, β-Ala³⁵, Lys³⁷(N^{ε}-dodecanoyl))hGLP-1(7-37)NH₂; (SEQ ID NO.371)

(Gly⁸, Arg^{26,34}, β-Ala³⁵, Lys³⁷(N^{ε}-dodecanoyl))hGLP-1(8-37)NH₂; (SEQ ID NO.372)

(Gly⁸, Arg²⁶, Aib³⁵)hGLP-1(7-36)NH₂; (SEQ ID NO.373)

(Gly⁸, Arg²⁶, Phe³¹, Aib³⁵)hGLP-1(7-36)NH₂; (SEQ ID NO.374)

(Gly⁸, Arg²⁶, Phe³¹, β-Ala³⁵)hGLP-1(7-36)NH₂; (SEQ ID NO.375)

(Gly⁸, Arg²⁶, β-Ala³⁵)hGLP-1(7-36)NH₂; (SEQ ID NO.376)

(Gly⁸, Arg^{26,34}, Phe³¹, Aib³⁵)hGLP-1(7-36)NH₂; (SEQ ID NO.377)

(Gly⁸, Arg^{26,34}, Phe³¹, β-Ala³⁵)hGLP-1(7-36)NH₂; (SEQ ID NO.378)

(Gly⁸, Arg³⁴, Aib³⁵)hGLP-1(7-36)NH₂; (SEQ ID NO.379)

(Gly⁸, Arg³⁴, β-Ala³⁵)hGLP-1(7-36)NH₂; (SEQ ID NO.380)

(Gly⁸, Phe³¹, Arg³⁴, Aib³⁵)hGLP-1(7-36)NH₂; (SEQ ID NO.381)

(Gly⁸, Phe³¹, Arg³⁴, β-Ala³⁵)hGLP-1(7-36)NH₂; (SEQ ID NO.382)

(Gly⁸, Arg^{26,34}, β-Ala³⁵, Lys³⁶(N^{ε}-Aec-decanoyl))hGLP-1(7-36)NH₂; (SEQ ID NO.383)

or (Gly⁸, β-Ala³⁵, Ser³⁷(O-decanoyl))hGLP1(7-37)-NH₂; (SEQ ID NO.384)

or a pharmaceutically acceptable salt thereof.

Another preferred compound of the group of compounds described in paragraph [067] is a compound according to the formula:

((N^{α}-Me-His)⁷, Ser⁸, Aib³⁵)hGLP-1(7-36)NH₂; (SEQ ID NO.385)

((N^{α}-Me-His)⁷, Ser⁸, β-Ala³⁵)hGLP-1(7-36)NH₂; (SEQ ID NO.386)

((N^{α}-Me-His)⁷, Ser⁸, Aib³⁵, Arg^{26,34})hGLP-1(7-36)NH₂; (SEQ ID NO.387)

((N^{α}-Me-His)⁷, Ser⁸, Arg^{26,34}, β-Ala³⁵)hGLP-1(7-36)NH₂; (SEQ ID NO.388)

(Ser⁸, A6c³⁵)hGLP-1(7-36)NH₂; (SEQ ID NO.389)

(Ser⁸, A5c³⁵)hGLP-1(7-36)NH₂; (SEQ ID NO.390)

(Ser⁸, D-Ala³⁵)hGLP-1(7-36)NH₂; (SEQ ID NO.391)

(Ser⁸, Aib³⁵, A6c³²)hGLP-1(7-36)NH₂; (SEQ ID NO.392)

(Ser⁸, Aib³⁵, A5c³²)hGLP-1(7-36)NH₂; (SEQ ID NO.393)

(Ser⁸, Aib³⁵, Glu²³)hGLP-1(7-36)NH₂; (SEQ ID NO.394)

(Ser⁸, Aib^{24,35})hGLP-1(7-36)NH₂; (SEQ ID NO.395)

(Ser⁸, Aib^{30,35})hGLP-1(7-36)NH₂; (SEQ ID NO.396)

(Ser⁸, Aib^{25,35})hGLP-1(7-36)NH₂; (SEQ ID NO.397)

(Ser⁸, Aib³⁵, A6c^{16,20})hGLP-1(7-36)NH₂; (SEQ ID NO.398)

(Ser⁸, Aib³⁵, A6c^{16,29,32})hGLP-1(7-36)NH₂; (SEQ ID NO.399)

(Ser⁸, Aib³⁵, A6c^{20,32})hGLP-1(7-36)NH₂; (SEQ ID NO.400)

(Ser⁸, Aib³⁵, A6c²⁰)hGLP-1(7-36)NH₂; (SEQ ID NO.401)

(Ser⁸, Aib³⁵, Lys²⁵)hGLP-1(7-36)NH₂; (SEQ ID NO.402)

(Ser⁸, Aib^{24,35}, A6c²⁰)hGLP-1(7-36)NH₂; (SEQ ID NO.403)

(Ser⁸, Aib³⁵, A6c^{29,32})hGLP-1(7-36)NH₂; (SEQ ID NO.404)

(Ser⁸, Aib^{24,35}, A6c^{29,32})hGLP-1(7-36)NH₂; (SEQ ID NO.405)

(Ser⁸, Aib³⁵, A6c¹²)hGLP-1(7-36)NH₂; (SEQ ID NO.406)

(Ser⁸, Aib³⁵, Cha²⁰)hGLP-1(7-36)NH₂; (SEQ ID NO.407)

(Ser⁸, Aib³⁵, A6c³³)hGLP-1(7-36)NH₂; (SEQ ID NO.408)

(Ser⁸, A6c^{16,20}, β-Ala³⁵)hGLP-1(7-36)NH₂; (SEQ ID NO.409)

(Ser⁸, Aib²⁵, β-Ala²²)hGLP-1(7-36)NH₂; (SEQ ID NO.410)

(Ser⁸, Aib^{22,35})hGLP-1(7-36)NH₂; (SEQ ID NO.411)

(Ser⁸, Aib³⁵, Glu²³, A6c³²)hGLP-1(7-36)NH₂; (SEQ ID NO.412)

(Ser⁸, Aib^{24,35}, Glu²³, A6c³²)hGLP-1(7-36)NH₂; (SEQ ID NO.413)

(Ser⁸, Aib^{24,25,35}, Glu²³, A6c³²)hGLP-1(7-36)NH₂; (SEQ ID NO.414)

(Ser⁸, Aib^{24,25,35}, A6c^{16,20,32}, Glu²³)hGLP-1(7-36)NH₂; (SEQ ID NO.415)

(Ser⁸, A6c³², β-Ala³⁵)hGLP-1(7-3 6)NH₂; (SEQ ID NO.416)

(Ser⁸, A5c³², β-Ala³⁵)hGLP-1(7-36)NH₂; (SEQ ID NO.417)

(Ser⁸, Glu²³, β-Ala³⁵)hGLP-1(7-36)NH₂; (SEQ ID NO.418)

(Ser⁸, Aib²⁴, β-Ala³⁵)hGLP-1(7-36)NH₂; (SEQ ID NO.419)

(Ser⁸, Alb³⁰, β-Ala³⁵)hGLP-1(7-36)NH₂; (SEQ ID NO.420)

(Ser⁸, Alb²⁵, β-Ala³⁵)hGLP-1(7-36)NH₂; (SEQ ID NO.421)

(Ser⁸, A6c^{16,29,32}, β-Ala³⁵)hGLP-1(7-36)NH₂; (SEQ ID NO.422)

(Ser⁸, A6c^{21,32}, β-Ala³⁵)hGLP-1(7-36)NH₂; (SEQ ID NO.423)

(Ser⁸, A6c²⁰, β-Ala³⁵)hGLP-1(7-36)NH₂; (SEQ ID NO.424)

(Ser⁸, Lys²⁵, β-Ala³⁵)hGLP-1(7-36)NH₂; (SEQ ID NO.425)

(Ser⁸, Aib²⁴, A6c²⁰, β-Ala³⁵)hGLP-1(7-36)NH₂; (SEQ ID NO.426)

(Ser⁸, A6c^{29,32}, β-Ala³⁵)hGLP-1(7-36)NH₂; (SEQ ID NO.427)

(Ser⁸, Aib²⁴, A6c^{29,32}, β-Ala³⁵)hGLP-1(7-36)NH₂; (SEQ ID NO.428)

(Ser⁸, A6c¹², β-Ala³⁵)hGLP-1(7-36)NH₂; (SEQ ID NO.429)

(Ser⁸, Cha²⁰, β-Ala³⁵)hGLP-1(7-36)NH₂; (SEQ ID NO.430)

(Ser⁸ , A6c³³, β-Ala³⁵)hGLP-1(7-36)NH₂; (SEQ ID NO.431)

(Ser⁸, β-Ala^{22,35})hGLP-1(7-36)NH₂; (SEQ ID NO.432)

(Ser⁸, Aib²², β-Ala³⁵)hGLP-1(7-36)NH₂; (SEQ ID NO.433)

(Ser⁸, Glu²³, A6c³², β-Ala³⁵)hGLP-1(7-36)NH₂; (SEQ ID NO.434)

(Ser⁸, Aib²⁴, Glu²³, A6c³², β-Ala³⁵)hGLP-1(7-36)NH₂; (SEQ ID NO.435)

(Ser⁸, Aib²⁴, Glu²³, A6c³², Lys³⁴(N^{e}-octanoyl), β-Ala³⁵)hGLP-1(7-36)NH₂; (SEQ ID NO.436)

(Ser⁸, Aib^{24,25}, Glu²³, A6c³², β-Ala³⁵)hGLP-1(7-36)NH₂; (SEQ ID NO.437)

(Ser⁸, Aib^{24,25}, A6c^{16,20,32}, Glu²³, β-Ala³⁵)hGLP-1(7-36)NH₂; (SEQ ID NO.438)

(Ser⁸, Aib³⁵, D-Arg³⁶)hGLP-1(7-36)NH₂; (SEQ ID NO.439)

(Ser⁸, Aib³⁵, D-Lys³⁶)hGLP-1(7-36)NH₂; (SEQ ID NO.440)

(Ser⁸, β-Ala³⁵, D-Arg³⁶)hGLP-1(7-36)NH₂; (SEQ ID NO.441)

(Ser⁸, β-Ala³⁵, D-Lys³⁶)hGLP-1(7-36)NH₂; (SEQ ID NO.442)

(Ser⁸, Aib³⁵, Arg^{26,34})hGLP-1(7-36)NH₂; (SEQ ID NO.443)

(Ser⁸, Arg^{26,34}, β-Ala³⁵)hGLP-1(7-36)NH₂; (SEQ ID NO.444)

(Ser⁸, Aib³⁵, Arg^{25,26,34})hGLP-1(7-36)NH₂; (SEQ ID NO.445)

(Ser⁸, Arg^{25,26,34}, β-Ala³⁵)hGLP-1(7-36)NH₂; (SEQ ID NO.446)

(Ser⁸, Arg^{26,34}, β-Ala³⁵, Lys³⁶(N^{ε}-tetradecanoyl))hGLP-1(7-36)OH; (SEQ ID NO.447)

(Ser⁸, Aib³⁵, Arg^{26,34}, Lys³⁶(N^{ε}-tetradecanoyl))hGLP-1(7-37)OH; (SEQ ID NO.448)

(Ser⁸, Aib^{35,37}, Arg^{26,34}, Lys³⁶(N^{ε}-tetradecanoyl))hGLP-1(7-37)OH; (SEQ ID NO.449)

(Ser⁸, Aib³⁵, Arg^{26,34}, Lys³⁶(N^{ε}-tetradecanoyl), D-Ala³⁷)hGLP-1(7-37)OH; (SEQ ID NO.450)

(Ser⁸, Aib^{35,37}, Arg^{26,34}, Lys³⁸(N^{ε}-tetradecanoyl))hGLP-1(7-38)OH; (SEQ ID NO.451)

(Ser⁸, Aib³⁵, Arg^{26,34} β-Ala³⁷, Lys³⁸(N^{ε}-tetradecanoyl))hGLP-1(7-38)OH; (SEQ ID NO.452)

(Ser⁸, Aib³⁵, Arg^{26,34}, Lys³⁸(N^{ε}-tetradecanoyl))hGLP-1(7-38)OH; (SEQ ID NO.453)

(Ser⁸, Arg^{26,34}, Lys³⁶(Nε-tetradecanoyly, β-Ala³⁷)hGLP-1(7-37)OH; (SEQ ID NO.454)

(Ser⁸, Aib³⁷, Arg^{26,34}, Lys³⁶(N^{ε}-tetradecanoyl))hGLP-1(7-37)OH; (SEQ ID NO.455)

(Ser⁸, Aib³⁵, Arg^{26,34}, Ado³⁷)hGLP-1(7-37)OH; (SEQ ID NO.456)

(Ser⁸, Aib³⁵, Arg^{26,34}, Ado³⁷)hGLP-1(7-37)NH₂; (SEQ ID NO.457)

(Ser⁸, Arg^{26,34}, Lys³⁶(N^{ε}-tetradecanoyl), D-Ala³⁷)hGLP-1(7-37)OH; (SEQ ID NO.458)

(Ser⁸, Aib³⁷, Arg^{26,34}, Lys³⁸(N^{ε}-etradecanoyl))hGLP-1(7-38)OH; (SEQ ID NO.459)

(Ser⁸, Arg^{26,34}, β-Ala³⁷, Lys³⁸(N^{ε}-tetradecanoyl))hGLP-1(7-38)OH; (SEQ ID NO.460)

(Ser⁸, Aib³⁵, Lys²⁶(N^{ε}-octanoyl))hGLP-1(7-36)NH₂; (SEQ ID NO.461)

(Ser⁸, Aib³⁵, Lys²⁶(N^{ε}-telradecanoyl))hGLP-1(7-36)NH₂; (SEQ ID NO.462)

(Ser⁸, Aib³⁵, Lys²⁶(N^{ε}-hexadecanoyl))hGLP-1(7-36)NH₂; (SEQ ID NO.463)

(Ser⁸, Lys²⁶(N^{ε}-octanoyl), β-Ala³⁵)hGLP-1(7-36)NH₂; (SEQ ID NO.464)

(Ser⁸, Lys²⁶(N^{ε}-tetradecanoyl), β-Ala³⁵)hGLP-1(7-36)NH₂; (SEQ ID NO.465)

(Ser⁸, Lys²⁶(N^{ε}-hexadecanoyl), β-Ala³⁵)hGLP-1(7-36)NH₂; (SEQ ID NO.466)

(Ser⁸, Aib³⁵, Lys²⁶(N^{ε}-octanoyl), Arg³⁴)hGLP-1(7-36)NH₂; (SEQ ID NO.467)

(Ser⁸, Aib³⁵, Lys²⁶(N^{ε}-tetradecanoyl), Arg³⁴)hGLP-1(7-36)NH₂; (SEQ ID NO.468)

(Ser⁸, Aib³⁵, Lys²⁶(N^{ε}-hexadecanoyl), Arg³⁴)hGLP-1(7-36)NH₂; (SEQ ID NO.469)

(Ser⁸, Aib³⁵, Lys²⁶(N^{ε}-decanoyl), Arg³⁴)hGLP-1(7-36)NH₂; (SEQ ID NO.470)

(Ser⁸, Aib³⁵, Lys²⁵, Lys²⁶(N^{ε}-octanoyl), Arg³⁴)hGLP-1(7-36)NH₂; (SEQ ID NO.471)

(Ser⁸, Aib³⁵, Lys²⁵, Lys²⁶(N^{ε}-tetradecanoyl), Arg³⁴)hGLP-1(7-36)NH₂; (SEQ ID NO.472)

(Ser⁸, Aib³⁵, Lys²⁵, Lys²⁶(N^{ε}-hexadecanoyl), Arg³⁴)hGLP-1(7-36)NH₂; (SEQ ID NO.473)

(Ser⁸, Aib³⁵, Arg^{25,34}, Lys²⁶(N^{ε}-octanoyl))hGLP-1(7-36)NH₂; (SEQ ID NO.474)

(Ser⁸, Aib³⁵, Arg^{25,34}, Lys²⁶(N^{ε}-etradecanoyl))hGLP-1(7-36)NH₂; (SEQ ID NO.475)

(Ser⁸, Aib³⁵, Arg^{25,34}, Lys²⁶(N^{ε}-hexadecanoyl))hGLP-1(7-36)NH₂; (SEQ ID NO.476)

(Ser⁸, Aib³⁵, Arg^{25,34}, Lys²⁶(N^{ε}-decanoyl))hGLP-1(7-36)NH₂; (SEQ ID NO.477)

(Ser⁸, Lys²⁶(N^{ε}-octanoyl), Arg³⁴, β-Ala³⁵)hGLP-1(7-36)NH₂; (SEQ ID NO.478)

(Ser⁸, Lys²⁶(N^{ε}-tetradecanoyl), Arg³⁴, β-Ala³⁵)hGLP-1(7-36)NH₂; (SEQ ID NO.479)

(Ser⁸, Lys²⁶(N^{ε}-hexadecanoyl), Arg³⁴, β-Ala³⁵)hGLP-1(7-36)NH₂; (SEQ ID NO.480)

(Ser⁸, Lys²⁶(N^{ε}-decanoyl), Arg³⁴, β-Ala³⁵)hGLP-1(7-36)NH₂; (SEQ ID NO.481)

(Ser⁸, Aib³⁵, Lys³⁴(N^{ε}-octanoyl))hGLP-1(7-36)NH₂; (SEQ ID NO.482)

(Ser⁸, Aib³⁵, Lys³⁴(N^{ε}-tetradecanoyl))hGLP-1(7-36)NH₂; (SEQ ID NO.483)

(Ser⁸, Aib³⁵, Lys³⁴(N^{ε}-hexadecanoyl))hGLP-1(7-36)NH₂; (SEQ ID NO.484)

(Ser⁸, Aib³⁵, Arg²⁶, Lys³⁴(N^{ε}-octanoyl))hGLP-1(7-36)NH₂; (SEQ ID NO.485)

(Ser⁸, Aib³⁵, Arg²⁶, Lys³⁴(N^{ε}-hexadecanoyl))hGLP-1(7-36)NH₂; (SEQ ID NO.486)

(Ser⁸, Aib³⁵, Arg²⁶, Lys³⁴(N^{ε}-decanoyl))hGLP-1(7-36)NH₂; (SEQ ID NO.487)

(Ser⁸, Aib³⁵, Arg^{25,26}, Lys³⁴(N^{ε}-octanoyl))hGLP-1(7-36)NH₂; (SEQ ID NO.488)

(Ser⁸, Aib³⁵, Arg^{25,26}, Lys³⁴(N^{ε}-tetradecanoyl))hGLP-1(7-36)NH₂; (SEQ ID NO.489)

(Ser⁸, Aib³⁵, Arg^{25,26}, Lys³⁴(N^{ε}-hexadecanoyl))hGLP-1(7-36)NH₂; (SEQ ID NO.490)

(Ser⁸, Aib³⁵, Arg^{25,26}, Lys³⁴(N^{ε}-decanoyl))hGLP-1(7-36)NH₂; (SEQ ID NO.491)

(Ser⁸, Aib³⁵, Lys²⁵, Arg²⁶, Lys³⁴(N^{ε}-octanoyl))hGLP-1(7-36)NH₂; (SEQ ID NO.492)

(Ser⁸, Aib³⁵, Lys²⁵, Arg²⁶, Lys³⁴(N^{ε}-tetradecancyl))hGLP-1(7-36)NH₂; (SEQ ID NO.493)

(Ser⁸, Aib³⁵, Lys²⁵, Arg²⁶, Lys³⁴(N^{ε}-hexadecanoyl))hGLP-1(7-36)NH₂; (SEQ ID NO.494)

(Ser⁸, Aib³⁵, Lys³⁶(N^{ε}-octanoyl))hGLP-1(7-36)NH₂; (SEQ ID NO.495)

(Ser⁸, Aib³⁵, Lys³⁶(N^{ε}-etradecanoyl))hGLP-1(7-36)NH₂; (SEQ ID NO.496)

(Ser⁸, Aib³⁵, Lys³⁶(N^{ε}-hexadecanoyl))hGLP-1(7-36)NH₂; (SEQ ID NO.497)

(Ser⁸, Aib³⁵, Arg²⁶, Lys³⁶(N^{ε}-octanoyl))hGLP-1(7-36)NH₂; (SEQ ID NO.498)

(Ser⁸, Aib³⁵, Arg²⁶, Lys³⁶(N^{ε}-tetradecanoyl))hGLP-1(7-36)NH₂; (SEQ ID NO.499)

(Ser⁸, Aib³⁵, Arg²⁶, Lys³⁶(N^{ε}-hexadecanoyl))hGLP-1(7-36)NH₂; (SEQ ID NO.500)

(Ser⁸, Aib³⁵, Arg^{26,34}, Lys³⁶(N^{ε}-octanoyl))hGLP-1(7-36)NH₂; (SEQ ID NO.501)

(Ser⁸, Aib³⁵, Arg^{26,34}, Lys³⁶(N^{ε}-hexadecanoyl))hGLP-1(7-36)NH₂; (SEQ ID NO.502)

(Ser⁸, Aib³⁵, Arg^{26,34}, Lys³⁸(N^{ε}-octanoyl))hGLP-1(7-38)NH₂; (SEQ ID NO.503)

(Ser⁸, Aib³⁵, Arg^{26,34}, Lys³⁸(N^{ε}-decanoyl))hGLP-1(7-38)NH₂; (SEQ ID NO.504)

(Ser⁸, Aib³⁵, Arg^{26,34}, Lys³⁸(N^{ε}-tetradecanoyl))hGLP-1(7-38)NH₂; (SEQ ID NO.505)

(Ser⁸, Aib³⁵, Arg^{26,34}, Lys³⁸(N^{ε}-hexadecanoyl))hGLP-1(7-38)NH₂; (SEQ ID NO.506)

(Ser⁸, Aib^{35,37}, Arg^{25,26,34}, Lys³⁸(N^{ε}-octanoyl))hGLP-1(7-38)NH₂; (SEQ ID NO.507)

(Ser⁸, Aib^{35,37}, Arg^{25,26,34}, Lys³⁸(N^{ε}-decanoyl))hGLP-1(7-38)NH₂; (SEQ ID NO.508)

(Ser⁸, Aib^{35,37}, Arg^{25,26,34}, Lys³⁸(N^{ε}-tetradecanoyl))hGLP-1(7-38)NH₂; (SEQ ID NO.509)

(Ser⁸, Aib^{35,37}, Arg^{25,26,34}, Lys³⁸(N^{ε}-hexadecanoyl))hGLP-1(7-38)NH₂; (SEQ ID NO.510)

(Ser⁸, Aib^{35,37}, Arg^{26,34}, Lys³⁸(N^{ε}-octanoyl))hGLP-1(7-38)NH₂; (SEQ ID NO.511)

(Ser⁸, Aib^{35,37}, Arg^{26,34}, Lys³⁸(N^{ε}-decanoyl))hGLP-1(7-38)NH₂; (SEQ ID NO.512)

(Ser⁸, Aib^{35,37}, Arg^{26,34}, Lys³⁸(N^{ε}-hexadecanoyl))hGLP-1(7-38)NH₂; (SEQ ID NO.513)

(Ser⁸, Aib³⁵, Lys²⁵, Arg^{26,34}, Lys³⁶(N^{ε}-octanoyl))hGLP-1(7-36)NH₂; (SEQ ID NO.514)

(Ser⁸, Aib³⁵, Lys²⁵, Arg^{26,34}, Lys³⁶(N^{ε}-tetradecanoylh))GLP-1(7-36)NH₂; (SEQ ID NO.515)

(Ser⁸, Aib³⁵, Lys²⁵, Arg^{26,34}, Lys³⁶(N^{ε}-hexadecanoyl)hGLP-1(7-36)NH₂; (SEQ ID NO.516)

(Ser⁸, Aib³⁵, Arg^{25,26,34}, Lys³⁶(N^{ε}-octanoyl))hGLP-1(7-36)NH₂; (SEQ ID NO.517)

(Ser⁸, Aib³⁵, Arg^{25,26,34}, Lys³⁶(N^{ε}-tetradecanoyl))hGLP-1(7-36)NH₂; (SEQ ID NO.518)

(Ser⁸, Aib³⁵, Arg^{25,26,34}, Lys³⁶(N^{ε}-hexadecanoyl))hGLP-1(7-36)NH₂; (SEQ ID NO.579)

(Ser⁸, Aib³⁵, Arg^{25,26,34}, Lys³⁶(N^{ε}-decanoyl))hGLP-1(7-36)NH₂; (SEQ ID NO.520)

(Ser⁸, Lys³⁴(N^{ε}-octanoyl), β-Ala³⁵)hGLP-1(7-36)NH₂; (SEQ ID NO.521)

(Ser⁸, Lys³⁴(N^{ε}-tetradecanoyl), β-Ala³⁵)hGLP-1(7-36)NH₂; (SEQ ID NO.522)

(Ser⁸, Lys³⁴(N^{ε}-hexadecanoyl), β-Ala³⁵)hGLP-1(7-36)NH₂; (SEQ ID NO.523)

(Ser⁸, A6c³², Lys³⁴(N^{e}-octanoyl), β-Ala³⁵)hGLP-1(7-36)NH₂; (SEQ ID NO.524)

(Ser⁸, Glu²³, Lys³⁴(N^{e}-octanoyl), β-Ala³⁵)hGLP-1(7-36)NH₂; (SEQ ID NO.525)

(Ser⁸, Glu²³, A6c³², Lys³⁴(N^{e}-octanoyl), β-Ala³⁵)hGLP-1(7-36)NH₂; (SEQ ID NO.526)

(Ser⁸, Arg²⁶, Lys³⁴(N^{ε}-octanoyl), β-Ala³⁵)hGLP-1(7-36)NH₂; (SEQ ID NO.527)

(Ser⁸, Arg²⁶, Lys³⁴(N^{ε}-tetradecanoyl), β-Ala³⁵)hGLP-1(7-36)NH₂; (SEQ ID NO.528)

(Ser⁸, Arg²⁶, Lys³⁴(N^{ε}-hexadecanoyl), β-Ala³⁵)hGLP-1(7-36)NH₂; (SEQ ID NO.529)

(Ser⁸, Arg²⁶, Lys³⁴(N^{ε}-decanoyl), β-Ala³⁵)hGLP-1(7-36)NH₂; (SEQ ID NO.530)

(Ser⁸, Arg^{25,26}, Lys³⁴(N^{ε}-octanoyl), β-Ala³⁵)hGLP-1(7-36)NH₂; (SEQ ID NO.531)

(Ser⁸, Arg^{25,26}, Lys³⁴(N^{ε}-tetradecanoyl), β-Ala³⁵)hGLP-1(7-36)NH₂; (SEQ ID NO.532)

(Ser⁸, Arg^{25,26}, Lys³⁴(N^{ε}-hexadecanoyl), β-Ala³⁵)hGLP-1(7-36)NH₂; (SEQ ID NO.533)

(Ser⁸, Arg^{25,26}, Lys³⁴(N^{ε}-decanoyl), β-Ala³⁵)hGLP-1(7-36)NH₂; (SEQ ID NO.534)

(Ser⁸, Lys²⁵, Arg²⁶, Lys³⁴(N^{ε}-octanoyl), β-Ala³⁵)hGLP-1(7-36)NH₂; (SEQ ID NO.535)

(Ser⁸, Lys²⁵, Arg²⁶, Lys³⁴(N^{ε}-tetractecanoyl), β-Ala³⁵)hGLP-1(7-36)NH₂; (SEQ ID NO.536)

(Ser⁸, Lys²⁵, Arg²⁶, Lys³⁴(N^{ε}-hexadecanoyl), β-Ala³⁵)hGLP-1(7-36)NH₂; (SEQ ID NO.537)

(Ser⁸, β-Ala³⁵, Lys³⁶(N^{ε}-octanoyl))hGLP-1(7-36)NH₂; (SEQ ID NO.538)

(Ser⁸, β-Ala³⁵, Lys³⁶(N^{ε}-tetradecanoyl))hGLP-1(7-36)NH₂; (SEQ ID NO.539)

(Ser⁸, β-Ala³⁵, Lys³⁶(N^{ε}-hexadecanoyl))hGLP-1(7-36)NH₂; (SEQ ID NO.540)

(Ser⁸, Arg²⁶, β-Ala³⁵, Lys³⁶(N^{ε}-octanoyl))hGLP-1(7-36)NH₂; (SEQ ID NO.541)

(Ser⁸, Arg²⁶, β-Ala³⁵, Lys³⁶(N^{ε}-tetradecanoyl))hGLP-1(7-36)NH₂; (SEQ ID NO.542)

(Ser⁸, Arg²⁶, β-Ala³⁵, Lys³⁶(N^{ε}-hexadecanoyl))hGLP-1(7-36)NH₂; (SEQ ID NO.543)

(Ser⁸, Arg^{26,34}, β-Ala³⁵, Lys³⁶(N^{ε}-octanoyl))hGLP-1(7-36)NH₂; (SEQ ID NO.544)

(Ser⁸, Arg^{26,34}, β-Ala³⁵, Lys³⁶(N^{ε}-tetradecanoyl))hGLP-1(7-36)NH₂; (SEQ ID NO.545)

(Ser⁸, Arg^{26,34}, β-Ala³⁵, Lys³⁶(N^{ε}-hexadecanoyl))hGLP-1(7-36)NH₂; (SEQ ID NO.546)

(Ser⁸, Arg^{26,34}, β-Ala³⁵, Lys³⁶(N^{ε}-decanoyl))hGLP-1(7-36)NH₂; (SEQ ID NO.547)

(Ser⁸, Lys²⁵, Arg^{26,34}, β-Ala³⁵, Lys³⁶(N^{ε}-octanoyl))hGLP-1(7-36)NH₂; (SEQ ID NO.548)

(Ser⁸, Lys²⁵, Arg^{26,34}, Lys³⁶(N^{ε}-tetractecanoyl), β-Ala³⁵)hGLP-1(7-36)NH₂; (SEQ ID NO.549)

(Ser⁸, Lys²⁵, Arg^{26,34}, β-Ala³⁵, Lys³⁶(N^{ε}-hexadecanoyl))hGLP-1(7-36)NH₂; (SEQ ID NO.550)

(Ser⁸, Arg^{25,26,34}, β-Ala³⁵, Lys³⁶(N^{ε}-octanoyl))hGLP-1(7-36)NH₂; (SEQ ID NO.551)

(Ser⁸, Arg^{25,26,34}, β-Ala³⁵, Lys³⁶(N^{ε}-tetradecanoyl))hGLP-1(7-36)NH₂; (SEQ ID NO.552)

(Ser⁸, Arg^{25,26,34}, β-Ala³⁵, Lys³⁶(N^{ε}-hexadecanoyl))hGLP-1(7-36)NH₂; (SEQ ID NO.553)

(Ser⁸, Arg^{25,26,34}, β-Ala³⁵, Lys³⁶(N^{ε}-decanoyl))hGLP-1(7-36)NH₂; (SEQ ID NO.554)

(Ser⁸, Aib³⁵, Lys²⁶(N^{ε}-octanoyl), A6c³², Arg³⁴)hGLP-1(7-36)NH₂; (SEQ ID NO.555)

(Ser⁸, Aib³⁵, Lys²⁶(N^{ε}-tetradecanoyl), A6c³², Arg³⁴)hGLP-1(7-36)NH₂; (SEQ ID NO.556)

(Ser⁸, Aib³⁵, Lys²⁶(N^{ε}-hexadecanoyl), A6c³², Arg³⁴)hGLP-1(7-36)NH₂; (SEQ ID NO.557)

(Ser⁸, Aib³⁵, A6c³², Lys³⁴(N^{ε}-octanoyl))hGLP-1(7-36)NH₂; (SEQ ID NO.558)

(Ser⁸, Aib³⁵, A6c³², Lys³⁴(N^{ε}-tetradecanoyl))hGLP-1(7-36)NH₂; (SEQ ID NO.559)

(Ser⁸, Aib³⁵, A6c³², Lys³⁴(N^{ε}-hexadecanoyl))hGLP-1(7-36)NH₂; (SEQ ID NO.560)

(Ser⁸, Aib³⁵, Arg²⁶, A6c³², Lys³⁴(N^{ε}-octanoyl))hGLP-1(7-36)NH₂; (SEQ ID NO.561)

(Ser⁸, Aib³⁵, Arg²⁶, A6c³², Lys³⁴(N^{ε}-tetradecanoyl))hGLP-1(7-36)NH₂; (SEQ ID NO.562)

(Ser⁸, Aib³⁵, A6c³², Lys³⁶(N^{ε}-octanoyl)hGLP-1(7-36)NH₂; (SEQ ID NO.563)

(Ser⁸, Aib³⁵, A6c³², Lys³⁶(N^{ε}-tetradecanoyl))hGLP-1(7-36)NH₂; (SEQ ID NO.564)

(Ser⁸, Aib³⁵, A6c³², Lys³⁶(N^{ε}-hexadecanoyl))hGLP-1(7-36)NH₂; (SEQ ID NO.565)

(Ser⁸, Aib³⁵, Arg²⁶, A6c³², Lys³⁶(N^{ε}-octanoyl))hGLP-1(7-36)NH₂; (SEQ ID NO.566)

(Ser⁸, Aib³⁵, Arg²⁶, A6c³², Lys³⁶(N^{ε}-tetradecanoyl))hGLP-1(7-36)NH₂; (SEQ ID NO.567)

(Ser⁸, Aib³⁵, Arg²⁶, A6c³², Lys³⁶(N^{ε}-hexadecanoyl))hGLP-1(7-36)NH₂; (SEQ ID NO.568)

(Ser⁸, Aib³⁵, Arg^{26,34}, A6c³², Lys³⁶(N^{ε}-octanoyl))hGLP-1(7-36)NH₂; (SEQ ID NO.569)

(Ser⁸, Aib³⁵, Arg^{26,34}, A6c³², Lys³⁶(N^{ε}-decanoyl))hGLP-1(7-36)NH₂; (SEQ ID NO.570)

(Ser⁸, Aib³⁵, Arg^{26,34}, A6c³², Lys³⁶(N^{ε}-tetradecanoyl))hGLP-1(7-36)NH₂; (SEQ ID NO.571)

(Ser⁸, Aib³⁵, Arg^{26,34}, A6c³², Lys³⁶(N^{ε}-hexadecanoyl))hGLP-1(7-36)NH₂; (SEQ ID NO.572)

(Ser⁸, Aib^{24,35}, Lys²⁶(N^{ε}-octanoyl), Arg³⁴)hGLP-1(7-36)NH₂; (SEQ ID NO.573)

(Ser⁸, Aib^{24,35}, Lys²⁶(N^{ε}-tetradecanoyl), Arg³⁴)hGLP-1(7-36)NH₂; (SEQ ID NO.574)

(Ser⁸, Aib^{24,35}, Lys²⁶(N^{ε}-hexadecanoyl), Arg³⁴)hGLP-1(7-36)NH₂, (SEQ ID NO.575)

(Ser⁸, Aib^{24,35}, Arg²⁶, Lys³⁴(N^{ε}-octanoyl))hGLP-1(7-36)NH₂; (SEQ ID NO.576)

(Ser⁸, Aib^{24,35}, Arg²⁶, Lys³⁴(N^{ε}-tetradecanoyl))hGLP-1(7-36)NH₂; (SEQ ID NO.577)

(Ser⁸, Aib^{24,35}, Arg²⁶, Lys³⁴(N^{ε}-hexadecanoyl))hGLP-1(7-36)NH₂; (SEQ ID NO.578)

(Ser⁸, Aib^{24,35}, Arg^{26,34}, Lys³⁶(N^{ε}-octanoyl))hGLP-1(7-36)NH₂; (SEQ ID NO.579)

(Ser⁸, Aib^{24,35}, Arg^{26,34}, Lys³⁶(N^{ε}-tetradecanoyl))hGLP-1(7-36)NH₂; (SEQ ID NO.580)

(Ser⁸, Aib^{24,35}, Arg^{26,34}, Lys³⁶(N^{ε}-hexadecanoyl))hGLP-1(7-36)NH₂; (SEQ ID NO.581)

(Ser⁸, Aib^{24,35}, Glu²³, A6c³², Lys³⁴(N^{e}-octanoyl))hGLP-1(7-36)NH₂; (SEQ ID NO.582)

(Ser⁸, Aib³⁵, Glu²³, Lys²⁶(N^{ε}-octanoyl), Arg³⁴)hGLP-1(7-36)NH₂; (SEQ ID NO.583)

(Ser⁸, Aib³⁵, Glu²³, Lys²⁶(N^{ε}-tetradecanoyl), Arg³⁴)hGLP-1(7-36)NH₂; (SEQ ID NO.584)

(Ser⁸, Aib³⁵, Glu²³, Lys²⁶(N^{ε}-hexadecanoyl), Arg³⁴)hGLP-1(7-36)NH₂; (SEQ ID NO.585)

(Ser⁸, Aib³⁵, Glu²³, Lys³⁴(N^{e}-octanoyl))hGLP-1(7-36)NH₂; (SEQ ID NO.586)

(Ser⁸, Aib³⁵, Glu²³, A6c³², Lys³⁴(N^{e}-octanoyl))hGLP-1(7-36)NH₂; (SEQ ID NO.587)

(Ser⁸, Aib³⁵, Glu²³, Arg²⁶, Lys³⁴(N^{ε}-octanoyl))hGLP-1(7-36)NH₂; (SEQ ID NO.588)

(Ser⁸, Aib³⁵, Glu²³, Arg²⁶, Lys³⁴(N^{ε}-tetradecanoyl))hGLP-1(7-36)NH₂; (SEQ ID NO.589)

(Ser⁸, Aib³⁵, Glu²³, Arg²⁶, Lys³⁴(N^{ε}-hexadecanoyl)hGLP-1(7-36)NH₂; (SEO ID NO.590)

(Ser⁸, Aib³⁵, Glu²³, Lys³⁶(N^{ε}-octanoyl))hGLP-1(7-36)NH₂; (SEQ ID NO.591)

(Ser⁸, Aib³⁵; Glu²³, Lys³⁶(N^{ε}-tetradecanoyl))hGLP-1(7-36)NH₂; (SEQ ID NO.592)

(Ser⁸, Aib³⁵, Glu²³, Lys³⁶(N^{ε}-hexadecanoyl))hGLP-1(7-36)NH₂; (SEQ ID NO.593)

(Ser⁸, Aib³⁵, Glu²³, Arg^{26,34}, Lys³⁶(N^{ε}-octanoyl))hGLP-1(7-36)NH₂; (SEQ ID NO.594)

(Ser⁸, Aib³⁵, Glu²³, Arg^{26,34}, Lys³⁶(N^{ε}-tetradecanoyl)hGLP-1(7-36)NH₂; (SEQ ID NO.595)

(Ser⁸, Aib³⁵, Glu²³, Arg^{26,34}, Lys³⁶(N^{ε}-hexadecanoyl))hGLP-1(7-36)NH₂; (SEQ ID NO.596)

(Ser⁸, Aib^{30,35}, Lys²⁶(N^{ε}-octanoyl), Arg³⁴)hGLP-1(7-36)NH₂; (SEQ ID NO.597)

(Ser⁸, Aib^{30,35}, Lys²⁶(N^{ε}-tetradecanoyl), Arg³⁴)hGLP-1(7-36)NH₂; (SEQ ID NO.598)

(Ser⁸, Aib^{30,35}, Lys²⁶(N^{ε}-hexadecanoyl), Arg³⁴)hGLP-1(7-36)NH₂; (SEQ ID NO.599)

(Ser⁸, Aib^{30,35}, Arg²⁶, Lys³⁴(N^{ε}-octanoyl))hGLP-1(7-36)NH₂; (SEQ ID NO.600)

(Ser⁸, Aib^{30,35}, Arg²⁶, Lys³⁴(N^{ε}-tetradecanoyl))hGLP-1(7-36)NH₂; (SEQ ID NO.601)

(Ser⁸, Aib^{30,35}, Arg²⁶, Lys³⁴(N^{ε}-hexadecanoy))hGLP-1(7-36)NH₂; (SEQ ID NO.602)

(Ser⁸, Aib^{30,35}, Arg^{26,34}, Lys³⁶(N^{ε}-octanoyl))hGLP-1(7-36)NH₂; (SEQ ID NO.603)

(Ser⁸, Aib^{30,35}, Arg^{26,34}, Lys³⁶(N^{ε}-tetradecanoyl))hGLP-1(7-36)NH₂; (SEQ ID NO.604)

(Ser⁸, Aib^{30,35}, Arg^{26,34}, Lys³⁶(N^{ε}-hexadecanoyl))hGLP-1(7-36)NH₂; (SEQ ID NO.605)

(Ser⁸, Aib³⁵, Glu²³, A6c³², Lys³⁶(N^{ε}-octanoyl))hGLP-1(7-36)NH₂; (SEQ ID NO.606)

(Ser⁸, Aib³⁵, Glu²³, A6c³², Lys³⁶(N^{ε}-tetradecanoyl))hGLP-1(7-36)NH₂; (SEQ ID NO.607)

(Ser⁸, Aib³⁵, Glu²³, A6c³², Lys³⁶(N^{ε}-hexadecanoyl))hGLP-1(7-36)NH₂; (SEQ ID NO.608)

(Ser⁸, Aib³⁵, Glu²³, Arg^{26,34}, A6c³², Lys³⁶(N^{ε}-octanoyl))hGLP-1 (7-36)NH₂; (SEQ ID NO.609)

(Ser⁸, Aib³⁵, Glu²³, Arg^{26,34}, A6c³², Lys³⁶(N^{ε}-tetradecanoyl))hGLP-1(7-36)NH₂; (SEQ ID NO.610)

(Ser⁸, Aib³⁵, Glu²³, Arg^{26,34}, A6c³², Lys³⁶(N^{ε}-hexadecanoyl))hGLP-1(7-36)NH₂; (SEQ ID NO.611)

(Ser⁸, Aib^{24,35}, Glu²³, Arg^{26,34}, A6c³², Lys³⁶(N^{ε}-octanoyl))hGLP-1(7-36)NH₂; (SEQ ID NO.612)

(Ser⁸, Aib^{24,35}, Glu²³, Arg^{26,34}, A6c³², Lys³⁶(N^{ε}-tetradecanoyl))hGLP-1(7-36)NH₂; (SEQ ID NO.613)

(Ser⁸, Aib^{24,35}, Glu²³, Arg^{26,34}, A6c³², Lys³⁶(N^{ε}-hexadecanoyl))hGLP-1(7-36)NH₂; (SEQ ID NO.614)

(Ser⁸, Aib^{24,30,35}, Glu²³, Arg^{26,34}, A6c³², Lys³⁶(N^{ε}-octanoyl))hGLP-1(7-36)NH₂; (SEQ ID NO.615)

(Ser⁸, Aib^{24,30,35}, Glu²³, Arg^{26,34}, A6c³², Lys³⁶(N^{ε}-tetradecanoyl)hGLP-1(7-36)NH₂; (SEQ ID NO.616)

(Ser⁸, Aib^{24,30,35}, Glu²³, Arg^{26,34}, A6c³²,Lys³⁶(N^{ε}-hexadecanoyl))hGLP-1(7-36)NH₂; (SEQ ID NO.617)

((N^{α}-HEPES-His)⁷, Ser⁸, β-Ala³⁵)hGLP-1(7-36)NH₂; (SEQ ID NO.618)

((N^{α}-HEPA-His)⁷, Ser⁸, β-Ala³⁵)hGLP-1(7-36)NH₂; (SEQ ID NO.619)

((N^{α}-tetradecanoyl-His)⁷, Ser⁸, Aib³⁵)hGLP-1(7-36)NH₂; (SEQ ID NO.620)

((N^{α}-tetradecanoyl-His)⁷, Ser⁸, β-Ala³⁵)hGLP-1(7-36)NH₂; (SEQ ID NO.621)

((N^{α}-tetradecanoyl-His)⁷, Ser⁸, Aib³⁵, Arg^{26,34})hGLP-1(7-36)NH₂; (SEQ ID NO.622)

((N^{α}-tetradecanoyl-His)⁷, Ser⁸, Arg^{26,34}, β-Ala³⁵)hGLP-1(7-36)NH₂; (SEQ ID NO.623)

((N^{α}-tetradecanoyl-His)⁷, Ser⁸, Aib³⁵, Arg^{25,26,34})hGLP-1(7-36)NH₂; (SEQ ID NO.624)

((N^{α}-tetradecanoyl-His)⁷, Ser⁸, Arg^{25,26,34}, β-Ala³⁵)hGLP-1(7-36)NH₂; (SEQ ID NO.625)

(Ser⁸, Aib³⁵, Lys²⁶(N^{ε}-octanesulfonyl), Arg³⁴)hGLP-1(7-36)NH₂; (SEQ ID NO.626)

(Ser⁸, Aib³⁵, Lys²⁶(N^{ε}-dodecanesulfonyl), Arg³⁴)hGLP-1(7-36)NH₂; (SEQ ID NO.627)

(Ser⁸, Aib³⁵, Lys²⁶(N^{ε}-hexadecanesulfonyl), Arg³⁴)hGLP-1(7-36)NH₂; (SEQ ID NO.628)

(Ser⁸, Aib³⁵, Arg²⁶, Lys³⁴(N^{ε}-octanesulfonyl))hGLP-1(7-36)NH₂; (SEQ ID NO.629)

(Ser⁸, Aib³⁵, Arg²⁶, Lys³⁴(N^{ε}-dodecanesulfonyl))hGLP-1(7-36)NH₂; (SEQ ID NO.630)

(Ser⁸, Aib³⁵, Arg²⁶, Lys³⁴(N^{ε}-hexadecanesulfonyl))hGLP-1(7-36)NH₂; (SEQ ID NO.631)

(Ser⁸, Aib³⁵, Arg^{26,34}, Lys³⁶(N^{ε}-octanesulfonyl))hGLP-1(7-36)NH₂; (SEQ ID NO.632)

(Ser⁸, Aib³⁵, Arg^{26,34}, Lys³⁶(N^{ε}-hexadecanesulfonyl))hGLP-1(7-36)NH₂; (SEQ ID NO.633)

(Ser⁸, Aib³⁵, Asp²⁶(1-(4-decylpiperazine)), Arg³⁴)hGLP-1(7-36)NH₂; (SEQ ID NO.634)

(Ser⁸, Aib³⁵, Asp²⁶(1-(4-dodecylpiperazine)), Arg³⁴)hGLP-1(7-36)NH₂; (SEQ ID NO.635)

(Ser⁸, Aib³⁵, Asp²⁶(1-(4-tetradecylpiperazine)), Arg³⁴)hGLP-1(7-36)NH₂; (SEQ ID NO.636)

(Ser⁸, Aib³⁵, Asp²⁸(1-(4-hexadecylpiperazine)), Arg³⁴)hGLP-1(7-36)NH₂; (SEQ ID NO.637)

(Ser⁸, Aib³⁵, Arg²⁶, Asp³⁴(1-(4-decylpiperazine)))hGLP-1(7-36)NH₂; (SEQ ID NO.638)

(Ser⁸, Aib³⁵, Arg²⁶, Asp³⁴(1-(4-dodecylpiperazine)))hGLP-1(7-36)NH₂; (SEQ ID NO.639)

(Ser⁸, Aib³⁵, Arg²⁶, Asp³⁴(1-(4-tetradecylpiperazine)))hGLP-1(7-36)NH₂; (SEQ ID NO.640)

(Ser⁸, Aib³⁵, Arg²⁶, Asp³⁴(1-(4-hexadecylpiperazine)))hGLP-1(7-36)NH₂; (SEQ ID NO.641)

(Ser⁸, Aib³⁵, Arg^{26,34}, Asp³⁶(1-(4-decylpiperazine)))hGLP-1(7-36)NH₂; (SEQ ID NO.642)

(Ser⁸, Aib³⁵, Arg^{26,34} Asp³⁶(1-(4-dodecylpiperazine)))hGLP-1(7-36)NH₂; (SEQ ID NO.643)

(Ser⁸, Aib³⁵, Arg^{26,34}, Asp³⁶(1-(4-hexadecylpiperazine)))hGLP-1(7-36)NH₂; (SEQ ID NO.644)

(Ser⁸, Aib³⁵, Arg^{26,34}, Asp³⁸(1-(4-decylpiperazine)))hGLP-1(7-38)NH₂; (SEQ ID NO.645)

(Ser⁸, Aib³⁵, Arg^{26,34}, Asp³⁸(1-(4-dodecylpiperazine)))hGLP-1(7-38)NH₂; (SEQ ID NO.646)

(Ser⁸, Aib³⁵, Arg^{26,34}, Asp³⁸(1-(4-tetradecylpiperazine)))hGLP-1(7-38)NH₂; (SEQ ID NO.647)

(Ser⁸, Aib³⁵, Arg^{26,34}, Asp³⁸(1-(4-hexadecylpiperazine)))hGLP-1(7-38)NH₂; (SEQ ID NO.648)

(Ser⁸, Aib^{35,37}, Arg^{26,34}, Asp³⁸(1-(4-decylpiperazine)))hGLP-1(7-38)NH₂; (SEQ ID NO.649)

(Ser⁸, Aib^{35,37}, Arg^{26,34}, Asp³⁸(1-(4-dodecylpiperazine)))hGLP-1(7-38)NH₂; (SEQ ID NO.650)

(Ser⁸, Aib^{35,37}, Arg^{26,34}, Asp³⁸(1-(4-tetradecylpiperazine)))hGLP-1(7-38)NH₂; (SEQ ID NO.651)

(Ser⁸, Aib^{35,37}, Arg^{26,34}, Asp³⁸(1-(4-hexadecylpiperazine))hGLP-1(7-38)NH₂; (SEQ ID NO.652)

(Ser⁸, Aib³⁵, Arg^{25,34}, Asp²⁶(1-(4-decylpiperazine)))hGLP-1(7-36)NH₂; (SEQ ID NO.653)

(Ser⁸, Aib³⁵, Arg^{25,34}, Asp²⁶(1-(4-dodecylpiperazine)))hGLP-1(7-36)NH₂; (SEQ ID NO,654)

(Ser⁸, Aib³⁵, Arg^{25,34}, Asp²⁶(1-(4-tetradecylpiperazine)))hGLP-1(7-36)NH₂; (SEQ ID NO.655)

(Ser⁸, Aib³⁵, Arg^{25,34}, Asp²⁶(1-(4-hexadecylpiperazine)))hGLP-1(7-36)NH₂; (SEQ ID NO.656)

(Ser⁸, Aib³⁵, Arg^{25,26}, Asp³⁴(1-(4-decylpiperazine)))hGLP-1(7-36)NH₂; (SEQ ID NO.657)

(Ser⁸, Aib³⁵, Arg^{25,26}, Asp³⁴(1-(4-dodecylpiperazine)))hGLP-1(7-36)NH₂; (SEQ ID NO.658)

(Ser⁸, Aib³⁵, Arg^{25,26}, Asp³⁴(1-(4-tetradecylpiperazine)))hGLP-1(7-36)NH₂; (SEQ ID NO.659)

(Ser⁸, Aib³⁵, Arg^{25,26}, Asp³⁴(1-(4-hexadecylpiperazine)))hGLP-1(7-36)NH₂; (SEQ ID NO.660)

(Ser⁸, Aib³⁵, Arg^{25,26,34}, Asp³⁶(1-(4-decylpiperatne)))hGLP-1(7-36)NH₂; (SEQ ID NO.661)

(Ser⁸, Aib³⁵, Arg^{25,26,34}, Asp³⁶(1-(4-dadecylpiperazine)))hGLP-1(7-36)NH₂; (SEQ ID NO.662)

(Ser⁸, Aib³⁵, Arg^{25,26,34}, Asp³⁶(1-(4-tetradecylpiperazine)))hGLP-1(7-36)NH₂; (SEQ ID NO.663)

(Ser⁸, Aib³⁵, Arg^{25,26,34}, Asp³⁶(1-(4-hexadecylpiperazine)))hGLP-1(7-36)NH₂; (SEQ ID NO.664)

(Ser⁸, Aib³⁵, Arg^{25,26,34}, Asp³⁸(1-(4-decylpiperazine)))hGLP-1(7-38)NH₂; (SEQ ID NO.665)

(Ser⁸, Aib³⁵, Arg^{25,26,34}, Asp³⁸(1-(4-dodecylpiperazine)))hGLP-1(7-38)NH₂; (SEQ ID NO.666)

(Ser⁸, Aib³⁵, Arg^{25,26,34}, Asp³⁸(1-(4-tetradecylpiperazine)))hGLP-1(7-38)NH₂; (SEQ ID NO.667)

(Ser⁸, Aib³⁵, Arg^{25,26,34}, Asp³⁸(1-(4-hexadecylpiperazine)))hGLP-1(7-38)NH₂; (SEQ ID NO.668)

(Ser⁸, Aib^{35,37}, Arg^{25,26,34}, Asp³⁸(1-(4-decylpiperazine)))hGLP-1(7-38)NH₂; (SEQ ID NO.669)

(Ser⁸, Aib^{35,37}, Arg^{25,26,34}, Asp³⁸(1-(4-dodecylpiperazine)))hGLP-1(7-38)NH₂; (SEQ ID NO.670)

(Ser⁸, Aib^{35,37}, Arg^{25,26,34}, Asp³⁸(1-(4-tetradecylpiperazine)))hGLP-1(7-38)NH₂; (SEQ ID NO.671)

(Ser⁸, Aib^{35,37}, Arg^{25,26,34}, Asp³⁸(1-(4-hexadecylpiperazine)))hGLP-1(7-38)NH₂; (SEQ ID NO.672)

(Ser⁸, Aib³⁵, Arg^{26,34}, Glu³⁶(1-dodecylamino))hGLP-1(7-36)NH₂; (SEQ ID NO.673)

(Ser⁸, Aib³⁵, Glu²⁶(1-dodecylamino), Arg³⁴)hGLP-1(7-36)NH₂; (SEQ ID NO.674)

(Ser⁸, Aib³⁵, Arg²⁶, Glu³⁴(1-dodecylamino))hGLP-1(7-36)NH₂; (SEQ ID NO.675)

(Ser⁸, Aib^{35,37}, Arg^{26,34}, Glu³⁸(1-dodecylamino))hGLP-1(7-38)NH₂; (SEQ ID NO.676)

(Ser⁸, Aib³⁵, Arg³⁴, Lys²⁶(N^{ε}-(2-(4-decyl-1-piperazine)-acetyl)))hGLP-1(7-36)NH₂; (SEQ ID NO.677)

(Ser⁸, Aib³⁵, Arg³⁴, Lys²⁶(N^{ε}-(2-(4-dodecyl-1-piperazine)-acetyl)))hGLP-1(7-36)NH₂; (SEQ ID NO.678)

(Ser⁸, Aib³⁵, Arg³⁴, Lys²⁶(N^{ε}-(2-(4-tetradecyl-1-piperazine)-acetyl)))hGLP-1(7-36)NH₂; (SEQ ID NO.679)

(Ser⁸, Aib³⁵, Arg³⁴, Lys²⁶(N^{ε}-(2-(4-hexadecyl-1-piperazine)-acetyl)))hGLP-1(7-36)NH₂; (SEQ ID NO.680)

(Ser⁸, Aib³⁵, Arg²⁶, Lys³⁴(N^{ε}-(4-decyl-1-piperazine)-acetyl)))hGLP-1(7-36)NH₂; (SEQ ID NO.681)

(Ser⁸, Aib³⁵, Arg²⁶, Lys³⁴(N^{ε}-(2-(4-dodecyl-1-piperazine)-acetyl))hGLP-1(7-36)NH₂; (SEQ ID NO.682)

(Ser⁸, Aib³⁵, Arg²⁶, Lys³⁴(N^{ε}-(2-(4-tetradecyl-1-piperazine)-acelyl)))hGLP-1(7-36)NH₂; (SEQ ID NO.683)

(Ser⁸, Aib³⁵, Arg²⁶, Lys³⁴(N^{ε}-(2-(4-hexadecyl-1-piperazine)-acetyl)))hGLP-1(7-36)NH₂; (SEQ ID NO.684)

(Ser⁸, Aib³⁵, Arg^{26,34}, Lys³⁶(N^{ε}-(2-(4-decyl-1-piperazine)-acetyl)))hGLP-1(7-36)NH₂; (SEQ ID NO.685)

(Ser⁸, Aib³⁵, Arg^{26,34}, Lys³⁶(N^{ε}-(2-(4-dodecyl-1-piperazine)-acetyl)))hGLP-1(7-36)NH₂, (SEQ ID NO.686)

(Ser⁸, Aib³⁵, Arg^{26,34}, Lys³⁶(N^{ε}-(2-(4-hexadecyl-1-piperazine)-acetyl)))hGLP-1(7-36)NH₂; (SEQ ID NO.687)

(Ser⁸, Aib³⁵, Arg^{26,34}, Lys³⁸(N^{ε}-(2-(4-decyl-1-piperazine)-acelyl)))hGLP-1(7-38)NH₂; (SEQ ID NO.688)

(Ser⁸, Aib³⁶, Arg^{26,34}, Lys³⁸(N^{ε}-(2-(4-dodecyl-1-piperazine)-acetyl)))hGLP-1(7-38)NH₂; (SEQ ID NO.689)

(Ser⁸, Aib³⁵, Arg^{26,34}, Lys³⁸(N^{ε}-(2-(4-tetradecyl-1-piperazine)-acetyl)))hGLP-1(7-38)NH₂; (SEQ ID NO.690)

(Ser⁸, Aib³⁵, Arg^{26,34}, Lys³⁸(N^{ε}-(2-(4-hexadecyl-1-piperazine)-acetyl)))hGLP-1(7-38)NH₂; (SEQ ID NO.691)

(Ser⁸, Aib^{35,37}, Arg^{26,34}, Lys³⁸(N^{ε}-(2-(4-decyl-1-piperazine)- (SEQ ID NO.692) acetyl)))hGLP-1(7-38)NH₂; (SEQ ID NO.692)

(Ser⁸, Aib^{35,37}, Arg^{26,34}, Lys³⁸(N^{ε}-(2-(4-dodecyl-1-piperazine)-acetyl)))hGLP-1(7-38)NH₂; (SEQ ID NO.693)

(Ser⁸, Aib^{35,37}, Arg^{26,34}, Lys³⁸(N^{ε}-(2-(4-tetradecyl-1-piperazine)-acetyl)))hGLP-1(7-38)NH₂; (SEQ ID NO.694)

(Ser⁸, Aib^{35,37}, Arg^{26,34}, Lys³⁸(N^{ε}-(2-(4-hexadecyl-1-piperazine)-acetyl)))hGLP-1(7-38)NH₂; (SEQ ID NO.695)

(Ser⁸, Aib³⁵, Arg^{25,34}, Lys²⁶(N^{ε}-(2-(4-decyl-1-piperazine)-acetyl)))hGLP-1(7-36)NH₂; (SEQ ID NO.696)

(Ser⁸, Aib³⁵, Arg^{25,34}, Lys²⁶(N^{ε}-(2-(4-dodecyl-1-piperazine)-acetyl)))hGLP-1(7-36)NH₂; (SEQ ID NO.697)

(Ser⁸, Aib³⁵, Arg^{25,34}, Lys²⁶(N^{ε}-(2-(4-tetradecyl-1-piperazine)-acetyl)))hGLP-1(7-36)NH₂; (SEQ ID NO.698)

(Ser⁸, Aib³⁵, Arg^{25,34}, Lys²⁶(N^{ε}-(2-(4-hexadecyl-1-piperazine)-acetyl)))hGLP-1(7-36)NH₂; (SEQ ID NO.699)

(Ser⁸, Aib³⁵, Arg^{25,26}, Lys³⁴(N^{ε}(2-(4-decy-1-piperazine)-acetyl)))hGLP-1(7-36)NH₂; (SEQ ID NO.700)

(Ser⁸, Aib³⁵, Arg^{25,26}, Lys³⁴(N^{ε}-(2-(4-dodecyl-1-piperazine)-acetyl)))hGLP-1(7-36)NH₂; (SEQ ID NO.701)

(Ser⁸, Aib³⁵, Arg^{25,26}, Lys³⁴(N^{ε}-(2-(4-tetradecyl-1-piperazine)-acetyl)))hGLP-1(7-36)NH₂; (SEQ ID NO.702)

(Ser⁸, Aib³⁵, Arg^{25,26},Lys³⁴(N^{ε}-(2-(4-hexadecyl-1-piperazine)-acetyl)))hGLP-1(7-36)NH₂; (SEQ ID NO.703)

(Ser⁸, Aib³⁵, Arg^{25,26,34}, Lys³⁶(N^{ε}-(2-(4-decyl-1-piperazine)-acetyl)))hGLP-1(7-36)NH₂; (SEQ ID NO.704)

(Ser⁸, Aib³⁵, Arg^{25,26,34}, Lys³⁶(N^{ε}-(2-(4-dodecyl-1-piperazine)-acetyl)))hGLP-1(7-36)NH₂; (SEQ ID NO.705)

(Ser⁸, Aib³⁵, Arg^{25,26,34}, Lys³⁶(N^{ε}-(2-(4-tetradecyl-1-piperazine)-acetyl)))hGLP-1(7-36)NH₂; (SEQ ID NO.706)

(Ser⁸, Aib³⁵, Arg^{25,26,34}, Lys³⁶(N^{ε}-(2-(4-hexadecyl-1-piperazine)- (SEQ ID NO.707) acetyl)))hGLP-1(7-36)NH₂;

(Ser⁸, Aib³⁵, Arg^{25,26,34}, Lys³⁸(N^{ε}(2-(4-decyl-1-piperazine)-acetyl)))hGLP-1(7-38)NH₂; (SEQ ID NO.708)

(Ser⁸, Aib³⁵, Arg^{25,26,34}, Lys³⁸(N^{ε}(2-(4-dodecyl-1-piperazine)- (SEQ ID NO.709) acetyl)))hGLP-1(7-38)NH₂;

(Ser⁸, Aib³⁵, Arg^{25,26,34}, Lys³⁸(N^{ε}(2-(4-tetradecyl-1-piperazine)- (SEQ ID NO.710) acetyl)))hGLP-1(7-38)NH₂;

(Ser⁸, Aib³⁵, Arg^{25,26,34}, Lys³⁸(N^{ε}(2-(4-hexadecyl-piperazine)- (SEQ ID NO.711) acetyl)))hGLP-1(7-38)NH₂;

(Ser⁸, Aib^{35,37}, Arg^{25,26,34}, Lys³⁸(N^{ε}-(2-(4-decyl-1-piperazine)-acetyl)))hGLP-1(7-38)NH₂; (SEQ ID NO.712)

(Ser⁸, Aib^{35,37}, Arg^{25,26,34}, Lys³⁸(N^{ε}(2-(4-dodecyl-1-piperazine)- (SEQ ID NO.713) acetyl)))hGLP-1(7-38)NH₂;

(Ser⁸, Aib^{35,37}, Arg^{25,26,34}, Lys³⁸(N^{ε}-(2-(4-tetradecyl-1-piperazine)-acetyl)))hGLP-1(7-38)NH₂; (SEQ ID NO.714)

(Ser⁸, Aib^{35,37}, Arg^{25,26,34}, Lys³⁸(N^{ε}-(2-(4-hexadecyl-1-piperazine)-acetyl)))hGLP-1(7-38)NH₂; (SEQ ID NO.715)

(Ser⁸, Aib³⁵, Arg^{26,34}, Lys³⁶(N^{ε}-decanoy))hGLP-1(7-36)OH; (SEQ ID NO.716)

(Ser⁸, Aib³⁵, Lys²⁶, Arg^{26,34}, Lys³⁶(N^{ε}-decanoyl))hGLP-1(7-36)OH; (SEQ ID NO.717)

(Ser⁸, Aib³⁵,Arg^{26,34}, Ava³⁷, Ado³⁸)hGLP-1(7-38)NN₂; (SEQ ID NO.718)

(Ser⁸, Aib³⁵,Arg^{26,34}, Asp³⁷, Ava³⁸, Ado³⁹)hGLP-1(7-39)NH₂; (SEQ ID NO.719)

(Ser⁸, Aib³⁵,Arg^{26,34}, Aun³⁷)hGLP-1(7-37)NH₂; (SEQ ID NO.720)

(Ser⁸, Aib^{17,35},)hGLP-1(7-36)NH₂; (SEQ ID NO.721)

(Ser⁸ ,Arg^{26,34}, β-Ala³⁵, D-Asp³⁷, Ava³⁸, Aun³⁹)hGLP-1(7-39)NH₂; (SEQ ID NO.722)

(Ser⁸, GlU^{22,23}, β-Ala³⁵)hGLP-1(7-36)NH₂; (SEQ ID NO.723)

(Ser⁸, Lys¹⁸, β-Ala³⁵)hGLP-1(7-36)NH₂; (SEQ ID NO.724)

(Ser⁸, Leu²⁷, β-Ala³⁵)hGLP-1(7-36)NH₂; (SEQ ID NO.725)

(Ser⁸, Lys³³, β-Ala³⁵)hGLP-1(7-36)NH₂; (SEQ ID NO.726)

(Ser⁸, Lys¹⁸, Leu²⁷, β-Ala³⁵)hGLP-1(7-36)NH₂; (SEQ ID NO.727)

(Ser⁸, D-Arg³⁶)hGLP-1(7-36)NH₂; (SEQ ID NO.728)

(Ser⁸, β-Ala³⁵, D-Arg³⁷)hGLP-1(7-37)NH₂; (SEQ ID NO.729)

(Ser⁸, Aib²⁷, β-Ala³⁵)hGLP-1(7-36)NH₂; (SEQ ID NO.730)

(Ser⁸, Aib²⁷, β-Ala^{35,37}, Arg³⁸)hGLP-1(7-38)NH₂; (SEQ ID NO.731)

(Ser⁸, Aib²⁷, β-Ala^{35,37}, Arg^{38,39})hGLP-1(7-39)NH₂; (SEQ ID NO.732)

(Ser⁸, Lys^{18,27}, β-Ala³⁵)hGLP-1(7-36)NH₂; (SEQ ID NO.733)

(Ser⁸, Lys²⁷, β-Ala³⁵)hGLP-1(7-36)NH₂; (SEQ ID NO.734)

(Ser⁸, β-Ala³⁵, Arg³⁸)hGLP-1(7-38)NH₂; (SEQ ID NO.735)

(Ser⁸, Arg^{26,34}, β-Ala³⁵,)hGLP-1(7-36)NH₂; (SEQ ID NO.736)

(Ser⁸, D-Arg³⁵)hGLP-1(7-36)NH₂; (SEQ ID NO.737)

(Ser⁸, β-Ala³⁵, Arg³⁷)hGLP-1(7-37)NH₂; (SEQ ID NO.738)

(Ser⁸, Phe³¹, β-Ala³⁵)hGLP-1(7-36)NH₂; (SEQ ID NO.739)

(Ser⁸, Aib³⁵, Phe³¹)hGLP-1(7-36)NH₂; (SEQ ID NO.740)

(Ser⁸, Aib³⁵, 2-Nal³¹)hGLP-1(7-36)NH₂; (SEQ ID NO.741)

(Ser⁸, Aib³⁵, 2-Nal^{28,31})hGLP-1(7-36)NH₂; (SEQ ID NO.742)

(Ser⁸, Aib³⁵, Arg^{26,34}, 2-Nal³¹)hGLP-1(7-36)NH₂; (SEQ ID NO.743)

(Ser⁸, Aib³⁵, Arg^{26,34}, Phe³¹)hGLP-1(7-36)NH₂; (SEQ ID NO.744)

(Ser⁸, Aib³⁵, 2-Nal^{19,31})hGLP-1(7-36)NH₂; (SEQ ID NO.745)

(Ser⁸, Aib³⁵, 2-Nal^{12,31})hGLP-1(7-36)NH₂; (SEQ ID NO.746)

(Ser⁸, Aib³⁵, Lys³⁶(N^{ε}-decanoyl))hGLP-1(7-36)NH₂; (SEQ ID NO.747)

(Ser⁸, Aib³⁵, Arg³⁴, Lys²⁶(N^{ε}-decanoyl))hGLP-1(7-36)NH₂; (SEQ ID NO.748)

(Ser⁸, Aib³⁵, Arg^{26,34}, Lys³⁶(N^{ε}-dodecanoyl))hGLP-1(7-36)NH₂; (SEQ ID NO.749)

(Ser⁸, β-Ala³⁵,Ser³⁷(O-decanoyl))hGLP1(7-37)-NH₂; (SEQ ID NO.750)

(Ser⁸, Aib²⁷, β-Ala^{35,37}, Arg³⁸, Lys³⁹(N^{ε}-octanoyl))hGLP-1(7-39)NH₂; (SEQ ID NO.751)

(Ser⁸, Arg^{26,34}, β-Ala³⁵, Lys³⁷(N^{ε}-octanoyl))hGLP-1(7-37)NH₂; (SEQ ID NO.752)

(Ser⁸, Arg^{26,34}, β-Ala³⁵, Lys³⁷(N^{ε}-decanoyl))hGLP-1(7-37)NH₂; (SEQ ID NO.753)

(Ser⁸, Arg^{26,34}, β-Ala³⁵, Lys³⁷(N^{ε}-tetradecanoyl))hGLP-1(7-37)NH₂; (SEQ ID NO.754)

(Ser⁸, Arg-^{26,34}, β-Ala³⁵, Lys³⁷(N^{ε}-dodecanoyl))hGLP-1(7-37)NH₂; (SEQ ID NO.755)

(Ser⁸, Arg^{26,34}, β-Ala³⁵, Lys³⁷(N^{ε}-dodecanoyl))hGLP-1(8-37)NH₂; (SEQ ID NO.756)

(Ser⁸, Arg^{26,34}, β-Ala³⁵, Lys³⁶(N^{ε}-Aec-decanoyl))hGLP-1(7-36)NH₂; (SEQ ID NO.757)

(Ser⁸, Arg²⁶, Aib³⁵)hGLP-1(7-36)NH₂; (SEQ ID NO.758)

(Ser⁸, Arg²⁶, Phe³¹, Aib³⁵)hGLP-1(7-36)NH₂; (SEQ ID NO.759)

(Ser⁸, Arg²⁶, Phe³¹, β-Ala³⁵)hGLP-1(7-36)NH₂; (SEQ ID NO.760)

(Ser⁸, Arg²⁶, β-Ala³⁵)hGLP-1(7-36)NH₂; (SEQ ID NO.761)

(Ser⁸, Arg^{26,34}, Phe³¹, β-Ala³⁵)hGLP-1(7-36)NH₂; (SEQ ID NO.762)

(Ser⁸, Arg³⁴, Aib³⁵)hGLP-1(7-36)NH₂; (SEQ ID NO.763)

(Ser⁸, Arg³⁴, β-Ala³⁵)hGLP-1(7-36)NH₂; (SEQ ID NO.764)

(Ser⁸, Phe³¹, Arg³⁴, Aib³⁵)hGLP-1(7-36)NH₂; (SEQ ID NO.765)

or (Ser⁸, Phe³¹, Arg³⁴, β-Ala³⁵)hGLP-1(7-36)NH₂; (SEQ ID NO.766)

or a pharmaceutically acceptable salt thereof.

A preferred compound of the group of compounds described in paragraphs [068] through [0453] is a compound according to the formula:

(Gly⁸, Aib³⁵, A6c³²)hGLP-1(7-36)NH₂; (SEQ ID NO.8)

(Gly⁸, Aib³⁵, Glu²³)hGLP-1(7-36)NH₂; (SEQ ID NO.10)

(Gly⁸, Aib^{24,35})hGLP-1(7-36)NH₂; (SEQ ID NO.11)

(Gly⁸, Aib³⁵, Glu²³, A6c³²)hGLP-1(7-36)NH₂; (SEQ ID NO.28)

(Gly⁸, Glu²³, β-Ala³⁵)hGLP-1(7-36)NH₂; (SEQ ID NO.34)

(Gly⁸, Aib³⁵, Arg^{26,34})hGLP-1(7-36)NH₂; (SEQ ID NO.59)

(Gly⁸, Aib³⁵, Arg^{26,34}, Lys³⁶(N^{ε}-octanoyl))hGLP-1(7-36)NH₂; (SEQ ID NO.117)

(Gly⁸, Aib³⁵, Arg^{26,34}, Lys³⁶(N^{ε}-decanoyl))hGLP-1(7-36)OH; (SEQ ID NO.332)

(Gly⁸, Aib³⁵, Lys²⁵, Arg^{26,34}, Lys³⁶(N^{ε}-decanoyl))hGLP-1(7-36)OH; (SEQ ID NO.333)

(Gly⁸, Arg^{26,34}, β-Ala³⁵, Lys³⁶(N^{ε}-Aec-decanoyl))hGLP-1(7-36)NH₂; (SEQ ID NO.383)

(Gly⁸, Aib³⁵,Arg^{26,34}, Ava³⁷, Ado³⁸)hGLP-1(7-38)NH₂; (SEQ ID NO.334)

(Gly⁸, Aib³⁵,Arg^{26,34}, Asp³⁷, Ava³⁸, Ado³⁹)hGLP-1(7-39)NH₂; (SEQ ID NO.335)

(Gly⁸, Aib³⁵,Arg^{26,34}, Aun³⁷)hGLP-1(7-37)NH₂; (SEQ ID NO.336)

(Gly⁸, Aib^{17,35})hGLP-1(7-36)NH₂; (SEQ ID NO.337)

(Gly⁸ ,Arg^{26,34}, β-Ala³⁵, O-Asp³⁷, Ava³⁸, Aun³⁹)hGLP-1(7-39)NH₂; (SEQ ID NO.338)

(Gly⁸, Glu^{22,23}, β-Ala³⁵)hGLP-1(7-36)NH₂; (SEQ ID NO.339)

(Gly⁸, Lye¹⁸, β-Ala³⁵)hGLP-1(7-36)NH₂; (SEQ ID NO.340)

(Gly⁸, Leu²⁷, β-Ala³⁵)hGLP-1(7-36)NH₂; (SEQ ID NO.341)

(Gly⁸, Lys³³, β-Ala³⁵)hGLP-1(7-36)NH₂; (SEQ ID NO.342)

(Gly⁸, Lys¹⁸, Leu²⁷, β-Ala³⁵)hGLP-1(7-36)NH₂; (SEQ ID NO.343)

(Gly⁸, D-Arg³⁶)hGLP-1(7-36)NH₂; (SEQ ID NO.344)

(Gly⁸, β-Ala³⁵, D-Arg³⁷)hGLP-1(7-37)NH₂; (SEQ ID NO.345)

(Gly⁸, Aib²⁷, β-Ala³⁵)hGLP-1(7-36)NH₂; (SEQ ID NO.346)

(Gly⁸, Aib²⁷, β-Ala^{35,37}, Arg³⁸)hGLP-1(7-38)NH₂; (SEQ ID NO.347)

(Gly⁸, Aib²⁷, β-Ala^{35,37}, Arg^{38,39})hGLP-1(7-39)NH₂; (SEQ ID NO.348)

(Gly⁸, Lys^{18,27}, β-Ala³⁵)hGLP-1(7-36)NH₂; (SEQ ID NO.349)

(Gly⁸, Lys²⁷, β-Ala³⁵)hGLP-1(7-36)NH₂; (SEQ ID NO.350)

(Gly⁸, β-Ala³⁵, Arg³⁸)hGLP-1(7-38)NH₂; (SEQ ID NO.351)

(Gly⁸, Arg^{26,34}, β-Ala³⁵,)hGLP-1(7-36)NH₂; (SEQ ID NO.352)

(Gly⁸, D-Arg³⁵)hGLP-1(7-36)NH₂; (SEQ ID NO.353)

(Gly⁸, β-Ala³⁵, Arg³⁷)hGLP-1(7-37)NH₂; (SEQ ID NO.354)

(Gly⁸, Phe³¹, β-Ala³⁵)hGLP-1(7-36)NH₂; (SEQ ID NO.355)

(Gly⁸, Aib³⁵, Phe³¹)hGLP-1(7-36)NH₂; (SEQ ID NO.356)

(Gly⁸, Aib³⁵, 2-Nal³¹)hGLP-1(7-36)NH₂; (SEQ ID NO.357)

(Gly⁸, Aib³⁵, 2-Nal^{28,31})hGLP-1(7-36)NH₂; (SEQ ID NO.358)

(Gly⁸, Aib³⁵, Arg^{26,34}, 2-Na³¹)hGLP-1(7-36)NH₂; (SEQ ID NO.359)

(Gly⁸, Aib³⁵, Arg^{26,34}, Phe³¹)hGLP-1(7-36)NH₂; (SEQ ID NO.360)

(Gly⁸, Aib³⁵, 2-Nal^{19,31})hGLP-1(7-36)NH₂; (SEQ ID NO.361)

(Gly⁸, Aib³⁵, 2-Nal^{12,31})hGLP-1(7-36)NH₂; (SEQ ID NO.362)

(Gly⁸, Aib³⁵, Lys³⁶(N^{ε}-decanoyl)hGLP-1(7-36)NH₂; (SEQ ID NO.99)

(Gly⁸, Aib³⁵, Arg³⁴, Lys²⁶(N^{ε}-decanoyl))hGLP-1(7-36)NH₂; (SEQ ID NO.364)

(Gly⁸, Aib³⁵, Arg^{26,34}, Lys³⁶(N^{ε}-dodecanoyl))hGLP-1(7-36)NH₂; (SEQ ID NO.365)

(Gly⁸, β-Ala³⁵, Ser³⁷(O-decanoyl))hGLP1(7-37)-NH₂; (SEQ ID NO.384)

(Gly⁸, Aib²⁷, β-Ala^{35,37}, Arg³⁸, Lys³⁹(N^{ε}-octanoyl))hGLP-1(7-39)NH₂; (SEQ ID NO.367)

(Gly⁸, Arg^{26,34}, β-Ala³⁵; Lys³⁷(N^{ε}-octanoyl))hGLP-1(7-37)NH₂; (SEQ ID NO.368)

(Gly⁸, Arg^{26,34}, β-Ala³⁵, Lys³⁷(N^{ε}-decanoyl))hGLP-1(7-37)NH₂; (SEQ ID NO.369)

or (Gly⁸, Arg^{26,34}, β-Ala³⁵, Lys³⁷(N^{ε}-tetrsdecanoyl))hGLP-1(7-37)NH₂; (SEQ ID NO.370)

or a pharmaceutically acceptable salt thereof.

A preferred compound of the group of compounds described in paragraphs [0455] through [0837] is a compound according to the formula:

(Ser⁸, Aib³⁵, A6c³²)hGLP-1(7-36)NH₂; (SEQ ID NO.392)

(Ser⁸, Aib³⁵, Glu²³)hGLP-1(7-36)NH₂; (SEQ ID NO.394)

(Ser⁸, Aib^{24,35})hGLP-1(7-36)NH₂; (SEQ ID NO.395)

(Ser⁸, Aib³⁵, Glu²³, A6c³²)hGLP-1(7-36)NH₂; (SEQ ID NO.412)

(Ser⁸, Glu²³, β-Ala³⁵)hGLP-1(7-36)NH₂; (SEQ ID NO.418)

(Ser⁸, Aib³⁵, Arg^{26,34})hGLP-1(7-36)NH₂; (SEQ ID NO.443)

(Ser⁸, Aib³⁵, Arg^{26,34}, Lys³⁶(N^{ε}-octanoyl))hGLP-1(7-36)NH₂; (SEQ ID NO.501)

(Ser⁸, Aib³⁵, Arg^{26,34}, Lys³⁶(N^{ε}-decanoyl))hGLP-1(7-36)OH; (SEQ ID NO.716)

(Ser⁸, Aib³⁵, Lys²⁵, Arg^{26,34} Lys³⁵(N^{ε}-decanoyl)hGLP-1(7-36)OH; (SEQ ID NO.717)

(Ser⁸, Arg^{26,34}, β-Ala³⁵, Lys³⁶(N^{ε}-Aec-decanoyl))hGLP-1(7-36)NH₂; (SEQ ID NO.757)

(Ser⁸, Aib³⁵,Arg^{26,34}, Ava³⁷, Ado³⁸)hGLP-1(7-38)NH₂; (SEQ ID NO.718)

(Ser⁸, Aib³⁵,Arg^{26,34}, Asp³⁷, Ava³⁸, Ado³⁹)hGLP-1(7-39)NH₂; (SEQ ID NO.719)

(Ser⁸, Aib³⁵,Arg^{26,34}, Aun³⁷)hGLP-1(7-37)NH₂; (SEQ ID NO.720)

(Ser⁸, Aib^{17,35})hGLP-1(7-36)NH₂; (SEQ ID NO.389)

(Ser⁸ ,Arg^{26,34}, β-Ala³⁵, D-Asp³⁷, Ava³⁸, Aun³⁹)hGLP-1(7-39)NH₂; (SEQ ID NO.722)

(Ser⁸, Glu^{22,23}, β-Ala³⁵)hGLP-1(7-36)NH₂; (SEQ ID NO.723)

(Ser⁸, Lys¹⁸, β-Ala³⁵)hGLP-1(7-36)NH₂; (SEQ ID NO.724)

(Ser⁸, Leu²⁷, β-Ala³⁵)hGLP-1(7-36)NH₂; (SEQ ID NO.725)

(Ser⁸, Lys³³, β-Ala³⁵)hGLP-1(7-36)NH₂; (SEQ ID NO.726)

(Ser⁸, Lys¹⁸, Leu²⁷, β-Ala³⁵)hGLP-1(7-36)NH₂; (SEQ ID NO.727)

(Ser⁸, D-Arg³⁶)hGLP-1(7-36)NH₂; (SEQ ID NO.728)

(Ser⁸, β-Ala³⁵, D-Arg³⁷)hGLP-1(7-37)NH₂; (SEQ ID NO.729)

(Ser⁸, Aib²⁷, β-Ala³⁵)hGLP-1(7-36)NH₂; (SEQ ID NO.730)

(Ser⁸, Aib²⁷, β-Ala^{35,37}, Arg³⁸)hGLP-1(7-38)NH₂; (SEQ ID NO.731)

(Ser⁸, Aib²⁷, β-Ala^{35,37}, Arg^{38,39})hGLP-1(7-39)NH₂; (SEQ ID NO.732)

(Ser⁸, Lys^{18,27}, β-Ala³⁵)hGLP-1(7-36)NH₂; (SEQ ID NO.733)

(Ser⁸, Lys²⁷, β-Ala³⁵)hGLP-1(7-36)NH₂; (SEQ ID NO.734)

(Ser⁸, β-Ala³⁵, Arg³⁸)hGLP-1(7-38)NH₂; (SEQ ID NO.735)

(Ser⁸, Arg^{26,34} β-Ala³⁵)hGLP-1(7-36)NH₂; (SEQ ID NO.736)

(Ser⁸, D-Arg³⁵)hGLP-1(7-36)NH₂; (SEQ ID NO.737)

(Ser⁸, β-Ala³⁵, Arg³⁷)hGLP-1(7-37)NH₂; (SEQ ID NO.738)

(Ser⁸, Phe³¹, β-Ala³⁵)hGLP-1(7-36)NH₂; (SEQ ID NO.739)

(Ser⁸, Aib35, Phe³¹)hGLP-1(7-36)NH₂; (SEQ ID NO.740)

(Ser⁸, Aib³⁵, 2-Nal³¹)hGLP-1(7-36)NH₂; (SEQ ID NO.741)

(Ser⁸, Aib³⁵, 2-Nal^{28,31})hGLP-1(7-36)NH₂; (SEQ ID NO.742)

(Ser⁸, Aib³⁵, Arg^{26,34}, 2-Nal³¹)hGLP-1(7-36)NH₂; (SEQ ID NO.743)

(Ser⁸, Aib³⁵, Arg^{26,34} Phe³¹)hGLP-1(7-36)NH₂; (SEQ ID NO.744)

(Ser⁸, Aib³⁵, 2-Nal^{19,31})hGLP-1(7-36)NH₂; (SEQ ID NO.745)

(Ser⁸, Aib³⁵, 2-Nal^{12,31})hGLP-1(7-36)NH₂; (SEQ ID NO.746)

(Ser⁸, Aib³⁵, Lys³⁶(N^{ε}-decanoyl))hGLP-1(7-36)NH₂; (SEQ ID NO.483)

(Ser⁸, Aib³⁵, Arg³⁴, Lys²⁶(N^{ε}-decanoyl))hGLP-1(7-36)NH₂; (SEQ ID NO.748)

(Ser⁸, Aib³⁵, Arg^{26,34}, Lys³⁶(N^{ε}-dodecanoyl))hGLP-1(7-36)NH₂; (SEQ ID NO.749)

(Ser⁸, β-Ala³⁵, Ser³⁷(O-decanoyl))hGLP1(7-37)-NH₂; (SEQ ID NO.539)

(Ser⁸, Aib²⁷, β-Ala^{35,37}, Arg³⁸, Lys³⁹(N^{ε}-octanoyl))hGLP-1(7-39)NH₂; (SEQ ID NO.751)

(Ser⁸, Arg^{26,34}, β-Ala³⁵, Lys³⁷(N^{ε}-octanoyl))hGLP-1(7-37)NH₂; (SEQ ID NO.752)

(Ser⁸, Arg^{26,34}, β-Ala³⁵, Lys³⁷(N^{ε}-decanoyl))hGLP-1(7-37)NH₂; (SEQ ID NO.753)

or (Ser⁸, Arg^{26,34}, β-Ala³⁵, Lys³⁷(N^{ε}-tetradecanoyl)) hGLP-1(7-37)NH₂; (SEQ ID NO.754)

or a pharmaceutically acceptable salt thereof.

In a more preferred embodiment the invention features a compound according to formula (I), wherein said compound is:

(Ser⁸, Aib³⁵)hGLP-1(7-36)NH₂; (SEQ ID NO.767)

(Abu⁸, β-Ala³⁵)hGLP-1(7-36)NH₂; (SEQ ID NO.768)

(Val⁸, β-Ala³⁵)hGLP-1(7-36)NH₂; (SEQ ID NO.769)

(β-Ala^{8,35})hGLP-1(7-36)NH₂; (SEQ ID NO.770)

(Abu⁸, Aib³⁵)hGLP-1(7-36)NH₂; (SEQ ID NO.771)

(Val⁸, Aib³⁵)hGLP-1(7-36)NH₂; (SEQ ID NO.772)

or (β-Ala⁸, Aib³⁵)hGLP-1(7-36)NH₂; (SEQ ID NO.773)

or a pharmaceutically acceptable salt thereof.

In a still more preferred embodiment the invention features a compound according to formula (I), wherein said compound is:

(Ser⁸, Aib³⁵)hGLP-1(7-36)NH₂; (SEQ ID NO.767)

(Abu⁸, β-Ala³⁵)hGLP-1(7-36)NH₂; (SEQ ID NO.768)

or (Abu⁸, Aib³⁵)hGLP-1(7-36)NH₂; (SEQ ID NO.771)

or a pharmaceutically acceptable salt thereof.

In yet a still more preferred embodiment the invention features a compound according to formula (I), wherein said compound is:

(Ser⁸, Aib³⁵)hGLP-1(7-36)NH₂; (SEQ ID NO.767)

or a pharmaceutically acceptable salt thereof.

In another aspect of the invention is featured a pharmaceutical composition comprising an effective amount of a compound according to formula (I), or a pharmaceutically acceptable salt thereof, and a pharmaceutically acceptable carrier or diluent.

In another aspect of the invention is featured a method of eliciting an agonist effect from a GLP-1 receptor in a subject in need thereof, said method comprising administering to said subject an effective amount of a compound according to formula (I) or a pharmaceutically acceptable salt thereof.

In another aspect of the invention is featured a method of treating a disease selected from the group consisting of Type I diabetes, Type II diabetes, obesity, glucagonomas, secretory disorders of the airway, metabolic disorder, arthritis, osteoporosis, central nervous system disease, restenosis and neurodegenerative disease, in a subject in need thereof, said method comprising administering to said subject an effective amount of a compound according to formula (I) or a pharmaceutically acceptable salt thereof. Preferably, said disease is Type I diabetes or Type II diabetes.

In a preferred embodiment of each of the aspects detailed in paragraphs [0953], [0955], and [0956] said compound according to formula (I) is a compound described in paragraph [060], [061], [062], [063], [063], [065], [066], [067], or [068], or a pharmaceutically acceptable salt thereof. More preferably said compound according to formula (I) is selected from among the compounds of Formula (I) that are specifically disclosed herein, or a pharmaceutically acceptable salt thereof. More preferably said compound according to formula (I) is selected from among the compounds disclosed in paragraphs [069] through [0452] or in paragraphs [0455] through [0836], or a pharmaceutically acceptable salt thereof. More preferably said compound according to formula (I) is selected from among the compounds disclosed in paragraphs [0839] through [0885] or in paragraphs [0888] through [0934], or a pharmaceutically acceptable salt thereof. Still more preferably said compound according to formula (I) is selected from among the compounds disclosed in paragraphs [0937] through [0943], or a pharmaceutically acceptable salt thereof. Still more preferably said compound according to formula (I) is selected from among the compounds disclosed in paragraphs [0946] through [0948], or a pharmaceutically acceptable salt thereof. In a most preferred embodiment said compound according to formula (I) is (Ser⁸, Aib³⁵)hGLP-1(7-36)NH₂ (SEQ ID NO. 767), or a pharmaceutically acceptable salt thereof.

With the exception of the N-terminal amino acid, all abbreviations (e.g., Ala) of amino acids in this disclosure stand for the structure of -NH-CH(R)-CO-, wherein R is the side chain of an amino acid (e.g., CH₃ for Ala). For the N-terminal amino acid, the abbreviation stands for the structure of (R²R³)-N-CH(R)-CO-, wherein R is a side chain of an amino acid and R² and R³ are as defined above, except when A⁷ is Ura, Paa or Pta, in which case R² and R³ are not present since Ura, Paa and Pta are considered here as des-amino amino acids. Certain other abbreviations for amino acids are employed herein with the following meanings:
- Abu: α-aminobutyric acid;
- Ado: 12-aminododecanoic acid;
- Aec: 4-(2-aminoethyl)-1-carboxymethyl-piperazine, represented by the structure: 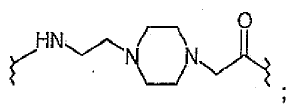
- Aib: α-aminoisobutyric acid;
- Aic: 2-aminoindane-2-carboxylic acid;
- β-Ala: β-alanine;
- Amp: 4-amino-phenylalanine;
- Aun: 11-aminoundecanoic acid;
- Ava: 5-aminovalerinc acid;
- Cha: cyclohexylalanine;
- Gaba: γ-aminobutyric acid;
- hArg: homoarginine;
- HEP: 4-(2-hydroxyethyl)-1-piperazimyl acetic acid;
- HEPES: 4-(2-hydroxyethyl)-1-piperazine-ethane sulfonic acid;
- N-Me-Ala: N-methyl-alanine;
- N-Me-Asp: N-methyl-aspartic acid;
- N-Me-Glu: N-methyl-glutamic acid;
- N-Me-Gly: N-methyl-glycine;
- N^{α}-Me-His: N^{α}-methyl-histidine;
- 1-Nal: β-(1-naphthyl)alanine;
- 2-Nal: β-(2-naphthyl)alanine;
- Nle: norleucine;
- Orn: ornithine;
- Paa: *trans*-3-(3-pyridyl) acrylic acid;
- 3-Pal: β-(3-pyridinyl)alanine;
- 4-Pal: β-(4-pyridinyl)alanine;
- Pta: (4-Pyridylthio) acetic acid;
- Tie: *tert*-butylglycine;
- Tma-His: N,N-tetramethylamidino-histidine;
- Tba: *tert*-butylalanine; and
- Ura: urocanic acid.

What is meant by Acc is an amino acid selected from the group of 1-amino-1-cyclopropanecarboxylic acid (A3c); 1-amino-1-cyclobutanecarboxylic acid (A4c); 1-amino-1-cyclopentanecarboxylic acid (A5c); 1-amino-1-cyclohexanecarboxylic acid (A6c); 1-amino-1-cycloheptanecarboxylic acid (A7c); 1-amino-1-cyclooctanecarboxylic acid (A8c); and 1-amino-1-cyclononanecarboxylic acid (A9c).

The terms hydroxyalkyl, hydroxyphenylalkyl, and hydroxynaphthylalkyl each denotes a moiety containing 1, 2, 3, or 4 hydroxy substituents.

The term COX⁵ stands for -C=O-X⁵. Examples of -C=O·X⁵ include, but are not limited to, acetyl and phenylpropionyl.

What is meant by Lys(N^{ε}-alkanoyl) is represented by the following structure:

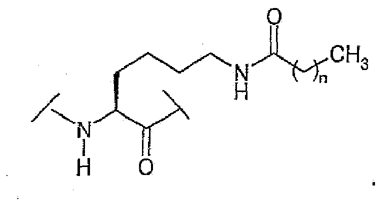

What is meant by Lys(N^{ε}-alkylsulfonyl) is represented by the following structure:

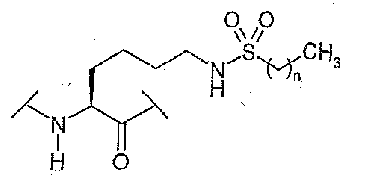

What is meant by Lys(N^{ε}-(2-(4-alkyl-1-piperazine)-acetyl)) is represented by the following structure:

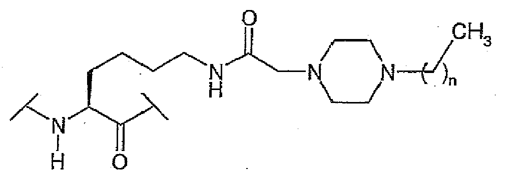

What is meant by Asp(1-(4-alkyl-piperazine)) is represented by the following structure:

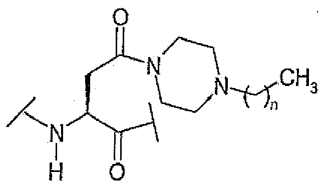

What is meant by Asp(1-alkylamino) is represented by the following structure:

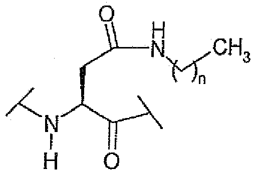

What is meant by Lys(N^{ε}-Aec-alkanoyl) is represented by the structure:

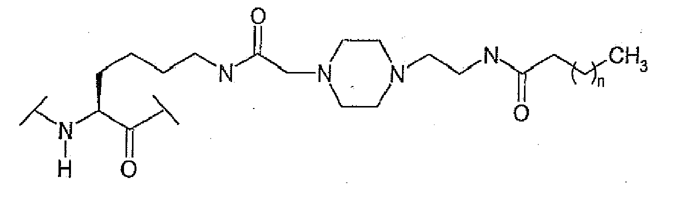

What is meant by Lys (N^{ε}-ace-alkanoyl) is represented by the structure:

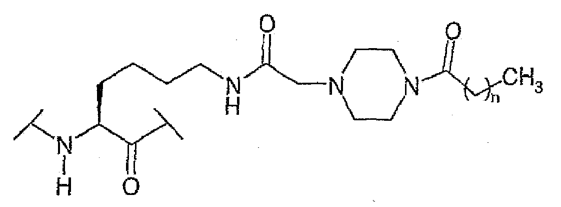

What is meant by Ser(O-alkanoyl) is represented by the structure:

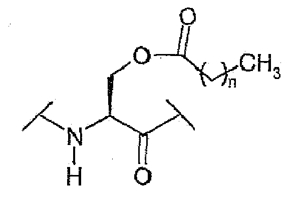

The variable n in the structure depicted for Lys(N^{ε}-alkanoyl), Lys(N^{ε}-alkylsulfonyl), Lys(N^{ε}-(2-(4-alkyl-1-piperazine)-acetyl)), Asp(1-(4-alkyl-piperazine)), Asp(1-alkylamino), Lys(N^{ε}-Aec-alkanoyl), (N^{ε}-ace-alkanoyl) and Ser(O-alkanoyl) is an integer from 1 through 30, inclusive.

The full names for other abbreviations used herein are as follows:
- Boc: t-butyloxycarbonyl;
- 2BrZ: 2-bromobenzyloxycarbonyl;
- Bzl: benzyl;
- 2CIZ: 2-chlorobenzyloxycarbonyl;
- DCM: dichloromethane;
- DIEA: diisopropylethylamine;
- DMF: dimethylformamide;
- DNP: 2,4-dinitrophenyl;
- Fm: formyl;
- Fmoc: 9-fluorenylmethoxycarbonyl;
- HBTU: 2-(1H-Benzotriazol-1-yl)-1,1,3,3-tetramethyl uronium hexafluorophosphate;
- HF: hydrogen fluoride;
- HOAc: acetic acid;
- HOBt: N-hydroxybenzotriazole;
- OcHex: O-cyclohexyl;
- PAM resin: 4-hydroxymethylphenylacetamidomethyl resin;
- TFA: trifluoroacetic acid;
- Tos: tosyl; and
- Xan: xanthyl.

The term "(C₁-C₃₀)hydrocarbon moiety" encompasses alkyl, alkenyl and alkynyl, and in the case of alkenyl and alkynyl there are C₂-C₃₀.

The term "halo" encompasses fluoro, chloro, bromo and iodo.

A peptide of this invention is also denoted herein by another format, e.g., (A5c⁸)hGLP-1(7-36)NH₂ (SEQ ID NO.774), with the substituted amino acids from the natural sequence placed between the first set of parentheses (e.g., A5c⁸ for Ala⁸ in hGLP-1). The abbreviation GLP-1 means glucagon-like peptide-1; hGLP-1 means human glucagon-like peptide-1. The numbers between the parentheses following "hGLP-1" refer to the number of amino acids present in the peptide. For example, hGLP-1(7-36) (SEQ ID NO.775) denotes amino acids 7 through 36 of the peptide sequence for human GLP-1. The sequence for hGLP-1(7-37) (SEQ ID NO.776) is listed in Mojsov, S., Int. J. Peptide Protein Res,. 40, 1992, pp. 333-342. The designation "NH₂" in hGLP-1(7-36)NH₂ indicates that the C-terminus of the peptide is amidated. hGLP-1(7-36) (SEQ ID NO.775) means that the C-terminus is the free acid. In hGLP-1(7-38) (SEQ ID NO.777), residues in positions 37 and 38 are Gly and Arg, respectively.

### Detailed Description

The peptides of this invention can be prepared by standard solid phase peptide synthesis. See, e.g., Stewart, J.M., et al., Solid Phase Synthesis (Pierce Chemical Co., 2d ed. 1984). The substituents R² and R³ of the above generic formula may be attached to the free amine of the N-terminal amino acid by standard methods known in the art. For example, alkyl groups, e.g., (C₁-C₃₀)alkyl, may be attached using reductive alkylation. Hydroxyalkyl groups, e.g., (C₁-C₃₀)hydroxyalkyl, may also be attached using reductive alkylation wherein the free hydroxy group is protected with a t-butyl ester. Acyl groups, e.g., COE¹, may be attached by coupling the free acid, e.g., E¹COOH, to the free amine of the N-terminal amino acid by mixing the completed resin with 3 molar equivalents of both the free acid and diisopropylcarbodiimide in methylene chloride for one hour. If the free acid contains a free hydroxy group, e.g., p-hydroxyphenylpropionic acid, then the coupling should be performed with an additional 3 molar equivalents of HOBT.

When R¹ is NH-X²-CH₂-CONH₂, (i.e., Z⁰=CONH₂), the synthesis of the peptide starts with BocHN-X²-CH₂-COOH which is coupled to the MBHA resin. If R¹ is NH-X²-CH₂-COOH, (i.e., Z⁰=COOH) the synthesis of the peptide starts with Boc-HN-X²-CH₂-COOH which is coupled to PAM resin. For this particular step, 4 molar equivalents of Boc-HN-X²-COOH, HBTU and HOBt and 10 molar equivalents of DIEA are used. The coupling time is about 8 hours.

The protected amino acid 1-(N-tert-butoxycarbonyl-amino)-1-cyclohexanecarboxylic acid (Boc-A6c-OH) was synthesized as follows. 19.9 g (0.133 mol) of 1-amino-1-cyclohexanecarboxylic acid (Acros Organics, Fisher Scientific, Pittsburgh, PA) was dissolved in 200 ml of dioxane and 100 ml of water. To this was added 67 ml of 2N NaOH and the solution was cooled in an ice-water bath. 32.0 g (0.147 mol) of di-tert-butyl-dicarbonate was added to the solution and the reaction mixture was stirred overnight at room temperature. Dioxane was then removed under reduced pressure and 200 ml of ethyl acetate was added to the remaining aqueous solution. The mixture was cooled in an ice-water bath and the pH of the aqueous layer was adjusted to about 3 by adding 4N HCl. The organic layer was separated and the aqueous layer was extracted with ethyl acetate (1 x 100 ml). The two organic layers were combined, washed with water (2 x 150 ml), dried over anhydrous MgSO₄, filtered, and concentrated to dryness under reduced pressure. The residue was recrystallized in ethyl acetate/hexanes to produce 9.2 g (0.0386 mol) of the purified product; i.e., a 29% yield from the starting material.

Boc-A5c-OH was synthesized in an analogous manner to that of Boc-A6c-OH. Other protected Acc amino acids can be prepared in an analogous manner by a person of ordinary skill in the art as enabled by the teachings herein.

In the synthesis of a GLP-1 analogue of this invention containing A5c, A6c, Aib, Boc-Lys(2CIZ)-OH and/or Boc-His(DNP)-OH, the coupling time is 2 hrs, for these residues and the residue immediately following them. Otherwise the coupling time is approximately 5 minutes. For the synthesis of (Tma-His⁷)hGLP-1(7-36)NH₂ (SEQ ID NO.778), HBTU (2 mmol) and DIEA (1.0 ml) in 4 ml DMF are used to react with the N-terminal free amine of the peptide-resin in the last coupling reaction; the coupling time is about 2 hours.

The substituents R² and R³ of the above generic formula can be attached to the free amine of the N-terminal amino acid by standard methods known in the art. For example, alkyl groups, e.g., (C₁-C₃₀)alkyl, can be attached using reductive alkylation. Hydroxyalkyl groups, e.g., (C₁-C₃₀)hydroxyalkyl, can also be attached using reductive alkylation wherein the free hydroxy group is protected with a t-butyl ester. Acyl groups, e.g., COX¹, can be attached by coupling the free acid, e.g., X¹COOH, to the free amine of the N-terminal amino acid by mixing the completed resin with 3 molar equivalents of both the free acid and diisopropylcarbodiimide in methylene chloride for about one hour. If the free acid contains a free hydroxy group, e.g., p-hydroxyphenylpropionic acid, then the coupling should be performed with an additional 3 molar equivalents of HOBT.

A compound of the present invention can be tested for activity as a GLP-1 binding compound according to the following procedure.

### Cell Culture:

RIN 5F rat insulinoma cells (ATCC-# CRL-2058, American Type Culture Collection, Manassas, VA), expressing the GLP-1 receptor, were cultured in Dulbecco's modified Eagle's medium (DMEM) containing 10% fetal calf serum, and maintained at about 37 °C in a humidifed atmosphere of 5% CO₂/95% air.

### Radioligand Binding:

Membranes were prepared for radioligand binding studies by homogenization of the RIN cells in 20 ml of ice-cold 50 mM Tris-HCl with a Brinkman Polytron (Westbury, NY) (setting 6, 15 sec). The homogenates were washed twice by centrifugation (39,000 g / 10 min), and the final pellets were resuspended in 50 mM Tris-HCl, containing 2.5 mM MgCl₂, 0.1 mg/ml bacitracin (Sigma Chemical, St. Louis, MO), and 0.1% BSA. For assay, aliquots (0.4 ml) were incubated with 0.05 nM (¹²⁵I)GLP-1(7-36) (-2200 Ci/mmol, New England Nuclear, Boston, MA), with and without 0.05 ml of unlabeled competing test peptides. After a 100 min incubation (25 °C), the bound (¹²⁵I)GLP-1(7-36) (SEQ ID NO: 775) was separated from the free by rapid filtration through GF/C filters (Brandel, Gaithersburg, MD), which had been previously soaked in 0.5% polyethyleneimine. The filters were then washed three times with 5 ml aliquots of ice-cold 50 mM Tris-HCl, and the bound radioactivity trapped on the filters was counted by gamma spectrometry (Wallac LKB, Gaithersburg, MD). Specific binding was defined as the total (¹²⁵I)GLP-1(7-36) (SEQ ID NO: 775) bound minus that bound in the presence of 1000 nM GLP1(7-36) (Bachem, Torrence, CA).

The peptides of this invention can be provided in the form of pharmaceutically acceptable salts. Examples of such salts include, but are not limited to, those formed with organic acids (e.g., acetic, lactic, maleic, citric, malic, ascorbic, succinic, benzoic, methanesulfonic, toluenesulfonic, or pamoic acid), inorganic acids (e.g., hydrochloric acid, sulfuric acid, or phosphoric acid), and polymeric acids (e.g., tannic acid, carboxymethyl cellulose, polylactic, polyglycolic, or copolymers of polylactic-glycolic acids). A typical method of making a salt of a peptide of the present invention is well known in the art and can be accomplished by standard methods of salt exchange. Accordingly, the TFA salt of a peptide of the present invention (the TFA salt results from the purification of the peptide by using preparative HPLC, eluting with TFA containing buffer solutions) can be converted into another salt, such as an acetate salt by dissolving the peptide in a small amount of 0.25 N acetic acid aqueous solution. The resulting solution is applied to a semi-prep HPLC column (Zorbax, 300 SB, C-8). The column is eluted with (1) 0.1N ammonium acetate aqueous solution for 0.5 hrs., (2) 0.25N acetic acid aqueous solution for 0.5 hrs. and (3) a linear gradient (20% to 100% of solution B over 30 min.) at a flow rate of 4 ml/min (solution A is 0.25N acetic acid aqueous solution; solution B is 0.25N acetic acid in acetonitrile/water, 80:20). The fractions containing the peptide are collected and lyophilized to dryness.

As is well known to those skilled in the art, the known and potential uses of GLP-1 is varied and multitudinous (See, Todd, J.F., et al., Clinical Science, 1998, 95, pp. 325-329; and Todd, J.F. et al., European Journal of Clinical Investigation, 1997, 27, pp.533-536). Thus, the administration of the compounds of this invention for purposes of eliciting an agonist effect can have the same effects and uses as GLP-1 itself. These varied uses of GLP-1 may be summarized as follows, treatment of: Type I diabetes, Type II diabetes, obesity, glucagonomas, secretory disorders of the airway, metabolic disorder, arthritis, osteoporosis, central nervous system diseases, restenosis, neurodegenerative diseases, renal failure, congestive heart failure, nephrotic syndrome, cirrhosis, pulmonary edema, hypertension, and disorders wherein the reduction of food intake is desired. GLP-1 analogues of the present invention that elicit an antagonist effect from a subject can be used for treating the following: hypoglycemia and malabsorption syndrome associated with gastroectomy or small bowel resection.

Accordingly, the present invention includes within its scope pharmaceutical compositions comprising, as an active ingredient, at least one of the compounds of formula (I) in association with a pharmaceutically acceptable carrier.

The dosage of active ingredient in the compositions of this invention may be varied; however, it is necessary that the amount of the active ingredient be such that a suitable dosage form is obtained. The selected dosage depends upon the desired therapeutic effect, on the route of administration, and on the duration of the treatment. In general, an effective dosage for the activities of this invention is in the range of 1x10⁻⁷ to 200 mg/kg/day, preferably 1x10⁻⁴ to 100 mg/kg/day, which can be administered as a single dose or divided into multiple doses.

The compounds of this invention can be administered by oral, parenteral (e.g., intramuscular, intraperitoneal, intravenous or subcutaneous injection, or implant), nasal, vaginal, rectal, sublingual or topical routes of administration and can be formulated with pharmaceutically acceptable carriers to provide dosage forms appropriate for each route of administration.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups, the elixirs containing inert diluents commonly used in the art, such as water. Besides such inert diluents, compositions can also include adjuvants, such as wetting agents, emulsifying and suspending agents, and sweetening, flavoring and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, excipients such as coca butter or a suppository wax.

Compositions for nasal or sublingual administration are also prepared with standard excipients well known in the art.

Further, a compound of this invention can be administered in a sustained release composition such as those described in the following patents and patent applications. U.S. Patent No. 5,672,659 teaches sustained release compositions comprising a bioactive agent and a polyester. U.S. Patent No. 5,595,760 teaches sustained release compositions comprising a bioactive agent in a gelable form. U.S. Application No. 08/929,363 filed September 9, 1997, teaches polymeric sustained release compositions comprising a bioactive agent and chitosan. U.S. Application No. 08/740,778 filed November 1, 1996, teaches sustained release compositions comprising a bioactive agent and cyclodextrin. U.S. Application No. 09/015,394 filed January 29, 1998, teaches absorbable sustained release compositions of a bioactive agent. U.S. Application No. 09/121,653 filed July 23, 1998, teaches a process for making microparticles comprising a therapeutic agent such as a peptide in an oil-in-water process. U.S. Application No. 09/131,472 filed August 10, 1998, teaches complexes comprising a therapeutic agent such as a peptide and a phosphorylated polymer. U.S. Application No. 09/184,413 filed November 2, 1998, teaches complexes comprising a therapeutic agent such as a peptide and a polymer bearing a non-polymerizable lactone. The teachings of the foregoing patents and applications are incorporated herein by reference.

Unless defined otherwise, all technical and scientific terms used herein have the same meaning as commonly understood by one of ordinary skill in the art to which this invention belongs. Also, all publications, patent applications, patents and other references mentioned herein are incorporated by reference.

### Synthesis:

The following example describes a synthetic method for making a peptide of the invention, which method is well-known to those skilled in the art of peptide synthesis. Where provided, the amounts of reagents are approximately those employed to carry out the synthesis on a 0.20 mmol scale. Other methods are also well known to those skilled in the art, thus the example is provided for the purpose of illustration only and is not meant to limit the scope of the present invention in any manner.

A great variety of amino acids, with and without side-chain protection, are available from a number of commercial sources. For example, the following amino acids, wherein parentheses designate the side-chain protecting group, if present, are variously available from Bachem, Torrance, CA; Nova Biochem., LaJolla, CA; Chem-Impex International; Wood Dale, IL; and Synthetech, Inc. Albany, OR: Boc-Ado-OH, Boc-Aib-OH, Boc-Ala-OH, Boc-βAla-OH, Boc-Arg(Tos)-OH, Boc-D-Arg(Tos)-OH, Boc-Asp(OcHex)-OH, Boc-D-Asp(OcHex)-OH, Boc-Aun-OH, Boc-Ava-OH, Boc-Gln-OH, Boc-Glu(OcHex)-OH, Boc-Gly-OH, Boc-His(DNP)-OH, Boc-Ile-OH, Boc-Leu-OH, Boc-Lys(2CIZ)-OH, Boc-Nal-OH, Boc-Phe-OH, Boc-Ser(Bzl)-OH, Boc-Thr(Bzl)-OH, Boc-Trp(Fm)-OH, Boc-Tyr(2BrZ)-OH, and Boc-Val-OH.

### Example 1: Synthesis of (Ser⁸, Aib³⁵)hGLP-1(7-36)NH₂

The title peptide may be synthesized on an Applied Biosystems (Foster City, CA) model 430A peptide synthesizer modified to do accelerated Boc-chemistry solid phase peptide synthesis. (See Schnolzer, et al., Int. J. Peptide Protein Res., 90:180 (1992).) 4-Methylbenzhydrylamine (MBHA) resin (Peninsula Laboratories, Belmont, CA) with the substitution of 0.91 mmol/g may be used. Boc amino acids (Bachem, CA, Torrance, CA; Nova Biochem., LaJolla, CA) may be used, e.g., with the following side chain protection: Boc-Ala-OH, Boc-Arg(Tos)-OH, Boc-Asp(OcHex)-OH, Boc-Tyr(2BrZ)-OH, Boc-His(DNP)-OH, Boc-Val-OH, Boc-Leu-OH, Boc-Gly-OH, Boc-Gln-OH, Boc-Ile-OH, Boc-Lys(2CIZ)-OH, Boc-Thr(Bzl)-OH, Boc-Ser(Bzl)-OH, Boc-Phe-OH, Boc-Glu(OcHex)-OH and Boc-Trp(Fm)-OH. The Boc groups are removed by treatment with 100% TFA for 2 x 1 min. Boc amino acids (2.5 mmol) are pre-activated with HBTU (2.0 mmol) and DIEA (1.0 mL) in 4 mL of DMF and are coupled without prior neutralization of the peptide-resin TFA salt.

At the end of the assembly of the peptide chain, the resin is treated with a solution of 20% mercaptoethanol/10% DIEA in DMF for 2 x 30 min. to remove the DNP group on the His side chain. The N-terminal Boc group is then removed by treatment with 100% TFA for 2 x 2 min. After neutralization of the peptide-resin with 10% DIEA in DMF (1 x 1 min), the formyl group on the side chain of Trp is removed by treatment with a solution of 15% ethanolamine/ 15% water/ 70% DMF for 2 x 30 min. The peptide-resin is then washed with DMF and DCM and dried under reduced pressure. The final cleavage is performed by stirring the peptide-resin in 10 mL of HF containing 1 mL of anisole and dithiothreitol (24 mg) at 0°C for approximately 75 min. HF is removed by a flow of nitrogen and the residue is then washed with ether (6 x 10 mL) and extracted with 4N HOAc (6 x 10 mL).

The peptide mixture in the aqueous extract is purified on reverse-phase preparative high pressure liquid chromatography (HPLC) using a reverse phase VYDAC® C₁₈ column (Nest Group, Southborough, MA). The column is eluted with a linear gradient (20% to 50% of solution B over 105 min.) at a flow rate of 10 mL/min (Solution A = water containing 0.1% TFA; Solution B = acetonitrile containing 0.1% of TFA). Fractions are collected and checked on analytical HPLC. Those fractions containing purified product are then combined and lyophilized to dryness. Electro-spray mass spectrometer (MS(ES)) analysis may be used to check the molecular weight of the final product.

### Examples 2 - 7

The following peptides may also be synthesized according to the procedure detailed in Example 1, substituting appropriate amino acids during peptide assembly.
Example 2: (Abu⁸, β-Ala³⁵)hGLP-1(7-36)NH₂; (SEQ ID NO.768)
Example 3: (Val⁸, β-Ala³⁵)hGLP-1(7-36)NH₂; (SEQ ID NO.769)
Example 4: (β-Ala^{8,35})hGLP-1(7-36)NH₂; (SEQ ID NO.770)
Example 5: (Abu⁵, Aib³⁵)hGLP-1(7-36)NH₂; (SEQ ID NO.771)
Example 6: (Val⁸, Aib³⁵)hGLP-1(7-36)NH₂, and (SEQ ID NO.772)
Example 7: (βAla⁸,Aib³⁵)hGLP-1(7-36)NH₂; (SEQ ID NO.773)

Each of the foregoing examples can be made according to the procedures described hereinabove, with appropriate substitutions in starting materials during peptide assembly.

### OTHER EMBODIMENTS

From the above description, one skilled in the art can easily ascertain the essential characteristics of the present invention, and without departing from the spirit and scope thereof, can make various changes and modifications of the invention to adapt it to various uses and conditions, Thus, other embodiments are also within the claims.
The invention further includes the subject matter of the claims of WO2004/074315 from which this application is derived, the content of which is reproduced below as numbered paragraphs.
Paragraph 1. A compound of formula (I),

   (R²R³)-A⁷⁻-⁸-A⁹-A¹⁰-A¹¹-A¹²-A¹³-A¹⁴-A¹⁵-A¹⁶-A¹⁷-A¹⁸-A¹⁹-A²⁰-A²¹-A²²-A²³-A²⁴-A²⁵-A²⁶-A²⁷-A²⁸-A²⁹-A³⁰-A³¹-A³²-A³³-A³⁴-A³⁵-A³⁶-A³⁷-A³⁸-A³⁹-R¹, (I)

   wherein:
   A⁷ is L-His, Ura, Paa, Pta, Amp, Tma-His, des-amino-His, or deleted;
   A⁸ is Abu, β-Ala, Ser, Gly or Val;
   A⁹ is Glu, N-Me-Glu, N-Me-Asp or Asp;
   A¹⁰ is Gly, Acc, β-Ala or Aib;
   A¹¹ is Thr or Ser;
   A¹² is Phe, Acc, Aic, Aib, 3-Pal, 4-Pal, 1-Nal, 2-Nal, Cha, Trp or X¹-Phe;
   A¹³ is Thr or Ser;
   A¹⁴ is Ser or Aib;
   A¹⁵ is Asp or Glu;
   A¹⁶ is Val, Acc, Aib, Leu, Ile, Tle, Nle, Abu, Ala or Cha;
   A¹⁷ is Ser or Thr;
   A¹⁸ is Ser or Thr;
   A¹⁹ is Tyr, Cha, Phe, 3-Pal, 4-Pal, Acc, 1-Nal, 2-Nal, or X¹-Phe;
   A²⁰ is Leu, Acc, Aib, Nle, Ile, Cha, Tle, Val, Phe or X¹-Phe;
   A²¹ is Glu or Asp;
   A²² is Gly, Ace, β-Ala, Glu or Aib;
   A²³ is Gln, Asp, Asn or Glu;
   A²⁴ is Ala, Aib, Val, Abu, Tle or Acc;
   A²⁵ is Ala, Aib, Val, Abu, Tle, Acc, Lys, Arg, hArg, Orn, HN-CH((CH₂)ₙ-N(R¹⁰R¹¹))-C(O) or HN-CH((CH₂),-X³)-C(O);
   A²⁶ is Lys, Arg, hArg, On, HN-CH((CH₂)ₙ-N(R¹⁰R¹¹))-C(O) or HN-CH((CH₂)ₑ-X³)-C(O);
   A²⁷ is Glu, Asp, Leu, Aib or Lys;
   A²⁸ is Phe, 3-Pal, 4-Pal, 1-Nal, 2-Nal, X¹-Phe, Aic, Acc, Aib, Cha or Trp;
   A²⁹ is Ile, Acc, Aib, Leu, Nle, Cha, Tle, Val, Abu, Ala or Phe;
   A³⁰ is Ala, Aib or Acc;
   A³¹ is Trp, 1-Nal, 2-Nal,3-Pal, 4-Pal, Phe, Acc, Aib or Cha;
   A³² is Leu, Acc, Aib, Nle, Ile, Cha, Tle, Phe, X¹-Phe or Ala;
   A³³ is Val, Acc, Aib, Leu, Ile, Tie, Nle, Cha, Ala, Phe, Abu, Lys or X¹-Phe;
   A³⁴ is Lys, Arg, hArg, Orn, HN-CH((CH₂)ₙ-N(R¹⁰R¹¹))-C(O) or HN-CH((CH₂)ₑ-X³)-C(O);
   A³⁵ is Gly, β-Ala, D-Ala, Gaba, Ava, HN-(CH₂)ₘ-C(O), Aib, Acc or a D-amino acid;
   A³⁶ is L- or D-Arg, D- or L-Lys, D- or L-hArg, D- or L-Orn, HN-CH((CH₂)ₙ-N(R¹⁰R¹¹))-C(O), HN-CH((CH₂)ₑ-X³)-C(O) or deleted;
   A³⁷ is Gly, β-Ala, Gaba, Ava, Aib, Acc, Ado, Arg, Asp, Aun, Aec, HN-(CH₂)ₘ-C(O), HN-CH((CH₂)ₙ-N(R¹⁰R¹¹))-C(O), HN-CH((CH₂)ₙ-O(R¹⁰))-C(O),a D-amino acid, or deleted;
   A³⁸ is D- or L-Lys, D- or L-Arg, D- or L-hArg, D- or L-Orn, HN-CH((CH₂)ₙ-N(R¹⁰R¹¹))-C(O), HN-CH((CH₂)ₑ-X³)-C(O) Ava, Ado, Aec or deleted;
   A³⁹ is D- or L-Lys, D- or L-Arg, HN-CH((CH₂)ₙN(R¹⁰R¹¹))-C(O), Ava, Ado, Aec, or Aun, or is deleted;
   X¹ is, independently for each occurrence, selected from the group consisting of (C₁-C₆)alkyl, OH and halo;
   R¹ is OH, NH₂, (C₁-C₃₀)alkoxy, or NH-X²-CH₂-Z⁰, wherein X² is a (C₁-C₁₂)hydrocarbon moiety, and Z⁰ is H, OH, CO₂H or CONH₂; 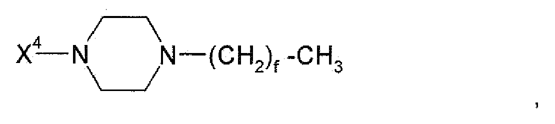
   X³ is 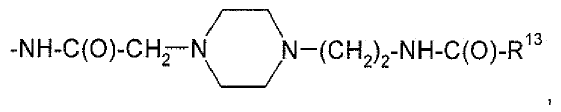 or -C(O)-NHR¹², wherein X⁴ is, independently for each occurrence, -C(O)-, -NH-C(O)- or -CH₂-, and wherein f is, independently for each occurrence, an integer from 1 to 29 inclusive;
each of R² and R³ is independently selected from the group consisting of H, (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, and hydroxynaphthyl(C₁-C₃₀)alkyl; or one of R² and R³ is 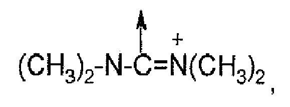 (C₁-C₃₀)acyl, (C₁-C₃₀)alkylsulfonyl, C(O)X⁵, 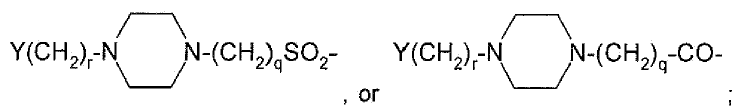 wherein Y is H, OH or NH₂; r is 0 to 4; q is 0 to 4; and X⁵ is (C₁-C₃₀)alkyl, (C₂-C₃₀)alkenyl, phenyl(C₁-C₃₀)alkyl, naphthyl(C₁C₃₀)alkyl, hydroxy(C₁-C₃₀)alkyl, hydroxy(C₂-C₃₀)alkenyl, hydroxyphenyl(C₁-C₃₀)alkyl or hydroxynaphthyl(C₁-C₃₀)alkyl;
e is, independently for each occurrence, an integer from 1 to 4 inclusive;
m is, independently for each occurrence, an integer from 5 to 24 inclusive;
n is, independently for each occurrence, an integer from 1 to 5, inclusive;
each of R¹⁰ and R¹¹ is, independently for each occurrence, H, (C₁-C₃₀)alkyl, (C₁-C₃₀)acyl, (C₁-C₃₀)alkylsulfonyl, -C((NH)(NH₂)) or 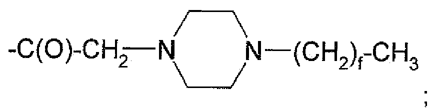 and
R¹² and R¹³ each is, independently for each occurrence, (C₁-C₃₀)alkyl;
provided that:
(i) said compound is not:
(Ser⁸, β-Ala³⁵)hGLP-1(7-36)NH₂(SEQ ID NO: 779);
(Gly⁸, Aib³⁵)hGLP-1(7-36)NH₂ (SEQ ID NO: 780); or
(Gly⁸, β-Ala³⁵)hGLP-1(7-36)NH₂ (SEQ ID NO: 781);
(ii) when A⁷ is Ura, Paa or Pta, then R² and R³ are deleted; and
(iii) when R¹⁰ is (C₁-C₃₀)acyl, (C₁-C₃₀)alkylsulfonyl, -C((NH)(NH₂)) or 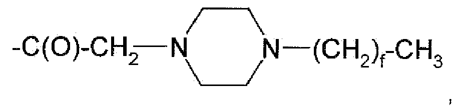 then R¹¹ is H or (C₁-C₃₀)alkyl;
or a pharmaceutically acceptable salt thereof.
Paragraph 2. A compound according to Paragraph 1, wherein A¹¹ is Thr; A¹³ is Thr; A¹⁵ is Asp; A¹⁷ is Ser; A¹⁸ is Ser; A²¹ is Glu; A²³ is Gln or Glu; A²⁷ is Glu; and A³¹ is Trp; or a pharmaceutically acceptable salt thereof.
Paragraph 3. A compound according to Paragraph 2, wherein A⁹ is Glu, N-Me-Glu or N-Me-Asp; A¹² is Phe, Acc or Aic; A¹⁶ is Val, Acc or Aib; A¹⁹ is Tyr; A²⁰ is Leu, Acc or Cha; A²⁴ is Ala, Aib or Acc; A²⁵ is Ala, Aib, Acc, Lys, Arg, hArg, Orn, HN-CH((CH₂)ₙN(R¹⁰R¹¹))-C(O) or HN-CH((CH₂)ₑ-X³)-C(O); A²⁸ is Phe; A²⁹ is Ile or Acc; A³⁰ is Ala or Aib; A³² is Leu, Acc or Cha; and A³³ is Val or Acc; or a pharmaceutically acceptable salt thereof.
Paragraph 4. A compound according to Paragraph 3, wherein A¹⁰ is Gly; A¹² is Phe, A6c or A5c; A¹⁶ is Val, A6c or A5c; A²⁰ is Leu, A6c, A5c or Cha; A²² is Gly, β-Ala or Aib; A²⁴ is Ala or Aib; A²⁹ is Ile, A6c or A5c; A³² is Leu, A6c, A5c or Cha; A³³ is Val, A6c or A5c; A³⁵ is Aib, β-Ala, Ado, A6c, A5c or Gly; and A³⁷ is Gly, Aib, β-Ala, Ado, D-Ala or deleted; or a pharmaceutically acceptable salt thereof.
Paragraph 5. A compound according to Paragraph 4, wherein X⁴ for each occurrence is -C(O)-; e for each occurrence is independently 1 or 2; and R¹ is OH or NH₂; or a pharmaceutically acceptable salt thereof.
Paragraph 6. A compound according to Paragraph 5, wherein R² is H and R³ is (C₁-C₃₀)alkyl, (C₂-C₃₀)alkenyl, (C₁-C₃₀)acyl, (C₁-C₃₀)alkylsuffonyl, 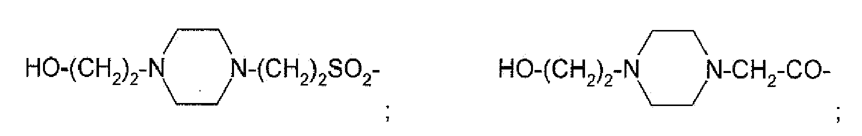 or 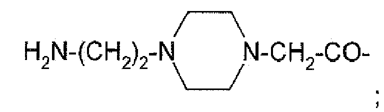 or a pharmaceutically acceptable salt thereof.
Paragraph 7. A compound according to Paragraph 5, wherein R¹⁰ is (C₁-C₃₀)acyl, (C₁-C₃₀))aikylsulfonyl or 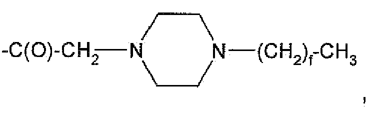 and R¹¹ is H; or a pharmaceutically acceptable salt thereof
Paragraph 8. A compound according to Paragraph 7, wherein R¹⁰ is (C₄-C₂₀)acyl, (C₄-C₂₀)alkylsulfonyl or 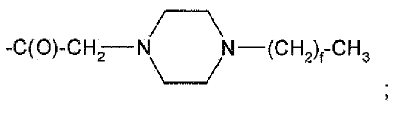 or a pharmaceutically acceptable salt thereof.
Paragraph 9. A compound according to Paragraph 5, wherein A⁸ is Gly, Ser, or Val; or a pharmaceutically acceptable salt thereof.
Paragraph 10. A compound according to Paragraph 9 wherein A⁸ is Gly or Ser; or a pharmaceutically acceptable salt thereof.
Paragraph 11. A compound according to Paragraph 10, wherein said compound is according to the formula:

| | |
|---|---|
| ((N^{α}-Me-His)⁷, Gly⁸, Aib³⁵)hGLP-1(7-36)NH₂; | (SEQ ID NO.1) |
| ((N^{α}-Me-His)⁷ Gly⁸, β-Ala³⁵)hGLP-1(7-36)NH₂; | (SEQ ID NO.2) |
| ((N^{α}-Me-His)⁷, Gly⁸, Aib³⁵, Arg^{26,34})hGLP-1(7-36)NH₂; | (SEQ ID NO.3) |
| ((N^{α}-Me-His)⁷, Gly⁸, Arg^{26,34}, β-Ala³⁵)hGLP-1(7-36)NH₂; | (SEQ ID NO.4) |
| (Gly⁸, A6c³⁵)hGLP-1(7-36)NH₂; | (SEQ ID NO.5) |
| (Gly⁸, A5c³⁵)hGLP-1(7-36)NH₂; | (SEQ ID NO.6) |
| (Gly⁸, D-Ala³⁵)hGLP-1(7-36)NH₂; | (SEQ ID NO.7) |
| (Gly⁸, Aib³⁵, A6c³²)hGLP-1(7-36)NH₂; | (SEQ ID NO.8) |
| (Gly⁸, Aib³⁵, A5c³²)hGLP-1(7-36)NH₂; | (SEQ ID NO.9) |
| (Gly⁸, Aib³⁵, Glu²³)hGLP-1(7-36)NH₂; | (SEQ ID NO.10) |
| (Gly⁸, Aib^{24,35})hGLP-1(7-36)NH₂; | (SEQ ID NO.11) |
| (Gly⁸, Aib^{30,35})hGLP-1(7-36)NH₂; | (SEQ ID NO.12) |
| (Gly⁸, Aib^{25,35})hGlP-1(7-36)NH₂; | (SEQ ID NO.13) |
| (Gly⁸, Aib³⁵, A6c^{16,20})hGLP-1(7-36)NH₂; | (SEQ ID NO.14) |
| (Gly⁸, Aib³⁵, A6c^{16,29,32})hGLP-1(7-36)NH₂; | (SEQ ID NO.15) |
| (Gly⁸, Aib³⁵, A6c^{20,32})hGLP-1(7-36)NH₂; | (SEQ ID NO.16) |
| (Gly⁸, Aib³⁵, A6c²⁰)hGLP-1(7-36)NH₂; | (SEQ ID NO.17) |
| (Gly⁸, Aib³⁵, Lys²⁵)hGLP-1(7-36)NH₂; | (SEQ ID NO.18) |
| (Gly⁸, Aib^{24,35}, A6c²⁰)hGLP-1(7-36)NH₂; | (SEQ ID NO.19) |
| (Gly⁸, Aib³⁵, A6c^{29,32})hGLP-1(7-36)NH₂; | (SEQ ID NO.20) |
| (Gly⁸, Aib^{24,35}, A6c^{29,32})hGLP-1(7-36)NH₂; | (SEQ ID NO.21) |
| (Giy⁸, Aib³⁵, A6c¹²)hGLP-1(7-36)NH₂; | (SEQ ID NO.22) |
| (Gly⁸, Aib³⁵, Cha²⁰)hGLP-1(7-36)NH₂; | (SEQ ID NO.23) |
| (Gly⁸, Aib³⁵, A6c³³)hGLP-1(7-36)NH₂; | (SEQ ID NO.24) |
| (Gly⁸, A6c^{16,20}, β-Ala³⁵)hGLP-1(7-36)NH₂; | (SEQ ID NO.25) |
| (Gly⁸, Aib³⁵, β-Ala²²)hGLP-1(7-36)NH₂; | (SEQ ID NO.26) |
| (Gly⁸, Aib^{22,35})hGLP-1(7-36)NH₂; | (SEQ ID NO.27) |
| (G!y⁸, Aib³⁵, Glu²³, A6c³²)hGLP-1(7-36)NH₂; | (SEQ ID NO.28) |
| (Gly⁸, Aib^{24,35}, Glu²³, A6c³²)hGLP-1(7-36)NH₂; | (SEQ ID NO.29) |
| (Gly⁸, Aib^{24,25,35}, Glu²³, A6c³²)hGLP-1(7-36)NH₂; | (SEQ ID NO.30) |
| (Gly⁸, Aib^{24,25,35}, A6c^{16,20,32}, Glu²³)hGLP-1(7-36)NH₂; | (SEQ ID NO.31) |
| (Gly⁸, A6c³², β-Ala³⁵)hGLP-1(7-36)NH₂; | (SEQ ID NO.32) |
| (Gly⁸, A5c³², β-Ala³⁵)hGLP-1(7-36)NH₂; | (SEQ ID NO.33) |
| (Gly⁸, Glu²³, β-Ala³⁵)hGLP-1(7-36)NH₂; | (SEQ ID NO.34) |
| (Gly⁸, Aib²⁴, β-Ala³⁵)hGLP-1(7-36)NH₂; | (SEQ ID NO.35) |
| (Gly⁸, Aib³⁰, β-Ala³⁵)hGLP-1(7-36)NH₂; | (SEQ ID NO.36) |
| (Gly⁸, Aib²⁵, β-Ala³⁵)hGLP-1(7-36)NH₂; | (SEQ ID NO.37) |
| (Gly⁸, A6c^{16,29,32}, β-Ala³⁵)hGLP-1(7-36)NH₂; | (SEQ ID NO.38) |
| (Gly⁸, A6c^{20,32}, β-Ala³⁵)hGLP-1(7-36)NH₂; | (SEQ ID NO.39) |
| (Gly⁸, A6c²⁰, β-Ala³⁵)hGLP-1(7-36)NH₂; | (SEQ ID NO.40) |
| (Gly⁸, Lys²⁵, β-Ala³⁵)hGLP-1(7-36)NH₂; | (SEQ ID NO.41 ) |
| (Gly⁸, Aib²⁴, A6c²⁰, β-Ala³⁵)hGLP-1(7-36)NH₂; | (SEQ ID NO.42) |
| (Gly⁸, A6c^{29,32}, β-Ala³⁵)hGLP-1(7-36)NH₂; | (SEQ ID NO.43) |
| (Gly⁸, Aib²⁴, A6c^{29,32}, β-Ala³⁵)hGLP-1(7-36)NH₂; | (SEQ ID NO.44) |
| (Gly⁸, A6c¹², β-Ala³⁵)hGLP-1(7-36)NH₂; | (SEQ ID NO.45) |
| (Gly⁸, Cha²⁰, β-Ala³⁵)hGLP-1(7-36)NH₂; | (SEQ ID NO.46) |
| (Gly⁸, A6c³³, β-Ala³⁵)hGLP-1(7-36)NH₂; | (SEQ ID NO.47) |
| (Gly⁸, β-Ala^{22,35})hGLP-1(7-36)NH₂; | (SEQ ID NO.48) |
| (Gly⁸, Aib²², β-Ala³⁵)hGLP-1(7-36)NH₂; | (SEQ ID NO.49) |
| (Gly⁸, Glu²³, A6c³², β-Ala³⁵)hGLP -1(7-36)NH₂; | (SEQ ID NO.50) |
| (Gly⁸, Aib²⁴, Glu²³, A6c³², β-Ala³⁵)hGLP-1(7-36)NH₂; | (SEQ ID NO.51) |
| (Gly⁸, Aib²⁴, Glu²³, A6c³², Lys³⁴(N^{e}-octanoyl), β-Ala³⁵) | |
| hGLP-1(7-36)NH₂; | (SEQ ID NO.52) |
| (Gly⁸, Aib^{24,25}, Glu²³, A6c³², β-Ala³⁵)hGLP-1(7-36)NH₂; | (SEQ ID NO.53) |
| (Gly⁸, Aib^{24,25}, A6c^{16,20,32}, Glu²³, β-Ala³⁵)hGLP-1(7-36)NH₂; | (SEQ ID NO.54) |
| (Gly⁸, Aib³⁵, D-Arg³⁶)hGLP-1(7-36)NH₂; | (SEQ ID NO.55) |
| (Gly⁸, Aib³⁵, D-Lys³⁶)hGLP-1(7-36)NH₂; | (SEQ ID NO.56) |
| (Gly⁸, β-Ala³⁵, D-Arg³⁶)hGLP-1(7-36)NH₂; | (SEQ ID NO.57) |
| (Gly⁸, β-Ala³⁵, D-Lys³⁶)hGLP-1(7-36)NH₂; | (SEQ ID NO.58) |
| (Gly⁸, Aib³⁵, Arg^{26,34})hGLP-1(7-36)NH₂; | (SEQ ID NO.59) |
| (Gly⁸, Arg^{26,34}, β-Ala³⁵)hGLP-1(7-36)NH₂; | (SEQ ID NO.60) |
| (Gly⁸, Aib³⁵, Arg^{25,26,34})hGLP-1(7-36)NH₂; | (SEQ ID NO.61) |
| (Gly⁸, Arg^{25,26,34}, β-Ala³⁵)hGLP-1(7-35)NH₂; | (SEQ ID NO.62) |
| (Gly⁸, Arg^{26,34}, β-Ala³⁵, Lys³⁶(N^{ε}-tetradecanoyl))hGLP-1(7-36)OH; | (SEQ ID NO.63) |
| (Gly⁸, Aib³⁵, Arg^{26,34},Lys³⁶(N^{ε}-tetradecanoyl))hGLP-1(7-37)OH; | (SEQ ID NO.64) |
| (Gly⁸, Aib^{35,37}, Arg^{26,34}, Lys³⁶(N^{ε}-tetradecanoyl))hGLP-1(7-37)OH; | (SEQ ID NO.65) |
| (Gly⁸, Aib³⁵, Arg^{26,34}, Lys³⁶(N^{ε}-tetradecanoyl), D-Ala³⁷) | |
| hGLP-1(7-37)OH; | (SEQ ID NO.66) |
| (Gly⁸, Aib^{35,37}, Arg^{26,34}, Lys³⁸(N^{ε}-tetradecanoyl))hGLP-1(7-38)OH; | (SEQ ID NO.67) |
| (Gly⁸, Aib³⁵, Arg^{26,34}, β-Ala³⁷, Lys³⁸(N^{ε}-tetradecanoyl)) | |
| hGLP-1 (7-38)OH; | (SEQ ID NO.68) |
| (Gly⁸, Aib³⁵, Arg^{26,34}, Lys³⁸(N^{ε}-tetradecanoyl))hGLP-1(7-38)OH; | (SEQ ID NO.69) |
| (Gly⁸, Arg^{26,34}, Lys³⁶(N^{ε}-tetradecanoyl), β-Ala³⁷)hGLP-1(7-37)OH; | (SEQ ID NO.70) |
| (Gly⁸, Aib³⁷, Arg^{26,34}, Lys³⁶(N^{ε}-tetradecanoyl))hGLP-1(7-37)OH; | (SEQ ID NO.71) |
| (Gly⁸, Aib³⁵, Arg^{26,34}, Ado³⁷)hGLP-1(7-37)OH; | (SEQ ID NO.72) |
| (Gly⁸, Aib³⁵, Arg^{26,34}, Ado³⁷)hGLP-1(7-37)NH₂; | (SEQ ID NO,73) |
| (Gly⁸, Arg^{26,34}, Lys³⁶(N^{ε}-tetradecanoyl), D-Ala³⁷)hGLP-1(7-37)OH; | (SEQ ID NO.74) |
| (Gly⁸, Aib³⁷, Arg^{26,34}, Lys³⁸(N^{ε}-tetradecanoyl))hGLP-1(7-38)OH; | (SEQ ID NO.75) |
| (Gly⁸, Arg^{26,34}, β-Ala³⁷, Lys³⁸(N^{ε}-tetradecanoyl))hGLP-1(7-38)OH; | (SEQ ID NO.76) |
| (Gly⁸, Aib³⁵, Lys²⁶(N^{ε}-octanoyl))hGLP-1(7-36)NH₂; | (SEQ ID NO.77) |
| (Gly⁸, Aib³⁵, Lys²⁶(N^{ε}-tetradecanoyl))hGLP-1(7-36)NH₂; | (SEQ ID NO.78) |
| (Gly⁸, Aib³⁵, Lys²⁶(N^{ε}-hexadecanoyl))hGLP-1(7-36)NH₂; | (SEQ ID NO.79) |
| (Gly⁸, Lys²⁶(N^{ε}-octanoyl), β-Ala³⁵)hGLP-1(7-36)NH₂; | (SEQ ID NO.80) |
| (Gly⁸, Lys²⁶(N^{ε}-tetradecanoyl), β-Ala³⁵)hGLP-1(7-36)NH₂; | (SEQ ID NO.81) |
| (Gly⁸, Lys²⁶(N^{ε}-hexadecanoyl), β-Ala³⁵)hGLP-1(7-36)NH₂; | (SEQ ID NO.82) |
| (Gly⁸, Aib³⁵, Lys²⁶(N^{ε}-octanoyl), Arg³⁴)hGLP-1(7-36)NH₂; | (SEQ ID NO.83) |
| (Gly⁸, Aib³⁵, Lys²⁶(N^{ε}-tetradecanoyl), Arg³⁴)hGLP-1(7-36)NH₂; | (SEQ ID NO.84) |
| (Gly⁸, Aib³⁵, Lys²⁶(N^{ε}-hexadecanoyl), Arg³⁴)hGLP-1(7-36)NH₂; | (SEQ ID NO.85) |
| (Gly⁸, Aib³⁵, Lys²⁶(N^{ε}-decanoyl), Arg³⁴)hGLP-1(7-36)NH₂; | (SEQ ID NO.86) |
| (Gly⁸, Aib³⁵, Lys²⁵, Lys²⁶(N^{ε}-octanoyl), Arg³⁴)hGLP-1(7-36)NH₂; | (SEQ ID NO.87) |
| (Gly⁸, Aib³⁵, Lys²⁵, Lys²⁶(N^{ε}-tetradecanoyl), Arg³⁴)hGLP-1(7-36)NH₂; | (SEQ ID NO.88) |
| (Gly⁸, Aib³⁵, Lys²⁵, Lys²⁶(N^{ε}-hexadecanoyl), Arg³⁴)hGLP-1(7-36)NH₂: | (SEQ ID NO.89) |
| (Gly⁸, Aib³⁵, Arg^{25,34}, Lys²⁶(N^{ε}-octanoyl))hGLP-1(7-36)NH₂; | (SEQ ID NO.90) |
| (Gly⁸, Aib³⁵, Arg^{25,34}, Lys²⁶(N^{ε}-tetradecanoyl))hGLP-1(7-36)NH₂; | (SEQ ID NO.91) |
| (Gly⁸, Aib³⁵, Arg^{25,34}, Lys²⁶(N^{ε}-hexadecanoyl))hGLP-1(7-36)NH₂; | (SEQ ID NO.92) |
| (Gly⁸, Aib³⁵, Arg^{25,34}, Lys²⁶(N^{ε}-decanoyl))hGLP-1(7-36)NH₂; | (SEQ ID NO.93) |
| (Gly⁸, Lys²⁶(N^{ε}-octanoyl), Arg³⁴, β-Ala³⁵)hGLP-1(7-36)NH₂; | (SEQ ID NO.94) |
| (Gly⁸, Lys²⁶(N^{ε}-tetradecanoyl), Arg³⁴, β-Ala³⁵)hGLP-1(7-36)NH₂; | (SEQ ID NO.95) |
| (Gly⁸, Lys²⁶(N^{ε}-hexadecanoyl), Arg³⁴, β-Ala³⁵)hGLP-1(7-36)NH₂; | (SEQ ID NO.96) |
| (Gly⁸, Lys²⁶(N^{ε}-decanoyl), Arg³⁴, β-Ala³⁵)hGLP-1(7-36)NH₂; | (SEQ ID NO.97) |
| (Gly⁸, Aib³⁵, Lys³⁴(N^{ε}-octanoyl))hGLP-1(7-36)NH₂; | (SEQ ID NO.98) |
| (Gly⁸, Aib³⁵, Lys³⁴(N^{ε}-tetradecanoyl))hGLP-1(7-36)NH₂; | (SEQ ID NO.99) |
| (Gly⁸, Aib³⁵, Lys³⁴(N^{ε}-hexadecanoyl))hGLP-1(7-36)NH₂; | (SEQ ID NO.100) |
| (Gly⁸, Aib³⁵, Arg²⁶, Lys³⁴(N^{ε}-octanoyl))hGLP-1(7-36)NH₂; | (SEQ ID NO.101) |
| (Gly⁸, Aib³⁵, Arg²⁶, Lys³⁴(N^{ε}-hexadecanoyl))hGLP-1(7-36)NH₂; | (SEQ ID NO.102) |
| (Gly⁸, Aib³⁵, Arg²⁶, Lys³⁴(N^{ε}-decanoyl))hGLP-1(7-36)NH₂; | (SEQ ID NO. 103) |
| (Gly⁸, Aib³⁵, Arg^{25,26}, Lys³⁴(N^{ε}-octanoyl))hGLP-1(7-36)NH₂; | (SEQ ID NO.104) |
| (Gly⁸, Aib³⁵, Arg^{25,26}, Lys³⁴(N^{ε}-tetradecanoyl))hGLP-1(7-36)NH₂; | (SEQ ID NO.105) |
| (Gly⁸, Aib³⁵, Arg^{25,26}, Lys³⁴(N^{ε}-hexadecanoyl))hGLP-1(7-36)NH₂; | (SEQ ID NO.106) |
| (Gly⁸, Aib³⁵, Arg^{25,26}, Lys³⁴(N^{ε}-decanoyl))hGLP-1(7-36)NH₂; | (SEQ ID NO.107) |
| (Gly⁸, Aib³⁵, Lys²⁵, Arg²⁶, Lys³⁴(N^{ε}-octanoyl))hGLP-1(7-36)NH₂; | (SEQ ID NO.108) |
| (Gly⁸, Aib³⁵, Lys²⁵, Arg²⁶, Lys³⁴(N^{ε}-tetradecanoyl))hGLP-1(7-36)NH₂; | (SEQ ID NO.109) |
| (Gly⁸, Aib³⁵, Lys²⁵, Arg²⁶, Lys³⁴(N^{ε}-hexadecanoyl))hGLP-1(7-36)NH₂; | (SEQ ID NO.110) |
| (Gly⁸, Aib³⁵, Lys³⁶(N^{ε}-octanoyl))hGLP-1(7-36)NH₂; | (SEQ ID NO.111) |
| (Gly⁸, Aib³⁵, Lys³⁶(N^{ε}-tetradecanoyl))hGLP-1(7-36)NH₂; | (SEQ ID NO.112) |
| (Gly⁸, Aib³⁵, Lys³⁶(N^{ε}-hexadecanoyl))hGLP-1(7-36)NH₂; | (SEQ ID NO.113) |
| (Gly⁸, Aib³⁵, Arg²⁶, Lys³⁶(N^{ε}-octanoyl))hGLP-1(7-36)NH₂; | (SEQ ID NO.114) |
| (Gly⁸, Aib³⁵, Arg²⁶, Lys³⁶(N^{ε}-tetradecanoyl))hGLP-1(7-36)NH₂; | (SEQ ID NO. 115) |
| (Gly⁸, Aib³⁵, Arg²⁶, Lys³⁶(N^{ε}-hexadecanoyl))hGLP-1(7-36)NH_{2;} | (SEQ ID NO.116) |
| (Gly⁸, Aib³⁵, Arg^{26,34}, Lys³⁶(N^{ε}-octanoyl))hGLP-1(7-36)NH₂; | (SEQ ID NO.117) |
| (Gly⁸, Aib³⁵, Arg^{26,34}, Lys³⁶(N^{ε}-hexadecanoyl))hGLP-1(7-36)NH₂; | (SEQ ID NO.118) |
| (Gly⁸, Aib³⁵, Arg^{26,34}, Lys³⁸(N^{ε}-octanoyl))hGLP-1(7-38)NH₂; | (SEQ ID NO. 119) |
| (Gly⁸, Aib³⁵, Arg^{26,34}, Lys³⁸(N^{ε}-decanoyl))hGLP-1(7-38)NH₂; | (SEQ ID NO.120) |
| (Gly⁸, Aib³⁵, Arg^{26,34}, Lys³⁸(N^{ε}-tetradecanoyl))hGLP-1(7-38)NH₂; | (SEQ ID NO.121) |
| (Gly⁸, Aib³⁵, Arg^{25,34}, Lys³⁸(N^{ε}-hexadecanoyl)hGLP-1(7-38)NH₂; | (SEQ ID NO.122) |
| (Gly⁸, Aib^{35,37}, Arg^{25,26,34}, Lys³⁸(N^{ε}-octanoyl))hGLP-1(7-38)NH₂; | (SEQ ID NO.123) |
| (Gly⁸, Aib^{35,37}, Arg^{25,26,34}, Lys³⁸N^{ε}-decanoyl))hGLP-1(7-38)NH₂; | (SEQ ID NO.124) |
| (Gly⁸, Aib^{35,37}, Arg^{25,26,34}, Lys³⁸(N^{ε}-tetradecanoyl))hGLP-1(7-38)NH₂; | (SEQ ID NO.125) |
| (Gly⁸, Aib^{35,37}, Arg^{25,26,34}, Lys³⁸(N^{ε}-hexadecanoyl))hGLP-1(7-38)NH₂; | (SEQ ID NO.126) |
| (Gly⁸, Aib^{35,37}, Arg^{26,34} Lys³⁸(N^{ε}-octanoy)))hGLP-1(7-38)NH₂; | (SEQ ID NO.127) |
| (Gly⁸, Aib^{35,37}, Arg^{26,34}, Lys³⁸(N^{ε}-decanoyl))hGLP-1(7-38)NH₂; | (SEQ ID NO.128) |
| (Gly⁸, Aib^{35,37}, Arg^{26,34}, Lys³⁸(N^{ε}-hexadecanoyl))hGLP-1(7-38)NH₂; | (SEQ ID NO.129) |
| (Gly⁸, Aib³⁵, Lys²⁵, Arg^{26,34}, Lys³⁶(N^{ε}-octanoy)))hGLP-1(7-36)NH₂; | (SEQ ID NO.130) |
| (Gly⁸, Aib³⁵, Lys²⁵, Arg^{26,34}, Lys³⁶(N^{ε}-tetradecanoyl)) | |
| hGLP-1(7-36)NH₂; | (SEQ ID NO.131) |
| (Gly⁸, Aib³⁵, Lys²⁵, Arg^{26,34}, Lys³⁶(N^{ε}-hexadecanoyl)) | |
| hGLP-1 (7-36)NH₂; | (SEQ ID NO.132) |
| (Gly⁸, Aib³⁵, Arg^{25,26,34}, Lys³⁶(N^{ε}-octanoyl))hGLP-1(7-36)NH₂; | (SEQ ID NO.133) |
| (Gly⁸, Aib³⁵, Arg^{25,26,34}, Lys³⁶(N^{ε}-tetradecanoyl))hGLP-1(7-36)NH₂; | (SEQ ID NO.134) |
| (Gly⁸, Aib³⁵, Arg^{25,26,34}, Lys³⁶(N^{ε}-hexadecanoyl))hGLP-1(7-36)NH₂; | (SEQ ID NO.135) |
| (Gly⁸, Aib³⁵, Arg^{25,26,34}, Lys³⁶(N^{ε}-decanoyl))hGLP-1(7-36)NH₂; | (SEQ ID NO.136) |
| (Gly⁸, Lys³⁴(N^{ε}-octanoyl), β-Ala³⁵)hGLP-1(7-36)NH₂; | (SEQ ID NO.137) |
| (Gly⁸, Lys³⁴(N^{ε}-tetradecanoyl), β-Ala³⁵)hGLP-1(7-36)NH₂; | (SEQ ID NO.138) |
| (Gly⁸, Lys³⁴(N^{ε}-hexadecanoyl), β-Ala³⁵)hGLP-1(7-36)NH₂; | (SEQ ID NO.139) |
| (Gly⁸, A6c³², Lys³⁴(N^{e}-octanoyl), β-Ala³⁵)hGLP-1(7-36)NH₂; | (SEQ ID NO.140) |
| (Gly⁸, Glu²³, Lys³⁴(N^{e}-octanoyl), β-Ala³⁵)hGLP-1(7-36)NH₂; | (SEQ ID NO.141) |
| (Gly⁸, Glu²³, A6c³², Lys³⁴(N^{e}-octanoyl), β-Ala³⁵)hGLP-1(7-36)NH₂; | (SEQ ID NO.142) |
| (Gly⁸, Arg²⁶, Lys³⁴(N^{ε}-octanoyl), β-Ala³⁵)hGLP-1(7-36)NH₂; | (SEQ ID NO.143) |
| (Gly⁸, Arg²⁶, Lys³⁴(N^{ε}-tetradecanoyl), β-Ala³⁵)hGLP-1(7-36)NH₂; | (SEQ ID NO.144) |
| (Gly⁸, Arg²⁶, Lys³⁴(N^{ε}-hexadecanoyl), β-Ala³⁵)hGLP-1(7-36)NH₂; | (SEQ ID NO.145) |
| (Gly⁸, Arg²⁶, Lys³⁴(N^{ε}-decanoyl), β-Ala³⁵)hGLP-1(7-36)NH₂; | (SEQ ID NO.146) |
| (Gly⁸, Arg^{25,26}, Lys³⁴(N^{ε}-octanoyl), β-Ala³⁵)hGLP-1(7-36)NH₂; | (SEQ ID NO.147) |
| (Gly⁸, Arg^{25,26}, Lys³⁴(N^{ε}-tetradecanoyl), β-Ala³⁵)hGLP-1(7-36)NH₂; | (SEQ ID NO.148) |
| (Gly⁸, Arg^{25,26}, Lys³⁴(N^{ε}-hexadecanoyl), β-Ala³⁵)hGLP-1(7-36)NH₂; | (SEQ ID NO.149) |
| (Gly⁸, Arg^{25,26}, Lys³⁴(NE-decanoyl), β-Ala³⁵)hGLP-1(7-36)NH₂; | (SEQ ID NO.150) |
| (Gly⁸, Lys²⁵, Arg²⁶, Lys³⁴(N^{ε}-octanoyl), β-Ala³⁵)hGLP-1(7-36)NH₂; | (SEQ ID NO.151) |
| (Gly⁸, Lys²⁵, Arg²⁶, Lys³⁴(N^{ε}-tetradecanoyl), β-Ala³⁵) | |
| hGLP-1(7-36)NH₂; | (SEQ ID NO.152) |
| (Gly⁸, Lys²⁵, Arg²⁶, Lys³⁴(N^{ε}-hexadecanoyl), β-Ala³⁵) | |
| hGLP-1(7-36)NH₂; | (SEQ ID NO.153) |
| (Gly⁸, β-Ala³⁵, Lys³⁶(N^{ε}-octanoyl))hGLP-1(7-36)NH₂; | (SEQ ID NO.154) |
| (Gly⁸, β-Ala³⁵, Lys³⁶(N^{ε}-tetradecanoyl))hGLP-1(7-36)NH₂; | (SEQ ID NO.155) |
| (Gly⁸, β-Ala³⁵, Lys³⁶(N^{ε}-hexadecanoyl))hGLP-1(7-36)NH₂; | (SEQ ID NO.156) |
| (Gly⁸, Arg²⁶, β-Ala³⁵, Lys³⁶(N^{ε}-octanoyl))hGLP-1(7-36)NH₂; | (SEQ ID NO.157) |
| (Gly⁸, Arg²⁶, β-Ala³⁵, Lys³⁶(N^{ε}-tetradecanoyl))hGLP-1(7-36)NH₂; | (SEQ ID NO.158) |
| (Gly⁸, Arg²⁶, β-Ala³⁵, Lys³⁶(N^{ε}-hexadecanoyl))hGLP-1(7-36)NH₂; | (SEQ ID NO.159) |
| (Gly⁸, Arg^{26,34}, β-Ala³⁵, Lys³⁶(N^{ε}-octanoyl))hGLP-1(7-36)NH₂; | (SEQ ID NO.160) |
| (Gly⁸, Arg^{26,34}, β-Ala³⁵, Lys³⁶(N^{ε}-tetradecanoyl))hGLP-1(7-36)NH₂; | (SEQ ID NO.161) |
| (Gly⁸, Arg^{26,34}, β-Ala³⁵, Lys³⁶(N^{ε}-hexadecanoyl))hGLP-1(7-36)NH₂; | (SEQ ID NO.162) |
| (Gly⁸, Arg^{26,34}, β-Ala³⁵, Lys³⁶(N^{ε}-decanoyl))hGLP-1(7-36)NH₂; | (SEQ ID NO.163) |
| (Gly⁸, Lys²⁵, Arg^{26,34}, β-Ala³⁵, Lys³⁶(N^{ε}-octanoyl))hGLP-1(7-36)NH₂; | (SEQ ID NO.164) |
| (Gly⁸, Lys²⁵, Arg^{26,34}, Lys³⁶(N^{ε}-tetradecanoyl), β-Ala³⁵) | |
| hGLP-1(7-36)NH₂; | (SEQ ID NO.165) |
| (Gly⁸, Lys²⁵, Arg^{26,34}, β-Ala³⁵, Lys³⁶(N^{ε}-hexadecanoyl)) | |
| hGLP-1(7-36)NH₂; | (SEQ ID NO.166) |
| (Gly⁸, Arg^{25,26,34}, β-Ala³⁵, Lys³⁶(N^{ε}-octanoyl)hGLP-1(7-36)NH₂; | (SEQ ID NO.167) |
| (Gly⁸, Arg^{25,26,34}, β-Ala³⁵, Lys³⁶(N^{ε}-tetradecanoyl))hGLP-1(7-36)NH₂; | (SEQ ID NO.168) |
| (Gly⁸, Arg^{25,26,34}, β-Ala³⁵, Lys³⁶(N^{ε}-hexadecanoyl))hGLP-1(7-36)NH₂; | (SEQ ID NO.169) |
| (Gly⁸, Arg^{25,26,34}, β-Ala³⁵, Lys³⁶(N^{ε}-decanoyl)hGLP-1(7-36)NH₂; | (SECT ID NO.170) |
| (Gly⁸, Aib³⁵, Lys²⁶(N^{ε}-octanoyl), A6c³², Arg³⁴)hGLP-1(7-36)NH₂; | (SEQ ID NO.171) |
| (Gly⁸, Aib³⁵, Lys²⁶(N^{ε}-tetradecanoyl), A6c³², Arg³⁴) | |
| hGLP-1(7-36)NH₂; | (SEQ ID NO.172) |
| (Gly⁸, Aib³⁵, Lys²⁶(N^{ε}-hexadecanoyl), A6c³², Arg³⁴) | |
| hGLP-1(7-36) NH₂; | (SEQ ID NO.173) |
| (Gly⁸, Aib³⁵, A6c³², Lys³⁴(N^{ε}-octanoyl))hGLP-1(7-35)NH₂; | (SEQ ID NO.174) |
| (Gly⁸, Aib³⁵, A6c³², Lys³⁴(N^{ε}-tetradecanoyl))hGLP-1(7-36)NH₂; | (SEQ ID NO.175) |
| (Gly⁸, Aib³⁵, A6C³², Lys³⁴(N^{ε}-hexadecanoyl))hGLP-1(7-36)NH₂; | (SEQ ID NO.176) |
| (Gly⁸, Aib³⁵, Arg²⁶, A6c³², Lys³⁴(N^{ε}-octanoyl))hGLP-1(7-36)NH₂; | (SEQ ID NO.177) |
| (Gly⁸, Aib³⁵, Arg²⁶, A6c³², Lys³⁴(N^{ε}-tetradecanoyl))hGLP-1(7-36)NH₂; | (SEQ ID NO.178) |
| (Gly⁸, Aib³⁵, A6c³², Lys³⁶(N^{ε}-octanoyl))hGLP-1(7-36)NH₂; | (SEQ ID NO.179) |
| (Gly⁸, Aib³⁵, A6c³², Lys³⁶(N^{ε}-tetradecanoyl))hGLP-1(7-36)NH₂; | (SEQ ID NO.180) |
| (Gly⁸, Aib³⁵, A6c³², Lys³⁶(N^{ε}-hexadecanoyl))hGLP-1(7-36)NH₂; | (SEQ ID NO.181) |
| (Gly⁸, Aib³⁵, Arg²⁶, A6C³², Lys³⁶(N^{ε}-octanoyl))hGLP-1(7-36)NH₂; | (SEQ 10 NO.182) |
| (Gly⁸, Aib³⁵, Arg²⁶, A6c³², Lys³⁶(N^{ε}-tetradecanoyl))hGLP-1(7-36)NH₂; | (SEQ ID NO.183) |
| (Gly⁸, Aib³⁵, Arg²⁶, A6c³², Lys³⁶(N^{ε}-hexadecanoyl))hGLP-1(7-36)NH₂; | (SEQ ID NO.184) |
| (Gly⁸, Aib³⁵, Arg^{26,34}, A6C³², Lys³⁶(N^{ε}-ocianoyl))hGLP-1(7-36)NH₂; | (SEQ ID NO.185) |
| (Gly⁸, Aib³⁵, Arg^{26,34}, A6c³², Lys³⁶(N^{ε}-decanoy))hGLP-1(7-36)NH₂; | (SEQ ID NO.186) |
| (Gly⁸, Aib³⁵, Arg^{26,34}, A6c³², Lys³⁶(N^{ε}-tetradecanoyl)) | |
| hGLP-1(7-36)NH₂; | (SEQ ID NO.187) |
| (Gly⁸, Aib³⁵, Arg^{26,34}, A6c³², Lys³⁶(N^{ε}-hexadecanoyl)) | |
| hGLP-1(7-36)NH₂; | (SEQ ID NO.188) |
| (Gly⁸, Aib^{24,35}, Lys²⁶(N^{ε}-octanoyl), Arg³⁴)hGLP-1(7-36)NH₂; | (SEQ ID NO.189) |
| (Gly⁸, Aib^{24,35}, Lys²⁶(N^{ε}-tetradecanoyl), Arg³⁴)hGLP-1(7-36)NH₂; | (SEQ ID NO. 190) |
| (Gly⁸, Aib^{24,35}, Lys²⁶(N^{ε}-hexadecanoyl), Arg³⁴)hGLP-1(7-36)NH₂; | (SEQ ID NO.191) |
| (Gly⁸, Aib^{24,35}, Arg²⁶, Lys³⁴(N^{ε}-octanoyl))hGLP-1(7-36)NH₂; | (SEQ ID NO.192) |
| (Gly⁸, Aib^{24,35}, Arg²⁶, Lys³⁴(N^{ε}-tetradecanoyl))hGLP-1(7-36)NH₂; | (SEQ ID NO.193) |
| (Gly⁸, Aib^{24,35}, Arg²⁶, Lys³⁴(N^{ε}-hexadecanoyl))hGLP-1(7-36)NH₂; | (SEQ ID NO.194) |
| (Gly⁸, Aib^{24,35}, Arg^{26,34}, Lys³⁶(N^{ε}-octanoyl))hGLP-1(7-36)NH₂; | (SEQ ID NO.195) |
| (Gly⁸, Aib^{24,35}, Arg^{26,34}, Lys³⁶(N^{ε}-tetradecanoyl))hGLP-1(7-36)NH₂; | (SEQ ID NO.196) |
| (Gly⁸, Aib^{24,35}, Arg^{26,34}, Lys³⁶(N^{ε}-hexadecanoyl))hGLP-1(7-36)NH₂; | (SEQ ID NO.197) |
| (Gly⁸, Aib^{24,35}, Glu²³, A6c³², Lys³⁴(N^{e}-octanoyl))hGLP-1(7-36)NH₂; | (SEQ ID NO.198) |
| (Gly⁸, Aib³⁵, Glu²³, Lys²⁶(N^{ε}-octanoyl), Arg³⁴)hGLP-1(7-36)NH₂; | (SEQ ID NO.199) |
| (Gly⁸, Aib³⁵, Glu²³, Lys²⁶(N^{ε}-tetradecanoyl), Arg³⁴)hGLP-1(7-36)NH₂; | (SEQ ID NO.200) |
| (Gly⁸, Aib³⁵, Glu²³, Lys²⁶(N^{ε}-hexadecanoyl), Arg³⁴)hGLP-1(7-36)NH₂; | (SEQ ID NO.201) |
| (Gly⁸, Aib³⁵, Glu²³, Lys³⁴(N^{e}-octanoyl))hGLP-1(7-36)NH₂; | (SEQ ID NO.202) |
| (Gly⁸, Aib³⁵, Glu²³, A6c³², Lys³⁴(N^{e}-octanoyl))hGLP-1(7-36)NH₂; | (SEQ ID NO.203) |
| (Gly⁸, Aib³⁵, Glu²³, Arg²⁶, Lys³⁴(N^{ε}-octanoyl))hGLP-1(7-36)NH₂; | (SEQ ID NO.204) |
| (Gly⁸, Aib³⁵, Glu²³, Arg²⁶, Lys³⁴(N^{ε}-tetradecanoyl))hGLP-1(7-36)NH₂; | (SEQ ID NO.205) |
| (Gly⁸, Aib³⁵, Glu²³, Arg²⁶, Lys³⁴(N^{ε}-hexadecanoyl))hGLP-1(7-36)NH₂; | (SEQ ID NO.206) |
| (Gly⁸, Aib³⁵, Glu²³, Lys³⁶(N^{ε}-octanoyl))hGLP-1(7-36)NH₂; | (SEQ ID NO.207) |
| (Gly⁸, Aib³⁵, Glu²³, Lys³⁶(N^{ε}-tetractecarfoyl))hGLP-1(7-36)NH₂; | (SEQ ID NO.208) |
| (Gly⁸, Aib³⁵, Glu²³, Lys³⁶(N^{ε}-hexadecanoyl))hGLP-1(7-36)NH₂; | (SEQ ID NO.209) |
| (Gly⁸, Aib³⁵, Glu²³, Arg^{26,34}, Lys³⁶(N^{ε}-octanoyl))hGLP-1(7-36)NH₂; | (SEQ ID NO.210) |
| (Gly⁸, Aib³⁵, Glu²³, Arg^{26,34}, Lys³⁶(N^{ε}-tetradecanoyl)) | |
| hGLP-1(7-36)NN₂; | (SEQ ID NO.211) |
| (Gly⁸, Aib³⁵, Glu²³, Arg^{26,34}, Lys³⁶(N^{ε}-hexadecanoyl)) | |
| hGLP-1(7-36)NH₂; | (SEQ ID NO.212) |
| (Gly⁸, Aib^{30,35}, Lys²⁶(N^{ε}-octanoyl), Arg³⁴)hGLP-1(7-36)NH₂; | (SEQ ID NO.213) |
| (Gly⁸, Aib^{30,35}, Lys²⁶(N^{ε}-tetradecanoyl), Arg³⁴)hGLP-1(7-36)NH₂; | (SEQ ID NO.214) |
| (Gly⁸, Aib^{30,35}, Lys²⁶(N^{ε}-hexadecanoyl), Arg³⁴)hGLP-1(7-36)NH₂; | (SEQ ID NO.215) |
| (Gly⁸, Aib^{30,35}, Arg²⁶, Lys³⁴(N^{ε}-octanoyl))hGLP-1(7-36)NH₂; | (SEQ ID NO.216) |
| (Gly⁸, Aib^{30,35}, Arg²⁶, Lys³⁴(N^{ε}-tetradecanoyl))hGLP-1(7-36)NH₂; | (SEQ ID NO.217) |
| (Gly⁸, Aib^{30,35}, Arg²⁶, Lys³⁴(N^{ε}-hexadecanoyl))hGLP-1(7-36)NH₂; | (SEQ ID NO.218) |
| (Gly⁸, Aib^{30,35}, Arg^{26,34}, Lys³⁶(N^{ε}-Octanoyl))hGLP-1(7-36)NH₂; | (SEQ ID NO.219) |
| (Gly⁸, Aib^{30,35}, Arg^{26,34}, Lys³⁶(N^{ε}-tetradecanoyl))hGLP-1(7-36)NH₂; | (SEQ ID NO.220) |
| (Gly⁸, Aib^{30,35}, Arg^{26,34} Lys³⁶(N^{ε}-hexadecanoyl))hGLP-1(7-36)NH₂; | (SEQ ID NO.221) |
| (Gly⁸, Aib³⁵, Glu²³, A6c³², Lys³⁶(N^{ε}-octanoyl))hGLP-1(7-36)NH₂; | (SEQ ID NO.222) |
| (Gly⁸, Aib³⁵, Glu²³, A6c³², Lys³⁶(N^{ε}-tetradecanoyl))hGLP-1(7-36)NH₂; | (SEQ ID NO.223) |
| (Gly⁸, Aib³⁵, Glu²³, A6c³², Lys³⁶(N^{ε}-hexadecanoyl))hGLP-1(7-36)NH₂; | (SEQ ID NO.224) |
| (Gly⁸, Aib³⁵, Glu²³, Arg^{26,34}, A6C³², Lys³⁶(N^{ε}-octanoyl)) | |
| hGLP-1(7-36)NH₂; | (SEQ ID NO.225) |
| (Gly⁸, Aib³⁵, Glu²³, Arg^{26,34}, A6c³² Lys³⁶(N^{ε}-tetradecanoyl)) | |
| hGLP-1(7-36)NH₂; | (SEQ ID NO.226) |
| (Gly⁸, Aib³⁵, Glu²³, Arg^{26,34}, A6c³², Lys³⁶(N^{ε}-hexadecanoyl)) | |
| hGLP-1(7-36)NH₂; | (SEQ ID NO.227) |
| (Gly⁸, Aib^{24,35}, Glu²³, Arg^{26,34}, A6C³², Lys³⁶(N^{ε}-octanoyl)) | |
| hGLP-1(7-36)NH₂; | (SEQ ID NO.228) |
| (Gly⁸, Aib^{24,35}, Glu²³, Arg^{26,34}, A6c³², Lys³⁶(N^{ε}-tetradecanoyl)) | |
| hGLP-1(7-36)NH₂; | (SEQ ID NO.229) |
| (Gly⁸, Aib^{24,35}, Glu²³, Arg^{26,34}, A6c³², Lys³⁶(N^{ε}-hexadecanoyl)) | |
| hGLP-1(7-36)NH₂; | (SEQ ID NO.230) |
| (Gly⁸, Aib^{24,30,35}, Glu²³, Arg^{26,34} A6c³², Lys³⁶(N^{ε}-octanoyl) | |
| hGLP-1(7-36)NH₂; | (SEQ ID NO.231) |
| (Gly⁸, Aib^{24,30,35}, Glu²³, Arg^{26,34}, A6c³², Lys³⁶(E^{ε}-tetradecanoyl)) | |
| hGLP-1(7-36)NH₂; | (SEQ ID NO.232) |
| (Gly⁸, Aib^{24,30,35}, Glu²³, Arg^{26,34}, A6c³², Lys³⁶(N^{ε}-hexadecanoyl)) | |
| hGLP-1(7-36)NH₂; | (SEQ ID NO.233) |
| ((N^{α}-HEPES-His)⁷, Gly⁸, β-Ala³⁵)hGLP-1(7-36)NH₂; | (SEQ ID NO.234) |
| ((N^{α}-HEPA-His)⁷, Gly⁸, β-Ala³⁵)hGLP-1(7-36)NH₂; | (SEQ ID NO.235) |
| ((N^{α}-tetradecanoyl-His)⁷, Gly⁸, Aib³⁵)hGLP-1(7-36)NH₂; | (SEQ ID NO.236) |
| ((N^{α}-tetradecanoyl-His)⁷, Gly⁸, β-Ala³⁵)hGLP-1(7-36)NH₂; | (SEQ ID NO.237) |
| ((N^{α}-tetradecanoyl-HiS)⁷, Gly⁸, Aib³⁵, Arg^{26,34}) | |
| hGLP-1(7-36)NH₂; | (SEQ ID NO.238) |
| ((N^{α}-tetradecanoyl-His)⁷, Gly⁸, Arg^{26,34}, β-Ala³⁵) | |
| hGLP-1(7-36)NH₂; | (SEQ ID NO.239) |
| ((N^{α}-tetradecanoyl-His)⁷, Gly⁸, Aib³⁵, Arg^{25,26,34}) | |
| hGLP-1(7-36)NH₂; | (SEQ ID NO.240) |
| ((N^{α}-tetradecanoyl-His)⁷, Gly⁸, Arg^{25,26,34}, β-Ala³⁵) | |
| hGLP-1(7-36)NH₂; | (SEQ ID NO.241) |
| (Gly⁸, Aib³⁵, Lys²⁶(N^{ε}-octanesuffonyl), Arg³⁴) | |
| hGLP-1(7-36)NH₂; | (SEQ ID NO.242) |
| (Gly⁸, Aib³⁵, Lys²⁶(N^{ε}-dodecanesulfonyl), Arg³⁴) | |
| hGLP-1(7-36)NH₂; | (SEQ ID NO.243) |
| (Gly⁸, Aib³⁵, Lys²⁶(N^{ε}-hexadecanesulfonyl), Arg³⁴) | |
| hGLP-1(7-36)NH₂; | (SEQ ID NO.244) |
| (Gly⁸, Aib³⁵, Arg²⁶, Lys³⁴(N^{ε}-octanesulfonyl)) | |
| hGLP-1(7-36)NH₂; | (SEQ ID NO.245) |
| (Gly⁸, Aib³⁵, Arg²⁶, Lys³⁴(N^{ε}-dodecanesulfonyl)) | |
| hGLP-1(7-36)NH₂; | (SEQ ID NO.246) |
| Gly⁸, Aib³⁵, Arg²⁶, Lys³⁴(N^{ε}-hexadecanesulfonyl)) | |
| hGLP-1(7-36)NH₂; | (SEQ ID NO.247) |
| (Gly⁸, Aib³⁵, Arg^{26,34}, Lys³⁶(N^{ε}-vctanesulfionyl)) | |
| hGLP-1(7-36)NH₂; | (SEQ ID NO.248) |
| (Gly⁸, Aib³⁵, Arg^{26,34}, Lys³⁶(N^{ε}-hexadecanesulfonyl)) | |
| hGLP-1(7-36)NH₂; | (SEQ ID NO.249) |
| (Gly⁸, Aib³⁵, Asp²⁶(1-(4-decylpiperazine)), Arg³⁴) | |
| hGLP-1(7-36)NH₂; | (SEQ ID NO.250) |
| (Gly⁸, Aib³⁵, Asp²⁶(1-(4-dodecylpiperazine)), Arg³⁴) | |
| hGLP-1(7-36)NH₂; | (SEQ ID NO.251) |
| (Gly⁸, Aib³⁵, Asp²⁶(1-(4-tetradecylpiperazine)), Arg³⁴) | |
| hGLP-1(7-36)NH₂; | (SEQ ID NO.252) |
| (Gly⁸, Aib³⁵, Asp²⁶(1-(4-hexadecylpiperazine)), Arg³⁴) | |
| hGLP-1(7-36)NH₂; | (SEQ ID NO.253) |
| (Gly⁸, Aib³⁵, Arg²⁶, Asp³⁴(1-(4-decylpiperazine))) | |
| hGLP-1(7-36)NH₂; | (SEQ ID NO.254) |
| (Gly⁸, Aib³⁵, Arg²⁶, Asp³⁴(1-(4-dodecylpiperazine))) | |
| hGLP-1(7-36)NH₂; | (SEQ ID NO.255) |
| (Gly⁸, Aib³⁵, Arg²⁶, Asp³⁴(1-(4-tetradecylpiperazine))) | |
| hGLP-1(7-36)NH₂; | (SEQ ID NO.256) |
| (Gly⁸, Aib³⁵, Arg²⁶, Asp³⁴(1-(4-hexadecylpiperazlne))) | |
| hGLP-1(7-36)NH₂; | (SEQ ID NO.257) |
| (Gly⁸, Aib³⁵, Arg^{26,34}, Asp³⁶(1-(4-decylpiperazine))) | |
| hGLP-1(7-36)NH₂; | (SEQ ID NO.258) |
| (Gly⁸, Aib³⁵, Arg^{26,34}, Asp³⁶(1-(4-dodecylpiperazine))) | |
| hGLP-1(7-36)NH₂; | (SEQ ID NO.259) |
| (Gly⁸, Aib³⁵, Arg^{26,34}, Asp³⁶(1-(4-hexadecylpiperazine))) | |
| hGLP-1(7-36)NH₂; | (SEQ ID NO.260) |
| (Gly⁸, Aib³⁵, Arg^{26,34}, Asp³⁸(1-(4-decylpiperazine))) | |
| hGLP-1(7-38)NH₂; | (SEQ ID NO.261) |
| (Gly⁸, Aib³⁵, Arg^{26,34}, Asp³⁸(1-(4-dodecylpiperazine))) | |
| hGLP-1(7-38)NH₂; | (SEQ ID NO.262) |
| (Gly⁸, Aib³⁵, Arg^{26,34}, Asp³⁸(1-(4-tetradecylpiperazine))) | |
| hGLP-1(7-38)NH₂; | (SEQ ID NO.263) |
| (Gly⁸, Aib³⁵, Arg^{26,34}, Asp³⁸(1-(4-hexadecylpiperazine))) | |
| hGLP-1(7-38)NH₂; | (SEQ ID NO.264) |
| (Gly⁸, Aib^{35,37}, Arg^{26,34}, Asp³⁸(1-(4-decylpiperazine))) | |
| hGLP-1(7-38)NH₂; | (SEQ ID NO.265) |
| (Gly⁸, Aib^{35,37}, Arg^{26,34}, Asp³⁸(1-(4-dodecylpiperazine))) | |
| hGLP-1(7-38)NH₂; | (SEQ ID NO.266) |
| (Gly⁸, Aib^{35,37}, Arg^{26,34}, Asp³⁸(1-(4-tetradecylpiperazine))) | |
| hGLP-1(7-38)NH₂; | (SEQ ID NO.267) |
| (Gly⁸, Aib^{35,37}, Arg^{26,34}, Asp³⁸(1-(4-hexadecylpiperazine))) | |
| hGLP-1(7-38)NH₂; | (SEQ ID NO.268) |
| (Gly⁸, Aib³⁵, Arg^{25,34}, Asp²⁶(1-(4-decylpiperazine))) | |
| hGLP-1(7-36)NH₂; | (SEQ ID NO.269) |
| (Gly⁸, Aib³⁵, Arg^{25,34}, Asp²⁶(1-(4-dodecylpiperazine))) | |
| hGLP-1(7-36)NH₂; | (SEQ ID NO.270) |
| (Gly⁸, Aib³⁵, Arg^{25,34}, Asp²⁶(1-(4-tetradecylpiperazine))) | |
| hGLP-1(7-36)NH₂; | (SEQ ID NO.271) |
| (Gly⁸, Aib³⁵, Arg^{25,34}, Asp²⁶(1-(4-hexadecyipiperazine))) | |
| hGLP-1(7-36)NH₂; | (SEQ ID NO.272) |
| (Gly⁸, Aib³⁵, Arg^{25,26}, Asp³⁴(1-(4-decylpiperazine))) | |
| hGLP-1(7-36)NH₂; | (SEQ ID NO.273) |
| (Gly⁸, Aib³⁵, Arg^{25,26}, Asp³⁴(1-(4-dodecylpiperazine))) | |
| hGLP-1(7-36)NH₂; | (SEQ ID NO.274) |
| (Gly⁸, Aib³⁵, Arg^{25,26}, Asp³⁴(1-(4-tetradecylpiperazine))) | |
| hGLP-1(7-36)NH₂; | (SEQ ID NO.275) |
| (Gly⁸, Aib³⁵, Arg^{25,26}, Asp³⁴(1-(4-hexadecylpiperazine))) | |
| hGLP-1(7-36)NH₂; | (SEQ ID NO.276) |
| (Gly⁸, Aib³⁵, Arg^{25,26,34}, Asp³⁶(1-(4-decylpiperazine))) | |
| hGLP-1(7-36)NH₂; | (SEQ ID NO.277) |
| (Gly⁸, Aib³⁵, Arg^{25,26,34}, Asp³⁶(1-(4-dodecylpiperazine))) | |
| hGLP-1(7-36)NH₂; | (SECT ID NO.278) |
| (Gly⁸, Aib³⁵, Arg^{25,26,34}, Asp³⁶(1-(4-tetradecylpiperazine))) | |
| hGLP-1(7-36)NH₂; | (SEQ ID NO.279) |
| (Gly⁸, Aib³⁵, Arg^{25,26,34}, Asp³⁶(1-(4-hexadecylpiperazine))) | |
| hGLP-1(7-36)NH₂; | (SEQ ID NO.280) |
| (Gly⁸, Aib³⁵, Arg^{25,26,34}, Asp³⁸(1-(4-decylpiperazine))) | |
| hGLP-1(7-38)NH₂: | (SEQ ID NO.281) |
| (Gly⁸, Aib³⁵, Arg^{25,26,34}, Asp³⁸(1-(4-dodecylpiperazine))) | |
| hGLP-1(7-38)NH₂; | (SEQ ID NO.282) |
| (Gly⁸, Aib³⁵, Arg^{25,26,34}, Asp³⁸(1-(4-tetradecylpiperazine))) | |
| hGLP-1(7-38)NH₂; | (SEQ ID NO.283) |
| (Gly⁸, Aib³⁵, Arg^{25,26,34}, Asp³⁸(1-(4-hexadecylpiperazine))) | |
| hGLP-1(7-38)NH₂; | (SEQ ID NO.284) |
| (Gly⁸, Aib^{35,37}, Arg^{25,26,34}, Asp³⁸(1-(4-decylpiperazine))) | |
| hGLP-1(7-38)NH₂; | (SEQ ID NO.285) |
| (Gly⁸, Aib^{35,37}, Arg^{25,26,34}, Asp³⁸(1-(4-dodecylpiperazine))) | |
| hGLP-1(7-38)NH₂; | (SEQ ID NO.286) |
| (Gly⁸, Aib^{35,37}, Arg^{25,26,34}, Asp³⁸(1-(4-tetradecylpiperazine))) | |
| hGLP-1(7-38)NH₂; | (SEQ ID NO.287) |
| (Gly⁸, Aib^{35,37}, Arg^{25,26,34}, Asp³⁸(1-(4-hexadecylpiperazine))) | |
| hGLP-1(7-38)NH₂; | (SEQ ID NO.288) |
| (Gly⁸, Aib³⁵, Arg^{26,34}, Glu³⁶(1-dodecylamino)) | |
| hGLP-1(7-36)NH₂; | (SEQ ID NO.289) |
| (Gly⁸_{,} Aib³⁵, Glu²⁶(1-dodecylamino), Arg³⁴) | |
| hGLP-1(7-36)NH₂; | (SEQ ID NO.290) |
| (Gly⁸, Aib³⁵, Arg²⁶, Glu³⁴(1-dodecylamino)) | |
| hGLP-1(7-36)NH₂; | (SEQ ID NO.291) |
| (Gly⁸, Aib^{35,37}, Arg^{26,34}, Glu³⁸(1-dodecylamino)) | |
| hGLP-1(7-38)NH₂; | (SEQ ID NO.292) |
| (Gly⁸, Aib³⁵, Arg³⁴, Lys²⁶(N^{ε}-(2-(4-decyl-1-piperazine)-acetyl))) | |
| hGLP-1(7-36)NH₂; | (SEQ ID NO.293) |
| (Gly⁸, Aib³⁵, Arg³⁴, Lys²⁶(N^{ε}-(2-(4-dodecyl-1-piperazine)-acetyl))) | |
| hGLP-1(7-36)NH₂; | (SEQ ID NO.294) |
| (Gly⁸, Aib³⁵, Arg³⁴, Lys²⁶(N^{ε}-(2-(4-tetradecyl-1-piperazine)-acetyl))) | |
| hGLP-1(7-36)NH₂; | (SEQ ID NO.295) |
| (Gly⁸, Aib³⁵, Arg³⁴, Lys²⁶(N^{ε}-(2-(4-hexadecyl-1-piperazine)-acetyl))) | |
| hGLP-1(7-36)NH₂; | (SEQ ID NO.296) |
| (Gly⁸, Aib³⁵, Arg²⁶, Lys³⁴(N^{ε}-(2-(4-decyl-1-piperazine)-acetyl))) | |
| hGLP-1(7-36)NH₂; | (SEQ ID NO.297) |
| (Gly⁸, Aib³⁵, Arg²⁶, Lys³⁴(N^{ε}-(2-(4-dodecyl-1-piperazine)-acetyl)) | |
| hGLP-1(7-36)NH₂; | (SEQ ID NO.298) |
| (Gly⁸, Aib³⁵, Arg²⁶, Lys³⁴(N^{ε}-(2-(4-tetradecyl-1-piperazine)-acetyl))) | |
| hGLP-1(7-36)NH₂; | (SEQ ID NO.299) |
| (Gly⁸, Aib³⁵, Arg²⁶, Lys³⁴(N^{ε}-(2-(4-hexadecyl-1-piperazine)-acetyl))) | |
| hGLP-1(7-36)NH₂; | (SEQ ID NO.300) |
| (Gly⁸, Aib³⁵, Arg^{26,34}, Lys³⁶(N^{ε}-(2-(4-decyl-1-piperazine)-acetyl))) | |
| hGLP-1(7-36)NH₂; | (SEQ ID NO.301) |
| (Gly⁸, Aib³⁵, Arg^{26,34}, Lys³⁶(N^{ε}-(2-(4-dodecyl-1-piperazine)-acetyl))) | |
| hGLP-1(7-36)NH₂; | (SEQ ID NO.302) |
| (Gly⁸, Aib³⁵, Arg^{26,34}, Lys³⁶(N^{ε}-(2-(4-hexadecyl-1-piperazine)-acetyl))) | |
| hGLP-1(7-36)NH₂; | (SEQ ID NO.303) |
| (Gly⁸, Aib³⁵, Arg^{26,34}, Lys³⁸(N^{ε}-(2-(4-decyl-1-piperazine)-acetyl))) | |
| hGLP-1(7-38)NH₂; | (SEQ ID NO.304) |
| (Gly⁸, Aib³⁵, Arg^{26,34}, Lys³⁸(N^{ε}-(2-(4-dodecyl-1-piperazine)-acetyl))) | |
| hGLP-1(7-38)NH₂; | (SEQ ID NO.305) |
| (Gly⁸, Aib³⁵, Arg^{26,34}, Lys³⁸(N^{ε}-(2-(4-tetradecyl-1-piperazine)-acetyl))) | |
| hGLP-1(7-38)NH₂; | (SEQ ID NO.306) |
| (Gly⁸, Aib³⁵, Arg^{26,34}, Lys³⁸(N^{ε}-(2-(4-hexadecyl-1-piperazine)-acetyl))) | |
| hGLP-1(7-38)NH₂; | (SEQ ID NO.307) |
| (Gly⁸, Aib^{35,37}, Arg^{26,34}, Lys³⁸(N^{ε}-(2-(4-decyl-1-piperazine)-acetyl))) | |
| hGLP-1(7-38)NH₂; | (SEQ ID NO.308) |
| (Gly⁸, Aib^{35,37}, Arg^{26,34}, Lys³⁸(N^{ε}-(2-(4-dodecyl-1-piperazine)-acetyl))) | |
| hGLP-1(7-38)NH₂; | (SEQ ID NO.309) |
| (Gly⁸, Aib^{35,37}, Arg^{26,34}, Lys³⁸(N^{ε}-(2-(4-tetradecyl-1-piperazine)-acetyl))) | |
| hGLP-1(7-38)NH₂; | (SEQ ID NO.310) |
| (Gly⁸, Aib^{35,37}, Arg^{26,34}, Lys³⁸(N^{ε}-(2-(4-hexadecyl-1-piperazine)-acetyl))) | |
| hGLP-1(7-38)NH₂; | (SEQ ID NO.311) |
| (Gly⁸, Aib³⁵, Arg^{25,34}, Lys²⁶(N^{ε}-(2-(4-decyl-1-piperazine)-acetyl))) | |
| hGLP-1(7-36)NH₂; | (SEQ ID NO.312) |
| (Gly⁸, Aib³⁵, Arg^{25,34}, Lys²⁶(N^{ε}-(2-(4-dodecyl-1-piperazine)-acetyl))) | |
| hGLP-1(7-36)NH₂; | (SEQ ID NO.313) |
| (Gly⁸, Aib³⁵, Arg^{25,34}, Lys²⁶(N^{ε}-(2-(4-tetradecyl-1-piperazine)-acetyl))) | |
| hGLP-1(7-36)NH₂; | (SEQ ID NO.314) |
| (Gly⁸, Aib³⁵, Arg^{25,34}, Lys²⁶(N^{ε}-(2-(4-hexadecyl-1-piperazine)-acetyl))) | |
| hGLP-1(7-36)NH₂; | (SEQ ID NO.315) |
| (Gly⁸, Aib³⁵, Arg^{25,26}, Lys³⁴(N^{ε}-(2-(4-decyl-1-piperazine)-acetyl))) | |
| hGLP-1(7-36)NH₂; | (SEQ ID NO.316) |
| (Gly⁸, Aib³⁵, Arg^{25,26}, Lys³⁴(N^{ε}-(2-(4-dodecyl-1-piperazine)-acetyl))) | |
| hGLP-1(7-36)NH₂; | (SEQ ID NO.317) |
| (Gly⁸, Aib³⁵, Arg^{25,26}, Lys³⁴(N^{ε}-(2-(4-tetradecyl-1-piperazine)-acetyl))) | |
| hGLP-1(7-36)NH₂; | (SEQ ID NO.318) |
| (Gly⁸, Aib³⁵, Arg^{25,26}, Lys³⁴(N^{ε}-(2-(4-hexadecyl-1-piperazine)-acetyl))) | |
| hGLP-1(7-36)NH₂; | (SEQ ID NO.319) |
| (Gly⁸, Aib³⁵, Arg^{25,26,34}, Lys³⁶(N^{ε}-(2-(4-decyl-1-piperazine)-acetyl))) | |
| hGLP-1(7-36)NH₂; | (SEQ ID NO.320) |
| (Gly⁸, Aib³⁵, Arg^{25,26,34}, Lys³⁶(N^{ε}-(2-(4-dodecyl-1-piperazine)-acetyl))) | |
| hGLP-1(7-36)NH₂; | (SEQ ID NO.321) |
| (Gly⁸, Aib³⁵, Arg^{25,26,34}, Lys³⁶(N^{ε}-(2-(4-tetradecyl-1-piperazine)-acetyl))) | |
| hGLP-1(7-36)NH₂; | (SEQ ID NO.322) |
| (Gly⁸, Aib³⁵, Arg^{25,26,34}, Lys³⁶(N^{ε}-(2-(4-hexadecyl-1-piperazine)-acetyl))) | |
| hGLP-1(7-36)NH₂; | (SEQ ID NO.323) |
| (Gly⁸, Aib³⁵, Arg^{25,26,34}, Lys³⁸(N^{ε}-(2-(4-decyl-1-piperazine)-acetyl))) | |
| hGLP-1(7-38)NH₂; | (SEQ ID NO.324) |
| (Gly⁸, Aib³⁵, Arg^{25,26,34}, Lys³⁸(N^{ε}-(2-(4-dodecyl-1-piperazine)-acetyl))) | |
| hGLP-1(7-38)NH₂; | (SEQ ID NO.325) |
| (Gly⁸, Aib³⁵, Arg^{25,26,34}, Lys³⁸(N^{ε}-(2-(4-tetradecyl-1-piperazine)-acetyl))) | |
| hGLP-1(7-38)NH₂; | (SEQ ID NO.326) |
| (Gly⁸, Aib³⁵, Arg^{25,26,34}, Lys³⁸(N^{ε}-(2-(4-hexadecy)-1-piperazine)-acetyl))) | |
| hGLP-1(7-38)NH₂; | (SEQ ID NO.327) |
| (Gly⁸, Aib^{35,37}, Arg^{25,26,34}, Lys³⁸(N^{ε}-(2-(4-decyl-1-piperazine)-acetyl))) | |
| hGLP-1(7-38)NH₂; | (SEQ ID NO.328) |
| (Gly⁸, Aib^{35,37}, Arg^{25,26,34}, Lys³⁸(N^{ε}-(2-(4-dodecyl-1-piperazine)-acetyl))) | |
| hGLP-1(7-38)NH₂; | (SEQ ID NO.329) |
| (Gly⁸, Aib^{35,37}, Arg^{25,26,34}, Lys³⁸(N^{ε}-(2-(4-tetradecyl-1-piperazine)-acetyl))) | |
| hGLP-1(7-38)NH₂; | (SEQ ID NO.330) |
| (Gly⁸, Aib^{35,37}, Arg^{25,26,34}, Lys³⁸(N^{ε}-(2-(4-hexadecyl-1-piperazine)-acetyl))) | |
| hGLP-1(7-38)NH₂; | (SEQ ID NO.331) |
| (Gly⁸, Aib³⁵, Arg^{26,34}, Lys³⁶(N^{ε}-decanoyl))hGLP-1(7-36)OH; | (SEQ ID NO.332) |
| (Gly⁸, Aib³⁵, Lys²⁵, Arg^{26,34}, Lys³⁶(N^{ε}-decanoyl))hGLP-1(7-36)OH; | (SEQ ID NO.333) |
| (Gly⁸, Aib³⁵,Arg^{26,34}, Ava³⁷, Ado³⁸)hGLP-1(7-38)NH₂; | (SEQ ID NO.334) |
| (Gly⁸, Aib³⁵,Arg^{26,34}, Asp³⁷, Ava³⁸, Ado³⁹)hGLP-1(7-39)NH₂; | (SEQ ID NO.335) |
| (Gly⁸, Aib³⁵,Arg^{26,34}, Aun³⁷)hGLP-1(7-37)NH₂; | (SEQ ID NO.336) |
| (Gly⁸, Aib^{17,35})hGLP-1(7-36)NH₂; | (SEQ ID NO.337) |
| (Gly⁸,Arg^{26,34}, β-Ala³⁵, D-Asp³⁷, Ava³⁸, Aun³⁹)hGLP-1(7-39)NH₂; | (SEQ ID NO.338) |
| (Gly⁸, Glu^{22,23}, β-Ala³⁵)hGLP-1(7-36)NH₂; | (SEQ ID NO.339) |
| (Gly⁸, Lys¹⁸, β-Ala³⁵)hGLP-1(7-36)NH₂; | (SEQ ID NO.340) |
| (Gly⁸, Leu²⁷, β-Ala³⁵)hGLP-1(7-36))NH₂; | (SEQ ID NO.341) |
| (Gly⁸, Lys³³, β-Ala³⁵)hGLP-1(7-36)NH₂; | (SEQ ID NO.342) |
| (Gly⁸, Lys¹⁸, Leu²⁷, β-Ala³⁵)hGLP-1(7-36)NH₂; | (SEQ ID NO.343) |
| (Gly⁸, D-Arg³⁶)hGLP-1(7-36)NH₂; | (SEQ ID NO.344) |
| (Gly⁸, β-Ala³⁵, D-Arg³⁷)hGLP-1(7-37)NH₂; | (SEQ ID NO.345) |
| (Gly⁸, Aib²⁷, β-Ala³⁵)hGLP-1(7-36)NH₂; | (SEQ ID NO.346) |
| (Gly⁸, Aib²⁷, β-Ala^{35,37}, Arg³⁸)hGLP-1(7-38)NH₂; | (SEQ ID NO.347) |
| (Gly⁸, Aib²⁷, β-Ala^{35,37}, Arg^{38,39})hGLP-1(7-39)NH₂; | (SEQ ID NO.348) |
| (Gly⁸, Lys^{18,27}, β-Ala³⁵)hGLP-1(7-36)NH₂; | (SEQ ID NO.349) |
| (Gly⁸, Lys²⁷, β-Ala³⁵)hGLP-1(7-36)NH₂; | (SEQ ID NO.350) |
| (Gly⁸, β-Ala³⁵, Arg³⁸)hGLP-1(7-38)NH₂; | (SEQ ID NO.351) |
| (Gly⁸, Arg^{26,34}, β-Ala³⁵,)hGLP-1(7-36)NH₂; | (SEQ ID NO.352) |
| (Gly⁸, D-Arg³⁵)hGLP-1(7-36)NH₂; | (SEQ ID NO.353) |
| (Gly⁸, β-Ala³⁵, Arg³⁷)hGLP-1(7-37)NH₂; | (SEQ ID NO.354) |
| (Gly⁸, Phe³¹, β-Ala³⁵)hGLP-1(7-36)NH₂; | (SEQ ID NO.355) |
| (Gly⁸, Aib³⁵, Phe³¹)hGLP-1(7-36)NH₂; | (SEQ ID NO.356) |
| (Gly⁸, Aib³⁵, 2-Nal³¹)hGLP-1(7-36)NH₂; | (SEQ ID NO.357) |
| (Gly⁸, Aib³⁵, 2-Nal^{28,31})hGLP-1(7-36)NH₂; | (SEQ ID NO.358) |
| (Gly⁸, Aib³⁵, Arg^{26,34}, 2-Nal³¹)hGLP-1(7-36)NH₂; | (SEQ ID NO.359) |
| (Gly⁸, Aib³⁵, Arg^{26,34}, Phe³¹)hGLP-1(7-36)NH₂; | (SEQ ID NO.360) |
| (Gly⁸, Aib³⁵, 2-Nal^{19,31})hGLP-1(7-36)NH₂; | (SEQ ID NO.361) |
| (Gly⁸, Aib³⁵, 2-Nal^{12,31})hGLP-1(7-36)NH₂; | (SEQ ID NO.362) |
| (Gly⁸, Aib³⁵, Lys³⁶(N^{ε}-decanoyl))hGLP-1(7-36)NH₂; | (SEQ ID NO.363) |
| (Gly⁸, Aib³⁵, Arg³⁴, Lys²⁶(N^{ε}-decanoyl))hGLP-1(7-36)NH₂; | (SEQ ID NO.364) |
| (Gly⁸, Aib³⁵, Arg^{26,34}, Lys³⁶(N^{ε}-dodecanoyl))hGLP-1(7-36)NH₂; | (SEQ ID NO.365) |
| (Gly⁸, β-Ala³⁵,Ser³⁷(O-decanoyl))hGLP1(7-37)-NH₂; | (SEQ ID NO.366) |
| (Gly⁸, Aib²⁷, β-Ala^{35,37}, Arg³⁸, Lys³⁹(N^{ε}-octanoyl))hGLP-1(7-39)NH₂; | (SEQ ID NO.367) |
| (Gly⁸, Arg^{26,34}, β-Ala³⁵, Lys³⁷(N^{ε}-octanoyl))hGLP-1(7-37)NH₂; | (SEQ ID NO.368) |
| (Gly⁸, Arg^{26,34}, β-Ala³⁵, Lys³⁷(N^{ε}-decanoyl))hGLP-1(7-37)NH₂; | (SEQ ID NO.369) |
| (Gly⁸, Arg^{26,34}, β-Ala³⁵, Lys³⁷(N^{ε}-tetradecanoyl))hGLP-1(7-37)NH₂; | (SEQ ID NO.370) |
| (Gly⁸, Arg^{26,34}, β-Ala³⁵, Lys³⁷(N^{ε}-dodecanoyl))hGLP-1(7-37)NH₂; | (SEQ ID NO.371) |
| (Gly⁸, Arg^{26,34}, β-Ala³⁵, Lys³⁷(N^{ε}-dodecanoyl))hGLP-1(8-37)NH₂; | (SEQ ID NO.372) |
| (Gly⁸, Arg²⁶, Aib³⁵)hGLP-1(7-36)NH₂; | (SEQ ID NO.373) |
| (Gly⁸, Arg²⁶, Phe³¹, Aib³⁵)hGLP-1(7-36)NH₂; | (SEQ ID NO.374) |
| (Gly⁸, Arg²⁶, Phe³¹, β-Ala³⁵)hGLP-1(7-36)NH₂; | (SEQ ID NO.375) |
| (Gly⁸, Arg²⁶, β-Ala³⁵)hGLP-1(7-36)NH₂; | (SEQ ID NO.376) |
| (Gly⁸, Arg^{26,34}, Phe³¹, Aib³⁵)hGLP-1(7-36)NH₂; | (SEQ ID NO.377) |
| (Gly⁸, Arg^{26,34}, Phe³¹, β-Ala³⁵)hGLP-1(7-36)NH₂; | (SEQ ID NO.378) |
| (Gly⁸, Arg³⁴, Aib³⁵)hGLP-1(7-36)NH₂; | (SEQ ID NO.379) |
| (Gly⁸, Arg³⁴, β-Ala³⁵)hGLP-1(7-36)NH₂; | (SEQ ID NO.380) |
| (Gly⁸, Phe³¹, Arg³⁴, Aib³⁵)hGLP-1(7-36)NH₂; | (SEQ ID NO.381) |
| (Gly⁸, Phe³¹, Arg³⁴, β-Ala³⁵)hGLP-1(7-36)NH₂; | (SEQ ID NO.382) |
| (Gly⁸, Arg^{26,34}, β-Ala³⁵, Lys³⁶(N^{ε}-Aec-decanoyl))hGLP-1(7-36)NH₂; | (SEQ ID NO.383) |
| or (Gly⁸, β-Ala³⁵, Ser³⁷(O-decanoyl))hGLP1(7-37)-NH₂; | (SEQ IDNO.384) |

or a pharmaceutically acceptable salt thereof.
Paragraph 12. A compound according to Paragraph 10, wherein said compound is according to the formula:

| | |
|---|---|
| ((N^{α}-Me-His)⁷, Ser⁸, Aib³⁵)hGLP-1(7-36)NH₂; | (SEQ ID NO.385) |
| ((N^{α}-Me-His)⁷, Ser⁸, β-Ala³⁵)hGLP-1(7-36)NH₂; | (SEQ ID NO.386) |
| ((N^{α}-Me-His)⁷, Ser⁸, Aib³⁵, Arg^{26,34})hGLP-1(7-36)NH₂; | (SEQ ID NO.387) |
| ((N^{α}-Me-His)⁷, Ser⁸, Arg^{26,34}, β-Ala³⁵)hGLP-1(7-36)NH₂; | (SEQ ID NO.388) |
| (Ser⁸, A6c³⁵)hGLP-1(7-36)NH₂; | (SEQ ID NO.389) |
| (Ser⁸, A5c³⁵)hGLP-1(7-36)NH₂; | (SEQ ID NO.390) |
| (Ser⁸, D-Ala³⁵)hGLP-1(7-36)NH₂; | (SEQ ID NO.391) |
| (Ser⁸, Aib³⁵, A6c³²)hGLP-1(7-36)NH₂; | (SEQ ID NO.392) |
| (Ser⁸, Aib³⁵, A5c³²)hGLP-1(7-36)NH₂; | (SEQ ID NO.393) |
| (Ser⁸, Aib³⁵, Glu²³)hGLP-1(7-36)NH₂; | (SEQ ID NO.394) |
| (Ser⁸, Aib^{24,35})hGLP-1(7-36)NH₂; | (SEQ ID NO.395) |
| (Ser⁸, Aib^{30,35})hGLP-1(7-36)NH₂; | (SEQ ID NO.396) |
| (Ser⁸, Aib^{25,35}hGLP-1(7-36)NH₂; | (SEQ ID NO.397) |
| (Ser⁸, Aib³⁵, A6c^{16,20})hGLP-1(7-36)NH₂; | (SEQ ID NO.398) |
| (Ser⁸, Aib³⁵, A6c^{16,29,32})hGLP-1(7-36)NH₂; | (SEQ ID NO.399) |
| (Ser⁸, Aib³⁵, A6c^{20,32})hGLP-1(7-36)NH₂; | (SEQ ID NO.400) |
| (Ser⁸, Aib³⁵, A6c²⁰)hGLP-1(7-36)NH₂; | (SEQ ID NO.401) |
| (Ser⁸, Aib³⁵, Lys²⁵)hGLP-1 (7-36)NH₂; | (SEQ ID NO.402) |
| (Ser⁸, Aib^{24,35}, A6c²⁰)hGLP-1(7-36)NH₂; | (SEQ ID NO.403) |
| (Ser⁸; Aib³⁵, A6c^{29,32})hGLP-1(7-36)NH₂; | (SEQ ID NO.404) |
| (Ser⁸, Aib^{24,35}, A6c^{29,32})hGLP-1(7-36)NH₂; | (SEQ ID NO.405) |
| (Ser⁸, Aib³⁵, A6c¹²)hGLP-1(7-36)NH₂; | (SEQ ID NO.406) |
| (Ser⁸, Aib³⁵, Cha²⁰)hGLP-1(7-36)NH₂; | (SEQ ID NO.407) |
| (Ser⁸, Aib³⁵, A6c³³)hGLP-1(7-36)NH₂; | (SEQ ID NO.408) |
| (Ser⁸, Aic^{16,20}, β-Ala³⁵)hGLP-1(7-36)NH₂; | (SEQ ID NO.409) |
| (Ser⁸, Aib³⁵, β-Ala²²)hGLP-1(7-36)NH₂; | (SEQ ID NO.410) |
| (Ser⁸, Aib^{22,35})hGLP-1(7-36)NH₂; | (SEQ ID NO.411) |
| (Ser⁸, Aib³⁵, Glu²³, A6c³²)hGLP-1(7-36)NH₂; | (SEQ ID NO.412) |
| (Ser⁸, Aib^{24,35}, Glu²³, A6c³²)hGLP-1(7-36)NH₂; | (SEQ ID NO.413) |
| (Ser⁸, Aib^{24,25,35}, Glu²³, A6c³²)hGLP-1(7-36)NH₂; | (SEQ ID NO.414) |
| (Ser⁸, Aib^{24,25,35}, A6c^{16,20,32}, Glu²³)hGLP-1(7-36)NH₂; | (SEQ ID NO.415) |
| (Ser⁸, A6c³², β-Ala³⁵)hGLP-1(7-36)NH₂; | (SEQ ID NO.416) |
| (Ser⁸, A5c³², β-Ala³⁵)hGLP-1(7-36)NH₂; | (SEQ ID NO.417) |
| (Ser⁸, Glu²³, β-Ala³⁵)hGLP-1(7-36)NH₂; | (SEQ ID NO.418) |
| (Ser⁸, Aib²⁴, β-Ala³⁵)hGLP-1(7-36)NH₂; | (SEQ ID NO.419) |
| (Ser⁸, Aib³⁰, β-Ala³⁵)hGLP-1(7-36)NH₂; | (SEQ ID NO.420) |
| (Ser⁸, Aib²⁵, β-Ala¹⁵)hGLP-1(7-36)NH₂; | (SEQ ID NO.421) |
| (Ser⁸, A6c^{16,29,32}, β-Ala³⁵)hGLP-1(7-36)NH₂; | (SEQ ID NO.422) |
| (Ser⁸, A6c^{20,32}, β-Ala³⁵)hGLP-1(7-36)NH₂; | (SEQ ID NO.423) |
| (Ser⁸, A6c²⁰, β-Ala³⁵)hGLP-1(7-36)NH₂; | (SEQ ID NO.424) |
| (Ser⁸, Lys²⁵, β-Ala³⁵)hGLP-1(7-36)NH₂; | (SEQ ID NO.425) |
| (Ser⁸, Aib²⁴, A6c²⁰, β-Ala³⁵)hGLP-1(7-36)NH₂; | (SEQ ID NO.426) |
| (Ser⁸, A6c^{29,32}, β-Ala³⁵)hGLP-1(7-36)NH₂; | (SEQ ID NO.427) |
| (Ser⁸, Aib²⁴, A6c²⁹,³², β-Ala³⁵)hGLP-1(7-36)NH₂; | (SEQ ID NO.428) |
| (Ser⁸, A6c¹², β-Ala³⁵)hGLP-1(7-36)NH₂; | (SEQ ID NO.429) |
| (Ser⁸, Cha²⁰, β-Ala³⁵)hGLP-1(7-36)NH₂; | (SEQ ID NO.430) |
| (Ser⁸, A6c³³, β-Ala³⁵)hGLP-1(7-36)NH₂; | (SEQ ID NO.431) |
| (Ser⁸, β-Ala^{22,35})hGLP-1(7-36)NH₂; | (SEQ ID NO.432) |
| (Ser⁸, Aib²², β-Ala³⁵)hGLP-1(7-36)NH₂; | (SEQ ID NO.433) |
| (Ser⁸, Glu²³, A6c³², β-Ala³⁵)hGLP-1(7-36)NH₂; | (SEQ ID NO.434) |
| (Ser⁸, Aib²⁴, Glu²³, A6c³², β-Ala³⁵)hGLP-1(7-36)NH₂; | (SEQ ID NO.435) |
| (Ser⁸, Aib²⁴, Glu²³, A6c³², Lys³⁴(N^{e}-octanoyl), β-Ala³⁵) | |
| hGLP-1(7-36)NH₂; | (SEQ ID NO.436) |
| (Ser⁸, Aib^{24,25}, Glu²³, A6c³², β-Ala³⁵)hGLP-1(7-36)NH₂; | (SEQ ID NO.437) |
| (Ser⁸, Aib^{24,25}, A6c^{16,20,32}, Glu²³, β-Ala³⁵)hGLP-1(7-36)NH₂; | (SEQ ID NO.438) |
| (Ser⁸, Aib³⁵, D-Arg³⁶)hGLP-1(7-36)NH₂; | (SEQ ID NO.439) |
| (Ser⁸, Aib³⁵, D-Lys³⁶)hGLP-1(7-36)NH₂; | (SEQ ID NO.440) |
| (Ser⁸, β-Ala³⁵, D-Arg³⁶)hGLP-1(7-36)NH₂; | (SEQ ID NO.441) |
| (Ser⁸, β-Ala³⁵, D-Lys³⁶)hGLP-1(7-36)NH₂; | (SEQ ID NO.442) |
| (Ser⁸, Aib³⁵, Arg^{26,34})hGLP-1(7-36)NH₂; | (SEQ ID NO.443) |
| (Ser⁸, Arg^{26,34}, β-Ala³⁵)hGLP-1(7-36)NH₂; | (SEQ ID NO.444) |
| (Ser⁸, Aib³⁵, Arg^{25,26,34})hGLP-1(7-36)NH₂; | (SEQ ID NO.445) |
| (Ser⁸, Arg^{25,26,34}, β-Ala³⁵)hGLP-1(7-36)NH₂; | (SEQ ID NO.446) |
| (Ser⁸, Arg^{26,34}, β-Ala³⁵, Lys³⁶(N^{ε}-tetradecanoyl))hGLP-1(7-36)OH; | (SEQ ID NO.447) |
| (Ser⁸, Aib³⁵, Arg^{26,34}, Lys³⁶(N^{ε}-tetradecanoyl))hGLP-1(7-37)OH; | (SEQ ID NO.448) |
| (Ser⁸, Aib^{35,37}, Arg^{26,34}, Lys³⁶(N^{ε}-tetradecanoyl))hGLP-1(7-37)OH; | (SEQ ID NO.449) |
| (Ser⁸, Aib³⁵, Arg^{26,34}, Lys³⁶(N^{ε}-tetradecanoyl), D-Ala³⁷) | |
| hGLP-1(7-37)OH; | (SEQ ID NO.450) |
| (Ser⁸, Aib^{35,37}, Arg^{26,34}, Lys³⁸(N^{ε}-tetradecanoyl))hGLP-1(7-38)OH; | (SEQ ID NO.451) |
| (Ser⁸, Aib³⁵, Arg^{26,34}, β-Ala³⁷, Lys³⁸(N^{ε}-tetradecanoyl)) | |
| hGLP-1(7-38)OH; | (SEQ ID NO.452) |
| (Ser⁸, Aib³⁵, Arg^{26,34}, Lys³⁸(N^{ε}-tetradecanoyl))hGLP-1 (7-38)OH; | (SEQ ID NO.453) |
| (Ser⁸, Arg^{26,34}, Lys³⁶(N^{ε}-tetradecanoyl), β-Ala³⁷)hGLP-1(7-37)OH; | (SEQ ID NO.454) |
| (Ser⁸, Aib³⁷, Arg^{26,34}, Lys³⁶(N^{ε}-tetradecanoyl))hGLP-1(7-37)OH; | (SEQ ID NO.455) |
| (Ser⁸, Aib³⁵, Arg^{26,34}, Ado³⁷)hGLP-1(7-37)OH; | (SEQ ID NO.456) |
| (Ser⁸, Aib³⁵, Arg^{26,34}, Ado³⁷)hGLP-1(7-37)NH₂; | (SEQ ID NO.457) |
| (Ser⁸, Arg^{26,34}, Lys³⁶(N^{ε}-tetradecanoyl), D-Ala³⁷)hGLP-1(7-37)OH; | (SEQ ID NO.458) |
| (Ser⁸, Aib³⁷, Arg^{26,34}, Lys³⁸(N^{ε}-tetradecanoyl))hGLP-1 (7-38)OH; | (SEQ ID NO.459) |
| (Ser⁸, Arg^{26,34}, β-Ala³⁷, Lys³⁸(N^{ε}-tetradecanoyl))hGLP-1(7-38)OH; | (SEQ ID NO.460) |
| (Ser⁸, Aib³⁵, Lys²⁶(N^{ε}-octanoyl))hGLP-1(7-36)NH₂; | (SEQ ID NO.461) |
| (Ser⁸, Aib³⁵, Lys²⁶(N^{ε}-tetradecanoyl))hGLP-1(7-36)NH₂; | (SEQ ID NO.462) |
| (Ser⁸, Aib³⁵, Lys²⁶(N^{ε}-hexadecanoyl)hGLP-1(7-36)NH₂; | (SEQ ID NO.463) |
| (Ser⁸, Lys²⁶(N^{ε}-octanoyl), β-Ala³⁵)hGLP-1(7-36)NH₂; | (SEQ ID NO.464) |
| (Ser⁸, Lys²⁶(N^{ε}-tetradecanoyl), β-Ala³⁵)hGLP-1(7-36)NH₂; | (SEQ ID NO.465) |
| (Ser⁸, Lys²⁶(N^{ε}-hexadecanoyl), β-Ala³⁵)hGLP-1(7-36)NH₂; | (SEQ ID NO.466) |
| (Ser⁸, Aib³⁵, Lys²⁶(N^{ε}-octanoyl), Arg³⁴)hGLP-1(7-36)NH₂; | (SEQ ID NO.467) |
| (Ser⁸, Aib³⁵, Lys²⁶(N^{ε}-tetradecanoyl), Arg³⁴)hGLP-1(7-36)NH₂; | (SEQ ID NO.468) |
| (Ser⁸, Aib³⁵, Lys²⁶(N^{ε}-hexadecanoyl), Arg³⁴)hGLP-1(7-36)NH₂; | (SEQ ID NO.469) |
| (Ser⁸, Aib³⁵, Lys²⁶(N^{ε}-decanoyl), Arg³⁴)hGLP-1(7-36)NH₂; | (SEQ ID NO.470) |
| (Ser⁸, Aib³⁵, Lys²⁵, Lys²⁶(N^{ε}-octanoyl), Arg³⁴)hGLP-1(7-36)NH₂; | (SEQ ID NO.471) |
| (Ser⁸, Aib³⁵, Lys²⁵, Lys²⁶(N^{ε}-tetradecanoyl), Arg³⁴)hGLP-1(7-36)NH₂; | (SEQ ID NO.472) |
| (Ser⁸, Aib³⁵, Lys²⁵, Lys²⁶(N^{ε}-hexadecanoyl), Arg³⁴)hGLP-1(7-36)NH₂; | (SEQ ID NO.473) |
| (Ser⁸, Aib³⁵, Arg^{25,34}, Lys²⁶(N^{ε}-octanoyl))hGLP-1(7-36)NH₂; | (SEQ ID NO.474) |
| (Ser⁸, Aib³⁵, Arg^{25,34}, Lys²⁶(N^{ε}-tetradecanoyl))hGLP-1(7-36)NH₂; | (SEQ ID NO.475) |
| (Ser⁸, Aib³⁵, Arg^{25,34}, Lys²⁶(N^{ε}-hexadecanoyl))hGLP-1(7-36)NH₂; | (SEQ ID NO.476) |
| (Ser⁸, Aib³⁵, Arg^{25,34}, Lys²⁶(N^{ε}-decanoyl))hGLP-1(7-36)NH₂; | (SEQ ID NO.477) |
| (Ser⁸, Lys²⁶(N^{ε}-octanoyl), Arg³⁴, β-Ala³⁵)hGLP-1(7-36)NH₂; | (SEQ ID NO.478) |
| (Ser⁸, Lys²⁶(N^{ε}-tetradecanoyl), Arg³⁴, β-Ala³⁵)hGLP-1(7-36)NH₂; | (SEQ ID NO.479) |
| (Ser⁸, Lys²⁶(N^{ε}-hexadecanoyl), Arg³⁴, β-Ala³⁵)hGLP-1(7-36)NH₂; | (SEQ ID NO.480) |
| (Ser⁶, Lys²⁶(N^{ε}-decanoyl), Arg³⁴; β-Ala³⁵)hGLP-1(7-36)NH₂; | (SEQ ID NO.481) |
| (Ser⁸, Aib³⁵, Lys³⁴(N^{ε}-octanoyl))hGLP-1(7-36)NH₂; | (SEQ ID NO.482) |
| (Ser⁸, Aib³⁵, Lys³⁴(N^{ε}-tetradecanoyl))hGLP-1(7-36)NH₂; | (SEQ ID NO.483) |
| (Ser⁸, Aib³⁵, Lys³⁴(N^{ε}-hexadecanoyl))hGLP-1(7-36)NH₂; | (SEQ ID NO.484) |
| (Ser⁸, Aib³⁵, Arg²⁶, Lys³⁴(N^{ε}-octanoyl))hGLP-1(7-36)NH₂; | (SEQ ID NO.485) |
| (Ser⁸, Aib³⁵, Arg²⁶, Lys³⁴(N^{ε}-hexadecanoyl))hGLP-1(7-36)NH₂; | (SEQ ID NO.486) |
| (Ser⁸, Aib³⁵, Arg²⁶, Lys³⁴(N^{ε}-decanoyl))hGLP-1(7-36)NH₂; | (SEQ ID NO.487) |
| (Ser⁸, Aib³⁵, Arg^{25,26}, Lys³⁴(N^{ε}-octanoyl))hGLP-1(7-36)NH₂; | (SEQ ID NO.488) |
| (Ser⁸, Aib³⁵, Arg^{25,26}, Lys³⁴(N^{ε}-tetradecanoyl))hGLP-1(7-36)NH₂; | (SEQ ID NO.489) |
| (Ser⁸, Aib³⁵, Arg^{25,26}, Lys³⁴(N^{ε}-hexadecanoyl))hGLP-1(7-36)NH₂; | (SEQ ID NO.490) |
| (Ser⁸, Aib³⁵, Arg^{25,26}, Lys³⁴(N^{ε}-decanoyl))hGLP-1(7-36)NH₂; | (SEQ ID NO.491) |
| (Ser⁸, Aib³⁵, Lys²⁵, Arg²⁶, Lys³⁴(N^{ε}-octanoyl))hGLP-1(7-36)NH₂; | (SEQ ID NO.492) |
| (Ser⁸, Aib³⁵, Lys²⁵, Arg²⁶, Lys³⁴(N^{ε}-tetradecanoyl))hGLP-1(7-36)NH₂; | (SEQ ID NO.493) |
| (Ser⁸, Aib³⁵, Lys²⁵, Arg²⁶, Lys³⁴(N^{ε}-hexadecanoyl))hGLP-1(7-36)NH₂; | (SEQ ID NO.494) |
| (Ser⁸, Aib³⁵, Lys³⁶(N^{ε}-octanoyl))hGLP-1(7-36)NH₂; | (SEQ ID NO.495) |
| (Ser⁸, Aib³⁵, Lys³⁶(N^{ε}-tetradecanoyl))hGLP-1(7-36)NH₂ | (SEQ ID NO.496) |
| (Ser⁸ Aib³⁵, Lys³⁶(N^{ε}-hexadecanoyl))hGLP-1(7-36)NH₂; | (SEQ ID NO.497) |
| (Ser⁸, Aib³⁵, Arg²⁶, Lys³⁶(N^{ε}-octanoyl))hGLP-1(7-36)NH₂; | (SEQ ID NO.498) |
| (Ser⁸, Aib³⁵, Arg²⁶, Lys³⁶(N^{ε}-tetradecanoyl))hGLP-1(7-36)NH₂; | (SEQ ID NO.499) |
| (Ser⁸, Aib³⁵, Arg²⁶, Lys³⁶(N^{ε}-hexadecanoyl))hGLP-1(7-36)NH₂; | (SEQ ID NO.500) |
| (Ser⁸, Aib³⁵, Arg^{26,34}, Lys³⁶(N^{ε}-octanoyl))hGLP-1(7-36)NH₂; | (SEQ ID NO.501) |
| (Ser⁶, Aib³⁵, Arg^{26,34}, Lys³⁶(N^{ε}-hexadecanoyl))hGLP-1(7-36)NH₂; | (SEQ ID NO.502) |
| (Ser⁸, Aib³⁵, Arg^{26,34}, Lys³⁸(N^{ε}-octanoyl))hGLP-1(7-38)NH₂; | (SEQ ID NO.503) |
| (Ser⁸, Aib³⁵, Arg^{26,34}, Lys³⁸(N^{ε}-decanoyl))hGLP-1(7-38)NH₂; | (SEQ ID NO.504) |
| (Ser⁸, Aib³⁵, Arg^{26,34}, Lys³⁸(N^{ε}-tetradecanoyl))hGLP-1(7-38)NH₂; | (SEQ ID NO.505) |
| (Ser⁸, Aib³⁵, Arg^{26,34}, Lys³⁸(N^{ε}-hexadecanoyl))hGLP-1(7-38)NH₂; | (SEQ ID NO.506) |
| (Ser⁸, Aib^{35,37}, Arg^{25,26,34}, Lys³⁸(N^{ε}-octanoyl))hGLP-1(7-38)NH₂; | (SEQ ID NO.507) |
| (Ser⁸, Aib^{35,37}, Arg^{25,26,34}, Lys³⁸(N^{ε}-decanoyl)hGLP-1(7-38)NH₂; | (SEQ ID NO.508) |
| (Ser⁸, Aib^{35,37}, Arg^{25,26,34}, Lys³⁸(N^{ε}-tetradecanoyl))hGLP-1(7-38)NH₂; | (SEQ ID NO.509) |
| (Ser⁸, Aib^{35,37}, Arg^{25,26,34}, Lys³⁸(N^{ε}-hexadecanoyl))hGLP-1(7-38)NH₂; | (SEQ ID NO.510) |
| (Ser⁸, Aib^{35,37}, Arg^{26,34}, Lys³⁸(N^{ε}-octanoyl))hGLP-1(7-38)NH₂; | (SEQ ID NO.511) |
| (Ser⁸, Aib^{35,37}, Arg^{26,34}, Lys³⁸(N^{ε}-decanoyl))hGLP-1(7-38)NH₂; | (SEQ ID NO.512) |
| (Ser⁸, Aib^{35,37}, Arg^{26,34}, Lys³⁸(N^{ε}-hexadecanoyl))hGLP-1(7-38)NH₂; | (SEQ ID NO.513) |
| (Ser⁸, Aib³⁵, Lys²⁵, Arg^{26,34}, Lys³⁶(N^{ε}-octanoyl))hGLP-1(7-36)NH₂; | (SEQ ID NO.514) |
| (Ser⁸, Aib³⁵, Lys²⁵, Arg^{26,34}, Lys³⁶(N^{ε}-tetradecanoyl)) | |
| hGLP-1(7-36)NH₂; | (SEQ ID NO:515) |
| (Ser⁸, Aib³⁵, Lys²⁵, Arg^{26,34}, Lys³⁶(N^{ε}-hexadecanoyl)) | |
| hGLP-1(7-36)NH₂; | (SEQ ID NO.516) |
| (Ser⁸, Aib³⁵, Arg^{25,26,34}, Lys³⁶(N^{ε}-octanoyl))hGLP-1(7-36)NH₂; | (SEQ ID NO.517) |
| (Ser⁸, Aib³⁵, Arg^{25,26,34}, Lys³⁶(N^{ε}-tetradecanoyl))hGLP-1(7-36)NH₂; | (SEQ ID NO.518) |
| (Ser⁸, Aib³⁵, Arg^{25,26,34}, Lys³⁶(N^{ε}-hexadecanoyl))hGLP-1(7-36)NH₂; | (SEQ ID NO.519) |
| (Ser⁸, Aib³⁵, Arg^{25,26,34}, Lys³⁶(N^{ε}-decanoyl))hGLP-1(7-36)NH₂; | (SEQ ID NO.520) |
| (Ser⁸, Lys³⁴(N^{ε}-octanoyl), β-Ala³⁵)hGLP-1(7-36)NH₂; | (SEQ ID NO.521) |
| (Ser⁸, Lys³⁴(N^{ε}-tetradecanoyl), β-Ala³⁵)hGLP-1(7-36)NH₂; | (SEQ ID NO.522) |
| (Ser⁸, Lys³⁴(N^{ε}-hexadecanoyl), β-Ala³⁵)hGLP-1(7-36)NH₂; | (SEQ ID NO.523) |
| (Ser⁸, A6c³², Lys³⁴(N^{e}-octanoyl), β-Ala³⁵)hGLP-1(7-36)NH₂; | (SEQ ID NO.524) |
| (Ser⁸, Glu²³, Lys³⁴(N^{e}-octanoyl), β-Ala³⁵)hGLP-1(7-36)NH₂; | (SEQ ID NO.525) |
| (Ser⁸, Glu²³, A6c³², Lys³⁴(N^{e}-octanoyl), β-Ala³⁵)hGLP-1(7-36)NH₂; | (SEQ ID NO.526) |
| (Ser⁸, Arg²⁶, Lys³⁴(N^{ε}-octanoyl), β-Ala³⁵)hGLP-1(7-36)NH₂; | (SEQ ID NO.527) |
| (Ser⁸, Arg²⁶, Lys³⁴(N^{ε}-tetradecanoyl), β-Ala³⁵)hGLP-1(7-36)NH₂; | (SEQ ID NO.528) |
| (Ser⁸, Arg²⁶, Lys³⁴(N^{ε}-hexadecanoyl), β-Ala³⁵)hGLP-1(7-36)NH₂; | (SEQ ID NO.529) |
| (Ser⁸, Arg²⁶, Lys³⁴(N^{ε}-decanoyl), β-Ala³⁵)hGLP-1(7-36)NH₂; | (SEQ ID NO.530) |
| (Ser⁸, Arg^{25,26}, Lys³⁴(N^{ε}-octanoyl), β-Ala³⁵)hGLP-1(7-36)NH₂; | (SEQ ID NO.531) |
| (Ser⁸, Arg^{25,26}, Lys³⁴(N^{ε}-tetradecanoyl), β-Ala³⁵)hGLP-1(7-36)NH₂; | (SEQ ID NO.532) |
| (Ser⁸, Arg^{25,26}, Lys³⁴(N^{ε}-hexadecanoyl), β-Ala³⁵)hGLP-1(7-36)NH₂; | (SEQ ID NO.533) |
| (Ser⁸, Arg^{25,26}, Lys³⁴(N^{ε}-decanoyl), β-Ala³⁵)hGLP-1(7-36)NH₂; | (SEQ ID NO.534) |
| (Ser⁸, Lys²⁵, Arg²⁶, Lys³⁴(N^{ε}-octanoyl), β-Ala³⁵)hGLP-1(7-36)NH₂; | (SEQ ID NO.535) |
| (Ser⁸, Lys²⁵, Arg²⁶, Lys³⁴(N^{ε}-tetradecanoyl), β-Ala³⁵) | |
| hGLP-1(7-36)NH₂; | (SEQ ID NO.536) |
| (Ser⁸, Lys²⁵, Arg²⁶, Lys³⁴(N^{ε}-hexadecanoyl), β-Ala³⁵) | |
| hGLP-1(7-36)NH₂; | (SEQ ID NO.537) |
| (Ser⁸, β-Ala³⁵, Lys³⁶(N^{ε}-octanoyl))hGLP-1(7-36)NH₂; | (SEQ ID NO.538) |
| (Ser⁸, β-Ala³⁵, Lys³⁶(N^{ε}-tetradecanoyl))hGLP-1(7-36)NH₂; | (SEQ ID NO.539) |
| (Ser⁸, β-Ala³⁵, Lys³⁶(N^{ε}-hexadecanoyl))hGLP-1(7-36)NH₂; | (SEQ ID NO.540) |
| (Ser⁸, Arg²⁶, β-Ala³⁵, Lys³⁶(N^{ε}-octanoyl))hGLP-1(7-36)NH₂; | (SEQ ID NO.541) |
| (Ser⁸, Arg²⁶, β-Ala³⁵, Lys³⁶(N^{ε}-tetradecanoyl))hGLP-1(7-36)NH₂; | (SEQ ID NO.542) |
| (Ser⁸, Arg²⁶, β-Ala³⁵, Lys³⁶(N^{ε}-hexadecanoyl))hGLP-1(7-36)NH₂; | (SEQ ID NO.543) |
| (Ser⁸, Arg^{26,34}, β-Ala³⁵, Lys³⁶(N^{ε}-octanoyl))hGLP-1(7-36)NH₂; | (SEQ ID NO.544) |
| (Ser⁸, Arg^{26,34}, β-Ala³⁵, Lys³⁶(N^{ε}-tetradecanoyl))hGLP-1(7-36)NH₂; | (SEQ ID NO.545) |
| (Ser⁸, Arg^{26,34}, β-Ala³⁵, Lys³⁶(N^{ε}- hexadecanoyl))hGLP-1(7-36)NH₂; | (SEQ ID NO.546) |
| (Ser⁸, Arg^{26,34}, β-Ala³⁵, Lys³⁶(N^{ε}-decanoyl))hGLP-1(7-36)NH₂; | (SEQ ID NO.547) |
| (Ser⁸, Lys²⁵, Arg^{26,34}, β-Ala³⁵, Lys³⁶(N^{ε}-octanoyl))hGLP-1(7-36)NH₂; | (SEQ ID NO.548) |
| (Ser⁸, Lys²⁵, Arg^{26,34}, Lys³⁶(N^{ε}-tetradecanoyl), β-Ala³⁵) | |
| hGLP-1(7-36)NH₂; | (SEQ ID NO.549) |
| (Ser⁸, Lys²⁵, Arg^{26,34}, β-Ala³⁵, Lys³⁶(N^{ε}-hexadecanoyl)) | |
| hGLP-1 (7-36)NH₂; | (SEQ ID NO.550) |
| (Ser⁸, Arg^{25,26,34}, β-Ala³⁵, Lys³⁶(N^{ε}-octanoyl))hGLP-1(7-36)NH₂; | (SEQ ID NO.551) |
| (Ser⁸, Arg^{25,26,34}, β-Ala³⁵, Lys³⁶(N^{ε}-tetradecanoyl))hGLP-1(7-36)NH₂; | (SEQ ID NO.552) |
| (Ser⁸, Arg^{25,26,34}, β-Ala³⁵, Lys³⁶(N^{ε}-hexadecanoyl))hGLP-1(7-36)NH₂; | (SEQ ID NO.553) |
| (Ser⁸, Arg^{25,26,34}, β-Ala³⁵, Lys³⁶(N^{ε}-decanoyl))hGLP-1(7-36)NH₂; | (SEQ ID NO.554) |
| (Ser⁸, Aib³⁵, Lys²⁶(N^{ε}-octanoyl), A6c³², Arg³⁴)hGLP-1(7-36)NH₂; | (SEQ ID NO.555) |
| (Ser⁸, Aib³⁵, Lys²⁶(N^{ε}-tetradecanoyl), A6c³², Arg³⁴)hGLP-1(7-36)NH₂; | (SEQ ID NO.556) |
| (Ser⁸, Aib³⁵, Lys²⁶(N^{ε}-hexadecanoyl), A6c³², Arg³⁴)hGLP-1(7-36)NH₂; | (SEQ ID NO.557) |
| (Ser⁸, Aib³⁵, A6c³², Lys³⁴(N^{ε}-octanoyl))hGLP-1(7-36)NH₂; | (SEQ ID NO.558) |
| (Ser⁸, Aib³⁵, A6c³², Lys³⁴(N^{ε}-tetradecanoyl))hGLP-1(7-36)NH₂; | (SEQ ID NO.559) |
| (Ser⁸, Aib³⁵, A6c³², Lys³⁴(N^{ε}-hexadecanoyl))hGLP-1(7-36)NH₂; | (SEQ ID NO.560) |
| (Ser⁸, Aib³⁵, Arg²⁶, A6c³², Lys³⁴(N^{ε}-octanoyl))hGLP-1(7-36)NH₂; | (SEQ ID NO.561) |
| (Ser⁸, Aib³⁵, Arg²⁶, A6c³², Lys³⁴(N^{ε}-tetradecanoyl))hGLP-1(7-36)NH₂; | (SEQ ID NO.562) |
| (Ser⁸, Aib³⁵, A6c³², Lys³⁶(N^{ε}-octanoyl)hGLP-1(7-36)NH₂; | (SEQ ID NO.563) |
| (Ser⁸, Aib³⁵, A6c³², Lys³⁶(N^{ε}-tetradecanoyl))hGLP-1(7-36)NH₂; | (SEQ ID NO.564) |
| (Ser⁸, Aib³⁵, A6c³², Lys³⁶(N^{ε}-hexadecanoyl))hGLP-1(7-36)NH₂; | (SEQ ID NO.565) |
| (Ser⁸, Aib³⁵, Arg²⁶, A6c³², Lys³⁶(N^{ε}-octanoyl))hGLP-1(7-36)NH₂; | (SEQ ID NO.566) |
| (Ser⁸, Aib³⁵, Arg²⁶, A6c³², Lys³⁶(N^{ε}-tetradecanoyl))hGLP-1(7-36)NH₂; | (SEQ ID NO.567) |
| (Ser⁸, Aib³⁵, Arg²⁶, A6c³², Lys³⁶(N^{ε}-hexadecanoyl))hGLP-1(7-36)NH₂; | (SEQ ID NO.568) |
| (Ser⁸, Aib³⁵, Arg^{26,34}, A6c³², Lys³⁶(N^{ε}-octanoyl))hGLP-1(7-36)NH₂; | (SEQ ID NO.569) |
| (Ser⁸, Aib³⁵, Arg^{26,34}, A6c³², Lys³⁶(N^{ε}-decanoyl))hGLP-1(7-36)NH₂; | (SEQ ID NO.570) |
| (Ser⁸, Aib³⁵, Arg^{26,34}, A6c³², Lys³⁶(N^{ε}-tetradecanoyl)) | |
| hGLP-1(7-38)NH₂; | (SEQ ID NO.571) |
| (Ser⁸, Aib³⁵, Arg^{26,34}, A6c³², Lys³⁶(N^{ε}-hexadecanoyl)) | |
| hGLP-1(7-36)NH₂; | (SEQ ID NO.572) |
| (Ser⁸, Aib^{24,35}, Lys²⁶(N^{ε}-octanoyl), Arg³⁴)hGLP-1(7-36)NH₂; | (SEQ ID NO.573) |
| (Ser⁸, Aib^{24,35}, Lys²⁶(N^{ε}-tetradecanoyl), Arg³⁴ )hGLP-1(7-38))NH₂; | (SEQ ID NO.574) |
| (Ser⁸, Aib^{24,35}, Lys²⁶(N^{ε}-hexadecanoyl), Arg³⁴)hGLP-1(7-36)NH₂; | (SEQ ID NO.575) |
| (Ser⁸, Aib^{24,35}, Arg²⁶, Lys³⁴(N^{ε}-octanoyl))hGLP-1(7-36)NH₂; | (SEQ ID NO.576) |
| (Ser⁸, Aib^{24,35}, Arg²⁶, Lys³⁴(N^{ε}-tetradecanoyl))hGLP-1(7-36)NH₂; | (SEQ ID NO.577) |
| (Ser⁸, Aib^{24,35}, Arg²⁶, Lys³⁴(N^{ε}-hexadecanoyl))hGLP-1(7-36)NH₂; | (SEQ ID NO.578) |
| (Ser⁸, Aib^{24,35}, Arg^{26,34}, Lys³⁶(N^{ε}-octanoyl))hGLP-1(7-36)NH₂; | (SEQ ID NO.579) |
| (Ser⁸, Aib^{24,35}, Arg^{26,34}, Lys³⁶(N^{ε}-tetradecanoyl))hGLP-1(7-36)NH₂; | (SEQ ID NO.580) |
| (Ser⁸, Aib^{24,35}, Arg^{26,34}, Lys³⁶(N^{ε}-hexadecanoyl))hGLP-1(7-36)NH₂; | (SEQ ID NO.581) |
| (Ser⁸, Aib^{24,35}, Glu²³, A6c³², Lys³⁴(N^{e}-octanoyl))hGLP-1(7-36)NH₂; | (SEQ ID NO.582) |
| (Ser⁸, Aib³⁵, Glu²³, Lys²⁶(N^{ε}-octanoyl), Arg³⁴)hGLP-1(7-36)NH₂; | (SEQ ID NO.583) |
| (Ser⁸, Aib³⁵, Glu²³, Lys²⁶(N^{ε}-tetradecanoyl), Arg³⁴)hGLP-1(7-36)NH₂; | (SEQ ID NO.584) |
| (Ser⁸, Aib³⁵, Glu²³, Lys²⁶(N^{ε}-hexadecanoyl), Arg³⁴)hGLP-1(7-36)NH₂; | (SEQ ID NO.585) |
| (Ser⁸, Aib³⁵, Glu²³, Lys³⁴(N^{ε}-octanoyl))hGLP-1(7-36)NH₂; | (SEQ ID NO.586) |
| (Ser⁸, Aib³⁵, Glu²³, A6c³², Lys³⁴(N^{ε}-octanoyl))hGLP-1(7-36)NH₂; | (SEQ ID NO.587) |
| (Ser⁸, Aib³⁵, Glu²³, Arg²⁶, Lys³⁴(N^{ε}-octanoyl))hGLP-1(7-36)NH₂; | (SEQ ID NO.588) |
| (Ser⁸, Aib³⁵, Glu²³, Arg²⁶, Lys³⁴(N^{ε}-tetradecanoyl))hGLP-1(7-36)NH₂; | (SEQ ID NO.589) |
| (Ser⁸, Aib³⁵, Glu²³, Arg²⁶, Lys³⁴(N^{ε}-hexadecanoyl))hGLP-1(7-36)NH₂; | (SEQ ID NO.590) |
| (Ser⁸, Aib³⁵, Glu²³, Lys³⁶(N^{ε}-octanoyl))hGLP-1(7-36)NH₂; | (SEQ ID NO.591) |
| (Ser⁸, Alb³⁵, Glu²³, Lys³⁶(N^{ε}-tetradecanoyl))hGLP-1(7-36))NH₂; | (SEQ ID NO.592) |
| (Ser⁸, Aib³⁵, Glu²³, Lys³⁶(N^{ε}-hexadecanoyl))hGLP-1(7-36)NH₂; | (SEQ ID NO.593) |
| (Ser⁸, Aib³⁵, (Glu²³, Arg^{26,34}, Lys³⁶(N^{ε}-octanoyl))hGLP-1(7-36)NH₂; | (SEQ ID NO.594) |
| (Ser⁸, Aib³⁵, Glu²³, Arg^{26,34}, Lys³⁶(N^{ε}-tetradecanoyl)) | |
| hGLP-1(7-36)NH₂; | (SEQ ID NO.595) |
| (Ser⁸, Aib³⁵, Glu²³, Arg^{26,34}, Lys³⁶(N^{ε}-hexadecanoyl)) | |
| hGLP-1(7-36)NH₂; | (SEQ ID NO.596) |
| (Ser⁸, Aib^{30,35}, Lys²⁶(N^{ε}-octanoyl), Arg³⁴)hGLP-1(7-36)NH₂; | (SEQ ID NO.597) |
| (Ser⁸, Aib^{30,35}, Lys²⁶(N^{ε}-tetradecanoyl), Arg³⁴)hGLP-1(7-36)NH₂; | (SEQ ID NO.598) |
| (Ser⁸, Aib^{30,35}, Lys²⁶(N^{ε}-hexadecanoyl), Arg³⁴)hGLP-1(7-36)NH₂; | (SEQ ID NO.599) |
| (Ser⁸, Aib^{30,35}, Arg²⁶, Lys³⁴(N^{ε}-octanoyl))hGLP-1(7-36)NH₂; | (SEQ ID NO.600) |
| (Ser⁸, Aib^{30,35}, Arg²⁶, Lys³⁴(N^{ε}-tetradecanoyl))hGLP-1(7-36)NH₂; | (SEQ ID NO.601) |
| (Ser⁸, Aib^{30,35} Arg²⁶, Lys³⁴(N^{ε}-hexadecanoyl))hGLP-1(7-36)NH₂; | (SEQ ID NO.602) |
| (Ser⁸, Aib^{30,35}, Arg^{26,34}, Lys³⁶(N^{ε}-octanoyl))hGLP-1(7-36)NH₂; | (SEQ ID NO.603) |
| (Ser⁸, Aib^{30,35}, Arg^{26,34}, Lys³⁶(N^{ε}-tetradecanoyl))hGLP-1(7-36)NH₂; | (SEQ ID NO.604) |
| (Ser⁸, Aib^{30,35}, Arg^{26,34}, Lys³⁶(N^{ε}-hexadecanoyl))hGLP-1(7-36)NH₂; | (SEQ ID NO.605) |
| (Ser⁸, Aib³⁵, Glu²³, A6c³², Lys³⁶(N^{ε}-octanoyl))hGLP-1(7-36)NH₂; | (SEQ ID NO.606) |
| (Ser⁸, Aib³⁵, Glu²³, A6c³², Lys³⁶(N^{ε}-tetradecanoyl))hGLP-1(7-36)NH₂; | (SEQ ID NO.607) |
| (Ser⁸, Aib³⁵, Glu²³, A6c³², Lys³⁶(N^{ε}-hexadecanoyl))hGLP-1(7-36)NH₂; | (SEQ ID NO.608) |
| (Ser⁸, Aib³⁵, Glu²³, Arg^{26,34}, A6c³², Lys³⁶(N^{ε}-octanoyl)) | |
| hGLP-1(7-36)NH₂; | (SEQ ID NO.609) |
| (Ser⁸, Aib³⁵, Glu²³, Arg^{26,34}, A6c³², Lys³⁶(N^{ε}-tetradecanoyl)) hGLP-1(7-36)NH₂; | (SEQ ID NO.610) |
| (Ser⁸, Aib³⁵, Glu²³, Arg^{26,34}, A6c³², Lys³⁶(N^{ε}-hexadecanoyl)) hGLP-1(7-36)NH₂; | (SEQ ID NO.611) |
| (Ser⁸, Aib^{24,35}, Glu²³, Arg^{26,34}, A6c³², Lys³⁶(N^{ε}-octanoyl)) | |
| hGLP-1(7-36)NH₂; | (SEQ ID NO.612) |
| (Ser⁸, Aib^{24,35}, Glu²³, Arg^{26,34}, A6c³², Lys³⁶(N^{ε}-tetradecanoyl)) | |
| hGLP-1(7-36)NH₂ ; | (SEQ ID NO.613) |
| (Ser⁸, Aib^{24,35}, Glu²³, Arg^{26,34}, A6c³², Lys³⁶(N^{ε}-hexadecanoyl) | |
| hGLP-1(7-36)NH₂; | (SEQ ID NO.614) |
| (Ser⁸, Aib^{24,30,35}, Glu²³, Arg^{26,34}, A6c³², Lys³⁶(N^{ε}-octanoyl)) | |
| hGLP-1(7-36)NH₂; | (SEQ ID NO.615) |
| (Ser⁸, Aib^{24,30,35}, Glu²³, Arg^{26,34}, A6c³², Lys³⁶(N^{ε}-tetradecanoyl)) | |
| hGLP-1(7-36)NH₂; | (SEQ ID NO.616) |
| (Ser⁸, Aib^{24,30,35}, Glu²³, Arg^{26,34}, A6C³², Lys³⁶(N^{ε}-hexadecanoyl)) | |
| hGLP-1(7-36)NH₂; | (SEQ ID NO.617) |
| ((N^{α}-HEPES-His)⁷, Ser⁸, β-Ala³⁵)hGLP-1(7-36)NH₂; | (SEQ ID NO.618) |
| ((N^{α}-HEPA-His)⁷, Ser⁸, β-Ala³⁵)hGLP-1(7-36)NH₂; | (SEQ ID NO.619) |
| ((N^{α}-tetradecanoyl-His)⁷, Ser⁸, Aib³⁵)hGLP-1(7-36)NH₂; | (SEQ ID NO.620) |
| ((N^{α}-tetradecanoyl-His)⁷, Ser⁸, β-Ala³⁵)hGLP-1(7-36)NH₂; | (SEQ ID NO.621) |
| ((N^{α}-tetradecanoyl-His)⁷, Ser⁸, Aib³⁵, Arg^{26,34})hGLP-1(7-36)NH₂; | (SEQ ID NO.622) |
| ((N^{α}-tetradecanoyl-His)⁷, Ser⁸, Arg^{26,34}, β-Ala³⁵)hGLP-1(7-36)NH₂; | (SEQ ID NO.623) |
| ((N^{α}-tetradecanoyl-His)⁷, Ser⁸, Aib³⁵, Arg^{25,26,34})hGLP-1(7-36)NH₂; | (SEQ ID NO.624) |
| ((N^{α}-tetradecanoyl-His)⁷, Ser⁸, Arg^{25,26,34}, β-Ala³⁵)hGLP-1(7-36)NH₂; | (SEQ ID NO.625) |
| (Ser⁸, Aib³⁵, Lys²⁶(N^{ε}-octanesulfonyl), Arg³⁴)hGLP-1(7-36)NH₂; | (SEQ ID NO.626) |
| (Ser⁸, Aib³⁵, Lys²⁶(N^{ε}-dodecanesulfonyl), Arg³⁴)hGLP-1(7-36)NH₂; | (SEQ ID NO.627) |
| (Ser⁸, Aib³⁵, Lys²⁶(N^{ε}-hexadecanesulfonyl), Arg³⁴)hGLP-1(7-36)NH₂; | (SEQ ID NO.628) |
| (Ser⁸, Aib³⁵, Arg²⁶, Lys³⁴(N^{ε}-octanesulfonyl)hGLP-1(7-36)NH₂; | (SEQ ID NO.629) |
| (Ser⁸, Aib³⁵, Arg²⁶, Lys³⁴(N^{ε}-dodecanesulfonyl))hGLP-1(7-36)NH₂; | (SEQ ID NO.630) |
| (Ser⁸, Aib⁵, Arg²⁶, Lys³⁴(N^{ε}-hexadecanesulfonyl)hGLP-1(7-36)NH₂; | (SEQ ID NO.631) |
| (Ser⁸, Aib³⁵, Arg^{26,34}, Lys³⁶(N^{ε}-octanesulfonyl))hGLP-1(7-36)NH₂; | (SEQ ID NO.632) |
| (Ser⁸, Aib³⁵, Arg^{26,34}, Lys³⁶(N^{ε}-hexadecanesulfonyl)) hGLP-1(7-36)NH₂; | (SEQ ID NO.633) |
| (Ser⁸, Aib³⁵, Asp²⁶(1-(4-decylpiperazine)), Arg³⁴)hGLP-1(7-36)NH₂; | (SEQ ID NO.634) |
| (Ser⁸, Aib³⁵, Asp²⁶(1-(4-dodecylpiperazine)), Arg³⁴)hGLP-1(7-36)NH₂; | (SEQ ID NO.635) |
| (Ser⁸, Aib³⁵, Asp²⁶(1-(4-tetradecylpiperazine)), Arg³⁴) hGLP-1(7-36)NH₂; | (SEQ ID NO.636) |
| (Ser⁸, Aib³⁵, Asp²⁶(1-(4-hexadecylpiperazine)), Arg³⁴) hGLP-1(7-36)NH₂; | (SEQ ID NO.637) |
| (Ser⁸, Aib³⁵, Arg²⁶, Asp³⁴(1-(4-decylpiperazine)))hGLP-1(7-36)NH₂; | (SEQ ID NO.638) |
| (Ser⁸, Aib³⁵, Arg²⁶, Asp³⁴(1-(4-dodecylpiperazine)))hGLP-1(7₋36)NH₂; | (SEQ ID NO.639) |
| (Ser⁸, Aib³⁵, Arg²⁶, Asp³⁴(1-(4-tetradecylpiperazine))) hGLP-1(7-36)NH₂; | (SEQ ID NO.640) |
| (Ser⁸, Aib³⁵, Arg²⁶, Asp³⁴(1-(4-hexadecylpiperazine))) hGLP-1(7-36)NH₂; | (SEQ ID NO.641) |
| (Ser⁸, Aib³⁵, Arg^{26,34}, Asp³⁶(1-(4-decylpiperazine)))hGLP-1(7-36)NH₂: | (SEQ ID NO.642) |
| (Ser⁸, Aib³⁵, Arg^{26,34}, Asp³⁶(1-(4-dodecylpiperazine))) hGLP-1(7-36)NH₂; | (SEQ ID NO.643) |
| (Ser⁸, Aib³⁵, Arg^{26,34}, Asp³⁶(1-(4-hexadecylpiperazine))) | |
| hGLP-1(7-36)NH₂; | (SEQ ID NO.644) |
| (Ser⁸, Aib³⁵ , Arg^{26,34} Asp³⁸(1-(4-decylpiperazine)))hGLP-1(7-38)NH₂; | (SEQ ID NO.645) |
| (Ser⁸, Aib³⁵, Arg^{26,34}, Asp³⁸(1-(4-dodecylpiperazine))) | |
| hGLP-1(7-38)NH₂; | (SEQ ID NO.646) |
| (Ser⁸, Aib³⁵, Arg^{26,34}, Asp³⁸(1-(4-tetradecylpiperazine))) | |
| hGLP-1(7-38)NH₂; | (SEQ ID NO.647) |
| (Ser⁸, Aib³⁵, Arg^{26,34}, Asp³⁸(1-(4-hexadecylpiperazine))) | |
| hGLP-1(7-38)NH₂; | (SEQ ID NO.648) |
| (Ser⁸, Aib^{35,37}, Arg^{26,34}, Asp³⁸(1-(4-decylpiperazine))) | |
| hGLP-1(7-38)NH₂; | (SEQ ID NO.649) |
| (Ser⁸, Aib^{35,37}, Arg^{26,34}, Asp³⁸(1-(4-dodecylpiperazine))) | |
| hGLP-1(7-38)NH₂; | (SEQ ID NO.650) |
| (Ser⁸, Aib^{35,37}, Arg^{26,34} Asp³⁸(1-(4-tetradecylpiperazine))) | |
| hGLP-1(7-38)NH₂; | (SEQ ID NO.651) |
| (Ser⁸, Aib^{35,37}, Arg^{26,34}, Asp³⁸(1-(4-hexadecylpiperazine))) | |
| hGLP-1(7-38)NH₂; | (SEQ ID NO.652) |
| (Ser⁸, Aib³⁵, Arg^{25,34}, Asp²⁶(1-(4-decylpiperazine)))hGLP-1(7-36)NH₂; | (SEQ ID NO.653) |
| (Ser⁸, Aib³⁵, Arg^{25,34}, Asp²⁶(1-(4-dodecylpiperazine))) | |
| hGLP-1(7-36)NH₂; | (SEQ ID NO.654) |
| (Ser⁸, Aib³⁵, Arg^{25,34}, Asp²⁶(1-(4-tetradecylpiperazine))) | |
| hGLP-1(7-36)NH₂; | (SEQ ID NO.655) |
| (Ser⁸, Aib³⁵, Arg^{25,34}, Asp²⁶(1-(4-hexadecylpiperazine))) | |
| hGLP-1(7-36)NH₂; | (SEQ ID NO.656) |
| (Ser⁸, Aib³⁵, Arg^{25,26}, Asp³⁴(1-(4-decylpiperazine)))hGLP-1(7-36)NH₂; | (SEQ ID NO.657) |
| (Ser⁸, Aib³⁵, Arg^{25,26}, Asp³⁴(1-(4-dodecylpiperazine))) | |
| hGLP-1(7-36)NH₂; | (SEQ ID NO.658) |
| (Ser⁸, Aib³⁵, Arg^{25,26}, Asp³⁴(1-(4-tetradecylpiperazine))) | |
| hGLP-1(7-36)NH₂; | (SEQ ID NO.659) |
| (Ser⁸, Aib³⁵, Arg^{25,26}, Asp³⁴(1-(4-hexadecylpiperazine))) | |
| hGLP-1(7-36)NN₂; | (SEQ ID NO.660) |
| (Ser⁸, Aib³⁵, Arg^{25,26,34}, Asp³⁶(1-(4-decylpiperazine))) | |
| hGLP-1(7-36)NH₂; | (SEQ ID NO.661) |
| (Ser⁸, Aib³⁵, Arg^{25,26,34}, Asp³⁶(1-(4-dodecylpiperazine)))hGLP-1(7-36) | |
| NH₂; | (SEQ ID NO.662) |
| (Ser⁸, Aib³⁵, Arg^{25,26,34}, Asp³⁶(1-(4-tetradecylpiperazine))) | |
| hGLP-1(7-36)NH₂; | (SEQ ID NO.663) |
| (Ser⁸, Aib³⁵, Arg^{25,26,34}, Asp³⁶(1-(4-hexadecylpiperazine))) | |
| hGLP-1(7-36)NH₂; | (SEQ ID NO.664) |
| (Ser⁸, Aib³⁵, Arg^{25,26,34}, Asp³⁸(1-(4-decylpiperazine))) | |
| hGLP-1(7-38)NH₂; | (SEQ ID NO.665) |
| (Ser⁸, Aib³⁵, Arg^{25,26,34}, Asp³⁸(1-(4-dodecylpiperazine))) | |
| hGLP-1(7-38)NH₂; | (SEQ ID NO.666) |
| (Ser⁸, Aib³⁵, Arg^{25,26,34}, Asp³⁸(1-(4-tetradecylpiperazine))) | |
| hGLP-1(7-38)NH₂; | (SEQ ID NO.667) |
| (Ser⁸, Ab³⁵, Arg^{25,26,34} Asp³⁸(1-(4-hexadecylpiperazine))) | |
| hGLP-1(7-38)NH₂; | (SEQ ID NO.668) |
| (Ser⁸, Aib^{35,37}, Arg^{25,26,34}, Asp³⁸(1-(4-decylpiperazine))) | |
| hGLP-1(7-38)NH₂; | (SEQ ID NO.669) |
| (Ser⁸, Aib^{35,37}, Arg^{25,26,34}, Asp³⁸(1-(4_cloclecyipiperazine))) | |
| hGLP-1(7-38)NH₂; | (SEQ ID NO.670) |
| (Ser⁸, Aib^{35,37}, Arg^{25,26,34}, Asp³⁸(1-(4-tetradecylpiperazine))) | |
| hGLP-1(7-38)NH₂; | (SEQ ID NO.671) |
| (Ser⁸, Aib^{35,37}, Arg^{25,26,34}, Asp³⁸(1-(4-hexadecylpiperazine))) | |
| hGLP-1(7-38)NH₂; | (SEQ ID NO.672) |
| (Ser⁸, Aib³⁵, Arg^{26,34}, Glu³⁶(1-dodecylamino))hGLP-1(7-36)NH₂; | (SEQ ID NO.673) |
| (Ser⁸, Aib³⁵, Glu²⁶(1-dodecylamino), Arg³⁴)hGLP-1(7-36)NH₂; | (SEQ ID NO.674) |
| (Ser⁸, Aib³⁵, Arg²⁶, Glu³⁴(1-dodecylamino))hGLP-1(7-36)NH₂; | (SEQ ID NO.675) |
| (Ser⁸, Aib^{35,37}, Arg^{26,34}, Glu³⁸(1-dodecylamino))hGLP-1(7-38)NH₂; | (SEQ ID NO.676) |
| (Ser⁸, Aib³⁵, Arg³⁴, Lys²⁶(N^{ε}-(2-(4-decyl-1-piperazine)-acetyl)) | |
| hGLP-1(7-36)NH₂; | (SEQ ID NO.677) |
| (Ser⁸, Aib³⁵, Arg³⁴, Lys²⁶(N^{ε}-(2-(4-dodecyl-1-piperazine)-acetyl))) | |
| hGLP-1(7-36)NH₂; | (SEQ ID NO.678) |
| (Ser⁸, Aib³⁵, Arg³⁴, Lys²⁶(N^{ε}-(2-(4-tetradecyl-1-piperazine)-acetyl))) | |
| hGLP-1(7-36)NH₂; | (SEQ ID NO.679) |
| (Ser⁸, Aib³⁵, Arg³⁴, Lys²⁶(N^{ε}-(2-(4-hexadecyl-1-piperazine)-acetyl))) | |
| hGLP-1(7-36)NH₂; | (SEQ ID NO.680) |
| (Ser⁸, Aib³⁵, Arg²⁶, Lys³⁴(N^{ε}-(2-(4-decyl-1-piperazine)-acetyl))) | |
| hGLP-1(7-36)NH₂; | (SEQ ID NO.681) |
| (Ser⁸, Aib³⁵, Arg²⁶, Lys³⁴(N^{ε}-(2-(4-dodecyl-1-piperazine)-acetyl)) | |
| hGLP-1(7-36)NH₂; | (SEQ ID NO.682) |
| (Ser⁸, Aib³⁵, Arg²⁶, Lys³⁴(N^{ε}-(2-(4-tetradecyl-1-piperazine)-acetyl))) | |
| hGLP-1(7-36)NH₂; | (SEQ ID NO.683) |
| (Ser⁸, Aib³⁵, Arg²⁶, Lys³⁴(N^{ε}-(2-(4-hexadecyl-1-piperazine)-acetyl))) | |
| hGLP-1(7-36)NH₂; | (SEQ ID NO.684) |
| (Ser⁸, Aib³⁵, Arg^{26,34}, Lys³⁶(N^{ε}-(2-(4-decy(-1-piperazine)-acetyl))) | |
| hGLP-1(7-36)NH₂; | (SEQ ID NO.685) |
| (Ser⁸, Aib³⁵, Arg^{26,34}, Lys³⁶(N^{ε}-(2-(4-dodecyl-1-piperazine)-acetyl))) | |
| hGLP-1(7-36)NH₂; | (SEQ ID NO.686) |
| (Ser⁸, Aib³⁵, Arg^{26,34}, Lys³⁶(N^{ε}-(2-(4-hexadecyl-1-piperazine)-acetyl))) | |
| hGLP-1(7-36)NH₂; | (SEQ ID NO.687) |
| (Ser⁸, Aib³⁵, Arg^{26,34}, Lys³⁸(N^{ε}-(2-(4-decyl-1-piperazine)-acetyl))) | |
| hGLP-1(7-38)NH₂; | (SEQ ID NO.688) |
| (Ser⁸, Aib³⁵, Arg^{26,34}, Lys³⁶(N^{ε}-(2-(4-dodecyl-1-piperazine)-acetyl))) | |
| hGLP-1(7-38)NH₂; | (SEQ ID NO.689) |
| (Ser⁵, Aib³⁵, Arg^{26,34}, Lys³⁸(N^{ε}-(2-(4-tetradecyl-1-piperazine)-acetyl)) | |
| )hGLP-1(7-38)NH₂; | (SEQ ID NO.690) |
| (Ser⁸, Aib³⁵, Arg^{26,34}, Lys³⁸(N^{ε}-(2-(4-hexadecyl-1-piperazine)-acetyl))) | |
| hGLP-1(7-38)NH₂; | (SEQ ID NO.691) |
| (Ser⁸, Aib^{35,37}, Arg^{26,34}, Lys³⁸(N^{ε}-(2-(4-decyl-1-piperazine)-acetyl))) | |
| hGLP-1(7-38)NH₂; | (SEQ ID NO.692) |
| (Ser⁸, Aib^{35,37}, Arg^{26,34}, Lys³⁸(N^{ε}-(2-(4-clodecyl-1-piperazine)-acetyl))) | |
| hGLP-1(7-38)NH₂; | (SEQ ID NO.693) |
| (Ser⁸, Aib^{35,37}, Arg^{26,34}, Lys³⁸(N^{ε}-(2-(4-tetradecyl-1-piperazine)-acetyl))) | |
| hGLP-1(7-38)NH₂; | (SEQ ID NO.694) |
| (Ser⁸, Aib^{35,37}, Arg^{26,34}, Lys³⁸(N^{ε}-(2-(4-hexadecyl-l-piperazine)-acetyl))) | |
| hGLP-1(7-38)NH₂; | (SEQ ID NO.695) |
| (Ser⁶, Aib³⁵, Arg^{25,34}, Lys²⁶(N^{ε}-(2-(4-decyt-1-piperazine)-acetyl))) | |
| hGLP-1(7-36)NH₂; | (SEQ ID NO.696) |
| (Ser⁸, Aib³⁵, Arg^{25,34}, Lys²⁶(N^{ε}-(2-(4-dodecyl-1-piperazine)-acetyl))) | |
| hGLP-1(7-36)NH₂; | (SEQ ID NO.697) |
| (Ser⁸, Aib³⁵, Arg^{25,34}, Lys²⁶(N^{ε}-(2-(4-tetradecyl-1-piperazine)-acetyl)) | |
| hGLP-1(7-36)NH₂; | (SEQ ID NO.698) |
| (Ser⁸, Aib³⁵, Ag^{25,34}, Lys²⁶(N^{ε}-(2-(4-hexadecyl-1-piperazine)-acetyl))) | |
| hGLP-1(7-36)NH₂; | (SEQ ID NO.699) |
| (Ser⁸, Aib³⁵, Arg^{25,26}, Lys³⁴(N^{ε}-(2-(4-decyl-1-piperazine)-acetyl))) | |
| hGLP-1(7-36)NH₂: | (SEQ ID NO.700) |
| (Ser⁸, Aib³⁵, Arg^{25,26}, Lys³⁴(N^{ε}-(2-(4-dodecyl-1-piperazine)-acetyl))) | |
| hGLP-1(7-36)NH_{2;} | (SEQ ID NO.701) |
| (Ser⁸, Aib³⁵, Arg^{25,26}, Lys³⁴(N^{ε}-(2-(4-tetradecyl-1-piperazine)-acetyl))) | |
| hGLP-1(7-36)NH₂; | (SEQ ID NO.702) |
| (Ser⁸, Aib³⁵, Arg^{25,26}, Lys³⁴(N^{ε}-(2-(4-hexadecyl-1-piperazine)-acetyl))) | |
| hGLP-1(7-36)NH₂; | (SEQ ID NO.703) |
| (Ser⁸, Aib³⁵, Arg^{25,26,34}, Lys³⁶(N^{ε}-(2-(4-decyl-1-piperazine)-acetyl))) | |
| hGLP-1(7-36)NH₂; | (SEQ ID NO.704) |
| (Ser⁸, Aib³⁵, Arg^{25,26,34}, Lys³⁶(N^{ε}-(2-(4-dodecyl-1-piperazine)-acety))) | |
| hGLP-1(7-36)NH₂; | (SEQ ID NO.705) |
| (Ser⁸, Aib³⁵, Arg^{25,26,34}, Lys³⁶(N^{ε}-(2-(4-tetradecyl-1-piperazine)-acetyl))) | |
| hGLP-1(7-36)NH₂; | (SEQ ID NO.706) |
| (Ser⁸, Aib³⁵, Arg^{25,26,34}, Lys³⁶(N^{ε}-(2-(4-hexadecyl-1-piperazine)-acetyl))) | |
| hGLP-1(7-36)NH₂; | (SEQ ID NO.707) |
| (Ser⁸, Aib³⁵, Arg^{25,26,34}, Lys³⁸(N^{ε}-(2-(4-decyl-piperazine)-acetyl))) | |
| hGLP-1(7-38)NH₂; | (SEQ ID NO.708) |
| (Ser⁸, Aib³⁵, Arg^{25,26,34}, Lys³⁸(N^{ε}-(2-(4-dodecyl-1-piperazine)-acetyl))) | |
| hGLP-1(7-38)NH₂; | (SEQ ID NO.709) |
| (Ser⁸, Aib³⁵, Arg^{25,26,34}, Lys³⁸(N^{ε}-(2-(4-tetradecyl-1-piperazine)-acetyl))) | |
| hGLP-1 (7-38)NH₂; | (SEQ ID NO.710) |
| (Ser⁸, Aib³⁵, Arg^{25,26,34}, Lys³⁸(N^{ε}-(2-(4-hexadecyl-1-piperazine)-acetyl))) | |
| hGLP-1 (7-38)NH₂; | (SEQ ID NO.711) |
| (Ser⁸, Aib^{35,37}, Arg^{25,26,34}, Lys³⁸(N^{ε}-(2-(4-decyl-1-piperazine)-acetyl))) | |
| hGLP-1(7-38)NH₂; | (SEQ ID NO.712) |
| (Ser⁸, Aib^{35,37}, Arg^{25,26,34}, Lys³⁸(N^{ε}-(2-(4-dodecyl-1-piperazine)-acetyl))) | |
| hGLP-1(7-38)NH₂; | (SEQ ID NO.713) |
| (Ser⁸, Aib^{35,37}, Arg^{25,26,34}, Lys³⁸(N^{ε}-(2-(4-tetradecyl-1-piperazine)-acetyl))) | |
| hGLP-1(7-38)NH₂; | (SEQ ID NO.714) |
| (Ser⁸, Aib^{35,37}, Arg^{25,26,34}, Lys³⁸(N^{ε}-2-(4-hexadecyl-1-piperazine)-acetyl))) | |
| hGLP-1(7-38)NH₂; | (SEQ ID NO.715) |
| (Ser⁸, Aib³⁵, Arg^{26,34}, Lys³⁶(N^{ε}-decanoyl))hGLP-1(7-36)OH; | (SEQ ID NO.716) |
| (Ser⁸, Aib³⁵, Lys²⁵, Arg^{26,34}, Lys³⁶(N^{ε}-decanayl))hGLP-1(7-36)OH; | (SEQ ID NO.717) |
| (Ser⁸, Aib³⁵,Arg^{26,34}, Ava³⁷, Ado³⁸)hGLP-1(7-38)NH₂; | (SEQ ID NO.718) |
| (Ser⁸, Aib³⁵,Arg^{26,34}, Asp³⁷, Ava³⁸, Ado³⁹)hGLP-1(7-39)NH₂; | (SEQ ID NO.719) |
| (Ser⁸, Aib³⁵,Arg^{26,34}, Aun³⁷)hGLP-1(7-37)NH₂; | (SEQ ID NO.720) |
| (Ser⁸, Aib^{17,35},)hGLP-1(7-36)NH₂; | (SEQ ID NO.721) |
| (Ser⁸ ,Arg^{26,34}, β-Ala³⁵, D-Asp³⁷, Ava³⁶, Aun³⁹)hGLP-1(7-39)NH₂; | (SEQ ID NO.722) |
| (Ser⁸, Glu^{22,23}, β-Ala³⁵)hGLP-1(7-36)NH₂; | (SEQ ID NO.723) |
| (Ser⁸, Lys¹⁸, β-Ala³⁵)hGLP-1(7-36)NH₂; | (SEQ ID NO.724) |
| (Ser⁸, Leu²⁷, β-Ala³⁵)hGLP-1(7-36)NH₂; | (SEQ ID NO. 725) |
| (Ser⁸, Lys³³, β-Ala³⁵)hGLP-1(7-36)NH₂; | (SEQ ID NO.726) |
| (Ser⁸, Lys¹⁸, Leu²⁷, β-Ala³⁵)hGLP-1(7-36)NH₂; | (SEQ ID NO.727) |
| (Ser⁸, D-Arg³⁶)hGLP-1(7-36)NH₂; | (SEQ ID NO.728) |
| (Ser⁸, β-Ala³⁵, D-Arg³⁷)hGLP-1(7-37)NH₂; | (SEQ ID NO.729) |
| (Ser⁸, Aib²⁷, β-Ala³⁵)hGLP-1(7-36)NH₂; | (SEQ ID NO.730) |
| (Ser⁸, Aib²⁷, β-Ala^{35,37}, Arg³⁸)hGLP-1(7-38)NH₂; | (SEQ lD NO.731) |
| (Ser⁸, Aib²⁷, β-Ala^{35,37}, Arg^{38,39})hGLP-1(7-39)NH₂; | (SEQ ID NO.732) |
| (Ser⁸, Lys^{18,27}, β-Ala³⁵)hGLP-1(7-36)NH₂; | (SEQ ID NO.733) |
| (Ser⁸, Lys²⁷, β-Ala³⁵)hGLP-1(7-36)NH₂; | (SEQ ID NO.734) |
| (Ser⁸, β-Ala³⁵, Arg³⁸)hGLP-1(7-38)NH₂; | (SEQ ID NO.735) |
| (Ser⁸, Arg^{26,34}, β-Ala³⁵,)hGLP-1(7-36)NH₂; | (SEQ ID NO.736) |
| (Ser⁸, D-Arg³⁵)hGLP-1(7-36)NH₂; | (SEQ ID NO.737) |
| (Ser⁸, β-Ala³⁵, Arg³⁷)hGLP-1(7-37)NH₂; | (SEQ ID NO.738) |
| (Ser⁸, Phe³¹; β-Ala³⁵)hGLP-1(7-36)NH₂; | (SEQ ID NO.739) |
| (Ser⁸, Aib³⁵, Phe³¹)hGLP-1(7-36)NH₂; | (SEQ ID NO.740) |
| (Ser⁸, Aib³⁵, 2-Nal³¹)hGLP-1(7-36)NH₂; | (SEQ ID NO.741) |
| (Ser⁸, Aib³⁵, 2-Nal^{28,31})hGLP-1(7-36)NH₂; | (SEQ ID NO.742) |
| (Ser⁸, Aib³⁵, Arg^{26,34}, 2-Nal³¹)hGLP-1(7-36)NH₂; | (SEQ ID NO.743) |
| (Ser⁸, Aib³⁵, Arg^{26,34}, Phe³¹)hGLP-1(7-36)NH₂; | (SEQ ID NO.744) |
| (Ser⁸, Aib³⁵, 2-Nal^{19,31})hGLP-1(7-36)NH₂; | (SEQ ID NO.745) |
| (Ser⁸, Aib³⁵, 2-Na^{12,31})hGLP-1(7-36)NH₂; | (SEQ ID NO.746) |
| (Ser⁸, Aib³⁵, Lys³⁶(N^{ε}-decanoyl))hGLP-1(7-36)NH₂; | (SEQ ID NO.747) |
| (Ser⁸, Aib³⁵; Arg³⁴, Lys²⁶(N^{ε}-decanoyl))hGLP-1(7-36)NH₂; | (SEQ ID NO.748) |
| (Ser⁸, Aib³⁵, Arg^{26,34}, Lys³⁶(N^{ε}-dodecanoyl))hGLP-1(7-36)NH₂; | (SEQ ID NO.749) |
| (Ser⁸, β-Ala³,Ser³⁷(O-decanoyl))hGLP1(7-37)-NH₂; | (SEQ ID NO.750) |
| (Ser⁸, Aib²⁷, β-Ala^{35,37}, Arg³⁸, Lys³⁹(N^{ε}-octanoyl))hGLP-1(7-39)NH₂; | (SEQ ID NO.751) |
| (Ser⁸, Arg^{26,34}, β-Ala³⁵, Lys³⁷(N^{ε}-octanoyl))hGLP-1(7-37)NH₂; | (SEQ ID NO.752) |
| (Ser⁸, Arg^{26,34}, β-Ala³⁵, Lys³⁷(N^{ε}-decanoyl))hGLP-1(7-37)NH₂; | (SEQ ID NO.753) |
| (Ser⁸, Arg^{26,34}, β-Ala³⁵, Lys³⁷(N^{ε}-tetradecanoyl))hGLP-1 (7-37)NH₂; | (SEQ ID NO.754) |
| (Ser⁸, Arg^{26,34}, β-Ala³⁵, Lys³⁷(N^{ε}-dodecanoyl))hGLP-1(7-37)NH₂; | (SEQ ID NO.755) |
| (Ser⁸, Arg^{26,34}, β-Ala³⁵, Lys³⁷(N^{ε}-dodecanoyl))hGLP-1(8-37)NH₂; | (SEQ ID NO.756) |
| (Ser⁸, Arg^{26,34}, β-Ala³⁵, Lys³⁶(N^{ε}-Aec-decanoyl))hGLP-1(7-36)NH₂; | (SEQ ID NO.757) |
| (Ser⁸, Arg²⁶, Aib³⁵)hGLP-1(7-36)NH₂; | (SEQ ID NO.758) |
| (Ser⁸, Arg²⁶, Phe³¹, Aib³⁵)hGLP-1(7-36)NH₂; | (SEQ ID NO.759) |
| (Ser⁸, Arg²⁶, Phe³¹, β-Ala³⁵)hGLP-1(7-36)NH₂; | (SEQ ID NO.760) |
| (Ser⁸, Arg²⁶, β-Ala³⁵)hGLP-1(7-35)NH₂; | (SEQ ID NO.761) |
| (Ser⁸, Arg^{26,34}, Phe³¹, β-Ala³⁵)hGLP-1(7-36)NH₂; | (SEQ ID NO.762) |
| (Ser⁸, Arg³⁴, Aib³⁵)hGLP-1(7-36)NH₂; | (SEQ ID NO.763) |
| (Ser⁸, Arg³⁴, β-Ala³⁵)hGLP-1(7-36)NH₂; | (SEQ ID NO.764) |
| (Ser⁸, Phe³¹, Arg³⁴, Aib³⁵)hGLP-1(7-36)NH₂; | (SEQ ID NO.765) |
| or (Ser⁸, Phe³¹, Arg³⁴, β-Ala³⁵)hGLP-1(7-36)NH₂; | (SEQ ID NO.766) |

or a pharmaceutically acceptable salt thereof.
Paragraph 13. A compound according to Paragraph 11 wherein said compound is:

| | |
|---|---|
| (Gly⁸, Aib³⁵, A6c³²)hGLP-1(7-36)NH₂; | (SEQ ID NO.8) |
| (Gly⁸, Aib³⁵, Glu²³)hGLP-1(7-36)NH₂; | (SEQ ID NO.10) |
| (Gly⁸, Aib^{24,35})hGLP-1(7-36)NH₂; | (SEQ ID NO.11) |
| (Gly⁸, Aib³⁵, Glu²³, A6c³²)hGLP-1(7-36)NH₂; | (SEQ ID NO.28) |
| (Gly⁸, Glu²³, β-Ala³⁵)hGLP-1(7-36)NH₂; | (SEQ ID NO.34) |
| (Gly⁸, Aib³⁵, Arg^{26,34})hGLP-1(7-36)NH₂; | (SEQ ID NO.59) |
| (Gly⁸, Aib³⁵, Arg^{26,34}, Lys³⁶(N^{ε}-octanoyl))hGLP-1(7-36)NH₂; | (SEQ ID NO.117) |
| (Gly⁸, Aib³⁵, Arg^{26,34}, Lys³⁶(N^{ε}-decanoyl))hGLP-1(7-36)OH; | (SEQ ID NO.332) |
| (Gly⁸, Aib³⁵, Lys²⁵, Arg^{26,34}, Lys³⁶(N^{ε}-decanoyl))hGLP-1(7-36)OH; | (SEQ ID NO.333) |
| (Gly⁸, Arg^{26,34}, β-Ala³⁵, Lys³⁶(N^{ε}-Aec-decanoyl))hGLP-1(7-36)NH₂; | (SEQ ID NO.383) |
| (Gly⁸, Aib³⁵,Arg^{26,34}, Ava³⁷, Ado³⁸)hGLP-1(7-38)NH₂; | (SEQ ID NO.334) |
| (Gly⁸, Aib³⁵,Arg^{26,34}, Asp³⁷, Ava³⁸, Ado³⁹)hGLP-1(7-39)NH₂; | (SEQ ID NO.335) |
| (Gly⁸, Aib³⁵,Arg^{26,34}, Aun³⁷)hGLP-1(7-37)NH₂; | (SEQ ID NO.336) |
| (Gly⁸, Aib^{17,35})hGLP-1(7-36)NH₂; | (SEQ ID NO.337) |
| (Gly⁸ ,Ar^{26,34}, β-Ala³⁵, D-Asp³⁷, Ava³⁸, Aun³⁹)hGLP-1(7-39)NH₂; | (SEQ ID NO.338) |
| (Gly⁸, Glu^{22,23}, β-Ala³⁵)hGLP-1(7-36)NH₂; | (SEQ ID NO.339) |
| (Gly⁸, Lys¹⁸, β-Ala³⁵)hGLP-1(7-36)NH₂; | (SEQ ID NO.340) |
| (Gly⁸, Leu²⁷, β-Ala³⁵)hGLP-1(7-36)NH₂; | (SEQ ID NO.341) |
| (Gly⁸, Lys³³, β-Ala³⁵)hGLP-1(7-36)NH₂; | (SEQ ID NO.342) |
| (Gly⁸, Lys¹⁸, Leu²⁷, β-Ala³⁵)hGLP-1(7-36)NH₂; | (SEQ ID NO.343) |
| (Gly⁸, D-Arg³⁶)hGLP-1(7-36)NH₂; | (SEQ ID NO.344) |
| (Gly⁸, β-Ala³⁵, D-Arg³⁷)hGLP-1(7-37)NH₂; | (SEQ ID NO.345) |
| (Gly⁸, Aib²⁷, β-Ala³⁵)hGLP-1(7-36)NH₂; | (SEQ ID NO.346) |
| (Gly⁸, Aib²⁷, β-Ala^{35,37}, Arg³⁸)hGLP-1(7-38)NH₂; | (SEQ ID NO.347) |
| (Gly⁸, Aib²⁷, β-Ala^{35,37}, Arg^{38,39})hGLP-1(7-39)NH₂; | (SEQ ID NO.348) |
| (Gly⁸, Lys^{18,27}, β-Ala³⁵)hGLP-1(7-36)NH₂; | (SEQ ID NO.349) |
| (Gly⁸, Lys²⁷, β-Ala³⁵)hGLP-1(7-36)NH₂; | (SEQ ID NO.350) |
| (Gly⁸, β-Ala³⁵, Arg³⁸)hGLP-1(7-38)NH₂; | (SEQ ID NO.351) |
| (Gly⁸, Arg^{26,34}, β-Ala³⁵,)hGLP-1(7-36)NH₂; | (SEQ ID NO.352) |
| (Gly⁸, D-Arg³⁵)hGLP-1(7-36)NH₂; | (SEQ ID NO.353) |
| (Gly⁸, β-Ala³⁵, Arg³⁷)hGLP-1(7-37)NH₂; | (SEQ ID NO.354) |
| (Gly⁸, Phe³¹, β-Ala³⁵)hGLP-1(7-36)NH₂; | (SEQ ID NO.355) |
| (Gly⁸, Aib³⁵, Phe³¹)hGLP-1(7-36)NH₂; | (SEQ ID NO.356) |
| (Gly⁸, Aib³⁵, 2-Nal³¹)hGLP-1(7-36)NH₂; | (SEQ ID NO.357) |
| (Gly⁸, Aib³⁵, 2-Nal^{28,31})hGLP-1(7-36)NH₂; | (SEQ ID NO.358) |
| (Gly⁸, Aib³⁵, Arg^{26,34}, 2-Nal³¹)hGLP-1(7-36)NH₂; | (SEQ ID NO.359) |
| (Gly⁸, Aib³⁵, Arg^{26,34}, Phe³¹)hGLP-1(7-36)NH₂; | (SEQ ID NO.360) |
| (Gly⁸, Aib³⁵, 2-Nal^{19,31})hGLP-1(7-36)NH₂; | (SEQ ID NO.361) |
| (Gly⁸, Aib³⁵, 2-Nal^{12,31})hGLP-1(7-36)NH₂; | (SEQ ID NO.362) |
| (Gly⁸, Aib³⁵, Lys³⁶(N^{ε}-decanoyl))hGLP-1(7-36) NH₂; | (SEQ ID NO.99) |
| (Gly⁸, Aib³⁵, Arg³⁴, Lys²⁶(N^{ε}-decanoyl))hGLP-1(7-36)NH₂; | (SEQ ID NO.364) |
| (Gly⁸, Aib³⁵, Arg^{26,34}, Lys³⁶(N^{ε}-dodecanoyl))hGLP-1(7-36)NH₂; | (SEQ ID NO.365) |
| (Gly⁸, β-Ala³⁵, Ser³⁷(O-decanoyl))hGLP1(7-37)-NH₂; | (SEQ ID NO.384) |
| (Gly⁸, Aib²⁷, β-Ala^{35,37}, Arg³⁸, Lys³⁹(N^{ε}-octanoyl))hGLP-1(7-39)NH₂; | (SEQ ID NO.367) |
| (Gly⁸, Arg^{26,34}, β-Ala³⁵, Lys³⁷(N^{ε}-octanoyl))hGLP-1(7-37)NH₂; | (SEQ ID NO.368) |
| (Gly⁸, Arg^{26,34}, β-Ala³⁵, Lys³⁷(N^{ε}-decanoyl))hGLP-1(7-37)NH₂; | (SEQ ID NO.369) |
| or (Gly⁸, Arg^{26,34}, β-Ala³⁵, Lys³⁷(N^{ε}-tetradecanoyl))hGLP-1(7-37)NH₂; | (SEQ ID NO.370) |

or a pharmaceutically acceptable salt thereof.
Paragraph 14. A compound according to Paragraph 12 wherein said compound is:

| | |
|---|---|
| (Ser⁸, Aib³⁵, A6c³²)hGLP-1(7-36)NH₂; | (SEQ ID NO.392) |
| (Ser⁸, Aib³⁵, Glu²³)hGLP-1(7-36)NH₂; | (SEQ ID NO.394) |
| (Ser⁸, Aib^{24,35})hGLP-1(7-36)NH₂; | (SEQ ID NO.395) |
| (Ser⁸, Aib³⁵, Glu²³, A6c³²)hGLP-1(7-36)NH₂; | (SEQ ID NO.412) |
| (Ser⁸, Glu²³, β-Ala³⁵)hGhP-1(7-36)NH₂; | (SEQ ID NO.418) |
| (Ser⁸, Aib³⁵, Arg^{26,34})hGLP-1(7-36)NH₂; | (SEQ ID NO.443) |
| (Ser⁸, Aib³⁵, Arg^{26,34}, Lys³⁶(N^{ε}-octanoyl))hGLP-1(7-36)NH₂; | (SEQ ID NO.501) |
| (Ser⁸, Aib³⁵, Arg^{26,34}, Lys³⁶(N^{ε}-decanoyl))hGLP-1(7-36)OH; | (SEQ ID NO.716) |
| (Ser⁸, Aib³⁵, Lys²⁵, Arg^{26,34}, Lys³⁶(N^{ε}-decanoyl))hGLP-1(7-36)OH; | (SEQ ID NO.717) |
| (Ser⁸, Arg^{26,34}, β-Ala³⁵, Lys³⁶(N^{ε}-Aec-decanoyl))hGLP-1(7-36)NH₂; | (SEQ ID NO.757) |
| (Ser⁸, Aib³⁵,Arg^{26,34}, Ava³⁷, Ado³⁸)hGLP-1(7-38)NH₂; | (SEQ ID NO.718) |
| (Ser⁸, Aib³⁵,Arg^{26,34}, Asp³⁷, Ava³⁸, Ado³⁹)hGLP-1(7-39)NH₂; | (SEQ ID NO.719) |
| (Ser⁸, Aib³⁵,Arg^{26,34}, Aun³⁷)hGLP-1(7-37)NH₂; | (SEQ ID NO.720) |
| (Ser⁸, Aib^{17,35})hGLP-1(7-36)NH₂; | (SEQ ID NO.389) |
| (Ser⁸ ,Arg^{26,34}, β-Ala³⁵, D-Asp³⁷, Ava³⁸, Aun³⁹)hGLP-1(7-39)NH₂; | (SEQ ID NO.722) |
| (Ser⁸, Glu^{22,23}, β-Ala³⁵)hGLP-1(7-36)NH₂; | (SEQ ID NO.723) |
| (Ser⁸, Lys¹⁸, β-Ala³⁵)hGLP-1(7-36)NH₂; | (SEQ ID NO.724) |
| (Ser⁸, Leu²⁷, β-Ala³⁵)hGLP-1(7-36)NH₂; | (SEQ ID NO.725) |
| (Ser⁸, Lys³³, β-Ala³⁵)hGLP-1(7-36)NH₂; | (SEQ ID NO.726) |
| (Ser⁸, Lys¹⁸, Leu²⁷, β-Ala³⁵)hGLP-1(7-36)NH₂; | (SEQ ID NO.727) |
| (Ser⁸, D-Arg³⁶)hGLP-1(7-36)NH₂; | (SEQ ID NO.728) |
| (Ser⁸, β-Ala³⁵, D-Arg³⁷)hGLP-1(7-37)NH₂; | (SEQ ID NO.729) |
| (Ser⁸, Aib²⁷, β-Ala³⁵)hGLP-1(7-36)NH₂; | (SEQ ID NO.730) |
| (Ser⁸, Aib²⁷, β-Ala^{35,37}, Arg³⁸)hGLP-1(7-38)NH₂; | (SEQ ID NO.731) |
| (Ser⁸, Aib²⁷, β-Ala^{35,37}, Arg^{38,39})hGLP-1(7-39)NH₂; | (SEQ ID NO.732) |
| (Ser⁸, Lys^{18,27}, β-Ala³⁵)hGLP-1(7-36)NH₂; | (SEQ ID NO.733) |
| (Ser⁸, Lys²⁷, β-Ala³⁵)hGLP-1(7-36)NH₂; | (SEQ ID NO.734) |
| (Ser⁸, β-Ala³⁵, Arg³⁸)hGLP-1(7-38)NH₂; | (SEQ ID NO.735) |
| (Ser⁸, Arg^{26,34}, β-Ala³⁵)hGLP-1(7-36)NH₂; | (SEQ ID NO.736) |
| (Ser⁸, D-Arg³⁵)hGLP-1(7-36)NH₂; | (SEQ ID NO.737) |
| (Ser⁸, β-Ala³⁵, Arg³⁷)hGLP-1(7-37)NH₂; | (SEQ ID NO.738) |
| (Ser⁸, Phe³¹, β-Ala³⁵)hGLP-1(7-36)NH₂; | (SEQ ID NO.739) |
| (Ser⁸, Aib³⁵, Phe³¹)hGLP-1(7-36)NH₂; | (SEQ ID NO.740) |
| (Ser⁸, Aib³⁵, 2-Nal³¹)hGLP-1(7-36)NH₂; | (SEQ ID NO.741) |
| (Ser⁸, Aib³⁵, 2-Nal^{28,31})hGLP-1(7-36)NH₂; | (SEQ ID NO.742) |
| (Ser⁸, Aib³⁵, Arg^{26,34}, 2-Nal³¹)hGLP-1(7-36)NH₂; | (SEQ ID NO.743) |
| (Ser⁸, Aib³⁵, Arg^{26,34}, Phe³¹)hGLP-1(7-36)NH₂; | (SEQ ID NO.744) |
| (Ser⁸, Aib³⁵, 2-Nal^{19,31})hGLP-1(7-36)NH₂; | (SEQ ID NO.745) |
| (Ser⁸, Aib³⁵, 2-Nal^{12,31})hGLP-1(7-36)NH₂; | (SEQ ID NO.746) |
| (Ser⁸, Aib³⁵, Lys³⁶(N^{ε}-decanoyl))hGLP-1(7-36) NH₂; | (SEQ ID NO.483) |
| (Ser⁸, Aib³⁵, Arg³⁴, Lys²⁶(N^{ε}-decanoyl))hGLP-1(7-36)NH₂; | (SEQ ID NO.748) |
| (Ser⁸, Aib³⁵, Arg^{26,34}, Lys³⁶(N^{ε}-dodecanoyl))hGLP-1(7-36)NH₂; | (SEQ ID NO.749) |
| (Ser⁸, β-Ala³⁵, Ser³⁷(O-decanoyl))hGLP1(7-37)-NH₂; | (SEQ ID NO.539) |
| (Ser⁸, Aib²⁷, β-Ala^{35,37}, Arg³⁸, Lys³⁹(N^{ε}-octanoyl))hGLP-1(7-39)NH₂; | (SEQ ID NO.751) |
| (Ser⁸, Arg^{26,34}, β-Ala³⁵, Lys³⁷(N^{ε}-octanoyl))hGLP-1(7-37)NH₂; | (SEQ ID NO.752) |
| (Ser⁸, Arg^{26,34}, β-Ala³⁵, Lys³⁷(N^{ε}-decanoyl))hGLP-1(7-37)NH₂; | (SEQ ID NO.753) |
| or (Ser⁸, Arg^{26,34}, β-Ala³⁵, Lys³⁷(N^{ε}-tetradecanoyl))hGLP-1(7-37)NH₂; | (SEQ ID NO.754) |

or a pharmaceutically acceptable salt thereof.
Paragraph 15. A compound according to Paragraph 1, wherein said compound is:

| | |
|---|---|
| (Ser⁸, Aib³⁵)hGLP-1(7-36)NH₂; | (SEQ ID NO.767) |
| (Abu⁸, β-Ala³⁵)hGLP-1(7-36)NH₂; | (SEQ ID NO.768) |
| (Val⁸, β-Ala³⁵)hGLP-1(7-36)NH₂; | (SEQ ID NO.769) |
| (β-Ala^{8,35})hGLP-1(7-36)NH₂; | (SEQ ID NO.770) |
| (Abu⁸, Aib³⁵)hGLP-1(7-36)NH₂; | (SEQ ID NO.771) |
| (Val⁸, Aib³⁵)hGLP-1(7-36)NH₂; | (SEQ ID NO.772) |
| or (β-Ala⁸, Aib³⁵)hGLP-1(7-36)NH₂; | (SEQ ID NO.773) |

or a pharmaceutically acceptable salt thereof.
Paragraph 16. A compound according to Paragraph 15, wherein said compound is:

| | |
|---|---|
| (Ser⁸, Aib³⁵)hGLP-1(7-36)NH₂; | (SEQ ID NO.767) |
| (Abu⁸, β-Ala³⁵)hGLP-1(7-36)NH₂; | (SEQ ID NO.768) |
| or (Abu⁸, Aib³⁵)hGLP-1(7-36)NH₂; | (SEQ ID NO.771) |

or a pharmaceutically acceptable salt thereof.
Paragraph 17. A compound according to Paragraph 16, wherein said compound is:

| | |
|---|---|
| (Ser⁸, Aib³⁵)hGhP-1(7-36)NH₂; | (SEQ ID NO.767) |

or a pharmaceutically acceptable salt thereof.
Paragraph 18. A pharmaceutical composition comprising an effective amount of a compound according to Paragraph 1 or a pharmaceutically acceptable salt thereof and a pharmaceutically acceptable carrier or diluent.
Paragraph 19. A method of eliciting an agonist effect from a GLP-1 receptor in a subject in need thereof which comprises administering to said subject an effective amount of a compound according to Paragraph 1 or a pharmaceutically acceptable salt thereof.
Paragraph 20. A method of treating a disease selected from the group consisting of Type I diabetes, Type II diabetes, obesity, glucagonomas, secretory disorders of the airway, metabolic disorder, arthritis, osteoporosis, central nervous system disease, restenosis and neurodegenerative disease, in a subject in need thereof which comprises administering to said subject an effective amount of a compound according to Paragraph 1 or a pharmaceutically acceptable salt thereof.
Paragraph 21. A method according to Paragraph 20 wherein said disease is Type I diabetes or Type II diabetes.

## Claims

1. A compound of formula (I),
(R²R³)-A⁷⁻A⁸-A⁹-A¹⁰-A¹¹-A¹²-A¹³-A¹⁴-A¹⁵-A¹⁶-A¹⁷⁻A¹⁸-A¹⁹-A²⁰-A²¹-A²²-A²³-A²⁴-A²⁵-A²⁶-A²⁷⁻A²⁸-A²⁹-A³⁰-A³¹-A³²-A³³-A³⁴-A³⁵-A³⁶-A³⁷⁻A³⁸-A³⁹-R¹, (I)
wherein:
A⁷ is L-His, Ura, Paa, Pta, Amp, Tma-His, des-amino-His, or deleted;
A⁸ is Abu, β-Ala, Ser, Gly or Val;
A⁹ is Glu, N-Me-Glu, N-Me-Asp or Asp;
A¹⁰ is Gly, Acc, β-Ala or Aib;
A¹¹ is Thr or Ser;
A¹² is Phe, Acc, Aic, Aib, 3-Pal, 4-Pal, 1-Nal, 2-Nal, Cha, Trp or X¹-Phe;
A¹³ is Thr or Ser;
A¹⁴ is Ser or Aib;
A¹⁵ is Asp or Glu;
A¹⁶ is Val, Acc, Aib, Leu, Ile, Tle, Nle, Abu, Ala or Cha;
A¹⁷ is Ser or Thr;
A¹⁸ is Ser or Thr;
A¹⁹ is Tyr, Cha, Phe, 3-Pal, 4-Pal, Acc, 1-Nal, 2-Nal, or X¹-Phe;
A²⁰ is Leu, Acc, Aib, Nle, Ile, Cha, Tle, Val, Phe or X¹-Phe;
A²¹ is Glu or Asp;
A²² is Gly, Acc, β-Ala, Glu or Aib;
A²³ is Gln, Asp, Asn or Glu;
A²⁴ is Ala, Aib, Val, Abu, Tle or Acc;
A²⁵ is Ala, Aib, Val, Abu, Tle, Acc, Lys, Arg, hArg, Orn, HN-CH((CH₂)ₙ-N(R¹⁰R¹¹))-C(O) or HN-CH((CH₂)ₑ-X³)-C(O);
A²⁶ is Lys, Arg, hArg, Orn, HN-CH((CH₂)ₙ-N(R¹⁰R¹¹))-C(O) or HN-CH((CH₂)ₑ-X³)-C(O);
A²⁷ is Glu, Asp, Leu, Aib or Lys;
A²⁸ is Phe, 3-Pal, 4-Pal, 1-Nal, 2-Nal, X¹-Phe, Aic, Acc, Aib, Cha or Trp;
A²⁹ is Ile, Acc, Aib, Leu, Nle, Cha, Tle, Val, Abu, Ala or Phe;
A³⁰ is Ala, Aib or Acc;
A³¹ is Trp, 1-Nal, 2-Nal,3-Pal, 4-Pal, Phe, Acc, Aib or Cha;
A³² is Leu, Acc, Aib, Nle, Ile, Cha, Tle, Phe, X¹-Phe or Ala;
A³³ is Val, Acc, Aib, Leu, Ile, Tle, Nle, Cha, Ala, Phe, Abu, Lys or X¹-Phe;
A³⁴ is Lys, Arg, hArg, Orn, HN-CH((CH₂)ₙ-N(R¹⁰R¹¹))-C(O) or HN-CH((CH₂)ₑ-X³)-C(O);
A³⁵ is Gly, β-Ala, D-Ala, Gaba, Ava, HN-(CH₂)ₘ-C(O), Aib, Acc or a D-amino acid;
A³⁶ is L- or D-Arg, D- or L-Lys, D- or L-hArg, D- or L-Orn, HN-CH((CH₂)ₙ-N(R¹⁰R¹¹))-C(O), HN-CH((CH₂)ₑ-X³)-C(O) or deleted;
A³⁷ is Gly, β-Ala, Gaba, Ava, Aib, Acc, Ado, Arg, Asp, Aun, Aec, HN-(CH₂)ₘ-C(O), HN-CH((CH₂)ₙ-N(R¹⁰R¹¹))-C(O), HN-CH((CH₂)ₙ-O(R¹⁰))-C(O),a D-amino acid, or deleted;
A³⁸ is D- or L-Lys, D- or L-Arg, D- or L-hArg, D- or L-Orn, HN-CH((CH₂)ₙ-N(R¹⁰R¹¹))-C(O), HN-CH((CH₂)ₑ-X³)-C(O) Ava, Ado, Aec or deleted;
A³⁹ is D- or L-Lys, D- or L-Arg, HN-CH((CH₂)ₙ-N(R¹⁰R¹¹))-C(O), Ava, Ado, Aec, or Aun, or is deleted;
X¹ is, independently for each occurrence, selected from the group consisting of (C₁-C₆)alkyl, OH and halo;
R¹ is OH, NH₂, (C₁-C₃₀)alkoxy, or NH-X²-CH₂-Z⁰, wherein X² is a (C₁-C₁₂)hydrocarbon moiety, and Z⁰ is H, OH, CO₂H or CONH₂;
X³ is 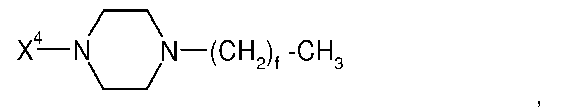 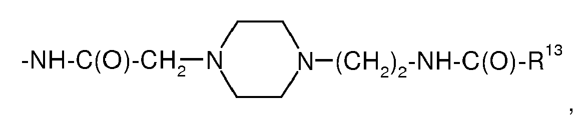 or -C(O)-NHR¹², wherein X⁴ is, independently for each occurrence, -C(O)-, -NH-C(O)- or -CH₂-, and wherein f is, independently for each occurrence, an integer from 1 to 29 inclusive;
each of R² and R³ is independently selected from the group consisting of H, (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, and hydroxynaphthyl(C₁-C₃₀)alkyl; or one of R² and
R³ is 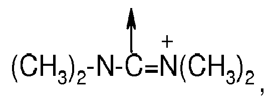 (C₁-C₃₀)acyl, (C₁-C₃₀)alkylsulfonyl, C(O)X⁵, 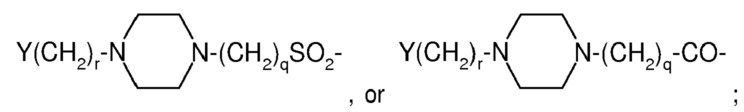 wherein Y is H, OH or NH₂; r is 0 to 4; q is 0 to 4; and X⁵ is (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl or hydroxynaphthyl(C₁-C₃₀)alkyl;
e is, independently for each occurrence, an integer from 1 to 4 inclusive;
m is, independently for each occurrence, an integer from 5 to 24 inclusive;
n is, independently for each occurrence, an integer from 1 to 5, inclusive;
each of R¹⁰ and R¹¹ is, independently for each occurrence, H, (C₁-C₃₀)alkyl, (C₁-C₃₀)acyl, (C₁-C₃₀)alkylsulfonyl, -C((NH)(NH₂)) or 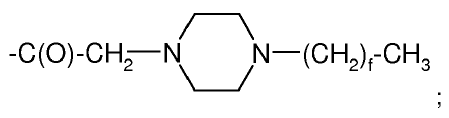 and
R¹² and R¹³ each is, independently for each occurrence, (C₁-C₃₀)alkyl;
provided that:
(i) said compound is not:
(Ser⁸, β-Ala³⁵)hGLP-1(7-36)NH₂ (SEQ ID NO: 779);
(Gly⁸, Aib³⁵)hGLP-1(7-36)NH₂ (SEQ ID NO: 780); or
(Gly⁸, β-Ala³⁵)hGLP-1(7-36)NH₂ (SEQ ID NO: 781);
(ii) when A⁷ is Ura, Paa or Pta, then R² and R³ are deleted;
(iii) when R¹⁰ is (C₁-C₃₀)acyl, (C₁-C₃₀)alkylsulfonyl, -C((NH)(NH₂)) or 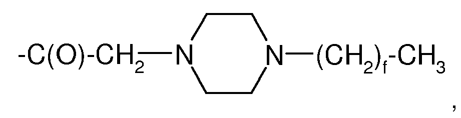 then R¹¹ is H or (C₁-C₃₀)alkyl; and
(iv) when A⁷ is L-His; A⁹ is Glu; A¹⁰ is Gly; A¹¹ is Thr; A¹² is Phe; A¹³ is Thr; A¹⁴ is Ser; A¹⁵ is Asp; A¹⁶ is Val; A¹⁷ is Ser; A¹⁸ is Ser; A¹⁹ is Tyr; A²⁰ is Leu; A²¹ is Glu; A²² is Gly; A²³ is Gln; A²⁴ is Ala; A²⁵ is Ala; A²⁶ is Lys; A²⁷ is Glu; A²⁸ is Phe; A²⁹ is Ile; A³⁰ is Ala; A³¹ is Trp; A³² is Leu; A³³ is Val; A³⁴ is Lys; A³⁶ is L- or D-Arg; A³⁷ is deleted; A³⁸ is deleted; and A³⁹ is deleted, A⁸ is not Abu, β-Ala, Ser or Val and A³⁵ is not β-Ala or Aib;
or a pharmaceutically acceptable salt thereof.

2. A compound according to claim 1, wherein A¹¹ is Thr; A¹³ is Thr; A¹⁵ is Asp; A¹⁷ is Ser; A¹⁸ is Ser; A²¹ is Glu; A²³ is Gln or Glu; A²⁷ is Glu; and A³¹ is Trp; or a pharmaceutically acceptable salt thereof.

3. A compound according to claim 2, wherein A⁹ is Glu, N-Me-Glu or N-Me-Asp; A¹² is Phe, Acc or Aic; A¹⁶ is Val, Acc or Aib; A¹⁹ is Tyr; A²⁰ is Leu, Acc or Cha; A²⁴ is Ala, Aib or Acc; A²⁵ is Ala, Aib, Acc, Lys, Arg, hArg, Orn, HN-CH((CH₂)ₙ-N(R¹⁰R¹¹))-C(O) or HN-CH((CH₂)ₑ-X³)-C(O); A²⁸ is Phe; A²⁹ is Ile or Acc; A³⁰ is Ala or Aib; A³² is Leu, Acc or Cha; and A³³ is Val or Acc; or a pharmaceutically acceptable salt thereof.

4. A compound according to claim 3, wherein A¹⁰ is Gly; A¹² is Phe, A6c or A5c; A¹⁶ is Val, A6c or A5c; A²⁰ is Leu, A6c, A5c or Cha; A²² is Gly, β-Ala or Aib; A²⁴ is Ala or Aib; A²⁹ is Ile, A6c or A5c; A³² is Leu, A6c, A5c or Cha; A³³ is Val, A6c or A5c; A³⁵ is Aib, β-Ala, Ado, A6c, A5c or Gly; and A³⁷ is Gly, Aib, β-Ala, Ado, D-Ala or deleted; or a pharmaceutically acceptable salt thereof.

5. A compound according to claim 4, wherein X⁴ for each occurrence is -C(O)-; e for each occurrence is independently 1 or 2; and R¹ is OH or NH₂; or a pharmaceutically acceptable salt thereof.

6. A compound according to claim 5, wherein R² is H and R³ is (C₁-C₃₀)alkyl, (C₂-C₃₀)alkenyl, (C₁-C₃₀)acyl, (C₁-C₃₀)alkylsulfonyl, 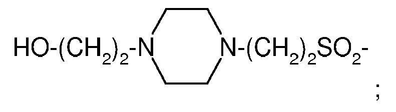 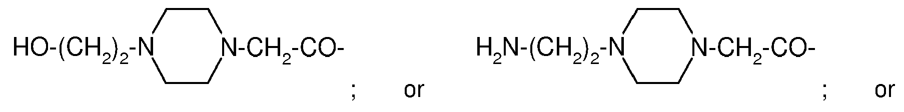 a pharmaceutically acceptable salt thereof.

7. A compound according to claim 5, wherein R¹⁰ is (C₁-C₃₀)acyl, (C₁-C₃₀)alkylsulfonyl or 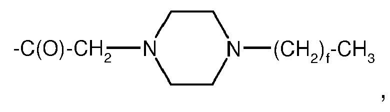 and R¹¹ is H; or a pharmaceutically acceptable salt thereof

8. A compound according to claim 7, wherein R¹⁰ is (C₄-C₂₀)acyl, (C₄-C₂₀)alkylsulfonyl or 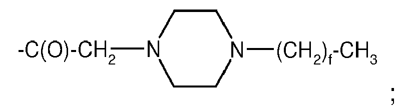 or a pharmaceutically acceptable salt thereof.

9. A compound according to claim 5, wherein A⁸ is Gly, Ser, or Val; or a pharmaceutically acceptable salt thereof.

10. A compound according to claim 9 wherein A⁸ is Gly or Ser; or a pharmaceutically acceptable salt thereof.

11. A compound according to claim 10, wherein said compound is according to the formula:
| | |
|---|---|
| ((N^{α}-Me-His)⁷, Gly⁸, Aib³⁵)hGLP-1(7-36)NH₂; | (SEQ ID NO.1) |
| ((N^{α}-Me-His)⁷, Gly⁸, β-Ala³⁵)hGLP-1(7-36)NH₂; | (SEQ ID NO.2) |
| ((N^{α}-Me-His)⁷, Gly⁸, Aib³⁵, Arg^{26,34})hGLP-1(7-36)NH₂; | (SEQ ID NO.3) |
| ((N^{α}-Me-His)⁷, Gly⁸, Arg^{26,34}, β-Ala³⁵)hGLP-1(7-36)NH₂; | (SEQ ID NO.4) |
| (Gly⁸, A6c³⁵)hGLP-1(7-36)NH₂; | (SEQ ID NO.5) |
| (Gly⁸, A5c³⁵)hGLP-1(7-36)NH₂; | (SEQ ID NO.6) |
| (Gly⁸, D-Ala³⁵)hGLP-1(7-36)NH₂; | (SEQ ID NO.7) |
| (Gly⁸, Aib³⁵, A6c³²)hGLP-1(7-36)NH₂; | (SEQ ID NO.8) |
| (Gly⁸, Aib³⁵, A5c³²)hGLP-1(7-36)NH₂; | (SEQ ID NO.9) |
| (Gly⁸, Aib³⁵, Glu²³)hGLP-1(7-36)NH₂; | (SEQ ID NO.10) |
| (Gly⁸, Aib^{24,35})hGLP-1(7-36)NH₂; | (SEQ ID NO.11) |
| (Gly⁸, Aib^{30,35})hGLP-1(7-36)NH₂; | (SEQ ID NO.12) |
| (Gly⁸, Aib^{25,35})hGLP-1(7-36)NH₂; | (SEQ ID NO.13) |
| (Gly⁸, Aib³⁵, A6c^{16,20})hGLP-1(7-36)NH₂; | (SEQ ID NO.14) |
| (Gly⁸, Aib³⁵, A6c^{16,29,32})hGLP-1(7-36)NH₂; | (SEQ ID NO.15) |
| (Gly⁸, Aib³⁵, A6c^{20,32})hGLP-1(7-36)NH₂; | (SEQ ID NO.16) |
| (Gly⁸, Aib³⁵, A6c²⁰)hGLP-1(7-36)NH₂; | (SEQ ID NO.17) |
| (Gly⁸, Aib³⁵, Lys²⁵)hGLP-1(7-36)NH₂; | (SEQ ID NO.18) |
| (Gly⁸, Aib^{24,35}, A6c²⁰)hGLP-1(7-36)NH₂; | (SEQ ID NO.19) |
| (Gly⁸, Aib³⁵, A6c^{29,32})hGLP-1(7-36)NH₂; | (SEQ ID NO.20) |
| (Gly⁸, Aib^{24,35}, A6c^{29,32})hGLP-1(7-36)NH₂; | (SEQ ID NO.21) |
| (Gly⁸, Aib³⁵, A6c¹²)hGLP-1(7-36)NH₂; | (SEQ ID NO.22) |
| (Gly⁸, Aib³⁵, Cha²⁰)hGLP-1(7-36)NH₂; | (SEQ ID NO.23) |
| (Gly⁸, Aib³⁵, A6c³³)hGLP-1(7-36)NH₂; | (SEQ ID NO.24) |
| (Gly⁸, A6c^{16,20}, β-Ala³⁵)hGLP-1(7-36)NH₂; | (SEQ ID NO.25) |
| (Gly⁸, Aib³⁵, β-Ala²²)hGLP-1(7-36)NH₂; | (SEQ ID NO.26) |
| (Gly⁸, Aib^{22,35})hGLP-1(7-36)NH₂; | (SEQ ID NO.27) |
| (Gly⁸, Aib³⁵, Glu²³, A6c³²)hGLP-1(7-36)NH₂; | (SEQ ID NO.28) |
| (Gly⁸, Aib^{24,35}, Glu²³, A6c³²)hGLP-1(7-36)NH₂; | (SEQ ID NO.29) |
| (Gly⁸, Aib^{24,25,35}, Glu²³, A6c³²)hGLP-1(7-36)NH₂; | (SEQ ID NO.30) |
| (Gly⁸, Aib^{24,25,35}, A6c^{16,20,32}, Glu²³)hGLP-1(7-36)NH₂; | (SEQ ID NO.31) |
| (Gly⁸, A6c³², β-Ala³⁵)hGLP-1(7-36)NH₂; | (SEQ ID NO.32) |
| (Gly⁸, A5c³², β-Ala³⁵)hGLP-1(7-36)NH₂; | (SEQ ID NO.33) |
| (Gly⁸, Glu²³, β-Ala³⁵)hGLP-1(7-36)NH₂; | (SEQ ID NO.34) |
| (Gly⁸, Aib²⁴, β-Ala³⁵)hGLP-1(7-36)NH₂; | (SEQ ID NO.35) |
| (Gly⁸, Aib³⁰, β-Ala³⁵)hGLP-1(7-36)NH₂; | (SEQ ID NO.36) |
| (Gly⁸, Aib²⁵, β-Ala³⁵)hGLP-1(7-36)NH₂; | (SEQ ID NO.37) |
| (Gly⁸, A6c^{16,29,32}, β-Ala³⁵)hGLP-1(7-36)NH₂; | (SEQ ID NO.38) |
| (Gly⁸, A6c^{20,32}, β-Ala³⁵)hGLP-1(7-36)NH₂; | (SEQ ID NO.39) |
| (Gly⁸, A6c²⁰, β-Ala³⁵)hGLP-1(7-36)NH₂; | (SEQ ID NO.40) |
| (Gly⁸, Lys²⁵, β-Ala³⁵)hGLP-1(7-36)NH₂; | (SEQ ID NO.41) |
| (Gly⁸, Aib²⁴, A6c²⁰, β-Ala³⁵)hGLP-1(7-36)NH₂; | (SEQ ID NO.42) |
| (Gly⁸, A6c^{29,32}, β-Ala³⁵)hGLP-1(7-36)NH₂; | (SEQ ID NO.43) |
| (Gly⁸, Aib²⁴, A6c^{29,32}, β-Ala³⁵)hGLP-1(7-36)NH₂; | (SEQ ID NO.44) |
| (Gly⁸, A6c¹², β-Ala³⁵)hGLP-1(7-36)NH₂; | (SEQ ID NO.45) |
| (Gly⁸, Cha²⁰, β-Ala³⁵)hGLP-1(7-36)NH₂; | (SEQ ID NO.46) |
| (Gly⁸, A6c³³, β-Ala³⁵)hGLP-1(7-36)NH₂; | (SEQ ID NO.47) |
| (Gly⁸, β-Ala^{22,35})hGLP-1(7-36)NH₂; | (SEQ ID NO.48) |
| (Gly⁸, Aib²², β-Ala³⁵)hGLP-1(7-36)NH₂; | (SEQ ID NO.49) |
| (Gly⁸, Glu²³, A6c³², β-Ala³⁵)hGLP-1(7-36)NH₂; | (SEQ ID NO.50) |
| (Gly⁸, Aib²⁴, Glu²³, A6c³², β-Ala³⁵)hGLP-1(7-36)NH₂; | (SEQ ID NO.51) |
| (Gly⁸, Aib²⁴, Glu²³, A6c³², Lys³⁴(N^{e}-octanoyl), β-Ala³⁵) | |
| hGLP-1(7-36)NH₂; | (SEQ ID NO.52) |
| (Gly⁸, Aib^{24,25}, Glu²³, A6c³², β-Ala³⁵)hGLP-1(7-36)NH₂; | (SEQ ID NO.53) |
| (Gly⁸, Aib^{24,25}, A6c^{16,20,32}, Glu²³, β-Ala³⁵)hGLP-1(7-36)NH₂; | (SEQ ID NO.54) |
| (Gly⁸, Aib³⁵, D-Arg³⁶)hGLP-1(7-36)NH₂; | (SEQ ID NO.55) |
| (Gly⁸, Aib³⁵, D-Lys³⁶)hGLP-1(7-36)NH₂; | (SEQ ID NO.56) |
| (Gly⁸, β-Ala³⁵, D-Arg³⁶)hGLP-1(7-36)NH₂; | (SEQ ID NO.57) |
| (Gly⁸, β-Ala³⁵, D-Lys³⁶)hGLP-1(7-36)NH₂; | (SEQ ID NO.58) |
| (Gly⁸, Aib³⁵, Arg^{26,34})hGLP-1(7-36)NH₂; | (SEQ ID NO.59) |
| (Gly⁸, Arg^{26,34}, β-Ala³⁵)hGLP-1(7-36)NH₂; | (SEQ ID NO.60) |
| (Gly⁸, Aib³⁵, Arg^{25,26,34})hGLP-1(7-36)NH₂; | (SEQ ID NO.61) |
| (Gly⁸, Arg^{25,26,34}, β-Ala³⁵)hGLP-1(7-36)NH₂; | (SEQ ID NO.62) |
| (Gly⁸, Arg^{26,34}, β-Ala³⁵, Lys³⁶(N^{ε}-tetradecanoyl))hGLP-1(7-36)OH; | (SEQ ID NO.63) |
| (Gly⁸, Aib³⁵, Arg^{26,34}, Lys³⁶(N^{ε}-tetradecanoyl))hGLP-1(7-37)OH; | (SEQ ID NO.64) |
| (Gly⁸, Aib^{35,37}, Arg^{26,34}, Lys³⁶(N^{ε}-tetradecanoyl))hGLP-1(7-37)OH; | (SEQ ID NO.65) |
| (Gly⁸, Aib³⁵, Arg^{26,34}, Lys³⁶(N^{ε}-tetradecanoyl), D-Ala³⁷) | |
| hGLP-1 (7-37)OH; | (SEQ ID NO.66) |
| (Gly⁸, Aib^{35,37}, Arg^{26,34}, Lys³⁸(N^{ε}-tetradecanoyl))hGLP-1(7-38)OH; | (SEQ ID NO.67) |
| (Gly⁸, Aib³⁵, Arg^{26,34}, β-Ala³⁷, Lys³⁸(N^{ε}-tetradecanoyl)) | |
| hGLP-1 (7-38)OH; | (SEQ ID NO.68) |
| (Gly⁸, Aib³⁵, Arg^{26,34}, Lys³⁸(N^{ε}-tetradecanoyl))hGLP-1(7-38)OH; | (SEQ ID NO.69) |
| (Gly⁸, Arg^{26,34}, Lys³⁶(N^{ε}-tetradecanoyl), β-Ala³⁷)hGLP-1(7-37)OH; | (SEQ ID NO.70) |
| (Gly⁸, Aib³⁷, Arg^{26,34}, Lys³⁶(N^{ε}-tetradecanoyl))hGLP-1(7-37)OH; | (SEQ ID NO.71) |
| (Gly⁸, Aib³⁵, Arg^{26,34}, Ado³⁷)hGLP-1(7-37)OH; | (SEQ ID NO.72) |
| (Gly⁸, Aib³⁵, Arg^{26,34}, Ado³⁷)hGLP-1(7-37)NH₂; | (SEQ ID NO.73) |
| (Gly⁸, Arg^{26,34}, Lys³⁶(N^{ε}-tetradecanoyl), D-Ala³⁷)hGLP-1(7-37)OH; | (SEQ ID NO.74) |
| (Gly⁸, Aib³⁷, Arg^{26,34}, Lys³⁸(N^{ε}-tetradecanoyl))hGLP-1(7-38)OH; | (SEQ ID NO.75) |
| (Gly⁸, Arg^{26,34}, β-Ala³⁷, Lys³⁸(N^{ε}-tetradecanoyl))hGLP-1(7-38)OH; | (SEQ ID NO.76) |
| (Gly⁸, Aib³⁵, Lys²⁶(N^{ε}-octanoyl))hGLP-1(7-36)NH₂; | (SEQ ID NO.77) |
| (Gly⁸, Aib³⁵, Lys²⁶(N^{ε}-tetradecanoyl))hGLP-1(7-36)NH₂; | (SEQ ID NO.78) |
| (Gly⁸, Aib³⁵, Lys²⁶(N^{ε}-hexadecanoyl))hGLP-1(7-36)NH₂; | (SEQ ID NO.79) |
| (Gly⁸, Lys²⁶(N^{ε}-octanoyl), β-Ala³⁵)hGLP-1(7-36)NH₂; | (SEQ ID NO.80) |
| (Gly⁸, Lys²⁶(N^{ε}-tetradecanoyl), β-Ala³⁵)hGLP-1(7-36)NH₂; | (SEQ ID NO.81) |
| (Gly⁸, Lys²⁶(N^{ε}-hexadecanoyl), β-Ala³⁵)hGLP-1(7-36)NH₂; | (SEQ ID NO.82) |
| (Gly⁸, Aib³⁵, Lys²⁶(N^{ε}-octanoyl), Arg³⁴)hGLP-1(7-36)NH₂; | (SEQ ID NO.83) |
| (Gly⁸, Aib³⁵, Lys²⁶(N^{ε}-tetradecanoyl), Arg³⁴)hGLP-1(7-36)NH₂; | (SEQ ID NO.84) |
| (Gly⁸, Aib³⁵, Lys²⁶(N^{ε}-hexadecanoyl), Arg³⁴)hGLP-1(7-36)NH₂; | (SEQ ID NO.85) |
| (Gly⁸, Aib³⁵, Lys²⁶(N^{ε}-decanoyl), Arg³⁴)hGLP-1(7-36)NH₂; | (SEQ ID NO.86) |
| (Gly⁸, Aib³⁵, Lys²⁵, Lys²⁶(N^{ε}-octanoyl), Arg³⁴)hGLP-1(7-36)NH₂; | (SEQ ID NO.87) |
| (Gly⁸, Aib³⁵, Lys²⁵, Lys²⁶(N^{ε}-tetradecanoyl), Arg³⁴)hGLP-1(7-36)NH₂; | (SEQ ID NO.88) |
| (Gly⁸, Aib³⁵, Lys²⁵, Lys²⁶(N^{ε}-hexadecanoyl), Arg³⁴)hGLP-1(7-36)NH₂; | (SEQ ID NO.89) |
| (Gly⁸, Aib³⁵, Arg^{25,34}, Lys²⁶(N^{ε}-octanoyl))hGLP-1(7-36)NH₂; | (SEQ ID NO.90) |
| (Gly⁸, Aib³⁵, Arg^{25,34}, Lys²⁶(N^{ε}-tetradecanoyl))hGLP-1(7-36)NH₂; | (SEQ ID NO.91) |
| (Gly⁸, Aib³⁵, Arg^{25,34}, Lys²⁶(N^{ε}-hexadecanoyl))hGLP-1(7-36)NH₂; | (SEQ ID NO.92) |
| (Gly⁸, Aib³⁵, Arg^{25,34}, Lys²⁶(N^{ε}-decanoyl))hGLP-1(7-36)NH₂; | (SEQ ID NO.93) |
| (Gly⁸, Lys²⁶(N^{ε}-octanoyl), Arg³⁴, β-Ala³⁵)hGLP-1(7-36)NH₂; | (SEQ ID NO.94) |
| (Gly⁸, Lys²⁶(N^{ε}-tetradecanoyl), Arg³⁴, β-Ala³⁵)hGLP-1(7-36)NH₂; | (SEQ ID NO.95) |
| (Gly⁸, Lys²⁶(N^{ε}-hexadecanoyl), Arg³⁴, β-Ala³⁵)hGLP-1(7-36)NH₂; | (SEQ ID NO.96) |
| (Gly⁸, Lys²⁶(N^{ε}-decanoyl), Arg³⁴, β-Ala³⁵)hGLP-1(7-36)NH₂; | (SEQ ID NO.97) |
| (Gly⁸, Aib³⁵, Lys³⁴(N^{ε}-octanoyl))hGLP-1(7-36)NH₂; | (SEQ ID NO.98) |
| (Gly⁸, Aib³⁵, Lys³⁴(N^{ε}-tetradecanoyl))hGLP-1(7-36)NH₂; | (SEQ ID NO.99) |
| (Gly⁸, Aib³⁵, Lys³⁴(N^{ε}-hexadecanoyl))hGLP-1(7-36)NH₂; | (SEQ ID NO.100) |
| (Gly⁸, Aib³⁵, Arg²⁶, Lys³⁴(N^{ε}-octanoyl))hGLP-1(7-36)NH₂; | (SEQ ID NO.101) |
| (Gly⁸, Aib³⁵, Arg²⁶, Lys³⁴(N^{ε}-hexadecanoyl))hGLP-1(7-36)NH₂; | (SEQ ID NO.102) |
| (Gly⁸, Aib³⁵, Arg²⁶, Lys³⁴(N^{ε}-decanoyl))hGLP-1(7-36)NH₂; | (SEQ ID NO.103) |
| (Gly⁸, Aib³⁵, Arg^{25,26}, Lys³⁴(N^{ε}-octanoyl))hGLP-1(7-36)NH₂; | (SEQ ID NO.104) |
| (Gly⁸, Aib³⁵, Arg^{25,26}, Lys³⁴(N^{ε}-tetradecanoyl))hGLP-1(7-36)NH₂; | (SEQ ID NO.105) |
| (Gly⁸, Aib³⁵, Arg^{25,26}, Lys³⁴(N^{ε}-hexadecanoyl))hGLP-1(7-36)NH₂; | (SEQ ID NO.106) |
| (Gly⁸, Aib³⁵, Arg^{25,26}, Lys³⁴(N^{ε}-decanoyl))hGLP-1(7-36)NH₂; | (SEQ ID NO.107) |
| (Gly⁸, Aib³⁵, Lys²⁵, Arg²⁶, Lys³⁴(N^{ε}-octanoyl))hGLP-1(7-36)NH₂; | (SEQ ID NO.108) |
| (Gly⁸, Aib³⁵, Lys²⁵, Arg²⁶, Lys³⁴(N^{ε}-tetradecanoyl))hGLP-1(7-36)NH₂; | (SEQ ID NO.109) |
| (Gly⁸, Aib³⁵, Lys²⁵, Arg²⁶, Lys³⁴(N^{ε}-hexadecanoyl))hGLP-1(7-36)NH₂; | (SEQ ID NO.110) |
| (Gly⁸, Aib³⁵, Lys³⁶(N^{ε}-octanoyl))hGLP-1(7-36)NH₂; | (SEQ ID NO.111) |
| (Gly⁸, Aib³⁵, Lys³⁶(N^{ε}-tetradecanoyl))hGLP-1(7-36)NH₂; | (SEQ ID NO.112) |
| (Gly⁸, Aib³⁵, Lys³⁶(N^{ε}-hexadecanoyl))hGLP-1(7-36)NH₂; | (SEQ ID NO.113) |
| (Gly⁸, Aib³⁵, Arg²⁶, Lys³⁶(N^{ε}-octanoyl))hGLP-1(7-36)NH₂; | (SEQ ID NO.114) |
| (Gly⁸, Aib³⁵, Arg²⁶, Lys³⁶(N^{ε}-tetradecanoyl))hGLP-1(7-36)NH₂; | (SEQ ID NO.115) |
| (Gly⁸, Aib³⁵, Arg²⁶, Lys³⁶(N^{ε}-hexadecanoyl))hGLP-1(7-36)NH₂; | (SEQ ID NO.116) |
| (Gly⁸, Aib³⁵, Arg^{26,34}, Lys³⁶(N^{ε}-octanoyl))hGLP-1(7-36)NH₂; | (SEQ ID NO.117) |
| (Gly⁸, Aib³⁵, Arg^{26,34}, Lys³⁶(N^{ε}-hexadecanoyl))hGLP-1(7-36)NH₂; | (SEQ ID NO.118) |
| (Gly⁸, Aib³⁵, Arg^{26,34}, Lys³⁸(N^{ε}-octanoyl))hGLP-1(7-38)NH₂; | (SEQ ID NO.119) |
| (Gly⁸, Aib³⁵, Arg^{26,34}, Lys³⁸(N^{ε}-decanoyl))hGLP-1(7-38)NH₂; | (SEQ ID NO.120) |
| (Gly⁸, Aib³⁵, Arg^{26,34}, Lys³⁸(N^{ε}-tetradecanoyl))hGLP-1(7-38)NH₂; | (SEQ ID NO.121) |
| (Gly⁸, Aib³⁵, Arg^{26,34}, Lys³⁸(N^{ε}-hexadecanoyl))hGLP-1(7-38)NH₂; | (SEQ ID NO.122) |
| (Gly⁸, Aib^{35,37}, Arg^{25,26,34}, Lys³⁸(N^{ε}-octanoyl))hGLP-1(7-38)NH₂; | (SEQ ID NO.123) |
| (Gly⁸, Aib^{35,37}, Arg^{25,26,34}, Lys³⁸(N^{ε}-decanoyl))hGLP-1(7-38)NH₂; | (SEQ ID NO.124) |
| (Gly⁸, Aib^{35,37}, Arg^{25,26,34}, Lys³⁸(N^{ε}-tetradecanoyl))hGLP-1(7-38)NH₂; | (SEQ ID NO.125) |
| (Gly⁸, Aib^{35,37}, Arg^{25,26,34}, Lys³⁸(N^{ε}-hexadecanoyl))hGLP-1(7-38)NH₂; | (SEQ ID NO.126) |
| (Gly⁸, Aib^{35,37}, Arg^{26,34}, Lys³⁸(N^{ε}-octanoyl))hGLP-1(7-38)NH₂; | (SEQ ID NO.127) |
| (Gly⁸, Aib^{35,37}, Arg^{26,34}, Lys³⁸(N^{ε}-decanoyl))hGLP-1(7-38)NH₂; | (SEQ ID NO.128) |
| (Gly⁸, Aib^{35,37}, Arg^{26,34}, Lys³⁸(N^{ε}-hexadecanoyl))hGLP-1(7-38)NH₂; | (SEQ ID NO.129) |
| (Gly⁸, Aib³⁵, Lys²⁵, Arg^{26,34}, Lys³⁶(N^{ε}-octanoyl))hGLP-1(7-36)NH₂; | (SEQ ID NO.130) |
| (Gly⁸, Aib³⁵, Lys²⁵, Arg^{26,34}, Lys³⁶(N^{ε}-tetradecanoyl)) | |
| hGLP-1(7-36)NH₂; | (SEQ ID NO.131) |
| (Gly⁸, Aib³⁵, Lys²⁵, Arg^{26,34}, Lys³⁶(N^{ε}-hexadecanoyl)) | |
| hGLP-1(7-36)NH₂; | (SEQ ID NO.132) |
| (Gly⁸, Aib³⁵, Arg^{25,26,34}, Lys³⁶(N^{ε}-octanoyl))hGLP-1(7-36)NH₂; | (SEQ ID NO.133) |
| (Gly⁸, Aib³⁵, Arg^{25,26,34}, Lys³⁶(N^{ε}-tetradecanoyl))hGLP-1(7-36)NH₂; | (SEQ ID NO.134) |
| (Gly⁸, Aib³⁵, Arg^{25,26,34}, Lys³⁶(N^{ε}-hexadecanoyl))hGLP-1(7-36)NH₂; | (SEQ ID NO.135) |
| (Gly⁸, Aib³⁵, Arg^{25,26,34}, Lys³⁶(N^{ε}-decanoyl))hGLP-1(7-36)NH₂; | (SEQ ID NO.136) |
| (Gly⁸, Lys³⁴(N^{ε}-octanoyl), β-Ala³⁵)hGLP-1(7-36)NH₂; | (SEQ ID NO.137) |
| (Gly⁸, Lys³⁴(N^{ε}-tetradecanoyl), β-Ala³⁵)hGLP-1(7-36)NH₂; | (SEQ ID NO.138) |
| (Gly⁸, Lys³⁴(N^{ε}-hexadecanoyl), β-Ala³⁵)hGLP-1(7-36)NH₂; | (SEQ ID NO.139) |
| (Gly⁸, A6c³², Lys³⁴(N^{e}-octanoyl), β-Ala³⁵)hGLP-1(7-36)NH₂; | (SEQ ID NO.140) |
| (Gly⁸, Glu²³, Lys³⁴(N^{e}-octanoyl), β-Ala³⁵)hGLP-1(7-36)NH₂; | (SEQ ID NO.141) |
| (Gly⁸, Glu²³, A6c³², Lys³⁴(N^{e}-octanoyl), β-Ala³⁵)hGLP-1(7-36)NH₂; | (SEQ ID NO.142) |
| (Gly⁸, Arg²⁶, Lys³⁴(N^{ε}-octanoyl), β-Ala³⁵)hGLP-1(7-36)NH₂; | (SEQ ID NO.143) |
| (Gly⁸, Arg²⁶, Lys³⁴(N^{ε}-tetradecanoyl), β-Ala³⁵)hGLP-1(7-36)NH₂; | (SEQ ID NO.144) |
| (Gly⁸, Arg²⁶, Lys³⁴(N^{ε}-hexadecanoyl), β-Ala³⁵)hGLP-1(7-36)NH₂; | (SEQ ID NO.145) |
| (Gly⁸, Arg²⁶ Lys³⁴(N^{ε}-decanoyl), β-Ala³⁵)hGLP-1(7-36)NH₂; | (SEQ ID NO.146) |
| (Gly⁸, Arg^{25,26}, Lys³⁴(N^{ε}-octanoyl), β-Ala³⁵)hGLP-1(7-36)NH₂; | (SEQ ID NO.147) |
| (Gly⁸, Arg^{25,26}, Lys³⁴(N^{ε}-tetradecanoyl), β-Ala³⁵)hGLP-1(7-36)NH₂; | (SEQ ID NO.148) |
| (Gly⁸, Arg^{25,26}, Lys³⁴(N^{ε}-hexadecanoyl), β-Ala³⁵)hGLP-1(7-36)NH₂; | (SEQ ID NO.149) |
| (Gly⁸, Arg^{25,26}, Lys³⁴(N^{ε}-decanoyl), β-Ala³⁵)hGLP-1(7-36)NH₂; | (SEQ ID NO.150) |
| (Gly⁸, Lys²⁵, Arg²⁶, Lys³⁴(N^{ε}-octanoyl), β-Ala³⁵)hGLP-1(7-36)NH₂; | (SEQ ID NO.151) |
| (Gly⁸, Lys²⁵, Arg²⁶, Lys³⁴(N^{ε}-tetradecanoyl), β-Ala³⁵) | |
| hGLP-1(7-36)NH₂; | (SEQ ID NO.152) |
| (Gly⁸, Lys²⁵, Arg²⁶, Lys³⁴(N^{ε}-hexadecanoyl), β-Ala³⁵) | |
| hGLP-1(7-36)NH₂; | (SEQ ID NO.153) |
| (Gly⁸, β-Ala³⁵, Lys³⁶(N^{ε}-octanoyl))hGLP-1(7-36)NH₂; | (SEQ ID NO.154) |
| (Gly⁸, β-Ala³⁵, Lys³⁶(N^{ε}-tetradecanoyl))hGLP-1(7-36)NH₂; | (SEQ ID NO.155) |
| (Gly⁸, β-Ala³⁵, Lys³⁶(N^{ε}-hexadecanoyl))hGLP-1(7-36)NH₂; | (SEQ ID NO.156) |
| (Gly⁸, Arg²⁶, β-Ala³⁵, Lys³⁶(N^{ε}-octanoyl))hGLP-1(7-36)NH₂; | (SEQ ID NO.157) |
| (Gly⁸, Arg²⁶, β-Ala³⁵, Lys³⁶(N^{ε}-tetradecanoyl))hGLP-1(7-36)NH₂; | (SEQ ID NO.158) |
| (Gly⁸, Arg²⁶, β-Ala³⁵, Lys³⁶(N^{ε}-hexadecanoyl))hGLP-1(7-36)NH₂; | (SEQ ID NO.159) |
| (Gly⁸, Arg^{26,34}, β-Ala³⁵, Lys³⁶(N^{ε}-octanoyl))hGLP-1(7-36)NH₂; | (SEQ ID NO.160) |
| (Gly⁸, Arg^{26,34}, β-Ala³⁵, Lys³⁶(N^{ε}-tetradecanoyl))hGLP-1(7-36)NH₂; | (SEQ ID NO.161) |
| (Gly⁸, Arg^{26,34}, β-Ala³⁵, Lys³⁶(N^{ε}- hexadecanoyl))hGLP-1(7-36)NH₂; | (SEQ ID NO.162) |
| (Gly⁸, Arg^{26,34}, β-Ala³⁵ Lys³⁶(N^{ε}-decanoyl))hGLP-1(7-36)NH₂; | (SEQ ID NO.163) |
| (Gly⁸, Lys²⁵, Arg^{26,34}, β-Ala³⁵, Lys³⁶(N^{ε}-octanoyl))hGLP-1(7-36)NH₂; | (SEQ ID NO.164) |
| (Gly⁸, Lys²⁵, Arg^{26,34}, Lys³⁶(N^{ε}-tetradecanoyl), β-Ala³⁵) | |
| hGLP-1(7-36)NH₂; | (SEQ ID NO.165) |
| (Gly⁸, Lys²⁵, Arg^{26,34}, β-Ala³⁵, Lyₛ³⁶(N^{ε}-hexadecanoyl)) | |
| hGLP-1(7-36)NH₂; | (SEQ ID NO.166) |
| (Gly⁸, Arg^{25,26,34}, β-Ala³⁵, Lys³⁶(N^{ε}-octanoyl))hGLP-1(7-36)NH₂; | (SEQ ID NO.167) |
| (Gly⁸, Arg^{25,26,34}, β-Ala³⁵, Lys³⁶(N^{ε}-tetradecanoyl))hGLP-1(7-36)NH₂; | (SEQ ID NO.168) |
| (Gly⁸, Arg^{25,26,34}, β-Ala³⁵, Lys³⁶(N^{ε}-hexadecanoyl))hGLP-1(7-36)NH₂; | (SEQ ID NO.169) |
| (Gly⁸, Arg^{25,26,34}, β-Ala³⁵, Lys³⁶(N^{ε}-decanoyl))hGLP-1(7-36)NH₂; | (SEQ ID NO.170) |
| (Gly⁸, Aib³⁵, LYS²⁶(N^{ε}-octanoyl), A6c³², Arg³⁴)hGLP-1(7-36)NH₂; | (SEQ ID NO.171) |
| (Gly⁸, Aib³⁵, Lys²⁶(N^{ε}-tetradecanoyl), A6c³², Arg³⁴) | |
| hGLP-1(7-36)NH₂ ; | (SEQ ID NO.172) |
| (Gly⁸, Aib³⁵, Lys²⁶(N^{ε}-hexadecanoyl), A6c³², Arg³⁴) | |
| hGLP-1(7-36) NH₂ ; | (SEQ ID NO.173) |
| (Gly⁸, Aib³⁵, A6c³², Lys³⁴(N^{ε}-octanoyl))hGLP-1(7-36)NH₂; | (SEQ ID NO.174) |
| (Gly⁸, Aib³⁵, A6c³², Lys³⁴(N^{ε}-tetradecanoyl))hGLP-1(7-36)NH₂; | (SEQ ID NO.175) |
| (Gly⁸, Aib³⁵, A6c³², Lys³⁴(N^{ε}-hexadecanoyl))hGLP-1(7-36)NH₂; | (SEQ ID NO.176) |
| (Gly⁸, Aib³⁵, Arg²⁶, A6c³², Lys³⁴(N^{ε}-octanoyl))hGLP-1(7-36)NH₂; | (SEQ ID NO.177) |
| (Gly⁸, Aib³⁵, Arg²⁶, A6c³², Lys³⁴(N^{ε}-tetradecanoyl))hGLP-1(7-36)NH₂; | (SEQ ID NO.178) |
| (Gly⁸, Aib³⁵, A6c³², Lys³⁶(N^{ε}-octanoyl))hGLP-1(7-36)NH₂; | (SEQ ID NO.179) |
| (Gly⁸, Aib³⁵, A6c³², Lys³⁶(N^{ε}-tetradecanoyl))hGLP-1(7-36)NH₂; | (SEQ ID NO.180) |
| (Gly⁸, Aib³⁵, A6c³², Lys³⁶(N^{ε}-hexadecanoyl))hGLP-1(7-36)NH₂; | (SEQ ID NO.181) |
| (Gly⁸, Aib³⁵, Arg²⁶, A6c³², Lys³⁶(N^{ε}-octanoyl))hGLP-1(7-36)NH₂; | (SEQ ID NO.182) |
| (Gly⁸, Aib³⁵, Arg²⁶, A6c³², Lys³⁶(N^{ε}-tetradecanoyl))hGLP-1(7-36)NH₂; | (SEQ ID NO.183) |
| (Gly⁸, Aib³⁵, Arg²⁶, A6c³², Lys³⁶(N^{ε}-hexadecanoyl))hGLP-1(7-36)NH₂; | (SEQ ID NO.184) |
| (Gly⁸, Aib³⁵, Arg^{26,34}, A6c³², Lys³⁶(N^{ε}-octanoyl))hGLP-1(7-36)NH₂; | (SEQ ID NO.185) |
| (Gly⁸, Aib³⁵, Arg^{26,34}, A6c³², Lys³⁶(N^{ε}-decanoyl))hGLP-17-36)NH₂; | (SEQ ID NO.186) |
| (Gly⁸, Aib³⁵, Arg^{26,34}, A6c³², Lys³⁶(N^{ε}-tetradecanoyl)) | |
| hGLP-1(7-36)NH₂; | (SEQ ID NO.187) |
| (Gly⁸, Aib³⁵, Arg^{26,34}, A6c³², Lys³⁶(N^{ε}-hexadecanoyl)) | |
| hGLP-1(7-36)NH₂; | (SEQ ID NO.188) |
| (Gly⁸, Aib^{24,35}, Lys²⁶(N^{ε}-octanoyl), Arg³⁴)hGLP-1(7-36)NH₂; | (SEQ ID NO.189) |
| (Gly⁸, Aib^{24,35}, Lys²⁶(N^{ε}-tetradecanoyl), Arg³⁴)hGLP-1(7-36)NH₂; | (SEQ ID NO.190) |
| (Gly⁸, Aib^{24,35}, Lys²⁶(N^{ε}-hexadecanoyl), Arg³⁴)hGLP-1(7-36)NH₂; | (SEQ ID NO.191) |
| (Gly⁸, Aib^{24,35}, Arg²⁶ Lys³⁴(N^{ε}-octanoyl))hGLP-1(7-36)NH₂; | (SEQ ID NO.192) |
| (Gly⁸, Aib^{24,35}, Arg²⁶ Lys³⁴(N^{ε}-tetradecanoyl))hGLP-1(7-36)NH₂; | (SEQ ID NO.193) |
| (Gly⁸, Aib^{24,35}, Arg²⁶ Lys³⁴(N^{ε}-hexadecanoyl))hGLP-1(7-36)NH₂; | (SEQ ID NO.194) |
| (Gly⁸, Aib^{24,35}, Arg^{26,34}, Lys³⁶(N^{ε}-octanoyl))hGLP-1(7-36)NH₂; | (SEQ ID NO.195) |
| (Gly⁸, Aib^{24,35}, Arg^{26,34}, Lys³⁶(N^{ε}-tetradecanoyl))hGLP-1(7-36)NH₂; | (SEQ ID NO.196) |
| (Gly⁸, Aib^{24,35}, Arg^{26,34}, Lys³⁶(N^{ε}-hexadecanoyl))hGLP-1(7-36)NH₂; | (SEQ ID NO.197) |
| (Gly⁸, Aib^{24,35}, Glu²³, A6c³², Lys³⁴(N^{e}-octanoyl))hGLP-1(7-36)NH₂; | (SEQ ID NO.198) |
| (Gly⁸, Aib³⁵, Glu²³, Lys²⁶(N^{ε}-octanoyl), Arg³⁴)hGLP-1(7-36)NH₂; | (SEQ ID NO.199) |
| (Gly⁸, Aib³⁵, Glu²³, Lys²⁶(N^{ε}-tetradecanoyl), Arg³⁴)hGLP-1(7-36)NH₂; | (SEQ ID NO.200) |
| (Gly⁸, Aib³⁵, Glu²³, Lys²⁶(N^{ε}-hexadecanoyl), Arg³⁴)hGLP-1(7-36)NH₂; | (SEQ ID NO.201) |
| (Gly⁸, Aib³⁵, Glu²³, Lys³⁴(N^{e}-octanoyl))hGLP-1(7-36)NH₂; | (SEQ ID NO.202) |
| (Gly⁸, Aib³⁵, Glu²³, A6c³², Lys³⁴(N^{e}-octanoyl))hGLP-1(7-36)NH₂; | (SEQ ID NO.203) |
| (Gly⁸, Aib³⁵, Glu²³, Arg²⁶, Lys³⁴(N^{ε}-octanoyl))hGLP-1(7-36)NH₂; | (SEQ ID NO.204) |
| (Gly⁸, Aib³⁵, Glu²³, Arg²⁶, Lys³⁴(N^{ε}-tetradecanoyl))hGLP-1(7-36)NH₂; | (SEQ ID NO.205) |
| (Gly⁸, Aib³⁵, Glu²³, Arg²⁶, Lys³⁴(N^{ε}-hexadecanoyl))hGLP-1(7-36)NH₂; | (SEQ ID NO.206) |
| (Gly⁸, Aib³⁵, Glu²³, Lys³⁶(N^{ε}-octanoyl))hGLP-1(7-36)NH₂; | (SEQ ID NO.207) |
| (Gly⁸, Aib³⁵, Glu²³, Lys³⁶(N^{ε}-tetradecanoyl))hGLP-1(7-36)NH₂; | (SEQ ID NO.208) |
| (Gly⁸, Aib³⁵, Glu²³, Lys³⁶(N^{ε}-hexadecanoyl))hGLP-1(7-36)NH₂; | (SEQ ID NO.209) |
| (Gly⁸, Aib³⁵, Glu²³, Arg^{26,34}, Lys³⁶(N^{ε}-octanoyl))hGLP-1(7-36)NH₂; | (SEQ ID NO.210) |
| (Gly⁸, Aib³⁵, Glu²³, Arg^{26,34}, Lys³⁶(N^{ε}-tetradecanoyl)) | |
| hGLP-1(7-36)NH₂; | (SEQ ID NO.211) |
| (Gly⁸, Aib³⁵, Glu²³, Arg^{26,34}, Lys³⁶(N^{ε}-hexadecanoyl)) | |
| hGLP-1(7-36)NH₂; | (SEQ ID NO.212) |
| (Gly⁸, Aib^{30,35}, Lys²⁶(N^{ε}-octanoyl), Arg³⁴)hGLP-1(7-36)NH₂; | (SEQ ID NO.213) |
| (Gly⁸, Aib^{30,35}, Lys²⁶(N^{ε}**-**tetradecanoyl), Arg³⁴)hGLP-1(7-36)NH₂; | (SEQ ID NO.214) |
| (Gly⁸, Aib^{30,35}, Lys²⁶(N^{ε}-hexadecanoyl), Arg³⁴)hGLP-1(7-36)NH₂; | (SEQ ID NO.215) |
| (Gly⁸, Aib^{30,35}, Arg²⁶ Lys³⁴(N^{ε}-octanoyl))hGLP-1(7-36)NH₂; | (SEQ ID NO.216) |
| (Gly⁸, Aib^{30,35}, Arg²⁶ Lys³⁴(N^{ε}-tetradecanoyl))hGLP-1(7-36)NH₂; | (SEQ ID NO.217) |
| (Gly⁸, Aib^{30,35}, Arg²⁶ Lys³⁴(N^{ε}-hexadecanoyl))hGLP-1(7-36)NH₂; | (SEQ ID NO.218) |
| (Gly⁸, Aib^{30,35}, Arg^{26,34}, Lys³⁶(N^{ε}-octanoyl))hGLP-1(7-36)NH₂; | (SEQ ID NO.219) |
| (Gly⁸, Aib^{30,35}, Arg^{26,34}, Lys³⁶(N^{ε}-tetradecanoyl))hGLP-1(7-36)NH₂; | (SEQ ID NO.220) |
| (Gly⁸, Aib^{30,35}, Arg^{26,34}, Lys³⁶(N^{ε}-hexadecanoyl))hGLP-1(7-36)NH₂; | (SEQ ID NO.221) |
| (Gly⁸, Aib³⁵, Glu²³, A6c³², Lys³⁶(N^{ε}-octanoyl))hGLP-1(7-36)NH₂; | (SEQ ID NO.222) |
| (Gly⁸, Aib³⁵, Glu²³, A6c³², Lys³⁶(N^{ε}-tetradecanoyl))hGLP-1(7-36)NH₂; | (SEQ ID NO.223) |
| (Gly⁸, Aib³⁵, Glu²³, A6c³², Lys³⁶(N^{ε}-hexadecanoyl))hGLP-1(7-36)NH₂; | (SEQ ID NO.224) |
| (Gly⁸, Aib³⁵, Glu²³, Arg^{26,34}, A6c³², Lys³⁶(N^{ε}-octanoyl)) | |
| hGLP-1(7-36)NH₂; | (SEQ ID NO.225) |
| (Gly⁸, Aib³⁵, Glu²³, Arg^{26,34}, A6c³², Lys³⁶(N^{ε}-tetradecanoyl)) | |
| hGLP-1(7-36)NH₂; | (SEQ ID NO.226) |
| (Gly⁸, Aib³⁵, Glu²³, Arg^{26,34}, A6c³², Lys³⁶(N^{ε}-hexadecanoyl)) | |
| hGLP-1(7-36)NH₂; | (SEQ ID NO.227) |
| (Gly⁸, Aib^{24,35}, Glu²³, Arg^{26,34}, A6c³², Lys³⁶(N^{ε}-octanoyl)) | |
| hGLP-1(7-36)NH₂; | (SEQ ID NO.228) |
| (Gly⁸, Aib^{24,35}, Glu²³, Arg^{26,34}, A6c³², Lys³⁶(N^{ε}-tetradecanoyl)) | |
| hGLP-1(7-36)NH₂; | (SEQ ID NO.229) |
| (Gly⁸, Aib^{24,35}, Glu²³, Arg^{26,34}, A6c³², Lys³⁶(N^{ε}-hexadecanoyl)) | |
| hGLP-1(7-36)NH₂; | (SEQ ID NO.230) |
| (Gly⁸, Aib^{24,30,35}, Glu²³, Arg^{26,34}, A6c³², Lys³⁶(N^{ε}-octanoyl)) | |
| hGLP-1(7-36)NH₂; | (SEQ ID NO.231) |
| (Gly⁸, Aib^{24,30,35}, Glu²³, Arg^{26,34}, A6c³², Lys³⁶(N^{ε}-tetradecanoyl)) | |
| hGLP-1(7-36)NH₂; | (SEQ ID NO.232) |
| (Gly⁸, Aib^{24,30,35}, Glu²³, Arg^{26,34}, A6c³², Lys³⁶(N^{ε}-hexadecanoyl)) | |
| hGLP-1(7-36)NH₂; | (SEQ ID NO.233) |
| ((N^{α}-HEPES-His)⁷, Gly⁸, β-Ala³⁵)hGLP-1(7-36)NH₂; | (SEQ ID NO.234) |
| ((N^{α}-HEPA-His)7, Gly⁸, β-Ala³⁵)hGLP-1(7-36)NH₂; | (SEQ ID NO.235) |
| ((N^{α}-tetradecanoyl-His)⁷ Gly⁸, Aib³⁵)hGLP-1(7-36)NH₂; | (SEQ ID NO.236) |
| ((N^{a}-tetradecanoyl-His)⁷ Gly⁸, β-Ala³⁵)hGLP-1(7-36)NH₂; | (SEQ ID NO.237) |
| ((N^{α}-tetradecanoyl-His)⁷ Gly⁸, Aib³⁵, Arg^{26,34}) | |
| hGLP-1(7-36)NH₂; | (SEQ ID NO.238) |
| ((N^{α}-tetradecanoyl-His)⁷, Gly⁸, Arg^{26,34}, β-Ala³⁵) | |
| hGLP-1(7-36)NH₂; | (SEQ ID NO.239) |
| ((N^{α}-tetradecanoyl-His)⁷, Gly⁸, Aib³⁵, Arg^{25,26,34}) | |
| hGLP-1(7-36)NH₂; | (SEQ ID NO.240) |
| ((N^{α}-tetradecanoyl-His)⁷, Gly⁸, Arg^{25,26,34}, β-Ala³⁵) | |
| hGLP-1(7-36)NH₂; | (SEQ ID NO.241) |
| (Gly⁸, Aib³⁵, Lys²⁶(N^{ε}-octanesulfonyl), Arg³⁴) | |
| hGLP-1(7-36)NH₂; | (SEQ ID NO.242) |
| (Gly⁸, Aib³⁵, Lys²⁶(N^{ε}-dodecanesulfonyl), Arg³⁴) | |
| hGLP-1(7-36)NH₂; | (SEQ ID NO.243) |
| (Gly⁸, Aib³⁵, Lys²⁶(N^{ε}-hexadecanesulfonyl), Arg³⁴) | |
| hGLP-1(7-36)NH₂; | (SEQ ID NO.244) |
| (Gly⁸, Aib³⁵, Arg²⁶, Lys³⁴(N^{ε}-octanesulfonyl)) | |
| hGLP-1(7-36)NH₂; | (SEQ ID NO.245) |
| (Gly⁸, Aib³⁵, Arg²⁶, Lys³⁴(N^{ε}-dodecanesulfonyl)) | |
| hGLP-1(7-36)NH₂; | (SEQ ID NO.246) |
| (Gly⁸, Aib³⁵, Arg²⁶, Lys³⁴(N^{ε}-hexadecanesulfonyl)) | |
| hGLP-1(7-36)NH₂; | (SEQ ID NO.247) |
| (Gly⁸, Aib³⁵, Arg^{26,34}, Lys³⁶(N^{ε}-octanesulfonyl)) | |
| hGLP-1(7-36)NH₂; | (SEQ ID NO.248) |
| (Gly⁸, Aib³⁵, Arg^{26,34}, Lys³⁶(N^{ε}-hexadecanesulfonyl)) | |
| hGLP-1(7-36)NH₂; | (SEQ ID NO.249) |
| (Gly⁸, Aib³⁵, Asp²⁶(1-(4-decylpiperazine)), Arg³⁴) | |
| hGLP-1(7-36)NH₂; | (SEQ ID NO.250) |
| (Gly⁸, Aib³⁵, Asp²⁶(1-(4-dodecylpiperazine)), Arg³⁴) | |
| hGLP-1(7-36)NH₂; | (SEQ ID NO.251) |
| (Gly⁸, Aib³⁵, Asp²⁶(1-(4-tetradecylpiperazine)), Arg³⁴) | |
| hGLP-1(7-36)NH₂; | (SEQ ID NO.252) |
| (Gly⁸, Aib³⁵, Asp²⁶(1-(4-hexadecylpiperazine)), Arg³⁴) | |
| hGLP-1(7-36)NH₂; | (SEQ ID NO.253) |
| (Gly⁸, Aib³⁵, Arg²⁶, Asp³⁴(1-(4-decylpiperazine))) | |
| hGLP-1(7-36)NH₂; | (SEQ ID NO.254) |
| (Gly⁸, Aib³⁵, Arg²⁶, Asp³⁴(1-(4-dodecylpiperazine))) | |
| hGLP-1(7-36)NH₂; | (SEQ ID NO.255) |
| (Gly⁸, Aib³⁵, Arg²⁶, Asp³⁴(1-(4-tetradecylpiperazine))) | |
| hGLP-1(7-36)NH₂; | (SEQ ID NO.256) |
| (Gly⁸, Aib³⁵, Arg²⁶, Asp³⁴(1-(4-hexadecylpiperazine))) | |
| hGLP-1(7-36)NH₂; | (SEQ ID NO.257) |
| (Gly⁸, Aib³⁵, Arg^{26,34}, Asp³⁶(1-(4-decylpiperazine))) | |
| hGLP-1(7-36)NH₂; | (SEQ ID NO.258) |
| (Gly⁸, Aib³⁵, Arg^{26,34}, Asp³⁶(1-(4-dodecylpiperazine))) | |
| hGLP-1(7-36)NH₂; | (SEQ ID NO.259) |
| (Gly⁸, Aib³⁵, Arg^{26,34}, Asp³⁶(1-(4-hexadecylpiperazine))) | |
| hGLP-1(7-36)NH₂; | (SEQ ID NO.260) |
| (Gly⁸, Aib³⁵, Arg^{26,34}, Asp³⁸(1-(4-decylpiperazine))) | |
| hGLP-1(7-38)NH₂; | (SEQ ID NO.261) |
| (Gly⁸, Aib³⁵, Arg^{26,34}, Asp³⁸(1-(4-dodecylpiperazine))) | |
| hGLP-1(7-38)NH₂; | (SEQ ID NO.262) |
| (Gly⁸, Aib³⁵, Arg^{26,34}, Asp³⁸(1-(4-tetradecylpiperazine))) | |
| hGLP-1(7-38)NH₂; | (SEQ ID NO.263) |
| (Gly⁸, Aib³⁵, Arg^{26,34}, Asp³⁸(1-(4-hexadecylpiperazine))) | |
| hGLP-1(7-38)NH₂; | (SEQ ID NO.264) |
| (Gly⁸, Aib^{35,37}, Arg^{26,34}, Asp³⁸(1-(4-decylpiperazine))) | |
| hGLP-1(7-38)NH₂; | (SEQ ID NO.265) |
| (Gly⁸, Aib^{35,37}, Arg^{26,34}, Asp³⁸(1-(4-dodecylpiperazine))) | |
| hGLP-1(7-38)NH₂; | (SEQ ID NO.266) |
| (Gly⁸, Aib^{35,37}, Arg^{26,34}, Asp³⁸(1-(4-tetradecylpiperazine))) | |
| hGLP-1(7-38)NH₂; | (SEQ ID NO.267) |
| (Gly⁸, Aib^{35,37}, Arg^{26,34}, Asp³⁸(1-(4-hexadecylpiperazine))) | |
| hGLP-1(7-38)NH₂; | (SEQ ID NO.268) |
| (Gly⁸, Aib³⁵, Arg^{25,34}, Asp²⁶(1-(4-decylpiperazine))) | |
| hGLP-1(7-36)NH₂; | (SEQ ID NO.269) |
| (Gly⁸, Aib³⁵, Arg^{25,34}, Asp²⁶(1-(4-dodecylpiperazine))) | |
| hGLP-1(7-36)NH₂; | (SEQ ID NO.270) |
| (Gly⁸, Aib³⁵, Arg^{25,34}, Asp²⁶(1-(4-tetradecylpiperazine))) | |
| hGLP-1(7-36)NH₂; | (SEQ ID NO.271) |
| (Gly⁸, Aib³⁵, Arg^{25,34}, Asp²⁶(1-(4-hexadecylpiperazine))) | |
| hGLP-1(7-36)NH₂; | (SEQ ID NO.272) |
| (Gly⁸, Aib³⁵, Arg^{25,26}, Asp³⁴(1-(4-decylpiperazine))) | |
| hGLP-1(7-36)NH₂; | (SEQ ID NO.273) |
| (Gly⁸, Aib³⁵, Arg^{25,26}, Asp³⁴(1-(4-dodecylpiperazine))) | |
| hGLP-1(7-36)NH₂; | (SEQ ID NO.274) |
| (Gly⁸, Aib³⁵, Arg^{25,26}, Asp³⁴(1-(4-tetradecylpiperazine))) | |
| hGLP-1(7-36)NH₂; | (SEQ ID NO.275) |
| (Gly⁸, Aib³⁵, Arg^{25,26}, Asp³⁴(1-(4-hexadecylpiperazine))) | |
| hGLP-1(7-36)NH₂; | (SEQ ID NO.276) |
| (Gly⁸, Aib³⁵, Arg^{25,26,34}, Asp³⁶(1-(4-decylpiperazine))) | |
| hGLP-1(7-36)NH₂; | (SEQ ID NO.277) |
| (Gly⁸, Aib³⁵, Arg^{25,26,34}, Asp³⁶(1-(4-dodecylpiperazine))) | |
| hGLP-1(7-36)NH₂; | (SEQ ID NO.278) |
| (Gly⁸, Aib³⁵, Arg^{25,26,34}, Asp³⁶(1-(4-tetradecylpiperazine))) | |
| hGLP-1(7-36)NH₂; | (SEQ ID NO.279) |
| (Gly⁸, Aib³⁵, Arg^{25,26,34}, Asp³⁶(1-(4-hexadecylpiperazine))) | |
| hGLP-1(7-36)NH₂; | (SEQ ID NO.280) |
| (Gly⁸, Aib³⁵, Arg^{25,26,34}, Asp³⁸(1-(4-decylpiperazine))) | |
| hGLP-1(7-38)NH₂; | (SEQ ID NO.281) |
| (Gly⁸, Aib³⁵, Arg^{25,26,34}, Asp³⁸(1-(4-dodecylpiperazine))) | |
| hGLP-1(7-38)NH₂; | (SEQ ID NO.282) |
| (Gly⁸, Aib³⁵, Arg^{25,26,34}, Asp³⁸(1-(4-tetradecylpiperazine))) | |
| hGLP-1(7-38)NH₂; | (SEQ ID NO.283) |
| (Gly⁸, Aib³⁵, Arg^{25,26,34}, Asp³⁸(1-(4-hexadecylpiperazine))) | |
| hGLP-1(7-38)NH₂; | (SEQ ID NO.284) |
| (Gly⁸, Aib^{35,37}, Arg^{25,26,34}, Asp³⁸(1-(4-decylpiperazine))) | |
| hGLP-1(7-38)NH₂; | (SEQ ID NO.285) |
| (Gly⁸, Aib^{35,37}, Arg^{25,26,34}, Asp³⁸(1-(4-dodecylpiperazine))) | |
| hGLP-1(7-38)NH₂; | (SEQ ID NO.286) |
| (Gly⁸, Aib^{35,37}, Arg^{25,26,34}, Asp³⁸(1-(4-tetradecylpiperazine))) | |
| hGLP-1(7-38)NH₂; | (SEQ ID NO.287) |
| (Gly⁸, Aib^{35,37}, Arg^{25,26,34}, Asp³⁸(1-(4-hexadecylpiperazine))) | |
| hGLP-1(7-38)NH₂; | (SEQ ID NO.288) |
| (Gly⁸, Aib³⁵, Arg^{26,34}, Glu³⁶(1-dodecylamino)) | |
| hGLP-1(7-36)NH₂; | (SEQ ID NO.289) |
| (Gly⁸, Aib³⁵, Glu²⁶(1-dodecylamino), Arg³⁴) | |
| hGLP-1(7-36)NH₂; | (SEQ ID NO.290) |
| (Gly⁸, Aib³⁵, Arg²⁶, Glu³⁴(1-dodecylamino)) | |
| hGLP-1(7-36)NH₂; | (SEQ ID NO.291) |
| (Gly⁸, Aib^{35,37}, Arg^{26,34}, Glu³⁸(1-dodecylamino)) | |
| hGLP-1(7-38)NH₂; | (SEQ ID NO.292) |
| (Gly⁸, Aib³⁵, Arg³⁴, Lys²⁶(N^{ε}-(2-(4-decyl-1-piperazine)-acetyl))) | |
| hGLP-1(7-36)NH₂; | (SEQ ID NO.293) |
| (Gly⁸, Aib³⁵, Arg³⁴, Lys²⁶(N^{ε}-(2-(4-dodecyl-1-piperazine)-acetyl))) | |
| hGLP-1(7-36)NH₂; | (SEQ ID NO.294) |
| (Gly⁸, Aib³⁵, Arg³⁴, Lys²⁶(N^{ε}-(2-(4-tetradecyl-1-piperazine)-acetyl))) | |
| hGLP-1(7-36)NH₂; | (SEQ ID NO.295) |
| (Gly⁸, Aib³⁵, Arg³⁴, Lys²⁶(N^{ε}-(2-(4-hexadecyl-1-piperazine)-acetyl))) | |
| hGLP-1(7-36)NH₂; | (SEQ ID NO.296) |
| (Gly⁸, Aib³⁵, Arg²⁶, Lys³⁴(N^{ε}-(2-(4-decyl-1-piperazine)-acetyl))) | |
| hGLP-1(7-36)NH₂; | (SEQ ID NO.297) |
| (Gly⁸, Aib³⁵, Arg²⁶, Lys³⁴(N^{ε}-(2-(4-dodecyl-1-piperazine)-acetyl)) | |
| hGLP-1(7-36)NH₂; | (SEQ ID NO.298) |
| (Gly⁸, Aib³⁵, Arg²⁶, Lys³⁴(N^{ε}-(2-(4-tetradecyl-1-piperazine)-acetyl))) | |
| hGLP-1(7-36)NH₂; | (SEQ ID NO.299) |
| (Gly⁸, Aib³⁵, Arg²⁶, Lys³⁴(N^{ε}-(2-(4-hexadecyl-1-piperazine)-acetyl))) | |
| hGLP-1(7-36)NH₂; | (SEQ ID NO.300) |
| (Gly⁸, Aib³⁵, Arg^{26,34}, Lys³⁶(N^{ε}-(2-(4-decyl-1-piperazine)-acetyl))) | |
| hGLP-1(7-36)NH₂; | (SEQ ID NO.301) |
| (Gly⁸, Aib³⁵, Arg^{26,34}, Lys³⁶(N^{ε}-(2-(4-dodecyl-1-piperazine)-acetyl))) | |
| hGLP-1(7-36)NH₂; | (SEQ ID NO.302) |
| (Gly⁸, Aib³⁵, Arg^{26,34}, Lys³⁶(N^{ε}-(2-(4-hexadecyl-1-piperazine)-acetyl))) | |
| hGLP-1(7-36)NH₂; | (SEQ ID NO.303) |
| (Gly⁸, Aib³⁵, Arg^{26,34}, Lys³⁸(N^{ε}-(2-(4-decyl-1-piperazine)-acetyl))) | |
| hGLP-1(7-38)NH₂; | (SEQ ID NO.304) |
| (Gly⁸, Aib³⁵, Arg^{26,34}, Lys³⁸(N^{ε}-(2-(4-dodecyl-1-piperazine)-acetyl))) | |
| hGLP-1(7-38)NH₂; | (SEQ ID NO.305) |
| (Gly⁸, Aib³⁵, Arg^{26,34}, Lys³⁸(N^{ε}-(2-(4-tetradecyl-1-piperazine)-acetyl))) | |
| hGLP-1(7-38)NH₂; | (SEQ ID NO.306) |
| (Gly⁸, Aib³⁵, Arg^{26,34}, Lys³⁸(N^{ε}-(2-(4-hexadecyl-1-piperazine)-acetyl))) | |
| hGLP-1(7-38)NH₂; | (SEQ ID NO.307) |
| (Gly⁸, Aib^{35,37}, Arg^{26,34}, Lys³⁸(N^{ε}-(2-(4-decyl-1-piperazine)-acetyl))) | |
| hGLP-1(7-38)NH₂; | (SEQ ID NO.308) |
| (Gly⁸, Aib^{35,37}, Arg^{26,34}, Lys³⁸(N^{ε}-(2-(4-dodecyl-1-piperazine)-acetyl))) | |
| hGLP-1(7-38)NH₂; | (SEQ ID NO.309) |
| (Gly⁸, Aib^{35,37}, Arg^{26,34}, Lys³⁸(N^{ε}-(2-(4-tetradecyl-1-piperazine)-acetyl))) | |
| hGLP-1(7-38)NH₂; | (SEQ ID NO.310) |
| (Gly⁸, Aib^{35,37}, Arg^{26,34}, Lys³⁸(N^{ε}-(2-(4-hexadecyl-1-piperazine)-acetyl))) | |
| hGLP-1(7-38)NH₂; | (SEQ ID NO.311) |
| (Gly⁸, Aib³⁵, Arg^{25,34}, Lys²⁶(N^{ε}-(2-(4-decyl-1-piperazine)-acetyl))) | |
| hGLP-1(7-36)NH₂; | (SEQ ID NO.312) |
| (Gly⁸, Aib³⁵, Arg^{25,34}, Lys²⁶(N^{ε}-(2-(4-dodecyl-1-piperazine)-acetyl))) | |
| hGLP-1(7-36)NH₂; | (SEQ ID NO.313) |
| (Gly⁸, Aib³⁵, Arg^{25,34}, Lys²⁶(N^{ε}-(2-(4-tetradecyl-1-piperazine)-acetyl))) | |
| hGLP-1(7-36)NH₂; | (SEQ ID NO.314) |
| (Gly⁸, Aib³⁵, Arg^{25,34}, Lys²⁶(N^{ε}-(2-(4-hexadecyl-1-piperazine)-acetyl))) | |
| hGLP-1(7-36)NH₂; | (SEQ ID NO.315) |
| (Gly⁸, Aib³⁵, Arg^{25,26}, Lys³⁴(N^{ε}-(2-(4-decyl-1-piperazine)-acetyl))) | |
| hGLP-1(7-36)NH₂; | (SEQ ID NO.316) |
| (Gly⁸, Aib³⁵, Arg^{25,26}, Lys³⁴(N^{ε}-(2-(4-dodecyl-1-piperazine)-acetyl))) | |
| hGLP-1(7-36)NH₂; | (SEQ ID NO.317) |
| (Gly⁸, Aib³⁵, Arg^{25,26}, Lys³⁴(N^{ε}-(2-(4-tetradecyl-1-piperazine)-acetyl))) | |
| hGLP-1(7-36)NH₂; | (SEQ ID NO.318) |
| (Gly⁸, Aib³⁵, Arg^{25,26}, Lys³⁴(N^{ε}-(2-(4-hexadecyl-1-piperazine)-acetyl))) | |
| hGLP-1(7-36)NH₂; | (SEQ ID NO.319) |
| (Gly⁸, Aib³⁵, Arg^{25,26,34}, Lys³⁶(N^{ε}-(2-(4-decyl-1-piperazine)-acetyl))) | |
| hGLP-1(7-36)NH₂; | (SEQ ID NO.320) |
| (Gly⁸, Aib³⁵, Arg^{25,26,34}, Lys³⁶(N^{ε}-(2-(4-dodecyl-1-piperazine)-acetyl))) | |
| hGLP-1(7-36)NH₂; | (SEQ ID NO.321) |
| (Gly⁸, Aib³⁵, Arg^{25,26,34}, Lys³⁶(N^{ε}-(2-(4-tetradecyl-1-piperazine)-acetyl))) | |
| hGLP-1(7-36)NH₂; | (SEQ ID NO.322) |
| (Gly⁸, Aib³⁵, Arg^{25,26,34}, Lys³⁶(N^{ε}-(2-(4-hexadecyl-1-piperazine)-acetyl))) | |
| hGLP-1(7-36)NH₂; | (SEQ ID NO.323) |
| (Gly⁸, Aib³⁵, Arg^{25,26,34}, Lys³⁸(N^{ε}-(2-(4-decyl-1-piperazine)-acetyl))) | |
| hGLP-1(7-38)NH₂; | (SEQ ID NO.324) |
| (Gly⁸, Aib³⁵, Arg^{25,26,34}, Lys³⁸(N^{ε}-(2-(4-dodecyl-1-piperazine)-acetyl))) | |
| hGLP-1(7-38)NH₂; | (SEQ ID NO.325) |
| (Gly⁸, Aib³⁵, Arg^{25,26,34}, Lys³⁸(N^{ε}-(2-(4-tetradecyl-1-piperazine)-acetyl))) | |
| hGLP-1(7-38)NH₂; | (SEQ ID NO.326) |
| (Gly⁸, Aib³⁵, Arg^{25,26,34}, Lys³⁸(N^{ε}-(2-(4-hexadecyl-1-piperazine)-acetyl))) | |
| hGLP-1(7-38)NH₂; | (SEQ ID NO.327) |
| (Gly⁸, Aib^{35,37}, Arg^{25,26,34}, Lys³⁸(N^{ε}-(2-(4-decyl-1-piperazine)-acetyl))) | |
| hGLP-1(7-38)NH₂; | (SEQ ID NO.328) |
| (Gly⁸, Aib^{35,37}, Arg^{25,26,34}, Lys³⁸(N^{ε}-(2-(4-dodecyl-1-piperazine)-acetyl))) | |
| hGLP-1(7-38)NH₂; | (SEQ ID NO.329) |
| (Gly⁸, Aib^{35,37}, Arg^{25,26,34}, Lys³⁸(N^{ε}-(2-(4-tetradecyl-1-piperazine)-acetyl))) | |
| hGLP-1(7-38)NH₂; | (SEQ ID NO.330) |
| (Gly⁸, Aib^{35,37}, Arg^{25,26,34}, Lys³⁸(N^{ε}-(2-(4-hexadecyl-1-piperazine)-acetyl))) | |
| hGLP-1(7-38)NH₂; | (SEQ ID NO.331) |
| (Gly⁸, Aib³⁵, Arg^{26,34}, Lys³⁶(N^{ε}-decanoyl))hGLP-1(7-36)OH; | (SEQ ID NO.332) |
| (Gly⁸, Aib³⁵, Lys²⁵, Arg^{26,34}, Lys³⁶(N^{ε}-decanoyl))hGLP-1(7-36)OH; | (SEQ ID NO.333) |
| (Gly⁸, Aib³⁵,Arg^{26,34}, Ava³⁷, Ado³⁸)hGLP-1(7-38)NH₂; | (SEQ ID NO.334) |
| (Gly⁸, Aib³⁵,Arg^{26,34}, Asp³⁷, Ava³⁸, Ado³⁹)hGLP-1(7-39)NH₂; | (SEQ ID NO.335) |
| (Gly⁸, Aib³⁵,Arg^{26,34}, Aun³⁷)hGLP-1(7-37)NH₂; | (SEQ ID NO.336) |
| (Gly⁸, Aib^{17,35})hGLP-1(7-36)NH₂; | (SEQ ID NO.337) |
| (Gly⁸ ,Arg^{26,34}, β-Ala³⁵, D-Asp³⁷, Ava³⁸, Aun³⁹)hGLP-1(7-39)NH₂; | (SEQ ID NO.338) |
| (Gly⁸, Glu^{22,23}, β-Ala³⁵)hGLP-1(7-36)NH₂; | (SEQ ID NO.339) |
| (Gly⁸, Lys¹⁸, β-Ala³⁵)hGLP-1(7-36)NH₂; | (SEQ ID NO.340) |
| (Gly⁸, Leu²⁷, β-Ala³⁵)hGLP-1(7-36)NH₂; | (SEQ ID NO.341) |
| (Gly⁸, Lys³³, β-Ala³⁵)hGLP-1(7-36)NH₂; | (SEQ ID NO.342) |
| (Gly⁸, Lys¹⁸, Leu²⁷, β-Ala³⁵)hGLP-1(7-36)NH₂; | (SEQ ID NO.343) |
| (Gly⁸, D-Arg³⁶)hGLP-1(7-36)NH₂; | (SEQ ID NO.344) |
| (Gly⁸, β-Ala³⁵, D-Arg³⁷)hGLP-1(7-37)NH₂; | (SEQ ID NO.345) |
| (Gly⁸, Aib²⁷, β-Ala³⁵)hGLP-1(7-36)NH₂; | (SEQ ID NO.346) |
| (Gly⁸, Aib²⁷, β-Ala^{35,37}, Arg³⁸)hGLP-1(7-38)NH₂; | (SEQ ID NO.347) |
| (Gly⁸, Aib²⁷, β-Ala^{35,37}, Arg^{38,39})hGLP-1(7-39)NH₂; | (SEQ ID NO.348) |
| (Gly⁸, Lys^{18,27}, β-Ala³⁵)hGLP-1(7-36)NH₂; | (SEQ ID NO.349) |
| (Gly⁸, Lys²⁷, β-Ala³⁵)hGLP-1(7-36)NH₂; | (SEQ ID NO.350) |
| (Gly⁸, β-Ala³⁵, Arg³⁸)hGLP-1(7-38)NH₂; | (SEQ ID NO.351) |
| (Gly⁸, Arg^{26,34}, β-Ala³⁵,)hGLP-1(7-36)NH₂; | (SEQ ID NO.352) |
| (Gly⁸, D-Arg³⁵)hGLP-1(7-36)NH₂; | (SEQ ID NO.353) |
| (Gly⁸, β-Ala³⁵, Arg³⁷)hGLP-1(7-37)NH₂; | (SEQ ID NO.354) |
| (Gly⁸, Phe³¹, β-Ala³⁵)hGLP-1(7-36)NH₂; | (SEQ ID NO.355) |
| (Gly⁸, Aib³⁵, Phe³¹)hGLP-1(7-36)NH₂; | (SEQ ID NO.356) |
| (Gly⁸, Aib³⁵, 2-Nal³¹)hGLP-1(7-36)NH₂; | (SEQ ID NO.357) |
| (Gly⁸, Aib³⁵, 2-Nal^{28,31})hGLP-1(7-36)NH₂; | (SEQ ID NO.358) |
| (Gly⁸, Aib³⁵, Arg^{26,34}, 2-Nal³¹)hGLP-1(7-36)NH₂; | (SEQ ID NO.359) |
| (Gly⁸, Aib³⁵, Arg^{26,34}, Phe³¹)hGLP-1(7-36)NH₂; | (SEQ ID NO.360) |
| (Gly⁸, Aib³⁵, 2-Nal^{19,31})hGLP-1(7-36)NH₂; | (SEQ ID NO.361) |
| (Gly⁸, Aib³⁵, 2-Nal^{12,31})hGLP-1(7-36)NH₂; | (SEQ ID NO.362) |
| (Gly⁸, Aib³⁵, Lys³⁶(N^{ε}-decanoyl))hGLP-1(7-36)NH₂; | (SEQ ID NO.363) |
| (Gly⁸, Aib³⁵, Arg³⁴, Lys²⁶(N^{ε}-decanoyl))hGLP-1(7-36)NH₂; | (SEQ ID NO.364) |
| (Gly⁸, Aib³⁵, Arg^{26,34}, Lys³⁶(N^{ε}-dodecanoyl))hGLP-1(7-36)NH₂; | (SEQ ID NO.365) |
| (Gly⁸, β-Ala³⁵,Ser³⁷(O-decanoyl))hGLP1(7-37)-NH₂; | (SEQ ID NO.366) |
| (Gly⁸, Aib²⁷, β-Ala^{35,37}, Arg³⁸, Lys³⁹(N^{ε}-octanoyl))hGLP-1(7-39)NH₂; | (SEQ ID NO.367) |
| (Gly⁸, Arg^{26,34}, β-Ala³⁵, Lys³⁷(N^{ε}-octanoyl))hGLP-1(7-37)NH₂; | (SEQ ID NO.368) |
| (Gly⁸, Arg^{26,34}, β-Ala³⁵, Lys³⁷(N^{ε}-decanoyl))hGLP-1(7-37)NH₂; | (SEQ ID NO.369) |
| (Gly⁸, Arg^{26,34}, β-Ala³⁵, Lys³⁷(N^{ε}-tetradecanoyl))hGLP-1(7-37)NH₂; | (SEQ ID NO.370) |
| (Gly⁸, Arg^{26,34}, β-Ala³⁵, Lys³⁷(N^{ε}-dodecanoyl))hGLP-1(7-37)NH₂; | (SEQ ID NO.371) |
| (Gly⁸, Arg^{26,34}, β-Ala³⁵, Lys³⁷(N^{ε}-dodecanoyl))hGLP-1(8-37)NH₂; | (SEQ ID NO.372) |
| (Gly⁸, Arg²⁶, Aib³⁵)hGLP-1(7-36)NH₂; | (SEQ ID NO.373) |
| (Gly⁸, Arg²⁶, Phe³¹, Aib³⁵)hGLP-1(7-36)NH₂; | (SEQ ID NO.374) |
| (Gly⁸, Arg²⁶, Phe³¹, β-Ala³⁵)hGLP-1(7-36)NH₂; | (SEQ ID NO.375) |
| (Gly⁸, Arg²⁶, β-Ala³⁵)hGLP-1(7-36)NH₂; | (SEQ ID NO.376) |
| (Gly⁸, Arg^{26,34}, Phe³¹, Aib³⁵)hGLP-1(7-36)NH₂; | (SEQ ID NO.377) |
| (Gly⁸, Arg^{26,34}, Phe³¹, β-Ala³⁵)hGLP-1(7-36)NH₂; | (SEQ ID NO.378) |
| (Gly⁸, Arg³⁴, Aib³⁵)hGLP-1(7-36)NH₂; | (SEQ ID NO.379) |
| (Gly⁸, Arg³⁴, β-Ala³⁵)hGLP-1(7-36)NH₂; | (SEQ ID NO.380) |
| (Gly⁸, Phe³¹, Arg³⁴, Aib³⁵)hGLP-1(7-36)NH₂; | (SEQ ID NO.381) |
| (Gly⁸, Phe ³¹, Arg³⁴, β-Ala³⁵)hGLP-1(7-36)NH₂; | (SEQ ID NO.382) |
| (Gly⁸, Arg^{26,34}, β-Ala³⁵, Lys³⁶(N^{ε}-Aec-decanoyl))hGLP-1(7-36)NH₂; | (SEQ ID NO.383) |
| or (Gly⁸, β-Ala³⁵, Ser³⁷(O-decanoyl))hGLP1(7-37)-NH₂; | (SEQ IDNO.384) |
or a pharmaceutically acceptable salt thereof.

12. A compound according to claim 10, wherein said compound is according to the formula:
| | |
|---|---|
| ((N^{α}-Me-His)⁷, Ser⁸, Aib³⁵)hGLP-1(7-36)NH₂; | (SEQ ID NO.385) |
| ((N^{α}-Me-His)⁷, Ser⁸, β-Ala³⁵)hGLP-1(7-36)NH₂; | (SEQ ID NO.386) |
| ((N^{α}-Me-His)⁷, Ser⁸, Aib³⁵, Arg^{26,34})hGLP-1(7-36)NH₂; | (SEQ ID NO.387) |
| ((N^{α}-Me-His)⁷, Ser⁸, Arg^{26,34}, β-Ala³⁵)hGLP-1(7-36)NH₂; | (SEQ ID NO.388) |
| (Ser⁸, A6c³⁵)hGLP-1(7-36)NH₂; | (SEQ ID NO.389) |
| (Ser⁸, A5c³⁵)hGLP-1(7-36)NH₂; | (SEQ ID NO.390) |
| (Ser⁸, D-Ala³⁵)hGLP-1(7-36)NH₂; | (SEQ ID NO.391) |
| (Ser⁸, Aib³⁵, A6c³²)hGLP-1(7-36)NH₂; | (SEQ ID NO.392) |
| (Ser⁸, Aib³⁵, A5c³²)hGLP-1(7-36)NH₂; | (SEQ ID NO.393) |
| (Ser⁸, Aib³⁵, Glu²³)hGLP-1(7-36)NH₂; | (SEQ ID NO.394) |
| (Ser⁸, Aib^{24,35})hGLP-1(7-36)NH₂; | (SEQ ID NO.395) |
| (Ser⁸, Aib^{30,35})hGLP-1(7-36)NH₂; | (SEQ ID NO.396) |
| (Ser⁸, Aib^{25,35})hGLP-1(7-36)NH₂; | (SEQ ID NO.397) |
| (Ser⁸, Aib³⁵, A6c^{16,20})hGLP-1(7-36)NH₂; | (SEQ ID NO.398) |
| (Ser⁸, Aib³⁵, A6c^{16,29,32})hGLP-1(7-36)NH₂; | (SEQ ID NO.399) |
| (Ser⁸, Aib³⁵, A6c^{20,32})hGLP-1(7-36)NH₂; | (SEQ ID NO.400) |
| (Ser⁸, Aib³⁵, A6c²⁰)hGLP-1(7-36)NH₂; | (SEQ ID NO.401) |
| (Ser⁸, Aib³⁵, Lys²⁵)hGLP-1(7-36)NH₂; | (SEQ ID NO.402) |
| (Ser⁸, Aib^{24,35}, A6c²⁰)hGLP-1(7-36)NH₂; | (SEQ ID NO.403) |
| (Ser⁸, Aib³⁵, A6c^{29,32})hGLP-1(7-36)NH₂; | (SEQ ID NO.404) |
| (Ser⁸, Aib^{24,35}, A6c^{29,32})hGLP-1(7-36)NH₂; | (SEQ ID NO.405) |
| (Ser⁸, Aib³⁵, A6c¹²)hGLP-1(7-36)NH₂; | (SEQ ID NO.406) |
| (Ser⁸, Aib³⁵, Cha²⁰)hGLP-1(7-36)NH₂; | (SEQ ID NO.407) |
| (Ser⁸, Aib³⁵, A6c³³)hGLP-1(7-36)NH₂; | (SEQ ID NO.408) |
| (Ser⁸, A6c^{16,20}, β-Ala³⁵)hGLP-1(7-36)NH₂; | (SEQ ID NO.409) |
| (Ser⁸, Aib³⁵, β-Ala²²)hGLP-1(7-36)NH₂; | (SEQ ID NO.410) |
| (Ser⁸, Aib^{22,35})hGLP-1(7-36)NH₂; | (SEQ ID NO.411) |
| (Ser⁸, Aib³⁵, Glu²³, A6c³²)hGLP-1(7-36)NH₂; | (SEQ ID NO.412) |
| (Ser⁸, Aib^{24,35}, Glu²³, A6c³²)hGLP-1(7-36)NH₂; | (SEQ ID NO.413) |
| (Ser⁸, Aib^{24,25,35}, Glu²³, A6c³²)hGLP-1(7-36)NH₂; | (SEQ ID NO.414) |
| (Ser⁸, Aib^{24,25,35}, A6c^{16,20,32}, Glu²³)hGLP-1(7-36)NH₂; | (SEQ ID NO.415) |
| (Ser⁸, A6c³², β-Ala³⁵)hGLP-1(7-36)NH₂; | (SEQ ID NO.416) |
| (Ser⁸, A5c³², β-Ala³⁵)hGLP-1(7-36)NH₂; | (SEQ ID NO.417) |
| (Ser⁸, Glu²³, β-Ala³⁵)hGLP-1(7-36)NH₂; | (SEQ ID NO.418) |
| (Ser⁸, Aib²⁴, β-Ala³⁵)hGLP-1(7-36)NH₂; | (SEQ ID NO.419) |
| (Ser⁸, Aib³⁰, β-Ala³⁵)hGLP-1(7-36)NH₂; | (SEQ ID NO.420) |
| (Ser⁸, Aib²⁵, β-Ala³⁵)hGLP-1(7-36)NH₂; | (SEQ ID NO.421) |
| (Ser⁸, A6c^{16,29,32}, β-Ala³⁵)hGLP-1(7-36)NH₂; | (SEQ ID NO.422) |
| (Ser⁸, A6c^{20,32}, β-Ala³⁵)hGLP-1(7-36)NH₂; | (SEQ ID NO.423) |
| (Ser⁸, A6c²⁰, β-Ala³⁵)hGLP-1(7-36)NH₂; | (SEQ ID NO.424) |
| (Ser⁸, Lys²⁵, β-Ala³⁵)hGLP-1(7-36)NH₂; | (SEQ ID NO.425) |
| (Ser⁸, Aib²⁴, A6c²⁰, β-Ala³⁵)hGLP-1(7-36)NH₂; | (SEQ ID NO.426) |
| (Ser⁸, A6c^{29,32}, β-Ala³⁵)hGLP-1(7-36)NH₂; | (SEQ ID NO.427) |
| (Ser⁸, Aib²⁴, A6c^{29,32}, β-Ala³⁵)hGLP-1(7-36)NH₂; | (SEQ ID NO.428) |
| (Ser⁸, A6c¹², β-Ala³⁵)hGLP-1(7-36)NH₂; | (SEQ ID NO.429) |
| (Ser⁸, Cha²⁰, β-Ala³⁵)hGLP-1(7-36)NH₂; | (SEQ ID NO.430) |
| (Ser⁸, A6c³³, β-Ala³⁵)hGLP-1(7-36)NH₂; | (SEQ ID NO.431) |
| (Ser⁸, β-Ala^{22,35})hGLP-1(7-36)NH₂; | (SEQ ID NO.432) |
| (Ser⁸, Aib²², β-Ala³⁵)hGLP-1(7-36)NH₂; | (SEQ ID NO.433) |
| (Ser⁸, Glu²³, A6c³², β-Ala³⁵)hGLP-1(7-36)NH₂; | (SEQ ID NO.434) |
| (Ser⁸, Aib²⁴, Glu²³, A6c³², β-Ala³⁵)hGLP-1(7-36)NH₂; | (SEQ ID NO.435) |
| (Ser⁸, Aib²⁴, Glu²³, A6c³², Lys³⁴(N^{e}-octanoyl), β-Ala³⁵) | |
| hGLP-1(7-36)NH₂; | (SEQ ID NO.436) |
| (Ser⁸, Aib^{24,25}, Glu²³, A6c³², β-Ala³⁵)hGLP-1(7-36)NH₂; | (SEQ ID NO.437) |
| (Ser⁸, Aib^{24,25}, A6c^{16,20,32}, Glu²³, β-Ala³⁵)hGLP-1(7-36)NH₂; | (SEQ ID NO.438) |
| (Ser⁸, Aib³⁵, D-Arg³⁶)hGLP-1(7-36)NH₂; | (SEQ ID NO.439) |
| (Ser⁸, Aib³⁵, D-Lys³⁶)hGLP-1(7-36)NH₂; | (SEQ ID NO.440) |
| (Ser⁸, β-Ala³⁵, D-Arg³⁶)hGLP-1(7-36)NH₂; | (SEQ ID NO.441) |
| (Ser⁸, β-Ala³⁵, D-Lys³⁶)hGLP-1(7-36)NH₂; | (SEQ ID NO.442) |
| (Ser⁸, Aib³⁵, Arg^{26,34})hGLP-1(7-36)NH₂; | (SEQ ID NO.443) |
| (Ser⁸, Arg^{26,34}, β-Ala³⁵)hGLP-1(7-36)NH₂; | (SEQ ID NO.444) |
| (Ser⁸, Aib³⁵, Arg^{25,26,34})hGLP-1(7-36)NH₂; | (SEQ ID NO.445) |
| (Ser⁸, Arg^{25,26,34}, β-Ala³⁵)hGLP-1(7-36)NH₂; | (SEQ ID NO.446) |
| (Ser⁸, Arg^{26,34}, β-Ala³⁵, Lys³⁶(N^{ε}-tetradecanoyl))hGLP-1(7-36)OH; | (SEQ ID NO.447) |
| (Ser⁸, Aib³⁵, Arg^{26,34}, Lys³⁶(N^{ε}-tetradecanoyl))hGLP-1(7-37)OH; | (SEQ ID NO.448) |
| (Ser⁸, Aib^{35,37}, Arg^{26,34}, Lys³⁶(N^{ε}-tetradecanoyl))hGLP-1(7-37)OH; | (SEQ ID NO.449) |
| (Ser⁸, Aib³⁵, Arg^{26,34}, Lys³⁶(N^{ε}-tetradecanoyl), D-Ala³⁷) | |
| hGLP-1(7-37)OH; | (SEQ ID NO.450) |
| (Ser⁸, Aib^{35,37}, Arg^{26,34}, Lys³⁸(N^{ε}-tetradecanoyl))hGLP-1(7-38)OH; | (SEQ ID NO.451) |
| (Ser⁸, Aib³⁵, Arg^{26,34}, β-Ala³⁷, Lys³⁸(N^{ε}-tetradecanoyl)) | |
| hGLP-1(7-38)OH; | (SEQ ID NO.452) |
| (Ser⁸, Aib³⁵, Arg^{26,34}, Lys³⁸(N^{ε}-tetradecanoyl))hGLP-1(7-38)OH; | (SEQ ID NO.453) |
| (Ser⁸, Arg^{26,34}, Lys³⁶(N^{ε}-tetradecanoyl), β-Ala³⁷)hGLP-1(7-37)OH; | (SEQ ID NO.454) |
| (Ser⁸, Aib³⁷, Arg^{26,34}, Lys³⁶(N^{ε}-tetradecanoyl))hGLP-1(7-37)OH; | (SEQ ID NO.455) |
| (Ser⁸, Aib³⁵, Arg^{26,34}, Ado³⁷)hGLP-1(7-37)OH; | (SEQ ID NO.456) |
| (Ser⁸, Aib³⁵, Arg^{26,34}, Ado³⁷)hGLP-1(7-37)NH₂; | (SEQ ID NO.457) |
| (Ser⁸, Arg^{26,34}, Lys³⁶(N^{ε}-tetradecanoyl), D-Ala³⁷)hGLP-1(7-37)OH; | (SEQ ID NO.458) |
| (Ser⁸, Aib³⁷, Arg^{26,34}, Lys³⁸(N^{ε}-tetradecanoyl))hGLP-1(7-38)OH; | (SEQ ID NO.459) |
| (Ser⁸, Arg^{26,34}, β-Ala³⁷, Lys³⁸(N^{ε}-tetradecanoyl))hGLP-1(7-38)OH; | (SEQ ID NO.460) |
| (Ser⁸, Aib³⁵, Lys²⁶(N^{ε}-octanoyl))hGLP-1(7-36)NH₂; | (SEQ ID NO.461) |
| (Ser⁸, Aib³⁵, Lys²⁶(N^{ε}-tetradecanoyl))hGLP-1(7-36)NH₂; | (SEQ ID NO.462) |
| (Ser⁸, Aib³⁵, Lys²⁶(N^{ε}-hexadecanoyl))hGLP-1(7-36)NH₂; | (SEQ ID NO.463) |
| (Ser⁸, Lys²⁶(N^{ε}-octanoyl), β-Ala³⁵)hGLP-1(7-36)NH₂; | (SEQ ID NO.464) |
| (Ser⁸, Lys²⁶(N^{ε}-tetradecanoyl), β-Ala³⁵)hGLP-1(7-36)NH₂; | (SEQ ID NO.465) |
| (Ser⁸, Lys²⁶(N^{ε}-hexadecanoyl), β-Ala³⁵)hGLP-1(7-36)NH₂; | (SEQ ID NO.466) |
| (Ser⁸, Aib³⁵, Lys²⁶(N^{ε}-octanoyl), Arg³⁴)hGLP-1(7-36)NH₂; | (SEQ ID NO.467) |
| (Ser⁸, Aib³⁵, Lys²⁶(N^{ε}-tetradecanoyl), Arg³⁴)hGLP-1(7-36)NH₂; | (SEQ ID NO.468) |
| (Ser⁸, Aib³⁵, Lys²⁶(N^{ε}-hexadecanoyl), Arg³⁴)hGLP-1(7-36)NH₂; | (SEQ ID NO.469) |
| (Ser⁸, Aib³⁵, Lys²⁶(N^{ε}-decanoyl), Arg³⁴)hGLP-1(7-36)NH₂; | (SEQ ID NO.470) |
| (Ser⁸, Aib³⁵, Lys²⁵, Lys²⁶(N^{ε}-octanoyl), Arg³⁴)hGLP-1(7-36)NH₂; | (SEQ ID NO.471) |
| (Ser⁸, Aib³⁵, Lys²⁵, Lys²⁶(N^{ε}-tetradecanoyl), Arg³⁴)hGLP-1(7-36)NH₂; | (SEQ ID NO.472) |
| (Ser⁸, Aib³⁵, Lys²⁵, Lys²⁶(N^{ε}-hexadecanoyl), Arg³⁴)hGLP-1(7-36)NH₂; | (SEQ ID NO.473) |
| (Ser⁸, Aib³⁵, Arg^{25,34}, Lys²⁶(N^{ε}-octanoyl))hGLP-1(7-36)NH₂; | (SEQ ID NO.474) |
| (Ser⁸, Aib³⁵, Arg^{25,34}, Lys²⁶(N^{ε}-tetradecanoyl))hGLP-1(7-36)NH₂; | (SEQ ID NO.475) |
| (Ser⁸, Aib³⁵, Arg^{25,34}, Lys²⁶(N^{ε}-hexadecanoyl))hGLP-1(7-36)NH₂; | (SEQ ID NO.476) |
| (Ser⁸, Aib³⁵, Arg^{25,34}, Lys²⁶(N^{ε}-decanoyl))hGLP-1(7-36)NH₂; | (SEQ ID NO.477) |
| (Ser⁸, Lys²⁶(N^{ε}-octanoyl), Arg³⁴, β-Ala³⁵)hGLP-1(7-36)NH₂; | (SEQ ID NO.478) |
| (Ser⁸, Lys²⁶(N^{ε}-tetradecanoyl), Arg³⁴, β-Ala³⁵)hGLP-1(7-36)NH₂; | (SEQ ID NO.479) |
| (Ser⁸, Lys²⁶(N^{ε}-hexadecanoyl), Arg³⁴, β-Ala³⁵)hGLP-1(7-36)NH₂; | (SEQ ID NO.480) |
| (Ser⁸, Lys²⁶(N^{ε}-decanoyl), Arg³⁴, β-Ala³⁵)hGLP-1(7-36)NH₂; | (SEQ ID NO.481) |
| (Ser⁸, Aib³⁵, Lys³⁴(N^{ε}-octanoyl))hGLP-1(7-36)NH₂; | (SEQ ID NO.482) |
| (Ser⁸, Aib³⁵, Lys³⁴(N^{ε}-tetradecanoyl))hGLP-1(7-36)NH₂; | (SEQ ID NO.483) |
| (Ser⁸, Aib³⁵, Lys³⁴(N^{ε}-hexadecanoyl))hGLP-1(7-36)NH₂; | (SEQ ID NO.484) |
| (Ser⁸, Aib³⁵, Arg²⁶, Lys³⁴(N^{ε}-octanoyl))hGLP-1(7-36)NH₂; | (SEQ ID NO.485) |
| (Ser⁸, Aib³⁵, Arg²⁶, Lys³⁴(N^{ε}-hexadecanoyl))hGLP-1(7-36)NH₂; | (SEQ ID NO.486) |
| (Ser⁸, Aib³⁵, Arg²⁶, Lys³⁴(N^{ε}-decanoyl))hGLP-1(7-36)NH₂; | (SEQ ID NO.487) |
| (Ser⁸, Aib³⁵, Arg^{25,26}, Lys³⁴(N^{ε}-octanoyl))hGLP-1(7-36)NH₂; | (SEQ ID NO.488) |
| (Ser⁸, Aib³⁵, Arg^{25,26}, Lys³⁴(N^{ε}-tetradecanoyl))hGLP-1(7-36)NH₂; | (SEQ ID NO.489) |
| (Ser⁸, Aib³⁵, Arg^{25,26}, Lys³⁴(N^{ε}-hexadecanoyl))hGLP-1(7-36)NH₂; | (SEQ ID NO.490) |
| (Ser⁸, Aib³⁵, Arg^{25,26}, Lys³⁴(N^{ε}-decanoyl))hGLP-1(7-36)NH₂; | (SEQ ID NO.491) |
| (Ser⁸, Aib³⁵, Lys²⁵, Arg²⁶, Lys³⁴(N^{ε}-octanoyl))hGLP-1(7-36)NH₂; | (SEQ ID NO.492) |
| (Ser⁸, Aib³⁵, Lys²⁵, Arg²⁶, Lys³⁴(N^{ε}-tetradecanoyl))hGLP-1(7-36)NH₂; | (SEQ ID NO.493) |
| (Ser⁸, Aib³⁵, Lys²⁵, Arg²⁶, Lys³⁴(N^{ε}-hexadecanoyl))hGLP-1(7-36)NH₂; | (SEQ ID NO.494) |
| (Ser⁸, Aib³⁵, Lys³⁶(N^{ε}-octanoyl))hGLP-1(7-36)NH₂; | (SEQ ID NO.495) |
| (Ser⁸, Aib³⁵, Lys³⁶(N^{ε}-tetradecanoyl))hGLP-1(7-36)NH₂; | (SEQ ID NO.496) |
| (Ser⁸, Aib³⁵, Lys³⁶(N^{ε}-hexadecanoyl))hGLP-1(7-36)NH₂; | (SEQ ID NO.497) |
| (Ser⁸, Aib³⁵, Arg²⁶, Lys³⁶(N^{ε}-octanoyl))hGLP-1(7-36)NH₂; | (SEQ ID NO.498) |
| (Ser⁸, Aib³⁵, Arg²⁶, Lys³⁶(N^{ε}-tetradecanoyl))hGLP-1(7-36)NH₂; | (SEQ ID NO.499) |
| (Ser⁸, Aib³⁵, Arg²⁶, Lys³⁶(N^{ε}-hexadecanoyl))hGLP-1(7-36)NH₂; | (SEQ ID NO.500) |
| (Ser⁸, Aib³⁵, Arg^{26,34}, Lys³⁶(N^{ε}-octanoyl))hGLP-1(7-36)NH₂; | (SEQ ID NO.501) |
| (Ser⁸, Aib³⁵, Arg^{26,34}, Lys³⁶(N^{ε}-hexadecanoyl))hGLP-1(7-36)NH₂; | (SEQ ID NO.502) |
| (Ser⁸, Aib³⁵, Arg^{26,34}, Lys³⁸(N^{ε}-octanoyl))hGLP-1(7-38)NH₂; | (SEQ ID NO.503) |
| (Ser⁸, Aib³⁵, Arg^{26,34}, Lys³⁸(N^{ε}-decanoyl))hGLP-1(7-38)NH₂; | (SEQ ID NO.504) |
| (Ser⁸, Aib³⁵, Arg^{26,34}, Lys³⁸(N^{ε}-tetradecanoyl))hGLP-1(7-38)NH₂; | (SEQ ID NO.505) |
| (Ser⁸, Aib³⁵, Arg^{26,34}, Lys³⁸(N^{ε}-hexadecanoyl))hGLP-1(7-38)NH₂; | (SEQ ID NO.506) |
| (Ser⁸, Aib^{35,37}, Arg^{25,26,34}, Lys³⁸(N^{ε}-octanoyl))hGLP-1(7-38)NH₂; | (SEQ ID NO.507) |
| (Ser⁸, Aib^{35,37}, Arg^{25,26,34}, Lys³⁸(N^{ε}-decanoyl))hGLP-1(7-38)NH₂; | (SEQ ID NO.508) |
| (Ser⁸, Aib^{35,37}, Arg^{25,26,34}, Lys³⁸(N^{ε}-tetradecanoyl))hGLP-1(7-38)NH₂; | (SEQ ID NO.509) |
| (Ser⁸, Aib^{35,37}, Arg^{25,26,34}, Lys³⁸(N^{ε}-hexadecanoyl))hGLP-1(7-38)NH₂; | (SEQ ID NO.510) |
| (Ser⁸, Aib^{35,37}, Arg^{26,34}, Lys³⁸(N^{ε}-octanoyl))hGLP-1(7-38)NH₂; | (SEQ ID NO.511) |
| (Ser⁸, Aib^{35,37}, Arg^{26,34}, Lys³⁸(N^{ε}-decanoyl))hGLP-1(7-38)NH₂; | (SEQ ID NO.512) |
| (Ser⁸, Aib^{35,37}, Arg^{26,34}, Lys³⁸(N^{ε}-hexadecanoyl))hGLP-1(7-38)NH₂; | (SEQ ID NO.513) |
| (Ser⁸, Aib³⁵, Lys²⁵, Arg^{26,34}, Lys³⁶(N^{ε}-octanoyl))hGLP-1(7-36)NH₂; | (SEQ ID NO.514) |
| (Ser⁸, Aib³⁵, Lys²⁵, Arg^{26,34}, Lys³⁶(N^{ε}-tetradecanoyl)) | |
| hGLP-1(7-36)NH₂; | (SEQ ID NO.515) |
| (Ser⁸, Aib³⁵, Lys²⁵, Arg^{26,34}, Lys³⁶(N^{ε}-hexadecanoyl)) | |
| hGLP-1(7-36)NH₂; | (SEQ ID NO.516) |
| (Ser⁸, Aib³⁵, Arg^{25,26,34}, Lys³⁶(N^{ε}-octanoyl))hGLP-1(7-36)NH₂; | (SEQ ID NO.517) |
| (Ser⁸, Aib³⁵, Arg^{25,26,34}, Lys³⁶(N^{ε}-tetradecanoyl))hGLP-1(7-36)NH₂; | (SEQ ID NO.518) |
| (Ser⁸, Aib³⁵, Arg^{25,26,34}, Lys³⁶(N^{ε}-hexadecanoyl))hGLP-1(7-36)NH₂; | (SEQ ID NO.519) |
| (Ser⁸, Aib³⁵, Arg^{25,26,34}, Lys³⁶(N^{ε}-decanoyl))hGLP-1(7-36)NH₂; | (SEQ ID NO.520) |
| (Ser⁸, Lys³⁴(N^{ε}-octanoyl), β-Ala³⁵)hGLP-1(7-36)NH₂; | (SEQ ID NO.521) |
| (Ser⁸, Lys³⁴(N^{ε}-tetradecanoyl), β-Ala³⁵)hGLP-1(7-36)NH₂; | (SEQ ID NO.522) |
| (Ser⁸, Lys³⁴(N^{ε}-hexadecanoyl), β-Ala³⁵)hGLP-1(7-36)NH₂; | (SEQ ID NO.523) |
| (Ser⁸, A6c³², Lys³⁴(N^{e}-octanoyl), β-Ala³⁵)hGLP-1(7-36)NH₂; | (SEQ ID NO.524) |
| (Ser⁸, Glu²³, Lys³⁴(N^{e}-octanoyl), β-Ala³⁵)hGLP-1(7-36)NH₂; | (SEQ ID NO.525) |
| (Ser⁸, Glu²³, A6c³², Lys³⁴(N^{e}-octanoyl), β-Ala³⁵)hGLP-1(7-36)NH₂; | (SEQ ID NO.526) |
| (Ser⁸, Arg²⁶, Lys³⁴(N^{ε}-octanoyl), β-Ala³⁵)hGLP-1(7-36)NH₂; | (SEQ ID NO.527) |
| (Ser⁸, Arg²⁶, Lys³⁴(N^{ε}-tetradecanoyl), β-Ala³⁵)hGLP-1(7-36)NH₂; | (SEQ ID NO.528) |
| (Ser⁸, Arg²⁶, Lys³⁴(N^{ε}-hexadecanoyl), β-Ala³⁵)hGLP-1(7-36)NH₂; | (SEQ ID NO.529) |
| (Ser⁸, Arg²⁶, Lys³⁴(N^{ε}-decanoyl), β-Ala³⁵)hGLP-1(7-36)NH₂; | (SEQ ID NO.530) |
| (Ser⁸, Arg^{25,26}, Lys³⁴(N^{ε}-octanoyl), β-Ala³⁵)hGLP-1(7-36)NH₂; | (SEQ ID NO.531) |
| (Ser⁸, Arg^{25,26}, Lys³⁴(N^{ε}-tetradecanoyl), β-Ala³⁵)hGLP-1(7-36)NH₂; | (SEQ ID NO.532) |
| (Ser⁸, Arg^{25,26}, Lys³⁴(N^{ε}-hexadecanoyl), β-Ala³⁵)hGLP-1(7-36)NH₂; | (SEQ ID NO.533) |
| (Ser⁸, Arg^{25,26}, Lys³⁴(N^{ε}-decanoyl), β-Ala³⁵)hGLP-1(7-36)NH₂; | (SEQ ID NO.534) |
| (Ser⁸, Lys²⁵, Arg²⁶, Lys³⁴(N^{ε}-octanoyl), β-Ala³⁵)hGLP-1(7-36)NH₂; | (SEQ ID NO.535) |
| (Ser⁸, Lys²⁵, Arg²⁶, Lys³⁴(N^{ε}-tetradecanoyl), β-Ala³⁵) | |
| hGLP-1(7-36)NH₂; | (SEQ ID NO.536) |
| (Ser⁸, Lys²⁵, Arg²⁶, Lys³⁴(N^{ε}-hexadecanoyl), β-Ala³⁵) | |
| hGLP-1(7-36)NH₂; | (SEQ ID NO.537) |
| (Ser⁸, β-Ala³⁵, Lys³⁶(N^{ε}-octanoyl))hGLP-1(7-36)NH₂; | (SEQ ID NO.538) |
| (Ser⁸, β-Ala³⁵, Lys³⁶(N^{ε}-tetradecanoyl))hGLP-1(7-36)NH₂; | (SEQ ID NO.539) |
| (Ser⁸, β-Ala³⁵, Lys³⁶(N^{ε}-hexadecanoyl))hGLP-1(7-36)NH₂; | (SEQ ID NO.540) |
| (Ser⁸, Arg²⁶, β-Ala³⁵, Lys³⁶(N^{ε}-octanoyl))hGLP-1(7-36)NH₂; | (SEQ ID NO.541) |
| (Ser⁸, Arg²⁶, β-Ala³⁵, Lys³⁶(N^{ε}-tetradecanoyl))hGLP-1(7-36)NH₂; | (SEQ ID NO.542) |
| (Ser⁸, Arg²⁶, β-Ala³⁵, Lys³⁶(N^{ε}-hexadecanoyl))hGLP-1(7-36)NH₂; | (SEQ ID NO.543) |
| (Ser⁸, Arg^{26,34}, β-Ala³⁵, Lys³⁶(N^{ε}-octanoyl))hGLP-1(7-36)NH₂; | (SEQ ID NO.544) |
| (Ser⁸, Arg^{26,34}, β-Ala³⁵, Lys³⁶(N^{ε}-tetradecanoyl))hGLP-1(7-36)NH₂; | (SEQ ID NO.545) |
| (Ser⁸, Arg^{26,34}, β-Ala³⁵, Lys³⁶(N^{ε}-hexadecanoyl))hGLP-1(7-36)NH₂; | (SEQ ID NO.546) |
| (Ser⁸, Arg^{26,34}, β-Ala³⁵, Lys³⁶(N^{ε}-decanoyl))hGLP-1(7-36)NH₂; | (SEQ ID NO.547) |
| (Ser⁸, Lys²⁵, Arg^{26,34}, β-Ala³⁵, Lys³⁶(N^{ε}-octanoyl))hGLP-1(7-36)NH₂; | (SEQ ID NO.548) |
| (Ser⁸, Lys²⁵, Arg^{26,34}, Lys³⁶(N^{ε}-tetradecanoyl), β-Ala³⁵) | |
| hGLP-1(7-36)NH₂; | (SEQ ID NO.549) |
| (Ser⁸, Lys²⁵, Arg^{26,34}, β-Ala³⁵, Lys³⁶(N^{ε}-hexadecanoyl)) | |
| hGLP-1(7-36)NH₂; | (SEQ ID NO.550) |
| (Ser⁸, Arg^{25,26,34}, β-Ala³⁵, Lys³⁶(N^{ε}-octanoyl))hGLP-1(7-36)NH₂; | (SEQ ID NO.551) |
| (Ser⁸, Arg^{25,26,34}, β-Ala³⁵, Lys³⁶(N^{ε}-tetradecanoyl))hGLP-1(7-36)NH₂; | (SEQ ID NO.552) |
| (Ser⁸, Arg^{25,26,34}, β-Ala³⁵, Lys³⁶(N^{ε}-hexadecanoyl))hGLP-1(7-36)NH₂; | (SEQ ID NO.553) |
| (Ser⁸, Arg^{25,26,34}, β-Ala³⁵, Lys³⁶(N^{ε}-decanoyl))hGLP-1(7-36)NH₂; | (SEQ ID NO.554) |
| (Ser⁸, Aib³⁵, Lys²⁶(N^{ε}-octanoyl), A6c³², Arg³⁴)hGLP-1(7-36)NH₂; | (SEQ ID NO.555) |
| (Ser⁸, Aib³⁵, Lys²⁶(N^{ε}-tetradecanoyl), A6c³², Arg³⁴)hGLP-1(7-36)NH₂; | (SEQ ID NO.556) |
| (Ser⁸, Aib³⁵, Lys²⁶(N^{ε}-hexadecanoyl), A6c³², Arg³⁴)hGLP-1(7-36)NH₂; | (SEQ ID NO.557) |
| (Ser⁸, Aib³⁵, A6c³², Lys³⁴(N^{ε}-octanoyl))hGLP-1(7-36)NH₂; | (SEQ ID NO.558) |
| (Ser⁸, Aib³⁵, A6c³², Lys³⁴(N^{ε}-tetradecanoyl))hGLP-1(7-36)NH₂; | (SEQ ID NO.559) |
| (Ser⁸, Aib³⁵, A6c³², Lys³⁴(N^{ε}-hexadecanoyl))hGLP-1(7-36)NH₂; | (SEQ ID NO.560) |
| (Ser⁸, Aib³⁵, Arg²⁶, A6c³², Lys³⁴(N^{ε}-octanoyl))hGLP-1(7-36)NH₂; | (SEQ ID NO.561) |
| (Ser⁸, Aib³⁵, Arg²⁶, A6c³², Lys³⁴(N^{ε}-tetradecanoyl))hGLP-1(7-36)NH₂; | (SEQ ID NO.562) |
| (Ser⁸, Aib³⁵, A6c³², Lys³⁶(N^{ε}-octanoyl))hGLP-1(7-36)NH₂; | (SEQ ID NO.563) |
| (Ser⁸, Aib³⁵, A6c³², Lys³⁶(N^{ε}-tetradecanoyl))hGLP-1(7-36)NH₂; | (SEQ ID NO.564) |
| (Ser⁸, Aib³⁵, A6c³², Lys³⁶(N^{ε}-hexadecanoyl))hGLP-1(7-36)NH₂; | (SEQ ID NO.565) |
| (Ser⁸, Aib³⁵, Arg²⁶, A6c³², Lys³⁶(N^{ε}-octanoyl))hGLP-1(7-36)NH₂; | (SEQ ID NO.566) |
| (Ser⁸, Aib³⁵, Arg²⁶, A6c³², Lys³⁶(N^{ε}-tetradecanoyl))hGLP-1(7-36)NH₂; | (SEQ ID NO.567) |
| (Ser⁸, Aib³⁵, Arg²⁶, A6c³², Lys³⁶(N^{ε}-hexadecanoyl))hGLP-1(7-36)NH₂; | (SEQ ID NO.568) |
| (Ser⁸, Aib³⁵, Arg^{26,34}, A6c³², Lys³⁶(N^{ε}-octanoyl))hGLP-1(7-36)NH₂; | (SEQ ID NO.569) |
| (Ser⁸, Aib³⁵, Arg^{26,34}, A6c³², Lys³⁶(N^{ε}-decanoyl))hGLP-1(7-36)NH₂; | (SEQ ID NO.570) |
| (Ser⁸, Aib³⁵, Arg^{26,34}, A6c³², Lys³⁶(N^{ε}-tetradecanoyl)) | |
| hGLP-1(7-36)NH₂; | (SEQ ID NO.571) |
| (Ser⁸, Aib³⁵, Arg^{26,34}, A6c³², Lys³⁶(N^{ε}-hexadecanoyl)) | |
| hGLP-1(7-36)NH₂; | (SEQ ID NO.572) |
| (Ser⁸, Aib^{24,35}, Lys²⁶(N^{ε}-octanoyl), Arg³⁴)hGLP-1(7-36)NH₂; | (SEQ ID NO.573) |
| (Ser⁸, Aib^{24,35}, Lys²⁶(N^{ε}-tetradecanoyl), Arg³⁴)hGLP-1(7-36)NH₂; | (SEQ ID NO.574) |
| (Ser⁸, Aib^{24,35}, Lys²⁶(N^{ε}-hexadecanoyl), Arg³⁴)hGLP-1(7-36)NH₂; | (SEQ ID NO.575) |
| (Ser⁸, Aib^{24,35}, Arg²⁶, Lys³⁴(N^{ε}-octanoyl))hGLP-1(7-36)NH₂; | (SEQ ID NO.576) |
| (Ser⁸, Aib^{24,35}, Arg²⁶, Lys³⁴(N^{ε}-tetradecanoyl))hGLP-1(7-36)NH₂; | (SEQ ID NO.577) |
| (Ser⁸, Aib^{24,35}, Arg²⁶, Lys³⁴(N^{ε}-hexadecanoyl))hGLP-1(7-36)NH₂; | (SEQ ID NO.578) |
| (Ser⁸, Aib^{24,35}, Arg^{26,34}, Lys³⁶(N^{ε}-octanoyl))hGLP-1(7-36)NH₂; | (SEQ ID NO.579) |
| (Ser⁸, Aib^{24,35}, Arg^{26,34}, Lys³⁶(N^{ε}-tetradecanoyl))hGLP-1(7-36)NH₂; | (SEQ ID NO.580) |
| (Ser⁸, Aib^{24,35}, Arg^{26,34}, Lys³⁶(N^{ε}-hexadecanoyl))hGLP-1(7-36)NH₂; | (SEQ ID NO.581) |
| (Ser⁸, Aib^{24,35}, Glu²³, A6c³², Lys³⁴(N^{ε}-octanoyl))hGLP-1(7-36)NH₂; | (SEQ ID NO.582) |
| (Ser⁸, Aib³⁵, Glu²³, Lys²⁶(N^{ε}-octanoyl), Arg³⁴)hGLP-1(7-36)NH₂; | (SEQ ID NO.583) |
| (Ser⁸, Aib³⁵, Glu²³, Lys²⁶(N^{ε}-tetradecanoyl), Arg³⁴)hGLP-1(7-36)NH₂; | (SEQ ID NO.584) |
| (Ser⁸, Aib³⁵, Glu²³, Lys²⁶(N^{ε}-hexadecanoyl), Arg³⁴)hGLP-1(7-36)NH₂; | (SEQ ID NO.585) |
| (Ser⁸, Aib³⁵, Glu²³, Lys³⁴(N^{ε}-octanoyl))hGLP-1(7-36)NH₂; | (SEQ ID NO.586) |
| (Ser⁸, Aib³⁵, Glu²³, A6c³², Lys³⁴(N^{ε}-octanoyl))hGLP-1(7-36)NH₂; | (SEQ ID NO.587) |
| (Ser⁸, Aib³⁵, Glu²³, Arg²⁶, Lys³⁴(N^{ε}-octanoyl))hGLP-1(7-36)NH₂; | (SEQ ID NO.588) |
| (Ser⁸, Aib³⁵, Glu²³, Arg²⁶, Lys³⁴ (N^{ε}-tetradecanoyl))hGLP-1(7-36)NH₂; | (SEQ ID NO.589) |
| (Ser⁸, Aib³⁵, Glu²³, Arg²⁶, Lys³⁴(N^{ε}-hexadecanoyl))hGLP-1(7-36)NH₂; | (SEQ ID NO.590) |
| (Ser⁸, Aib³⁵, Glu²³, Lys³⁶(N^{ε}-octanoyl))hGLP-1(7-36)NH₂; | (SEQ ID NO.591) |
| (Ser⁸, Aib³⁵, Glu²³, Lys³⁶(N^{ε}-tetradecanoyl))hGLP-1(7-36)NH₂; | (SEQ ID NO.592) |
| (Ser⁸, Aib³⁵, Glu²³, Lys³⁶(N^{ε}-hexadecanoyl))hGLP-1(7-36)NH₂; | (SEQ ID NO.593) |
| (Ser⁸, Aib³⁵, Glu²³, Arg^{26,34}, Lys³⁶(N^{ε}-octanoyl))hGLP-1(7-36)NH₂; | (SEQ ID NO.594) |
| (Ser⁸, Aib³⁵, Glu²³, Arg^{26,34}, Lys³⁶(N^{ε}-tetradecanoyl)) | |
| hGLP-1(7-36)NH₂; | (SEQ ID NO.595) |
| (Ser⁸, Aib³⁵, Glu²³, Arg^{26,34}, Lys³⁶(N^{ε}-hexadecanoyl)) | |
| hGLP-1(7-36)NH₂; | (SEQ ID NO.596) |
| (Ser⁸, Aib^{30,35}, Lys²⁶(N^{ε}-octanoyl), Arg³⁴)hGLP-1(7-36)NH₂; | (SEQ ID NO.597) |
| (Ser⁸, Aib^{30,35}, Lys²⁶(N^{ε}-tetradecanoyl), Arg³⁴)hGLP-1(7-36)NH₂; | (SEQ ID NO.598) |
| (Ser⁸, Aib^{30,35}, Lys²⁶(N^{ε}-hexadecanoyl), Arg³⁴)hGLP-1(7-36)NH₂; | (SEQ ID NO.599) |
| (Ser⁸, Aib^{30,35}, Arg²⁶, Lys³⁴(N^{ε}-octanoyl))hGLP-1(7-36)NH₂; | (SEQ ID NO.600) |
| (Ser⁸, Aib^{30,35}, Arg²⁶, Lys³⁴(N^{ε}-tetradecanoyl))hGLP-1(7-36)NH₂; | (SEQ ID NO.601) |
| (Ser⁸, Aib^{30,35}, Arg²⁶, Lys³⁴(N^{ε}-hexadecanoyl))hGLP-1(7-36)NH₂; | (SEQ ID NO.602) |
| (Ser⁸, Aib^{30,35}, Arg^{26,34}, Lys³⁶(N^{ε}-octanoyl))hGLP-1(7-36)NH₂; | (SEQ ID NO.603) |
| (Ser⁸, Aib^{30,35}, Arg^{26,34}, Lys³⁶(N^{ε}-tetradecanoyl))hGLP-1(7-36)NH₂; | (SEQ ID NO.604) |
| (Ser⁸, Aib^{30,35}, Arg^{26,34}, Lys³⁶(N^{ε}-hexadecanoyl))hGLP-1(7-36)NH₂; | (SEQ ID NO.605) |
| (Ser⁸, Aib³⁵, Glu²³, A6c³², Lys³⁶(N^{ε}-octanoyl))hGLP-1(7-36)NH₂; | (SEQ ID NO.606) |
| (Ser⁸, Aib³⁵, Glu²³, A6c³², Lys³⁶(N^{ε}-tetradecanoyl))hGLP-1(7-36)NH₂; | (SEQ ID NO.607) |
| (Ser⁸, Aib³⁵, Glu²³, A6c³², Lys³⁶(N^{ε}-hexadecanoyl))hGLP-1(7-36)NH₂; | (SEQ ID NO.608) |
| (Ser⁸, Aib³⁵, Glu²³, Arg^{26,34}, A6c³², Lys³⁶(N^{ε}-octanoyl)) | |
| hGLP-1(7-36)NH₂; | (SEQ ID NO.609) |
| (Ser⁸, Aib³⁵, Glu²³, Arg^{26,34}, A6c³², Lys³⁶(N^{ε}-tetradecanoyl)) | |
| hGLP-1(7-36)NH₂; | (SEQ ID NO.610) |
| (Ser⁸, Aib³⁵, Glu²³, Arg^{26,34}, A6c³², Lys³⁶(N'-hexadecanoyl)) | |
| hGLP-1(7-36)NH₂; | (SEQ ID NO.611) |
| (Ser⁸, Aib^{24,35}, Glu²³, Arg^{26,34}, A6c³², Lys³⁶(N^{ε}-octanoyl)) | |
| hGLP-1(7-36)NH₂; | (SEQ ID NO.612) |
| (Ser⁸, Aib^{24,35}, Glu²³, Arg^{26,34}, A6c³², Lys³⁶(N^{ε}-tetradecanoyl)) | |
| hGLP-1(7-36)NH₂; | (SEQ ID NO.613) |
| (Ser⁸, Aib^{24,35}, Glu²³, Arg^{26,34}, A6c³², Lys³⁶(N^{ε}-hexadecanoyl)) | |
| hGLP-1(7-36)NH₂; | (SEQ ID NO.614) |
| (Ser⁸, Aib^{24,30,35}, Glu²³, Arg^{26,34}, A6c³², Lys³⁶(N^{ε}-octanoyl)) | |
| hGLP-1(7-36)NH₂; | (SEQ ID NO.615) |
| (Ser⁸, Aib^{24,30,35}, Glu²³, Arg^{26,34}, A6c³², Lys³⁶(N^{ε}-tetradecanoyl)) | |
| hGLP-1(7-36)NH₂; | (SEQ ID NO.616) |
| (Ser⁸, Aib^{24,30,35}, Glu²³, Arg^{26,34}, A6c³², Lys³⁶(N^{ε}-hexadecanoyl)) | |
| hGLP-1(7-36)NH₂; | (SEQ ID NO.617) |
| ((N^{α}-HEPES-His)⁷, Ser⁸, β-Ala³⁵)hGLP-1(7-36)NH₂; | (SEQ ID NO.618) |
| ((N^{α}-HEPA-His)⁷, Ser⁸, β-Ala³⁵)hGLP-1(7-36)NH₂; | (SEQ ID NO.619) |
| ((N^{α}-tetradecanoyl-His)⁷, Ser⁸, Aib³⁵)hGLP-1(7-36)NH₂; | (SEQ ID NO.620) |
| ((N^{α}-tetradecanoyl-His)⁷, Ser⁸, β-Ala³⁵)hGLP-1(7-36)NH₂; | (SEQ ID NO.621) |
| ((N^{α}-tetradecanoyl-His)⁷, Ser⁸, Aib³⁵, Arg^{26,34})hGLP-1(7-36)NH₂; | (SEQ ID NO.622) |
| ((N^{α}-tetradecanoyl-His)⁷, Ser⁸, Arg^{26,34}, β-Ala³⁵)hGLP-1(7-36)NH₂; | (SEQ ID NO.623) |
| ((N^{α}-tetradecanoyl-His)⁷, Ser⁸, Aib³⁵, Arg^{25,26,34})hGLP-(7-36)NH₂; | (SEQ ID NO.624) |
| ((N^{α}-tetradecanoyl-His)⁷, Ser⁸, Arg^{25,26,34}, β-Ala³⁵)hGLP-1(7-36)NH₂; | (SEQ ID NO.625) |
| (Ser⁸, Aib³⁵, Lys²⁶(N^{ε}-octanesulfonyl), Arg³⁴)hGLP-1(7-36)NH₂; | (SEQ ID NO.626) |
| (Ser⁸, Aib³⁵, Lys²⁶(N^{ε}-dodecanesulfonyl), Arg³⁴)hGLP-1(7-36)NH₂; | (SEQ ID NO.627) |
| (Ser⁸, Aib³⁵, Lys²⁶(N^{ε}-hexadecanesulfonyl), Arg³⁴)hGLP-1(7-36)NH₂; | (SEQ ID NO.628) |
| (Ser⁸, Aib³⁵, Arg²⁶, Lys³⁴(N^{ε}-octanesulfonyl))hGLP-1(7-36)NH₂; | (SEQ ID NO.629) |
| (Ser⁸, Aib³⁵, Arg²⁶, Lys³⁴(N^{ε}-dodecanesulfonyl))hGLP-1(7-36)NH₂; | (SEQ ID NO.630) |
| (Ser⁸, Aib³⁵, Arg²⁶, Lys³⁴(N^{ε}-hexadecanesulfonyl))hGLP-1(7-36)NH₂; | (SEQ ID NO.631) |
| (Ser⁸, Aib³⁵, Arg^{26,34}, Lys³⁶(N^{ε}-octanesulfonyl))hGLP-1(7-36)NH₂; | (SEQ ID NO.632) |
| (Ser⁸, Aib³⁵, Arg^{26,34}, Lys³⁶(N^{ε}-hexadecanesulfonyl)) | |
| hGLP-1(7-36)NH₂; | (SEQ ID NO.633) |
| (Ser⁸, Aib³⁵, Asp²⁶(1-(4-decylpiperazine)), Arg³⁴ )hGLP-1(7-36)NH₂; | (SEQ ID NO.634) |
| (Ser⁸, Aib³⁵, Asp²⁶(1-(4-dodecylpiperazine)), Arg³⁴)hGLP-1(7-36)NH₂; | (SEQ ID NO.635) |
| (Ser⁸, Aib³⁵, Asp²⁶(1-(4-tetradecylpiperazine)), Arg³⁴) hGLP-1(7-36)NH₂; | (SEQ ID NO.636) |
| (Ser⁸, Aib³⁵, Asp²⁶(1-(4-hexadecylpiperazine)), Arg³⁴)hGLP-1(7-36)NH₂; | (SEQ ID NO.637) |
| (Ser⁸, Aib³⁵, Arg²⁶, Asp³⁴(1-(4-decylpiperazine)))hGLP-1(7-36)NH₂; | (SEQ ID NO.638) |
| (Ser⁸, Aib³⁵, Arg²⁶, Asp³⁴(1-(4-dodecylpiperazine)))hGLP-1(7-36)NH₂; | (SEQ ID NO.639) |
| (Ser⁸, Aib³⁵, Arg²⁶, Asp³⁴(1-(4-tetradecylpiperazine))) | |
| hGLP-1(7-36)NH₂; | (SEQ ID NO.640) |
| (Ser⁸, Aib³⁵, Arg²⁶, Asp³⁴(1-(4-hexadecylpiperazine))) | |
| hGLP-1(7-36)NH₂; | (SEQ ID NO.641) |
| (Ser⁸, Aib³⁵, Arg^{26,34}, Asp³⁶(1-(4-decylpiperazine)))hGLP-1(7-36)NH₂; | (SEQ ID NO.642) |
| (Ser⁸, Aib³⁵, Arg^{26,34}, Asp³⁶(1-(4-dodecylpiperazine))) | |
| hGLP-1(7-36)NH₂; | (SEQ ID NO.643) |
| (Ser⁸, Aib³⁵, Arg^{26,34}, Asp³⁶(1-(4-hexadecylpiperazine))) | |
| hGLP-1(7-36)NH₂; | (SEQ ID NO.644) |
| (Ser⁸, Aib³⁵, Arg^{26,34}, Asp³⁸(1-(4-decylpiperazine)))hGLP-1(7-38)NH₂; | (SEQ ID NO.645) |
| (Ser⁸, Aib³⁵, Arg^{26,34}, Asp³⁸(1-(4-dodecylpiperazine))) | |
| hGLP-1(7-38)NH₂; | (SEQ ID NO.646) |
| (Ser⁸, Aib³⁵, Arg^{26,34}, Asp³⁸(1-(4-tetradecylpiperazine))) | |
| hGLP-1(7-38)NH₂; | (SEQ ID NO.647) |
| (Ser⁸, Aib³⁵, Arg^{26,34}, Asp³⁸(1-(4-hexadecylpiperazine))) | |
| hGLP-1(7-38)NH₂; | (SEQ ID NO.648) |
| (Ser⁸, Aib^{35,37}, Arg^{26,34}, Asp³⁸(1-(4-decylpiperazine))) | |
| hGLP-1(7-38)NH₂; | (SEQ ID NO.649) |
| (Ser⁸, Aib^{35,37}, Arg^{26,34}, Asp³⁸(1-(4-dodecylpiperazine))) | |
| hGLP-1(7-38)NH₂; | (SEQ ID NO.650) |
| (Ser⁸, Aib^{35,37}, Arg^{26,34}, Asp³⁸(1-(4-tetradecylpiperazine))) | |
| hGLP-1(7-38)NH₂; | (SEQ ID NO.651) |
| (Ser⁸, Aib^{35,37}, Arg^{26,34}, Asp³⁸(1-(4-hexadecylpiperazine))) | |
| hGLP-1(7-38)NH₂; | (SEQ ID NO.652) |
| (Ser⁸, Aib³⁵, Arg^{25,34}, Asp²⁶(1-(4-decylpiperazine)))hGLP-1(7-36)NH₂; | (SEQ ID NO.653) |
| (Ser⁸, Aib³⁵, Arg^{25,34}, Asp²⁶(1-(4-dodecylpiperazine))) | |
| hGLP-1(7-36)NH₂; | (SEQ ID NO.654) |
| (Ser⁸, Aib³⁵, Arg^{25,34}, Asp²⁶(1-(4-tetradecylpiperazine))) | |
| hGLP-1(7-36)NH₂; | (SEQ ID NO.655) |
| (Ser⁸, Aib³⁵, Arg^{25,34}, Asp²⁶(1-(4-hexadecylpiperazine))) | |
| hGLP-1(7-36)NH₂; | (SEQ ID NO.656) |
| (Ser⁸, Aib³⁵, Arg^{25,26}, Asp³⁴(1-(4-decylpiperazine)))hGLP-1(7-36)NH₂; | (SEQ ID NO.657) |
| (Ser⁸, Aib³⁵, Arg^{25,26}, Asp³⁴(1-(4-dodecylpiperazine))) | |
| hGLP-1(7-36)NH₂; | (SEQ ID NO.658) |
| (Ser⁸, Aib³⁵, Arg^{25,26}, Asp³⁴(1-(4-tetradecylpiperazine))) | |
| hGLP-1(7-36)NH₂; | (SEQ ID NO.659) |
| (Ser⁸, Aib³⁵, Arg^{25,26}, Asp³⁴(1-(4-hexadecylpiperazine))) | |
| hGLP-1(7-36)NH₂; | (SEQ ID NO.660) |
| (Ser⁸, Aib³⁵, Arg^{25,26,34}, Asp³⁶(1-(4-decylpiperazine))) | |
| hGLP-1(7-36)NH₂; | (SEQ ID NO.661) |
| (Ser⁸, Aib³⁵, Arg^{25,26,34}, Asp³⁶(1-(4-dodecylpiperazine)))hGLP-1(7-36) | |
| NH₂; | (SEQ ID NO.662) |
| (Ser⁸, Aib³⁵, Arg^{25,26,34}, Asp³⁶(1-(4-tetradecylpiperazine))) | |
| hGLP-1(7-36)NH₂; | (SEQ ID NO.663) |
| (Ser⁸, Aib³⁵, Arg^{25,26,34}, Asp³⁶(1-(4-hexadecylpiperazine))) | |
| hGLP-1(7-36)NH₂; | (SEQ ID NO.664) |
| (Ser⁸, Aib³⁵, Arg^{25,26,34}, Asp³⁸(1-(4-decylpiperazine))) | |
| hGLP-1(7-38)NH₂; | (SEQ ID NO.665) |
| (Ser⁸, Aib³⁵, Arg^{25,26,34}, Asp³⁸(1-(4-dodecylpiperazine))) | |
| hGLP-1(7-38)NH₂; | (SEQ ID NO.666) |
| (Ser⁸, Aib³⁵, Arg^{25,26,34}, Asp³⁸(1-(4-tetradecylpiperazine))) | |
| hGLP-1(7-38)NH₂; | (SEQ ID NO.667) |
| (Ser⁸, Aib³⁵, Arg^{25,26,34}, Asp³⁸(1-(4-hexadecylpiperazine))) | |
| hGLP-1 (7-38)NH₂; | (SEQ ID NO.668) |
| (Ser⁸, Aib^{35,37}, Arg^{25,26,34}, Asp³⁸(1-(4-decylpiperazine))) | |
| hGLP-1(7-38)NH₂; | (SEQ ID NO.669) |
| (Ser⁸, Aib^{35,37}, Arg^{25,26,34}, Asp³⁸(1-(4-dodecylpiperazine))) | |
| hGLP-1 (7-38)NH₂; | (SEQ ID NO.670) |
| (Ser⁸, Aib^{35,37}, Arg^{25,26,34}, Asp³⁸(1-(4-tetradecylpiperazine))) | |
| hGLP-1 (7-38)NH₂; | (SEQ ID NO.671) |
| (Ser⁸, Aib^{35,37}, Arg^{25,26,34}, Asp³⁸(1-(4-hexadecylpiperazine))) | |
| hGLP-1 (7-38)NH₂; | (SEQ ID NO.672) |
| (Ser⁸, Aib³⁵, Arg^{26,34}, Glu³⁶(1-dodecylamino))hGLP-1(7-36)NH₂; | (SEQ ID NO.673) |
| (Ser⁸, Aib³⁵, Glu²⁶(1-dodecylamino), Arg³⁴)hGLP-1(7-36)NH₂; | (SEQ ID NO.674) |
| (Ser⁸, Aib³⁵, Arg²⁶ , Glu³⁴(1-dodecylamino))hGLP-1(7-36)NH₂; | (SEQ ID NO.675) |
| (Ser⁸, Aib^{35,37}, Arg^{26,34}, Glu³⁸(1-dodecylamino))hGLP-1(7-38)NH₂; | (SEQ ID NO.676) |
| (Ser⁸, Aib³⁵, Arg³⁴, Lys²⁶(N^{ε}-(2-(4-decyl-1-piperazine)-acetyl))) | |
| hGLP-1 (7-36)NH₂; | (SEQ ID NO.677) |
| (Ser⁸, Aib³⁵, Arg³⁴, Lys²⁶(N^{ε}-(2-(4-dodecyl-1-piperazine)-acetyl))) | |
| hGLP-1 (7-36)NH₂; | (SEQ ID NO.678) |
| (Ser⁸, Aib³⁵, Arg³⁴, Lys²⁶(N^{ε}-(2-(4-tetradecyl-1-piperazine)-acetyl))) | |
| hGLP-1 (7-36)NH₂; | (SEQ ID NO.679) |
| (Ser⁸, Aib³⁵, Arg³⁴, Lys²⁶(N^{ε}-(2-(4-hexadecyl-1-piperazine)-acetyl))) | |
| hGLP-1 (7-36)NH₂; | (SEQ ID NO.680) |
| (Ser⁸, Aib³⁵, Arg²⁶, Lys³⁴(N^{ε}-(2-(4-decyl-1-piperazine)-acetyl))) | |
| hGLP-1(7-36)NH₂; | (SEQ ID NO.681) |
| (Ser⁸, Aib³⁵, Arg²⁶, Lys³⁴(N^{ε}-(2-(4-dodecyl-1-piperazine)-acetyl)) | |
| hGLP-1(7-36)NH₂; | (SEQ ID NO.682) |
| (Ser⁸, Aib³⁵, Arg²⁶, Lys³⁴(N^{ε}-(2-(4-tetradecyl-1-piperazine)-acetyl))) | |
| hGLP-1(7-36)NH₂; | (SEQ ID NO.683) |
| (Ser⁸, Aib³⁵, Arg²⁶, Lys³⁴(N^{ε}-(2-(4-hexadecyl-1-piperazine)-acetyl))) | |
| hGLP-1(7-36)NH₂; | (SEQ ID NO.684) |
| (Ser⁸, Aib³⁵, Arg^{26,34}, Lys³⁶(N^{ε}-(2-(4-decyl-1-piperazine)-acetyl))) | |
| hGLP-1(7-36)NH₂; | (SEQ ID NO.685) |
| (Ser⁸, Aib³⁵, Arg^{26,34}, Lys³⁶(N^{ε}-(2-(4-dodecyl-1-piperazine)-acetyl))) | |
| hGLP-1 (7-36)NH₂; | (SEQ ID NO.686) |
| (Ser⁸, Aib³⁵, Arg^{26,34}, Lys³⁶(N^{ε}-(2-(4-hexadecyl-1-piperazine)-acetyl))) | |
| hGLP-1(7-36)NH₂; | (SEQ ID NO.687) |
| (Ser⁸, Aib³⁵, Arg^{26,34}, Lys³⁸(N^{ε}-(2-(4-decyl-1-piperazine)-acetyl))) | |
| hGLP-1(7-38)NH₂; | (SEQ ID NO.688) |
| (Ser⁸, Aib³⁵, Arg^{26,34}, Lys³⁸(N^{ε}-(2-(4-dodecyl-1-piperazine)-acetyl))) | |
| hGLP-1(7-38)NH₂; | (SEQ ID NO.689) |
| (Ser⁸, Aib³⁵, Arg^{26,34}, Lys³⁸(N^{ε}-(2-(4-tetradecyl-1-piperazine)-acetyl)) | |
| )hGLP-1(7-38)NH₂; | (SEQ ID NO.690) |
| (Ser⁸, Aib³⁵, Arg^{26,34}, Lys³⁸(N^{ε}-(2-(4-hexadecyl-1-piperazine)-acetyl))) | |
| hGLP-1(7-38)NH₂; | (SEQ ID NO.691) |
| (Ser⁸, Aib^{35,37}, Arg^{26,34}, Lys³⁸(N^{ε}-(2-(4-decyl-1-piperazine)-acetyl))) | |
| hGLP-1(7-38)NH₂; | (SEQ ID NO.692) |
| (Ser⁸, Aib^{35,37}, Arg^{26,34}, Lys³⁸(N^{ε}-(2-(4-dodecyl-1-piperazine)-acetyl))) | |
| hGLP-1(7-38)NH₂; | (SEQ ID NO.693) |
| (Ser⁸, Aib^{35,37}, Arg^{26,34}, Lys³⁸(N^{ε}-(2-(4-tetradecyl-1-piperazine)-acetyl))) | |
| hGLP-1(7-38)NH₂; | (SEQ ID NO.694) |
| (Ser⁸, Aib^{35,37}, Arg^{26,34}, Lys³⁸(N^{ε}-(2-(4-hexadecyl-1-piperazine)-acetyl))) | |
| hGLP-1(7-38)NH₂; | (SEQ ID NO.695) |
| (Ser⁸, Aib³⁵, Arg^{25,34}, Lys²⁶(N^{ε}-(2-(4-decyl-1-piperazine)-acetyl))) | |
| hGLP-1(7-36)NH₂; | (SEQ ID NO.696) |
| (Ser⁸, Aib³⁵, Arg^{25,34}, Lys²⁶(N^{ε}-(2-(4-dodecyl-1-piperazine)-acetyl))) | |
| hGLP-1(7-36)NH₂; | (SEQ ID NO.697) |
| (Ser⁸, Aib³⁵, Arg^{25,34}, Lys²⁶(N^{ε}-(2-(4-tetradecyl-1-piperazine)-acetyl))) | |
| hGLP-1(7-36)NH₂; | (SEQ ID NO.698) |
| (Ser⁸, Aib³⁵, Arg^{25,34}, Lys²⁶(N^{ε}-(2-(4-hexadecyl-1-piperazine)-acetyl))) | |
| hGLP-1(7-36)NH₂; | (SEQ ID NO.699) |
| (Ser⁸, Aib³⁵, Arg^{25,26}, Lys³⁴(N^{ε}-(2-(4-decyl-1-piperazine)-acetyl))) | |
| hGLP-1(7-36)NH₂; | (SEQ ID NO.700) |
| (Ser⁸, Aib³⁵, Arg^{25,26}, Lys³⁴(N^{ε}-(2-(4-dodecyl-1-piperazine)-acetyl))) | |
| hGLP-1(7-36)NH₂; | (SEQ ID NO.701) |
| (Ser⁸, Aib³⁵, Arg^{25,26}, Lys³⁴(N^{ε}-(2-(4-tetradecyl-1-piperazine)-acetyl))) | |
| hGLP-1(7-36)NH₂; | (SEQ ID NO.702) |
| (Ser⁸, Aib³⁵, Arg^{25,26}, Lys³⁴(N^{ε}-(2-(4-hexadecyl-1-piperazine)-acetyl))) | |
| hGLP-1(7-36)NH₂; | (SEQ ID NO.703) |
| (Ser⁸, Aib³⁵, Arg^{25,26,34}, Lys³⁶(N^{ε}-(2-(4-decyl-1-piperazine)-acetyl))) | |
| hGLP-1(7-36)NH₂; | (SEQ ID NO.704) |
| (Ser⁸, Aib³⁵, Arg^{25,26,34}, Lys³⁶(N^{ε}-(2-(4-dodecyl-1-piperazine)-acetyl))) | |
| hGLP-1(7-36)NH₂; | (SEQ ID NO.705) |
| (Ser⁸, Aib³⁵, Arg^{25,26,34}, Lys³⁶(N^{ε}-(2-(4-tetradecyl-1-piperazine)-acetyl))) | |
| hGLP-1(7-36)NH₂; | (SEQ ID NO.706) |
| (Ser⁸, Aib³⁵, Arg^{25,26,34}, Lys³⁶(N^{ε}-(2-(4-hexadecyl-1-piperazine)-acetyl))) | |
| hGLP-1(7-36)NH₂; | (SEQ ID NO.707) |
| (Ser⁸, Aib³⁵, Arg^{25,26,34}, Lys³⁸(N^{ε}-(2-(4-decyl-1-piperazine)-acetyl))) | |
| hGLP-1(7-38)NH₂; | (SEQ ID NO.708) |
| (Ser⁸, Aib³⁵, Arg^{25,26,34}, Lys³⁸(N^{ε}-(2-(4-dodecyl-1-piperazine)-acetyl))) | |
| hGLP-1(7-38)NH₂; | (SEQ ID NO.709) |
| (Ser⁸, Aib³⁵, Arg^{25,26,34}, Lys³⁸(N^{ε}-(2-(4-tetradecyl-1-piperazine)-acetyl))) | |
| hGLP-1(7-38)NH₂; | (SEQ ID NO.710) |
| (Ser⁸, Aib³⁵, Arg^{25,26,34}, Lys³⁸(N^{ε}-(2-(4-hexadecyl-1-piperazine)-acetyl))) | |
| hGLP-1(7-38)NH₂; | (SEQ ID NO.711) |
| (Ser⁸, Aib^{35,37}, Arg^{25,26,34}, Lys³⁸(N^{ε}-(2-(4-decyl-1-piperazine)-acetyl))) | |
| hGLP-1(7-38)NH₂; | (SEQ ID NO.712) |
| (Ser⁸, Aib^{35,37}, Arg^{25,26,34}, Lys³⁸(N^{ε}-(2-(4-dodecyl-1-piperazine)-acetyl))) | |
| hGLP-1(7-38)NH₂; | (SEQ ID NO.713) |
| (Ser⁸, Aib^{35,37}, Arg^{25,26,34}, Lys³⁸(N^{ε}-(2-(4-tetradecyl-1-piperazine)-acetyl))) | |
| hGLP-1(7-38)NH₂; | (SEQ ID NO.714) |
| (Ser⁸, Aib^{35,37}, Arg^{25,26,34}, Lys³⁸(N^{ε}-(2-(4-hexadecyl-1-piperazine)-acetyl))) | |
| hGLP-1(7-38)NH₂; | (SEQ ID NO.715) |
| (Ser⁸, Aib³⁵, Arg^{26,34}, Lys³⁶(N^{ε}-decanoyl))hGLP-1(7-36)OH; | (SEQ ID NO.716) |
| (Ser⁸, Aib³⁵, Lys²⁵, Arg^{26,34}, Lys³⁶(N^{ε}-decanoyl))hGLP-1(7-36)OH; | (SEQ ID NO.717) |
| (Ser⁸, Aib³⁵,Arg^{26,34}, Ava³⁷, Ado³⁸)hGLP-1(7-38)NH₂; | (SEQ ID NO.718) |
| (Ser⁸, Aib³⁵,Arg^{26,34}, Asp³⁷, Ava³⁸, Ado³⁹)hGLP-1(7-39)NH₂; | (SEQ ID NO.719) |
| (Ser⁸, Aib³⁵,Arg^{26,34}, Aun³⁷)hGLP-1(7-37)NH₂; | (SEQ ID NO.720) |
| (Ser⁸, Aib^{17,35},)hGLP-1(7-36)NH₂; | (SEQ ID NO.721) |
| (Ser⁸ ,Arg^{26,34}, β-Ala³⁵, D-ASp³⁷, Ava³⁸, Aun³⁹)hGLP-1(7-39)NH₂; | (SEQ ID NO.722) |
| (Ser⁸, GlU^{22,23}, β-Ala³⁵)hGLP-1(7-36)NH₂; | (SEQ ID NO.723) |
| (Ser⁸, Lys¹⁸, β-Ala³⁵)hGLP-1(7-36)NH₂; | (SEQ ID NO.724) |
| (Ser⁸, Leu²⁷, β-Ala³⁵)hGLP-1(7-36)NH₂; | (SEQ ID NO.725) |
| (Ser⁸, Lys³³, β-Ala³⁵)hGLP-1(7-36)NH₂; | (SEQ ID NO.726) |
| (Ser⁸, Lys¹⁸, Leu²⁷, β-Ala³⁵)hGLP-1(7-36)NH₂; | (SEQ ID NO.727) |
| (Ser⁸, D-Arg³⁶)hGLP-1(7-36)NH₂; | (SEQ ID NO.728) |
| (Ser⁸, β-Ala³⁵, D-Arg³⁷)hGLP-1(7-37)NH₂; | (SEQ ID NO.729) |
| (Ser⁸, Aib²⁷, β-Ala³⁵)hGLP-1(7-36)NH₂; | (SEQ ID NO.730) |
| (Ser⁸, Aib²⁷, β-Ala^{35,37}, Arg³⁸)hGLP-1(7-38)NH₂; | (SEQ ID NO.731) |
| (Ser⁸, Aib²⁷, β-Ala^{35,37}, Arg^{38,39})hGLP-1(7-39)NH₂; | (SEQ ID NO.732) |
| (Ser⁸, Lys^{18,27}, β-Ala³⁵)hGLP-1(7-36)NH₂; | (SEQ ID NO.733) |
| (Ser⁸, Lys²⁷, β-Ala³⁵)hGLP-1(7-36)NH₂; | (SEQ ID NO.734) |
| (Ser⁸, β-Ala³⁵, Arg³⁸)hGLP-1(7-38)NH₂; | (SEQ ID NO.735) |
| (Ser⁸, Arg^{26,34}, β-Ala³⁵,)hGLP-1(7-36)NH₂; | (SEQ ID NO.736) |
| (Ser⁸, D-Arg³⁵)hGLP-1(7-36)NH₂; | (SEQ ID NO.737) |
| (Ser⁸, β-Ala³⁵, Arg³⁷)hGLP-1(7-37)NH₂; | (SEQ ID NO.738) |
| (Ser⁸, Phe³¹, β-Ala³⁵)hGLP-1(7-36)NH₂; | (SEQ ID NO.739) |
| (Ser⁸, Aib³⁵, Phe³¹)hGLP-1(7-36)NH₂; | (SEQ ID NO.740) |
| (Ser⁸, Aib³⁵, 2-Nal³¹)hGLP-1(7-36)NH₂; | (SEQ ID NO.741) |
| (Ser⁸, Aib³⁵, 2-Nal^{28,31})hGLP-1(7-36)NH₂; | (SEQ ID NO.742) |
| (Ser⁸, Aib³⁵, Arg^{26,34}, 2-Nal³¹)hGLP-1(7-36)NH₂; | (SEQ ID NO.743) |
| (Ser⁸, Aib³⁵, Arg^{26,34}, Phe³¹)hGLP-1(7-36)NH₂; | (SEQ ID NO.744) |
| (Ser⁸, Aib³⁵, 2-Nal^{19,31})hGLP-1(7-36)NH₂; | (SEQ ID NO.745) |
| (Ser⁸, Aib³⁵, 2-Nal^{12,31})hGLP-1(7-36)NH₂; | (SEQ ID NO.746) |
| (Ser⁸, Aib³⁵, Lys³⁶(N^{ε}-decanoyl))hGLP-1(7-36)NH₂; | (SEQ ID NO.747) |
| (Ser⁸, Aib³⁵, Arg³⁴, Lys²⁶(N^{ε}-decanoyl))hGLP-1(7-36)NH₂; | (SEQ ID NO.748) |
| (Ser⁸, Aib³⁵, Arg^{26,34}, Lys³⁶(N^{ε}-dodecanoyl))hGLP-1(7-36)NH₂; | (SEQ ID NO.749) |
| (Ser⁸, β-Ala³⁵,Ser³⁷(O-decanoyl))hGLP1(7-37)-NH₂; | (SEQ ID NO.750) |
| (Ser⁸, Aib²⁷, β-Ala^{35,37}, Arg³⁸, Lys³⁹(N^{ε}-octanoyl))hGLP-1(7-39)NH₂; | (SEQ ID NO.751) |
| (Ser⁸, Arg^{26,34}, β-Ala³⁵, Lys³⁷(N^{ε}-octanoyl))hGLP-1(7-37)NH₂; | (SEQ ID NO.752) |
| (Ser⁸, Arg^{26,34}, β-Ala³⁵, Lys³⁷(N^{ε}-decanoyl))hGLP-1(7-37)NH₂; | (SEQ ID NO.753) |
| (Ser⁸, Arg^{26,34}, β-Ala³⁵, Lys³⁷(N^{ε}-tetradecanoyl))hGLP-1(7-37)NH₂; | (SEQ ID NO.754) |
| (Ser⁸, Arg^{26,34}, β-Ala³⁵, Lys³⁷(N^{ε}-dodecanoyl))hGLP-1(7-37)NH₂; | (SEQ ID NO.755) |
| (Ser⁸, Arg^{26,34}, β-Ala³⁵, Lys³⁷(N^{ε}-dodecanoyl))hGLP-1(8-37)NH₂; | (SEQ ID NO.756) |
| (Ser⁸, Arg^{26,34}, β-Ala³⁵, Lys³⁶(N^{ε}-Aec-decanoyl))hGLP-1(7-36)NH₂; | (SEQ ID NO.757) |
| (Ser⁸, Arg²⁶, Aib³⁵)hGLP-1(7-36)NH₂; | (SEQ ID NO.758) |
| (Ser⁸, Arg²⁶, Phe³¹, Aib³⁵)hGLP-1(7-36)NH₂; | (SEQ ID NO.759) |
| (Ser⁸, Arg²⁶, Phe³¹, β-Ala³⁵)hGLP-1(7-36)NH₂; | (SEQ ID NO.760) |
| (Ser⁸, Arg²⁶, β-Ala³⁵)hGLP-1(7-36)NH₂; | (SEQ ID NO.761) |
| (Ser⁸, Arg^{26,34}, Phe³¹, β-Ala³⁵)hGLP-1(7-36)NH₂; | (SEQ ID NO.762) |
| (Ser⁸, Arg³⁴, Aib³⁵)hGLP-1(7-36)NH₂; | (SEQ ID NO.763) |
| (Ser⁸, Arg³⁴, β-Ala³⁵)hGLP-1(7-36)NH₂; | (SEQ ID NO.764) |
| (Ser⁸, Phe³¹, Arg³⁴, Aib³⁵)hGLP-1(7-36)NH₂; | (SEQ ID NO.765) |
| or (Ser⁸, Phe³¹, Arg³⁴, β-Ala³⁵)hGLP-1(7-36)NH₂; | (SEQ ID NO.766) |
or a pharmaceutically acceptable salt thereof.

13. A compound according to claim 11 wherein said compound is:
| | |
|---|---|
| (Gly⁸, Aib³⁵, A6c³²)hGLP-1(7-36)NH₂; | (SEQ ID NO.8) |
| (Gly⁸, Aib³⁵, Glu²³)hGLP-1(7-36)NH₂; | (SEQ ID NO.10) |
| (Gly⁸, Aib^{24,35})hGLP-1(7-36)NH₂; | (SEQ ID NO.11) |
| (Gly⁸, Aib³⁵, GlU²³, A6c³²)hGLP-1(7-36)NH₂; | (SEQ ID NO.28) |
| (Gly⁸, Glu²³, β-Ala³⁵)hGLP-1(7-36)NH₂; | (SEQ ID NO.34) |
| (Gly⁸, Aib³⁵, Arg^{26,34})hGLP-1(7-36)NH₂; | (SEQ ID NO.59) |
| (Gly⁸, Aib³⁵, Arg^{26,34}, Lys³⁶(N^{ε}-octanoyl))hGLP-1(7-36)NH₂; | (SEQ ID NO.117) |
| (Gly⁸, Aib³⁵, Arg^{26,34}, Lys³⁶(N^{ε}-decanoyl))hGLP-1(7-36)OH; | (SEQ ID NO.332) |
| (Gly⁸, Aib³⁵, Lys²⁵, Arg^{26,34}, Lys³⁶(N^{ε}-decanoyl))hGLP-1(7-36)OH; | (SEQ ID NO.333) |
| (Gly⁸, Arg^{26,34}, β-Ala³⁵, Lys³⁶(N^{ε}-Aec-decanoyl))hGLP-1(7-36)NH₂; | (SEQ ID NO.383) |
| (Gly⁸, Aib³⁵,Arg^{26,34}, Ava³⁷, Ado³⁸)hGLP-1(7-38)NH₂; | (SEQ ID NO.334) |
| (Gly⁸, Aib³⁵,Arg^{26,34}, Asp³⁷, Ava³⁸, Ado³⁹)hGLP-1(7-39)NH₂; | (SEQ ID NO.335) |
| (Gly⁸, Aib³⁵,Arg^{26,34}, Aun³⁷)hGLP-1(7-37)NH₂; | (SEQ ID NO.336) |
| (Gly⁸, Aib^{17,35})hGLP-1(7-36)NH₂; | (SEQ ID NO.337) |
| (Gly⁸ ,Arg^{26,34}, β-Ala³⁵, D-Asp³⁷, Ava³⁸, Aun³⁹)hGLP-1(7-39)NH₂; | (SEQ ID NO.338) |
| (Gly⁸, GlU^{22,23}, β-Ala³⁵)hGLP-1(7-36)NH₂; | (SEQ ID NO.339) |
| (Gly⁸, Lys¹⁸, β-Ala³⁵)hGLP-1(7-36)NH₂; | (SEQ ID NO.340) |
| (Gly⁸, Leu²⁷, β-Ala³⁵)hGLP-1(7-36)NH₂; | (SEQ ID NO.341) |
| (Gly⁸, Lys³³, β-Ala³⁵)hGLP-1(7-36)NH₂; | (SEQ ID NO.342) |
| (Gly⁸, Lys¹⁸, Leu²⁷, β-Ala³⁵)hGLP-1(7-36)NH₂; | (SEQ ID NO.343) |
| (Gly⁸, D-Arg³⁶)hGLP-1(7-36)NH₂; | (SEQ ID NO.344) |
| (Gly⁸, β-Ala³⁵, D-Arg³⁷)hGLP-1(7-37)NH₂; | (SEQ ID NO.345) |
| (Gly⁸, Aib²⁷, β-Ala³⁵)hGLP-1(7-36)NH₂; | (SEQ ID NO.346) |
| (Gly⁸, Aib²⁷, β-Ala^{35,37}, Arg³⁸)hGLP-1(7-38)NH₂; | (SEQ ID NO.347) |
| (Gly⁸, Aib²⁷, β-Ala^{35,37}, Arg^{38,39})hGLP-1(7-39)NH₂; | (SEQ ID NO.348) |
| (Gly⁸, Lys^{18,27}, β-Ala³⁵)hGLP-1(7-36)NH₂; | (SEQ ID NO.349) |
| (Gly⁸, Lys²⁷, β-Ala³⁵)hGLP-1(7-36)NH₂; | (SEQ ID NO.350) |
| (Gly⁸, β-Ala³⁵, Arg³⁸)hGLP-1(7-38)NH₂; | (SEQ ID NO.351) |
| (Gly⁸, Arg^{26,34}, β-Ala³⁵,)hGLP-1(7-36)NH₂; | (SEQ ID NO.352) |
| (Gly⁸, D-Arg³⁵)hGLP-1(7-36)NH₂; | (SEQ ID NO.353) |
| (Gly⁸, β-Ala³⁵, Arg³⁷)hGLP-1(7-37)NH₂; | (SEQ ID NO.354) |
| (Gly⁸, Phe³¹, β-Ala³⁵)hGLP-1(7-36)NH₂; | (SEQ ID NO.355) |
| (Gly⁸, Aib³⁵, Phe³¹)hGLP-1(7-36)NH₂; | (SEQ ID NO.356) |
| (Gly⁸, Aib³⁵, 2-Nal³¹)hGLP-1(7-36)NH₂; | (SEQ ID NO.357) |
| (Gly⁸, Aib³⁵, 2-Nal^{28,31})hGLP-1(7-36)NH₂; | (SEQ ID NO.358) |
| (Gly⁸, Aib³⁵, Arg^{26,34}, 2-Nal³¹)hGLP-1(7-36)NH₂; | (SEQ ID NO.359) |
| (Gly⁸ Aib³⁵, Arg^{26,34}, Phe³¹)hGLP-1(7-36)NH₂; | (SEQ ID NO.360) |
| (Gly⁸, Aib³⁵, 2-Nal^{19,31})hGLP-1(7-36)NH₂; | (SEQ ID NO.361) |
| (Gly⁸, Aib³⁵, 2-Nal^{12,31})hGLP-1(7-36)NH₂; | (SEQ ID NO.362) |
| (Gly⁸, Aib³⁵, Lys³⁶(N^{ε}-decanoyl))hGLP-1(7-36) NH₂; | (SEQ ID NO.99) |
| (Gly⁸, Aib³⁵, Arg³⁴, Lys²⁶(N^{ε}-decanoyl))hGLP-1(7-36)NH₂; | (SEQ ID NO.364) |
| (Gly⁸, Aib³⁵, Arg^{26,34}, Lys³⁶(N^{ε}-dodecanoyl))hGLP-1(7-36)NH₂; | (SEQ ID NO.365) |
| (Gly⁸, β-Ala³⁵, Ser³⁷(O-decanoyl))hGLP1(7-37)-NH₂; | (SEQ ID NO.384) |
| (Gly⁸, Aib²⁷, β-Ala^{35,37}, Arg³⁸, Lys³⁹(N^{ε}-octanoyl))hGLP-1(7-39)NH₂; | (SEQ ID NO.367) |
| (Gly⁸, Arg^{26,34}, β-Ala³⁵, Lys³⁷(N^{ε}-octanoyl))hGLP-1(7-37)NH₂; | (SEQ ID NO.368) |
| (Gly⁸, Arg^{26,34}, β-Ala³⁵, Lys³⁷(N^{ε}-decanoyl))hGLP-1(7-37)NH₂; | (SEQ ID NO.369) |
| or (Gly⁸, Arg^{26,34}, β-Ala³⁵, Lys³⁷(N^{ε}-tetradecanoyl))hGLP-1(7-37)NH₂; | (SEQ ID NO.370) |
or a pharmaceutically acceptable salt thereof.

14. A compound according to claim 12 wherein said compound is:
| | |
|---|---|
| (Ser⁸, Aib³⁵, A6c³²)hGLP-1(7-36)NH₂; | (SEQ ID NO.392) |
| (Ser⁸, Aib³⁵, Glu²³)hGLP-1(7-36)NH₂; | (SEQ ID NO.394) |
| (Ser⁸, Aib^{24,35})hGLP-1(7-36)NH₂; | (SEQ ID NO.395) |
| (Ser⁸, Aib³⁵, Glu²³, A6c³²)hGLP-1(7-36)NH₂; | (SEQ ID NO.412) |
| (Ser⁸, Glu²³, β-Ala³⁵)hGLP-1(7-36)NH₂; | (SEQ ID NO.418) |
| (Ser⁸, Aib³⁵, Arg^{26,34} )hGLP-1(7-36)NH₂; | (SEQ ID NO.443) |
| (Ser⁸, Aib³⁵, Arg^{26,34}, Lys³⁶(N^{ε}-octanoyl))hGLP-1(7-36)NH₂; | (SEQ ID NO.501) |
| (Ser⁸, Aib³⁵, Arg^{26,34}, Lys³⁶(N^{ε}-decanoyl))hGLP-1(7-36)OH; | (SEQ ID NO.716) |
| (Ser⁸, Aib³⁵, Lys²⁵, Arg^{26,34}, Lys³⁶(N^{ε}-decanoyl))hGLP-1(7-36)OH; | (SEQ ID NO.717) |
| (Ser⁸, Arg^{26,34}, β-Ala³⁵, Lys³⁶(N^{ε}-Aec-decanoyl))hGLP-1(7-36)NH₂; | (SEQ ID NO.757) |
| (Ser⁸, Aib³⁵,Arg^{26,34}, Ava³⁷, Ado³⁸ )hGLP-1(7-38)NH₂; | (SEQ ID NO.718) |
| (Ser⁸, Aib³⁵,Arg^{26,34}, Asp³⁷, Ava³⁸, Ado³⁹)hGLP-1(7-39)NH₂; | (SEQ ID NO.719) |
| (Ser⁸, Aib³⁵,Arg^{26,34}, Aun³⁷)hGLP-1(7-37)NH₂; | (SEQ ID NO.720) |
| (Ser⁸, Aib^{17,35})hGLP-1(7-36)NH₂; | (SEQ ID NO.389) |
| (Ser⁸, Arg^{26,34}, β-Ala³⁵, D-Asp³⁷, Ava³⁸, Aun³⁹)hGLP-1(7-39)NH₂; | (SEQ ID NO.722) |
| (Ser⁸, Glu^{22,23}, β-Ala³⁵)hGLP-1(7-36)NH₂; | (SEQ ID NO.723) |
| (Ser⁸, Lys¹⁸, β-Ala³⁵)hGLP-1(7-36)NH₂; | (SEQ ID NO.724) |
| (Ser⁸, Leu²⁷, β-Ala³⁵)hGLP-1(7-36)NH₂; | (SEQ ID NO.725) |
| (Ser⁸, Lys³³, β-Ala³⁵)hGLP-1 (7-36)NH₂; | (SEQ ID NO.726) |
| (Ser⁸, Lys¹⁸, Leu²⁷, β-Ala³⁵)hGLP-1(7-36)NH₂; | (SEQ ID NO.727) |
| (Ser⁸, D-Arg³⁶)hGLP-1(7-36)NH₂; | (SEQ ID NO.728) |
| (Ser⁸, β-Ala³⁵, D-Arg³⁷)hGLP-1(7-37)NH₂; | (SEQ ID NO.729) |
| (Ser⁸, Aib²⁷, β-Ala³⁵)hGLP-1(7-36)NH₂; | (SEQ ID NO.730) |
| (Ser⁸, Aib²⁷, β-Ala^{35,37}, Arg³⁸ )hGLP-1(7-38)NH₂; | (SEQ ID NO.731) |
| (Ser⁸, Aib²⁷, β-Ala^{35,37}, Arg^{38,39})hGLP-1(7-39)NH₂; | (SEQ ID NO.732) |
| (Ser⁸, Lys^{18,27}, β-Ala³⁵)hGLP-1(7-36)NH₂; | (SEQ ID NO.733) |
| (Ser⁸, Lys²⁷, β-Ala³⁵)hGLP-1(7-36)NH₂; | (SEQ ID NO.734) |
| (Ser⁸, β-Ala³⁵, Arg³⁸)hGLP-1(7-38)NH₂; | (SEQ ID NO.735) |
| (Ser⁸, Arg^{26,34}, β-Ala³⁵)hGLP-1(7-36)NH₂; | (SEQ ID NO.736) |
| (Ser⁸, D-Arg³⁵)hGLP-1(7-36)NH₂; | (SEQ ID NO.737) |
| (Ser⁸, β-Ala³⁵, Arg³⁷)hGLP-1(7-37)NH₂; | (SEQ ID NO.738) |
| (Ser⁸, Phe³¹, β-Ala³⁵)hGLP-1(7-36)NH₂; | (SEQ ID NO.739) |
| (Ser⁸, Aib³⁵, Phe³¹)hGLP-1(7-36)NH₂; | (SEQ ID NO.740) |
| (Ser⁸, Aib³⁵, 2-Nal³¹)hGLP- 1(7-36)NH₂; | (SEQ ID NO.741) |
| (Ser⁸, Aib³⁵, 2-Nal^{28,31})hGLP-1(7-36)NH₂; | (SEQ ID NO.742) |
| (Ser⁸, Aib³⁵, Arg^{26,34}, 2-Nal³¹)hGLP-1(7-36)NH₂; | (SEQ ID NO.743) |
| (Ser⁸, Aib³⁵, Arg^{26,34}, Phe³¹)hGLP-1(7-36)NH₂; | (SEQ ID NO.744) |
| (Ser⁸, Aib³⁵, 2-Nal^{19,31})hGLP-1(7-36)NH₂; | (SEQ ID NO.745) |
| (Ser⁸, Aib³⁵, 2-Nal^{12,31})hGLP-1(7-36)NH₂; | (SEQ ID NO.746) |
| (Ser⁸, Aib³⁵, Lys³⁶(N^{ε}-decanoyl))hGLP-1(7-36)NH₂; | (SEQ ID NO.483) |
| (Ser⁸, Aib³⁵, Arg³⁴, Lys²⁶(N^{ε}-decanoyl))hGLP-1(7-36)NH₂; | (SEQ ID NO.748) |
| (Ser⁸, Aib³⁵, Arg^{26,34}, Lys³⁶(N^{ε}-dodecanoyl))hGLP-1(7-36)NH₂; | (SEQ ID NO.749) |
| (Ser⁸, β-Ala³⁵, Ser³⁷(O-decanoyl))hGLP1(7-37)-NH₂; | (SEQ ID NO.539) |
| (Ser⁸, Aib²⁷, β-Ala^{35,37}, Arg³⁸ , Lys³⁹(N^{ε}-octanoyl))hGLP-1(7-39)NH₂; | (SEQ ID NO.751) |
| (Ser⁸, Arg^{26,34}, β-Ala³⁵, Lys³⁷(N^{ε}-octanoyl))hGLP-1(7-37)NH₂; | (SEQ ID NO.752) |
| (Ser⁸, Arg^{26,34}, β-Ala³⁵, Lys³⁷(N^{ε}-decanoyl))hGLP-1(7-37)NH₂; | (SEQ ID NO.753) |
| or (Ser⁸, Arg^{26,34}, β-Ala³⁵, Lys³⁷(N^{ε}-tetradecanoyl))hGLP-1(7-37)NH₂; | (SEQ ID NO.754) |
or a pharmaceutically acceptable salt thereof.

15. A pharmaceutical composition comprising an effective amount of a compound according to claim 1 or a pharmaceutically acceptable salt thereof and a pharmaceutically acceptable carrier or diluent.

16. A compound according to claim 1 or a pharmaceutically acceptable salt thereof for eliciting an agonist effect from a GLP-1 receptor in a subject in need thereof..

17. A compound according to claim 1 or a pharmaceutically acceptable salt thereof for treating a disease selected from the group consisting of Type I diabetes, Type II diabetes, obesity, glucagonomas, secretory disorders of the airway, metabolic disorder, arthritis, osteoporosis, central nervous system disease, restenosis and neurodegenerative disease, in a subject in need thereof.

18. A compound according to claim 17 wherein said disease is Type I diabetes or Type II diabetes.


SEQUENCE LISTING

<110> SOCIETE DE CONSEILS DE RECHERCHES ET D'APPLICATIONS
      SCIENTIFIQUES, S.A.S
      DONG, ZHENG ZIN

<120> ANALOGUES OF GLP-1

<130> 129P-PCT2

<160> 781

<140>
<141>

<150> 60/449,203
<151> 2003-02-19

<170> PatentIn version 3.2

<210> 1
<211> 30
<212> PRT
<213> Artificial Sequence

<220>
<223> Description of Artificial Sequence: Synthetic modified hGLP-1
      peptide


<220>
<221> MOD_RES
<222> (1)..(1)
<223> N-Me-His

<220>
<221> MOD_RES
<222> (29)..(29)
<223> Aib

<220>
<223> c-term amidation

<400> 1
Xaa Gly Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Arg
            20                  25                  30


<210> 2
<211> 30
<212> PRT
<213> Artificial Sequence

<220>
<223> Description of Artificial Sequence: Synthetic modified hGLP-1
      peptide


<220>
<221> MOD_RES
<222> (1)..(1)
<223> N-Me-His

<220>

<221> MOD_RES
<222> (29)..(29)
<223> Beta-Ala

<220>
<223> c-term amidation

<400> 2
Xaa Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 3
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-Me-His

<220>
<221> MOD_RES
<222> (29)..(29)
<223> Aib

<220>
<223> c-term amidation

<400> 3
Xaa Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 4
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-Me-His

<220>
<221> MOD_RES
<222> (29)..(29)
<223> Beta-Ala

<220>
<223> c-term amidation

<400> 4
Xaa Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 5
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> A6c

<220>
<223> c-term amidation

<400> 5
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 6
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> A5c

<220>
<223> c-term amidation

<400> 6
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 7
<211> 30
<212> PRT

<213> Artificial Sequence

<220>
<223> Description of Artificial Sequence: Synthetic modified hGLP-1
      peptide

<220>
<221> MOD_RES
<222> (29)..(29)
<223> D-Ala

<220>
<223> c-term amidation

<400> 7
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 8
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<223> c-term amidation

<400> 8
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Lys Xaa Arg
            20                  25                  30

<210> 9
<211> 30
<212> PRT
<213> Artificial Sequence

<220>
<223> Description of Artificial Sequence: Synthetic modified hGLP-1
      peptide

<220>
<221> MOD_RES
<222> (26)..(26)

<223> A5c

<220>
<221> MOD_RES
<222> (29)..(29)
<223> Aib

<220>
<223> C-term amidation

<400> 9
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Lys Xaa Arg
            20                  25                  30

<210> 10
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> C-term amidation

<400> 10
His Gly Glu Gly Thr Phe Thr Ser Asp Val Ser Ser Tyr Leu Glu Gly
1               5                   10                  15

Glu Ala Ala Lys Glu Phe Ile Ala Trp Leu Val Lys Xaa Arg
            20                  25                  30

<210> 11
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Aib

<220>

<223> c-term amidation

<400> 11
His Gly Glu Gly Thr Phe Thr Ser Asp Val Ser Ser Tyr Leu Glu Gly
1               5                   10                  15

Gln Xaa Ala Lys Glu Phe Ile Ala Trp Leu Val Lys Xaa Arg
            20                  25                  30

<210> 12
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Aib

<220>
<223> c-term amidation

<400> 12
His Gly Glu Gly Thr Phe Thr Ser Asp Val Ser Ser Tyr Leu Glu Gly
1               5                   10                  15

Gln Ala Ala Lys Glu Phe Ile Xaa Trp Leu Val Lys Xaa Arg
            20                  25                  30

<210> 13
<211> 30
<212> PRT
<213> Artificial Sequence

<220>
<223> Description of Artificial Sequence: Synthetic modified hGLP-1
      peptide

<220>
<221> MOD_RES
<222> (19)..(19)
<223> Aib

<220>
<221> MOD_RES
<222> (29)..(29)
<223> Aib

<220>
<223> c-term amidation

<400> 13
His Gly Glu Gly Thr Phe Thr Ser Asp Val Ser Ser Tyr Leu Glu Gly

1                   5                   10                  15

Gln Ala Xaa Lys Glu Phe Ile Ala Trp Leu Val Lys Xaa Arg
            20                  25                  30

<210> 14
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (14)..(14)
<223> A6c

<220>
<221> MOD_RES
<222> (29)..(29)
<223> Aib

<220>
<223> c-term amidation

<400> 14
His Gly Glu Gly Thr Phe Thr Ser Asp Xaa Ser Ser Tyr Xaa Glu Gly
1               5                   10                  15

Gln Ala Ala Lys Glu Phe Ile Ala Trp Leu Val Lys Xaa Arg
            20                  25                  30

<210> 15
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (23)..(23)
<223> A6c

<220>
<221> MOD_RES
<222> (26)..(26)

<223> A6c

<220>
<221> MOD_RES
<222> (29)..(29)
<223> Aib

<220>
<223> c-term amidation

<400> 15
His Gly Glu Gly Thr Phe Thr Ser Asp Xaa Ser Ser Tyr Leu Glu Gly
1               5                   10                  15

Gln Ala Ala Lys Glu Phe Xaa Ala Trp Xaa Val Lys Xaa Arg
            20                  25                  30

<210> 16
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (26)..(26)
<223> A6c

<220>
<221> MOD_RES
<222> (29)..(29)
<223> Aib

<220>
<223> c-term amidation

<400> 16
His Gly Glu Gly Thr Phe Thr Ser Asp Val Ser Ser Tyr Xaa Glu Gly
1               5                   10                  15

Gln Ala Ala Lys Glu Phe Ile Ala Trp Xaa Val Lys Xaa Arg
            20                  25                  30

<210> 17
<211> 30
<212> PRT
<213> Artificial Sequence

<220>
<223> Description of Artificial Sequence: Synthetic modified hGLP-1
      peptide

<220>
<221> MOD_RES

<222> (14)..(14)
<223> A6c

<220>
<221> MOD_RES
<222> (29)..(29)
<223> Aib

<220>
<223> c-term amidation

<400> 17
His Gly Glu Gly Thr Phe Thr Ser Asp Val Ser Ser Tyr Xaa Glu Gly
1               5                   10                  15

Gln Ala Ala Lys Glu Phe Ile Ala Trp Leu Val Lys Xaa Arg
            20                  25                  30

<210> 18
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 18
His Gly Glu Gly Thr Phe Thr Ser Asp Val Ser Ser Tyr Leu Glu Gly
1               5                   10                  15

Gln Ala Lys Lys Glu Phe Ile Ala Trp Leu Val Lys Xaa Arg
            20                  25                  30

<210> 19
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (18)..(18)
<223> Aib

<220>

<221> MOD_RES
<222> (29)..(29)
<223> Aib


<220>
<223> c-term amidation

<400> 19
His Gly Glu Gly Thr Phe Thr Ser Asp Val Ser Ser Tyr Xaa Glu Gly
1               5                   10                  15


Gln Xaa Ala Lys Glu Phe Ile Ala Trp Leu Val Lys Xaa Arg
            20                  25                  30


<210> 20
<211> 30
<212> PRT
<213> Artificial Sequence

<220>
<223> Description of Artificial Sequence: Synthetic modified hGLP-1
      peptide


<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Aib

<220>
<223> c-term amidation

<400> 20
His Gly Glu Gly Thr Phe Thr Ser Asp Val Ser Ser Tyr Leu Glu Gly
1               5                   10                  15


Gln Ala Ala Lys Glu Phe Xaa Ala Trp Xaa Val Lys Xaa Arg
            20                  25                  30


<210> 21
<211> 30
<212> PRT
<213> Artificial Sequence

<220>
<223> Description of Artificial Sequence: Synthetic modified hGLP-1
      peptide


<220>
<221> MOD_RES
<222> (18)..(18)
<223> Aib

<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Aib

<220>
<223> c-term amidation

<400> 21
His Gly Glu Gly Thr Phe Thr Ser Asp Val Ser Ser Tyr Leu Glu Gly
1               5                   10                  15

Gln Xaa Ala Lys Glu Phe Xaa Ala Trp Xaa Val Lys Xaa Arg
            20                  25                  30

<210> 22
<211> 30
<212> PRT
<213> Artificial Sequence

<220>
<223> Description of Artificial Sequence: Synthetic modified hGLP-1
      peptide

<220>
<221> MOD_RES
<222> (6)..(6)
<223> A6c

<220>
<221> MOD_RES
<222> (29)..(29)
<223> Aib

<220>
<223> c-term amidation

<400> 22
His Gly Glu Gly Thr Xaa Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 23
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> Cha

<220>
<221> MOD_RES
<222> (29)..(29)
<223> Aib

<220>
<223> c-term amidation

<400> 23
His Gly Glu Gly Thr Phe Thr Ser Asp Val Ser Ser Tyr Xaa Glu Gly
1               5                   10                  15

Gln Ala Ala Lys Glu Phe Ile Ala Trp Leu Val Lys Xaa Arg
            20                  25                  30

<210> 24
<211> 30
<212> PRT
<213> Artificial Sequence

<220>
<223> Description of Artificial Sequence: Synthetic modified hGLP-1
      peptide

<220>
<221> MOD_RES
<222> (27)..(27)
<223> A6c

<220>
<221> MOD_RES
<222> (29)..(29)
<223> Aib

<220>
<223> c-term amidation

<400> 24
His Gly Glu Gly Thr Phe Thr Ser Asp Val Ser Ser Tyr Leu Glu Gly
1               5                   10                  15

Gln Ala Ala Lys Glu Phe Ile Ala Trp Leu Xaa Lys Xaa Arg
            20                  25                  30

<210> 25
<211> 30
<212> PRT
<213> Artificial Sequence

<220>
<223> Description of Artificial Sequence: Synthetic modified hGLP-1
      peptide

<220>
<221> MOD_RES

<222> (10)..(10)
<223> A6c

<220>
<221> MOD_RES
<222> (14)..(14)
<223> A6c

<220>
<221> MOD_RES
<222> (29)..(29)
<223> Beta-Ala

<220>
<223> c-term amidation

<400> 25
His Gly Glu Gly Thr Phe Thr Ser Asp Xaa Ser Ser Tyr Xaa Glu Gly
1               5                   10                  15

Gln Ala Ala Lys Glu Phe Ile Ala Trp Leu Val Lys Xaa Arg
            20                  25                  30

<210> 26
<211> 30
<212> PRT
<213> Artificial Sequence

<220>
<223> Description of Artificial Sequence: Synthetic modified hGLP-1
      peptide

<220>
<221> MOD_RES
<222> (16)..(16)
<223> Beta-Ala

<220>
<221> MOD_RES
<222> (29)..(29)
<223> Aib

<220>
<223> c-term amidation

<400> 26
His Gly Glu Gly Thr Phe Thr Ser Asp Val Ser Ser Tyr Leu Glu Xaa
1               5                   10                  15

Gln Ala Ala Lys Glu Phe Ile Ala Trp Leu Val Lys Xaa Arg
            20                  25                  30

<210> 27
<211> 30
<212> PRT
<213> Artificial Sequence

<220>
<223> Description of Artificial Sequence: Synthetic modified hGLP-1
      peptide

<220>

<221> MOD_RES
<222> (16)..(16)
<223> Aib

<220>
<221> MOD_RES
<222> (29)..(29)
<223> Aib

<220>
<223> C-term amidation

<400> 27
His Gly Glu Gly Thr Phe Thr Ser Asp Val Ser Ser Tyr Leu Glu Xaa
1               5                   10                  15

Gln Ala Ala Lys Glu Phe Ile Ala Trp Leu Val Lys Xaa Arg
            20                  25                  30

<210> 28
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<223> C-term amidation

<400> 28
His Gly Glu Gly Thr Phe Thr Ser Asp Val Ser Ser Tyr Leu Glu Gly
1               5                   10                  15

Glu Ala Ala Lys Glu Phe Ile Ala Trp Xaa Val Lys Xaa Arg
            20                  25                  30

<210> 29
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Aib

<220>
<223> c-term amidation

<400> 29
His Gly Glu Gly Thr Phe Thr Ser Asp Val Ser Ser Tyr Leu Glu Gly
1               5                   10                  15

Glu Xaa Ala Lys Glu Phe Ile Ala Trp Xaa Val Lys Xaa Arg
            20                  25                  30

<210> 30
<211> 30
<212> PRT
<213> Artificial Sequence

<220>
<223> Description of Artificial Sequence: Synthetic modified hGLP-1
      peptide

<220>
<221> MOD_RES
<222> (18)..(19)
<223> Aib

<220>
<221> MOD_RES
<222> (26)..(26)
<223> Ac6

<220>
<221> MOD_RES
<222> (29)..(29)
<223> Aib

<220>
<223> c-term amidation

<400> 30
His Gly Glu Gly Thr Phe Thr Ser Asp Val Ser Ser Tyr Leu Glu Gly
1               5                   10                  15

Glu Xaa Xaa Lys Glu Phe Ile Ala Trp Xaa Val Lys Xaa Arg
            20                  25                  30

<210> 31
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (14)..(14)
<223> A6c

<220>
<221> MOD_RES
<222> (18)..(19)
<223> Aib

<220>
<221> MOD_RES
<222> (26)..(26)
<223> A6c

<220>
<221> MOD_RES
<222> (29)..(29)
<223> Aib

<220>
<223> c-term amidation

<400> 31
His Gly Glu Gly Thr Phe Thr Ser Asp Xaa Ser Ser Tyr Xaa Glu Gly
1               5                   10                  15

Glu Xaa Xaa Lys Glu Phe Ile Ala Trp Xaa Val Lys Xaa Arg
            20                  25                  30

<210> 32
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Beta-Ala

<220>
<223> c-term amidation

<400> 32
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Lys Xaa Arg

20                   25                   30

<210> 33
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A5c

<220>
<221> MOD_RES
<222> (29)..(29)
<223> Beta-Ala

<220>
<223> c-term amidation

<400> 33
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Lys Xaa Arg
            20                  25                  30

<210> 34
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 34
His Gly Glu Gly Thr Phe Thr Ser Asp Val Ser Ser Tyr Leu Glu Gly
1               5                   10                  15

Glu Ala Ala Lys Glu Phe Ile Ala Trp Leu Val Lys Xaa Arg
            20                  25                  30

<210> 35
<211> 30
<212> PRT
<213> Artificial Sequence

<220>

<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Beta-Ala

<220>
<223> c-term amidation

<400> 35
His Gly Glu Gly Thr Phe Thr Ser Asp Val Ser Ser Tyr Leu Glu Gly
1               5                   10                  15

Gln Xaa Ala Lys Glu Phe Ile Ala Trp Leu Val Lys Xaa Arg
            20                  25                  30

<210> 36
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Beta-Ala

<220>
<223> c-term amidation

<400> 36
His Gly Glu Gly Thr Phe Thr Ser Asp Val Ser Ser Tyr Leu Glu Gly
1               5                   10                  15

Gln Ala Ala Lys Glu Phe Ile Xaa Trp Leu Val Lys Xaa Arg
            20                  25                  30

<210> 37
<211> 30
<212> PRT
<213> Artificial Sequence

<220>
<223> Description of Artificial Sequence: Synthetic modified hGLP-1
      peptide

<220>
<221> MOD_RES
<222> (19)..(19)
<223> Aib

<220>
<221> MOD_RES
<222> (29)..(29)
<223> Beta-Ala

<220>
<223> c-term amidation

<400> 37
His Gly Glu Gly Thr Phe Thr Ser Asp Val Ser Ser Tyr Leu Glu Gly
1               5                   10                  15

Gln Ala Xaa Lys Glu Phe Ile Ala Trp Leu Val Lys Xaa Arg
            20                  25                  30

<210> 38
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Beta-Ala

<220>
<223> c-term amidation

<400> 38
His Gly Glu Gly Thr Phe Thr Ser Asp Xaa Ser Ser Tyr Leu Glu Gly
1               5                   10                  15

Gln Ala Ala Lys Glu Phe Xaa Ala Trp Xaa Val Lys Xaa Arg
            20                  25                  30

<210> 39
<211> 30

<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (26)..(26)
<223> A6c

<220>
<221> MOD_RES
<222> (29)..(29)
<223> Beta-Ala

<220>
<223> c-term amidation

<400> 39
His Gly Glu Gly Thr Phe Thr Ser Asp Val Ser Ser Tyr Xaa Glu Gly
1               5                   10                  15

Gln Ala Ala Lys Glu Phe Ile Ala Trp Xaa Val Lys Xaa Arg
            20                  25                  30

<210> 40
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (29)..(29)
<223> Beta-Ala

<220>
<223> c-term amidation

<400> 40
His Gly Glu Gly Thr Phe Thr Ser Asp Val Ser Ser Tyr Xaa Glu Gly
1               5                   10                  15

Gln Ala Ala Lys Glu Phe Ile Ala Trp Leu Val Lys Xaa Arg
            20                  25                  30

<210> 41
<211> 30

<212> PRT
<213> Artificial Sequence

<220>
<223> Description of Artificial Sequence: Synthetic modified hGLP-1
      peptide


<220>
<221> MOD_RES
<222> (29)..(29)
<223> B-Ala

<220>
<223> c-term amidation

<400> 41
His Gly Glu Gly Thr Phe Thr Ser Asp Val Ser Ser Tyr Leu Glu Gly
1               5                   10                  15


Gln Ala Lys Lys Glu Phe Ile Ala Trp Leu Val Lys Xaa Arg
            20                  25                  30




<210> 42
<211> 30
<212> PRT
<213> Artificial Sequence

<220>
<223> Description of Artificial Sequence: Synthetic modified hGLP-1
      peptide


<220>
<221> MOD_RES
<222> (14)..(14)
<223> A6c

<220>
<221> MOD_RES
<222> (18)..(18)
<223> Aib


<220>
<221> MOD_RES
<222> (29)..(29)
<223> Beta-Ala

<220>
<223> c-term amidation

<400> 42
His Gly Glu Gly Thr Phe Thr Ser Asp Val Ser Ser Tyr Xaa Glu Gly
1               5                   10                  15


Gln Xaa Ala Lys Glu Phe Ile Ala Trp Leu Val Lys Xaa Arg
            20                  25                  30


<210> 43
<211> 30
<212> PRT
<213> Artificial Sequence

<220>
<223> Description of Artificial Sequence: Synthetic modified hGLP-1
      peptide

<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Beta-Ala

<220>
<223> c-term amidation

<400> 43
His Gly Glu Gly Thr Phe Thr Ser Asp Val Ser Ser Tyr Leu Glu Gly
1               5                   10                  15

Gln Ala Ala Lys Glu Phe Xaa Ala Trp Xaa Val Lys Xaa Arg
            20                  25                  30

<210> 44
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Beta-Ala

<220>
<223> c-term amidation

<400> 44

His Gly Glu Gly Thr Phe Thr Ser Asp Val Ser Ser Tyr Leu Glu Gly
1               5                   10                  15

Gln Xaa Ala Lys Glu Phe Xaa Ala Trp Xaa Val Lys Xaa Arg
            20                  25                  30

<210> 45
<211> 30
<212> PRT
<213> Artificial Sequence

<220>
<223> Description of Artificial Sequence: Synthetic modified hGLP-1
      peptide

<220>
<221> MOD_RES
<222> (6)..(6)
<223> A6c

<220>
<221> MOD_RES
<222> (29)..(29)
<223> Beta-Ala

<220>
<223> c-term amidation

<400> 45
His Gly Glu Gly Thr Xaa Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 46
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> Cha

<220>
<221> MOD_RES
<222> (29)..(29)
<223> Beta-Ala

<220>
<223> c-term amidation

<400> 46
His Gly Glu Gly Thr Phe Thr Ser Asp Val Ser Ser Tyr Xaa Glu Gly
1               5                   10                  15

Gln Ala Ala Lys Glu Phe Ile Ala Trp Leu Val Lys Xaa Arg
            20                  25                  30

<210> 47
<211> 30
<212> PRT
<213> Artificial Sequence

<220>
<223> Description of Artificial Sequence: Synthetic modified hGLP-1
      peptide

<220>
<221> MOD_RES
<222> (27)..(27)
<223> A6c

<220>
<221> MOD_RES
<222> (29)..(29)
<223> Beta-Ala

<220>
<223> c-term amidation

<400> 47
His Gly Glu Gly Thr Phe Thr Ser Asp Val Ser Ser Tyr Leu Glu Gly
1               5                   10                  15

Gln Ala Ala Lys Glu Phe Ile Ala Trp Leu Xaa Lys Xaa Arg
            20                  25                  30

<210> 48
<211> 30
<212> PRT
<213> Artificial Sequence

<220>
<223> Description of Artificial Sequence: Synthetic modified hGLP-1
      peptide

<220>
<221> MOD_RES
<222> (16)..(16)
<223> Beta-Ala

<220>
<221> MOD_RES
<222> (29)..(29)
<223> Beta-Ala

<220>
<223> c-term amidation

<400> 48
His Gly Glu Gly Thr Phe Thr Ser Asp Val Ser Ser Tyr Leu Glu Xaa
1               5                   10                  15

Gln Ala Ala Lys Glu Phe Ile Ala Trp Leu Val Lys Xaa Arg
            20                  25                  30

<210> 49
<211> 30
<212> PRT
<213> Artificial Sequence

<220>
<223> Description of Artificial Sequence: Synthetic modified hGLP-1
      peptide


<220>
<221> MOD_RES
<222> (16)..(16)
<223> Aib

<220>
<221> MOD_RES
<222> (29)..(29)
<223> Beta-Ala

<220>
<223> c-term amidation

<400> 49
His Gly Glu Gly Thr Phe Thr Ser Asp Val Ser Ser Tyr Leu Glu Xaa
1               5                   10                  15


Gln Ala Ala Lys Glu Phe Ile Ala Trp Leu Val Lys Xaa Arg
            20                  25                  30


<210> 50
<211> 30
<212> PRT
<213> Artificial Sequence

<220>
<223> Description of Artificial Sequence: Synthetic modified hGLP-1
      peptide


<220>
<221> MOD_RES
<222> (26)..(26)
<223> A6c

<220>
<221> MOD_RES
<222> (29)..(29)
<223> Beta-Ala

<220>
<223> c-term amidation

<400> 50
His Gly Glu Gly Thr Phe Thr Ser Asp Val Ser Ser Tyr Leu Glu Gly
1               5                   10                  15


Glu Ala Ala Lys Glu Phe Ile Ala Trp Xaa Val Lys Xaa Arg
            20                  25                  30


<210> 51
<211> 30
<212> PRT

<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Beta-Ala

<220>
<223> c-term amidation

<400> 51
His Gly Glu Gly Thr Phe Thr Ser Asp Val Ser Ser Tyr Leu Glu Gly
1               5                   10                  15

Glu Xaa Ala Lys Glu Phe Ile Ala Trp Xaa Val Lys Xaa Arg
            20                  25                  30

<210> 52
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 52

His Gly Glu Gly Thr Phe Thr Ser Asp Val Ser Ser Tyr Leu Glu Gly
1               5                   10                  15

Glu Xaa Ala Lys Glu Phe Ile Ala Trp Xaa Val Xaa Xaa Arg
            20                  25                  30

<210> 53
<211> 30
<212> PRT
<213> Artificial Sequence

<220>
<223> Description of Artificial Sequence: Synthetic modified hGLP-1
      peptide

<220>
<221> MOD_RES
<222> (18)..(19)
<223> Aib

<220>
<221> MOD_RES
<222> (26)..(26)
<223> A6c

<220>
<221> MOD_RES
<222> (29)..(29)
<223> Beta-Ala

<220>
<223> c-term amidation

<400> 53
His Gly Glu Gly Thr Phe Thr Ser Asp Val Ser Ser Tyr Leu Glu Gly
1               5                   10                  15

Glu Xaa Xaa Lys Glu Phe Ile Ala Trp Xaa Val Lys Xaa Arg
            20                  25                  30

<210> 54
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (14)..(14)
<223> A6c

<220>
<221> MOD_RES
<222> (18)..(19)

<223> Aib

<220>
<221> MOD_RES
<222> (26)..(26)
<223> A6c

<220>
<221> MOD_RES
<222> (29)..(29)
<223> Beta-Ala

<220>
<223> c-term amidation

<400> 54
His Gly Glu Gly Thr Phe Thr Ser Asp Xaa Ser Ser Tyr Xaa Glu Gly
1               5                   10                  15

Glu Xaa Xaa Lys Glu Phe Ile Ala Trp Xaa Val Lys Xaa Arg
            20                  25                  30

<210> 55
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> D-Arg

<220>
<223> c-term amidation

<400> 55
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 56
<211> 30
<212> PRT
<213> Artificial Sequence

<220>
<223> Description of Artificial Sequence: Synthetic modified hGLP-1
      peptide

<220>
<221> MOD_RES

<222> (29)..(29)
<223> Aib

<220>
<221> MOD_RES
<222> (30)..(30)
<223> D-Lys


<220>
<223> c-term amidation

<400> 56
His Gly Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Xaa
            20                  25                  30


<210> 57
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> D-Arg

<220>
<223> c-term amidation

<400> 57
His Gly Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Xaa
            20                  25                  30


<210> 58
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>

<221> MOD_RES
<222> (30)..(30)
<223> D-Lys

<220>
<223> c-term amidation

<400> 58
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 59
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 59
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 60
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 60
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 61
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<223> c-term amidation

<400> 61
His Gly Glu Gly Thr Phe Thr Ser Asp Val Ser Ser Tyr Leu Glu Gly
1               5                   10                  15


Gln Ala Arg Arg Glu Phe Ile Ala Trp Leu Val Arg Xaa Arg
            20                  25                  30


<210> 62
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 62
His Gly Glu Gly Thr Phe Thr Ser Asp Val Ser Ser Tyr Leu Glu Gly
1               5                   10                  15


Gln Ala Arg Arg Glu Phe Ile Ala Trp Leu Val Arg Xaa Arg
            20                  25                  30


<210> 63
<211> 30
<212> PRT
<213> Artificial Sequence

<220>
<223> Description of Artificial Sequence: Synthetic modified hGLP-1
      peptide

<220>
<221> MOD_RES
<222> (29)..(29)

<223> Beta-Ala

<220>
<221> MOD_RES
<222> (30)..(30)
<223> Lys(N-tetradecanoyl)

<400> 63
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 64
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<400> 64
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa Gly
            20                  25                  30

<210> 65
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<221> MOD_RES
<222> (31)..(31)

<223> Aib

<400> 65
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa Xaa
            20                  25                  30

<210> 66
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<221> MOD_RES
<222> (31)..(31)
<223> D-Ala

<400> 66
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa Xaa
            20                  25                  30

<210> 67
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES

<222> (32)..(32)
<223> Lys(N-tetradecanoyl)

<400> 67
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 68
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Beta-Ala

<220>
<221> MOD_RES
<222> (32)..(32)
<223> Lys(N-tetradecanoyl)

<400> 68
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 69
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-tetradecanoyl)

<400> 69
His Gly Glu Gly Thr Phe Thr Ser Asp Val Ser Ser Tyr Leu Glu Gly

1                   5                   10                  15

Gln Ala Ala Arg Glu Phe Ile Ala Trp Leu Val Arg Xaa Arg Gly Xaa
            20                  25                  30

<210> 70
<211> 31
<212> PRT
<213> Artificial Sequence

<220>
<223> Description of Artificial Sequence: Synthetic modified hGLP-1
      peptide

<220>
<221> MOD_RES
<222> (30)..(30)
<223> Lys(N-tetradecanoyl)

<220>
<221> MOD_RES
<222> (31)..(31)
<223> Beta-Ala

<400> 70
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Xaa Xaa
            20                  25                  30

<210> 71
<211> 31
<212> PRT
<213> Artificial Sequence

<220>
<223> Description of Artificial Sequence: Synthetic modified hGLP-1
      peptide

<220>
<221> MOD_RES
<222> (30)..(30)
<223> Lys(N-tetradecanoyl)

<220>
<221> MOD_RES
<222> (31)..(31)
<223> Aib

<400> 71
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Xaa Xaa
            20                  25                  30

<210> 72
<211> 31

<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<221> MOD_RES
<222> (31)..(31)
<223> Ado

<400> 72
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Arg Xaa
            20                  25                  30


<210> 73
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Ado

<400> 73
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Arg Xaa
            20                  25                  30


<210> 74
<211> 31
<212> PRT
<213> Artificial Sequence

<220>
<223> Description of Artificial Sequence: Synthetic modified hGLP-1
      peptide


<220>
<221> MOD_RES
<222> (30)..(30)

<223> Lys(N-tetradecanoyl)

<220>
<221> MOD_RES
<222> (31)..(31)
<223> D-Ala

<400> 74
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Xaa Xaa
            20                  25                  30

<210> 75
<211> 32
<212> PRT
<213> Artificial Sequence

<220>
<223> Description of Artificial Sequence: Synthetic modified hGLP-1
      peptide

<220>
<221> MOD_RES
<222> (31)..(31)
<223> Aib

<220>
<221> MOD_RES
<222> (32)..(32)
<223> Lys(N-tetradecanoyl)

<400> 75
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Arg Xaa Xaa
            20                  25                  30

<210> 76
<211> 32
<212> PRT
<213> Artificial Sequence

<220>
<223> Description of Artificial Sequence: Synthetic modified hGLP-1
      peptide

<220>
<221> MOD_RES
<222> (31)..(31)
<223> Beta-Ala

<220>
<221> MOD_RES
<222> (32)..(32)
<223> Lys(N-tetradecanoyl)

<400> 76
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Arg Xaa Xaa
            20                  25                  30

<210> 77
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> C-term amidation

<400> 77
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Xaa Arg
            20                  25                  30

<210> 78
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)
<223> Aib

<220>
<223> C-term amidation

<400> 78
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Xaa Arg

20                  25                  30

<210> 79
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib

<220>
<223> c-term amidation

<400> 79
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Xaa Arg
            20                  25                  30

<210> 80
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Beta-Ala

<220>
<223> c-term amidation

<400> 80
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Xaa Arg
            20                  25                  30

<210> 81

<211> 30
<212> PRT
<213> Artificial Sequence

<220>
<223> Description of Artificial Sequence: Synthetic modified hGLP-1
      peptide


<220>
<221> MOD_RES
<222> (20)..(20)
<223> Lys(N-tetradecanoyl)

<220>
<221> MOD_RES
<222> (29)..(29)
<223> Beta-Ala

<220>
<223> C-term amidation

<400> 81
His Gly Glu Gly Thr Phe Thr Ser Asp Val Ser Ser Tyr Leu Glu Gly
1               5                   10                  15


Gln Ala Ala Xaa Glu Phe Ile Ala Trp Leu Val Lys Xaa Arg
            20                  25                  30


<210> 82
<211> 30
<212> PRT
<213> Artificial Sequence

<220>
<223> Description of Artificial Sequence: Synthetic modified hGLP-1
      peptide


<220>
<221> MOD_RES
<222> (20)..(20)
<223> Lys(N-hexadecanoyl)

<220>
<221> MOD_RES
<222> (29)..(29)
<223> Beta-Ala

<220>
<223> C-term amidation

<400> 82
His Gly Glu Gly Thr Phe Thr Ser Asp Val Ser Ser Tyr Leu Glu Gly
1               5                   10                  15


Gln Ala Ala Xaa Glu Phe Ile Ala Trp Leu Val Lys Xaa Arg
            20                  25                  30


<210> 83
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 83
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 84
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)
<223> Aib

<220>
<223> c-term amidation

<400> 84
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 85
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib

<220>
<223> c-term amidation

<400> 85
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 86
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-decanoyl)

<220>
<221> MOD_RES
<222> (29)..(29)
<223> Aib

<220>
<223> c-term amidation

<400> 86
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 87
<211> 30
<212> PRT
<213> Artificial Sequence

<220>
<223> Description of Artificial Sequence: Synthetic modified hGLP-1
      peptide

<220>

<221> MOD_RES
<222> (20)..(20)
<223> Lys(N-octanoyl)

<220>
<221> MOD_RES
<222> (29)..(29)
<223> Aib

<220>
<223> c-term amidation

<400> 87
His Gly Glu Gly Thr Phe Thr Ser Asp Val Ser Ser Tyr Leu Glu Gly
1               5                   10                  15

Gln Ala Lys Xaa Glu Phe Ile Ala Trp Leu Val Arg Xaa Arg
            20                  25                  30

<210> 88
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)
<223> Aib

<220>
<223> c-term amidation

<400> 88
His Gly Glu Gly Thr Phe Thr Ser Asp Val Ser Ser Tyr Leu Glu Gly
1               5                   10                  15

Gln Ala Lys Xaa Glu Phe Ile Ala Trp Leu Val Arg Xaa Arg
            20                  25                  30

<210> 89
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib

<220>
<223> c-term amidation

<400> 89
His Gly Glu Gly Thr Phe Thr Ser Asp Val Ser Ser Tyr Leu Glu Gly
1               5                   10                  15

Gln Ala Lys Xaa Glu Phe Ile Ala Trp Leu Val Arg Xaa Arg
            20                  25                  30

<210> 90
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 90
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 91
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)

<223> Aib

<220>
<223> c-term amidation

<400> 91
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 92
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib

<220>
<223> c-term amidation

<400> 92
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 93
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-decanoyl)

<220>
<221> MOD_RES
<222> (29)..(29)
<223> Aib

<220>
<223> c-term amidation

<400> 93
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 94
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Beta-Ala

<220>
<223> C-term amidation

<400> 94
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 95
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)
<223> Beta-Ala

<220>
<223> C-term amidation

<400> 95
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 96
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Beta-Ala

<220>
<223> c-term amidation

<400> 96
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 97
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-decanoyl)

<220>
<221> MOD_RES
<222> (29)..(29)
<223> Beta-Ala

<220>
<223> c-term amidation

<400> 97
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 98
<211> 30
<212> PRT
<213> Artificial Sequence

<220>
<223> Description of Artificial Sequence: Synthetic modified hGLP-1
      peptide


<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 98
His Gly Glu Gly Thr Phe Thr Ser Asp Val Ser Ser Tyr Leu Glu Gly
1               5                   10                  15


Gln Ala Ala Lys Glu Phe Ile Ala Trp Leu Val Xaa Xaa Arg
            20                  25                  30


<210> 99
<211> 30
<212> PRT
<213> Artificial Sequence

<220>
<223> Description of Artificial Sequence: Synthetic modified hGLP-1
      peptide


<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 99
His Gly Glu Gly Thr Phe Thr Ser Asp Val Ser Ser Tyr Leu Glu Gly
1               5                   10                  15


Gln Ala Ala Lys Glu Phe Ile Ala Trp Leu Val Xaa Xaa Arg
            20                  25                  30



<210> 100

<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 100
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Xaa Xaa Arg
            20                  25                  30

<210> 101
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 101
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 102
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 102
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 103
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-decanoyl)

<220>
<221> MOD_RES
<222> (29)..(29)
<223> Aib

<220>
<223> c-term amidation

<400> 103
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 104
<211> 30
<212> PRT
<213> Artificial Sequence

<220>
<223> Description of Artificial Sequence: Synthetic modified hGLP-1

peptide

<220>
<221> MOD_RES
<222> (28)..(28)
<223> Lys(N-octanoyl)


<220>
<221> MOD_RES
<222> (29)..(29)
<223> Aib


<220>
<223> c-term amidation

<400> 104
His Gly Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 105
<211> 30
<212> PRT
<213> Artificial Sequence

<220>
<223> Description of Artificial Sequence: Synthetic modified hGLP-1
      peptide


<220>
<221> MOD_RES
<222> (28)..(28)
<223> Lys(N-tetradecanoyl)


<220>
<221> MOD_RES
<222> (29)..(29)
<223> Aib


<220>
<223> c-term amidation

<400> 105
His Gly Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 106
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)


<220>
<221> MOD_RES
<222> (29)..(29)
<223> Aib


<220>
<223> c-term amidation

<400> 106
His Gly Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 107
<211> 30
<212> PRT
<213> Artificial Sequence

<220>
<223> Description of Artificial Sequence: Synthetic modified hGLP-1
      peptide


<220>
<221> MOD_RES
<222> (28)..(28)
<223> Lys(N-decanoyl)


<220>
<221> MOD_RES
<222> (29)..(29)
<223> Aib


<220>
<223> c-term amidation

<400> 107
His Gly Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 108
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)


<220>
<221> MOD_RES
<222> (29)..(29)
<223> Aib


<220>
<223> c-term amidation

<400> 108
His Gly Glu Gly Thr Phe Thr Ser Asp Val Ser Ser Tyr Leu Glu Gly
1               5                   10                  15


Gln Ala Lys Arg Glu Phe Ile Ala Trp Leu Val Xaa Xaa Arg
            20                  25                  30


<210> 109
<211> 30
<212> PRT
<213> Artificial Sequence

<220>
<223> Description of Artificial Sequence: Synthetic modified hGLP-1
      peptide


<220>
<221> MOD_RES
<222> (28)..(28)
<223> Lys(N-tetradecanoyl)


<220>
<221> MOD_RES
<222> (29)..(29)
<223> Aib


<220>
<223> c-term amidation

<400> 109
His Gly Glu Gly Thr Phe Thr Ser Asp Val Ser Ser Tyr Leu Glu Gly
1               5                   10                  15


Gln Ala Lys Arg Glu Phe Ile Ala Trp Leu Val Xaa Xaa Arg
            20                  25                  30


<210> 110
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 110
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 111
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 111
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 112
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 112
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 113
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 113
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 114
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib


<220>
<221> MOD_RES
<222> (30)..(30)
<223> Lys(N-octanoyl)


<220>
<223> c-term amidation

<400> 114
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Lys Xaa Xaa
            20                  25                  30


<210> 115
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib


<220>
<221> MOD_RES
<222> (30)..(30)
<223> Lys(N-tetradecanoyl)


<220>
<223> c-term amidation

<400> 115
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Lys Xaa Xaa
            20                  25                  30


<210> 116
<211> 30
<212> PRT
<213> Artificial Sequence

<220>
<223> Description of Artificial Sequence: Synthetic modified hGLP-1
      peptide


<220>

<221> MOD_RES
<222> (29)..(29)
<223> Aib


<220>
<221> MOD_RES
<222> (30)..(30)
<223> Lys(N-hexadecanoyl)


<220>
<223> c-term amidation

<400> 116
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Lys Xaa Xaa
            20                  25                  30


<210> 117
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib


<220>
<221> MOD_RES
<222> (30)..(30)
<223> Lys(N-octanoyl)


<220>
<223> c-term amidation

<400> 117
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Xaa
            20                  25                  30


<210> 118
<211> 30
<212> PRT
<213> Artificial Sequence

<220>
<223> Description of Artificial Sequence: Synthetic modified hGLP-1
      peptide


<220>

<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 118
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 119
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-octanoyl)

<220>
<223> c-term amidation

<400> 119
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 120
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-decanoyl)

<220>
<223> c-term amidation

<400> 120
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 121
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-tetradecanoyl)

<220>
<223> c-term amidation

<400> 121
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 122
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)

<223> Lys(N-hexadecanoyl)

<220>
<223> c-term amidation

<400> 122
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 123
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-octanoyl)

<220>
<223> c-term amidation

<400> 123
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 124
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-decanoyl)

<220>
<223> c-term amidation

<400> 124
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 125
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-tetradecanoyl)

<220>
<223> c-term amidation

<400> 125
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 126
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-hexadecanoyl)

<220>
<223> c-term amidation

<400> 126
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 127
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-octanoyl)

<220>
<223> c-term amidation

<400> 127
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 128
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-decanoyl)

<220>
<223> c-term amidation

<400> 128
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 129
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>

<221> MOD_RES
<222> (32)..(32)
<223> Lys(N-hexadecanoyl)

<220>
<223> c-term amidation

<400> 129
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 130
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 130
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 131
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)

<223> Lys(N-tetradecanoyl)

<220>
<223> c-term amidation

<400> 131
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 132
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 132
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 133
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 133
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 134
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 134
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 135
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>

<223> c-term amidation

<400> 135
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 136
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-decanoyl)

<220>
<223> c-term amidation

<400> 136
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 137
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 137
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Xaa Xaa Arg
            20                  25                  30

<210> 138
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Beta-Ala

<220>
<223> c-term amidation

<400> 138
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Xaa Xaa Arg
            20                  25                  30

<210> 139
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Beta-Ala

<220>
<223> c-term amidation

<400> 139
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Xaa Xaa Arg
            20                  25                  30

<210> 140
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 140
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 141
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 141
His Gly Glu Gly Thr Phe Thr Ser Asp Val Ser Ser Tyr Leu Glu Gly
1               5                   10                  15

Glu Ala Ala Lys Glu Phe Ile Ala Trp Leu Val Xaa Xaa Arg
            20                  25                  30

<210> 142
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 142
His Gly Glu Gly Thr Phe Thr Ser Asp Val Ser Ser Tyr Leu Glu Gly
1               5                   10                  15

Glu Ala Ala Lys Glu Phe Ile Ala Trp Xaa Val Xaa Xaa Arg
            20                  25                  30

<210> 143
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>

<221> MOD_RES
<222> (29)..(29)
<223> Beta-Ala

<220>
<223> C-term amidation

<400> 143
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 144
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Beta-Ala

<220>
<223> C-term amidation

<400> 144
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 145
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Beta-Ala

<220>
<223> c-term amidation

<400> 145
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 146

<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-decanoyl)

<220>
<221> MOD_RES
<222> (29)..(29)
<223> Beta-Ala

<220>
<223> c-term amidation

<400> 146
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 147
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)

<223> Beta-Ala

<220>
<223> c-term amidation

<400> 147
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 148
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Beta-Ala

<220>
<223> c-term amidation

<400> 148
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 149
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Beta-Ala

<220>

<223> c-term amidation

<400> 149
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 150
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-decanoyl)

<220>
<221> MOD_RES
<222> (29)..(29)
<223> Beta-Ala

<220>
<223> c-term amidation

<400> 150
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 151
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 151

His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 152
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Beta-Ala

<220>
<223> c-term amidation

<400> 152
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 153
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Beta-Ala

<220>
<223> c-term amidation

<400> 153
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 154
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 154
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 155
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> c-term amidation

<400> 155
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 156
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-hexadecanoyl)

<220>
<223> c-term amidation

<400> 156
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 157
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 157
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 158
<211> 30
<212> PRT
<213> Artificial Sequence

<220>

<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> c-term amidation

<400> 158
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 159
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-hexadecanoyl)

<220>
<223> c-term amidation

<400> 159
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 160
<211> 30
<212> PRT
<213> Artificial Sequence

<220>
<223> Description of Artificial Sequence: Synthetic modified hGLP-1
      peptide

<220>

<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 160
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 161
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> c-term amidation

<400> 161
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 162
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES

<222> (30)..(30)
<223> Lys(N-hexadecanoyl)

<220>
<223> c-term amidation

<400> 162
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 163
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-decanoyl)

<220>
<223> c-term amidation

<400> 163
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 164
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 164
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 165
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> c-term amidation

<400> 165
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 166
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-hexadecanoyl)

<220>
<223> c-term amidation

<400> 166
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 167
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 167
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 168
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> c-term amidation

<400> 168
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa

20                  25                  30

<210> 169
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-hexadecanoyl)

<220>
<223> c-term amidation

<400> 169
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 170
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-decanoyl)

<220>
<223> c-term amidation

<400> 170
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 171

<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (26)..(26)
<223> A6c

<220>
<221> MOD_RES
<222> (29)..(29)
<223> Aib

<220>
<223> c-term amidation

<400> 171
His Gly Glu Gly Thr Phe Thr Ser Asp Val Ser Ser Tyr Leu Glu Gly
1               5                   10                  15

Gln Ala Ala Xaa Glu Phe Ile Ala Trp Xaa Val Arg Xaa Arg
            20                  25                  30

<210> 172
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (26)..(26)
<223> A6c

<220>
<221> MOD_RES
<222> (29)..(29)
<223> Aib

<220>
<223> c-term amidation

<400> 172
His Gly Glu Gly Thr Phe Thr Ser Asp Val Ser Ser Tyr Leu Glu Gly
1               5                   10                  15

Gln Ala Ala Xaa Glu Phe Ile Ala Trp Xaa Val Arg Xaa Arg
            20                  25                  30

<210> 173
<211> 30
<212> PRT

<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (26)..(26)
<223> A6c

<220>
<221> MOD_RES
<222> (29)..(29)
<223> Aib

<220>
<223> c-term amidation

<400> 173
His Gly Glu Gly Thr Phe Thr Ser Asp Val Ser Ser Tyr Leu Glu Gly
1               5                   10                  15

Gln Ala Ala Xaa Glu Phe Ile Ala Trp Xaa Val Arg Xaa Arg
            20                  25                  30

<210> 174
<211> 30
<212> PRT
<213> Artificial Sequence

<220>
<223> Description of Artificial Sequence: Synthetic modified hGLP-1

      peptide

<220>
<221> MOD_RES
<222> (26)..(26)
<223> A6c

<220>
<221> MOD_RES
<222> (28)..(28)
<223> Lys(N-octanoyl)

<220>
<221> MOD_RES

<222> (29)..(29)
<223> Aib

<220>
<223> c-term amidation

<400> 174
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 175
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 175
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 176
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 176
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 177
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 177
His Gly Glu Gly Thr Phe Thr Ser Asp Val Ser Ser Tyr Leu Glu Gly
1               5                   10                  15

Gln Ala Ala Arg Glu Phe Ile Ala Trp Xaa Val Xaa Xaa Arg
            20                  25                  30

<210> 178
<211> 30
<212> PRT
<213> Artificial Sequence

<220>

<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 178
His Gly Glu Gly Thr Phe Thr Ser Asp Val Ser Ser Tyr Leu Glu Gly
1               5                   10                  15

Gln Ala Ala Arg Glu Phe Ile Ala Trp Xaa Val Xaa Xaa Arg
            20                  25                  30

<210> 179
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 179
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Lys Xaa Xaa
            20                  25                  30

<210> 180
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 180
His Gly Glu Gly Thr Phe Thr Ser Asp Val Ser Ser Tyr Leu Glu Gly
1               5                   10                  15


Gln Ala Ala Lys Glu Phe Ile Ala Trp Xaa Val Lys Xaa Xaa
            20                  25                  30


<210> 181
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 181
His Gly Glu Gly Thr Phe Thr Ser Asp Val Ser Ser Tyr Leu Glu Gly
1               5                   10                  15

Gln Ala Ala Lys Glu Phe Ile Ala Trp Xaa Val Lys Xaa Xaa
            20                  25                  30


<210> 182
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 182
His Gly Glu Gly Thr Phe Thr Ser Asp Val Ser Ser Tyr Leu Glu Gly
1               5                   10                  15


Gln Ala Ala Arg Glu Phe Ile Ala Trp Xaa Val Lys Xaa Xaa
            20                  25                  30


<210> 183
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 183

His Gly Glu Gly Thr Phe Thr Ser Asp Val Ser Ser Tyr Leu Glu Gly
1               5                   10                  15

Gln Ala Ala Arg Glu Phe Ile Ala Trp Xaa Val Lys Xaa Xaa
            20                  25                  30

<210> 184
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 184
His Gly Glu Gly Thr Phe Thr Ser Asp Val Ser Ser Tyr Leu Glu Gly
1               5                   10                  15

Gln Ala Ala Arg Glu Phe Ile Ala Trp Xaa Val Lys Xaa Xaa
            20                  25                  30

<210> 185
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 185
His Gly Glu Gly Thr Phe Thr Ser Asp Val Ser Ser Tyr Leu Glu Gly
1               5                   10                  15

Gln Ala Ala Arg Glu Phe Ile Ala Trp Xaa Val Arg Xaa Xaa
            20                  25                  30

<210> 186
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-decanoyl)

<220>
<223> c-term amidation

<400> 186
His Gly Glu Gly Thr Phe Thr Ser Asp Val Ser Ser Tyr Leu Glu Gly
1               5                   10                  15

Gln Ala Ala Arg Glu Phe Ile Ala Trp Xaa Val Arg Xaa Xaa
            20                  25                  30

<210> 187
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>

<221> MOD_RES
<222> (30)..(30)
<223> Lys(N-tetradecanoyl)

<220>
<223> c-term amidation

<400> 187
His Gly Glu Gly Thr Phe Thr Ser Asp Val Ser Ser Tyr Leu Glu Gly
1               5                   10                  15

Gln Ala Ala Arg Glu Phe Ile Ala Trp Xaa Val Arg Xaa Xaa
            20                  25                  30

<210> 188
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 188
His Gly Glu Gly Thr Phe Thr Ser Asp Val Ser Ser Tyr Leu Glu Gly
1               5                   10                  15

Gln Ala Ala Arg Glu Phe Ile Ala Trp Xaa Val Arg Xaa Xaa
            20                  25                  30

<210> 189
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>

<221> MOD_RES
<222> (20)..(20)
<223> Lys(N-octanoyl)

<220>
<221> MOD_RES
<222> (29)..(29)
<223> Aib

<220>
<223> c-term amidation

<400> 189
His Gly Glu Gly Thr Phe Thr Ser Asp Val Ser Ser Tyr Leu Glu Gly
1               5                   10                  15

Gln Xaa Ala Xaa Glu Phe Ile Ala Trp Leu Val Arg Xaa Arg
            20                  25                  30

<210> 190
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (20)..(20)
<223> Lys(N-tetradecanoyl)

<220>
<221> MOD_RES
<222> (29)..(29)
<223> Aib

<220>
<223> c-term amidation

<400> 190
His Gly Glu Gly Thr Phe Thr Ser Asp Val Ser Ser Tyr Leu Glu Gly
1               5                   10                  15

Gln Xaa Ala Xaa Glu Phe Ile Ala Trp Leu Val Arg Xaa Arg
            20                  25                  30

<210> 191
<211> 30
<212> PRT
<213> Artificial Sequence

<220>
<223> Description of Artificial Sequence: Synthetic modified hGLP-1
      peptide

<220>

<221> MOD_RES
<222> (18)..(18)
<223> Aib

<220>
<221> MOD_RES
<222> (20)..(20)
<223> Lys(N-hexadecanoyl)

<220>
<221> MOD_RES
<222> (29)..(29)
<223> Aib


<220>
<223> c-term amidation

<400> 191
His Gly Glu Gly Thr Phe Thr Ser Asp Val Ser Ser Tyr Leu Glu Gly
1               5                   10                  15


Gln Xaa Ala Xaa Glu Phe Ile Ala Trp Leu Val Arg Xaa Arg
            20                  25                  30


<210> 192
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (28)..(28)
<223> Lys(N-octanoyl)

<220>
<221> MOD_RES
<222> (29)..(29)
<223> Aib


<220>
<223> c-term amidation

<400> 192
His Gly Glu Gly Thr Phe Thr Ser Asp Val Ser Ser Tyr Leu Glu Gly
1               5                   10                  15


Gln Xaa Ala Arg Glu Phe Ile Ala Trp Leu Val Xaa Xaa Arg
            20                  25                  30


<210> 193
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (28)..(28)
<223> Lys(N-tetradecanoyl)

<220>
<221> MOD_RES
<222> (29)..(29)
<223> Aib


<220>
<223> c-term amidation

<400> 193
His Gly Glu Gly Thr Phe Thr Ser Asp Val Ser Ser Tyr Leu Glu Gly
1               5                   10                  15


Gln Xaa Ala Arg Glu Phe Ile Ala Trp Leu Val Xaa Xaa Arg
            20                  25                  30


<210> 194
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (28)..(28)
<223> Lys(N-hexadecanoyl)

<220>
<221> MOD_RES
<222> (29)..(29)
<223> Aib


<220>
<223> c-term amidation

<400> 194
His Gly Glu Gly Thr Phe Thr Ser Asp Val Ser Ser Tyr Leu Glu Gly
1               5                   10                  15


Gln Xaa Ala Arg Glu Phe Ile Ala Trp Leu Val Xaa Xaa Arg

20                  25                  30

<210> 195
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 195
His Gly Glu Gly Thr Phe Thr Ser Asp Val Ser Ser Tyr Leu Glu Gly
1               5                   10                  15

Gln Xaa Ala Arg Glu Phe Ile Ala Trp Leu Val Arg Xaa Xaa
            20                  25                  30

<210> 196
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 196

His Gly Glu Gly Thr Phe Thr Ser Asp Val Ser Ser Tyr Leu Glu Gly
1               5                   10                  15

Gln Xaa Ala Arg Glu Phe Ile Ala Trp Leu Val Arg Xaa Xaa
            20                  25                  30

<210> 197
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 197
His Gly Glu Gly Thr Phe Thr Ser Asp Val Ser Ser Tyr Leu Glu Gly
1               5                   10                  15

Gln Xaa Ala Arg Glu Phe Ile Ala Trp Leu Val Arg Xaa Xaa
            20                  25                  30

<210> 198
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 198
His Gly Glu Gly Thr Phe Thr Ser Asp Val Ser Ser Tyr Leu Glu Gly
1               5                   10                  15

Glu Xaa Ala Lys Glu Phe Ile Ala Trp Xaa Val Xaa Xaa Arg
            20                  25                  30

<210> 199
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 199
His Gly Glu Gly Thr Phe Thr Ser Asp Val Ser Ser Tyr Leu Glu Gly
1               5                   10                  15

Glu Ala Ala Xaa Glu Phe Ile Ala Trp Leu Val Arg Xaa Arg
            20                  25                  30

<210> 200
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES

<222> (29)..(29)
<223> Aib


<220>
<223> c-term amidation

<400> 200
His Gly Glu Gly Thr Phe Thr Ser Asp Val Ser Ser Tyr Leu Glu Gly
1               5                   10                  15


Glu Ala Ala Xaa Glu Phe Ile Ala Trp Leu Val Arg Xaa Arg
            20                  25                  30


<210> 201
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib


<220>
<223> c-term amidation

<400> 201
His Gly Glu Gly Thr Phe Thr Ser Asp Val Ser Ser Tyr Leu Glu Gly
1               5                   10                  15


Glu Ala Ala Xaa Glu Phe Ile Ala Trp Leu Val Arg Xaa Arg
            20                  25                  30


<210> 202
<211> 30
<212> PRT
<213> Artificial Sequence

<220>
<223> Description of Artificial Sequence: Synthetic modified hGLP-1
      peptide


<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 202
His Gly Glu Gly Thr Phe Thr Ser Asp Val Ser Ser Tyr Leu Glu Gly
1               5                   10                  15

Glu Ala Ala Lys Glu Phe Ile Ala Trp Leu Val Xaa Xaa Arg
            20                  25                  30

<210> 203
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 203
His Gly Glu Gly Thr Phe Thr Ser Asp Val Ser Ser Tyr Leu Glu Gly
1               5                   10                  15

Glu Ala Ala Lys Glu Phe Ile Ala Trp Xaa Val Xaa Xaa Arg
            20                  25                  30

<210> 204
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES

<222> (29)..(29)
<223> Aib

<220>
<223> c-term amidation

<400> 204
His Gly Glu Gly Thr Phe Thr Ser Asp Val Ser Ser Tyr Leu Glu Gly
1               5                   10                  15

Glu Ala Ala Arg Glu Phe Ile Ala Trp Leu Val Xaa Xaa Arg
            20                  25                  30

<210> 205
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 205
His Gly Glu Gly Thr Phe Thr Ser Asp Val Ser Ser Tyr Leu Glu Gly
1               5                   10                  15

Glu Ala Ala Arg Glu Phe Ile Ala Trp Leu Val Xaa Xaa Arg
            20                  25                  30

<210> 206
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib

<220>

<223> c-term amidation

<400> 206
His Gly Glu Gly Thr Phe Thr Ser Asp Val Ser Ser Tyr Leu Glu Gly
1               5                   10                  15

Glu Ala Ala Arg Glu Phe Ile Ala Trp Leu Val Xaa Xaa Arg
            20                  25                  30

<210> 207
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 207
His Gly Glu Gly Thr Phe Thr Ser Asp Val Ser Ser Tyr Leu Glu Gly
1               5                   10                  15

Glu Ala Ala Lys Glu Phe Ile Ala Trp Leu Val Lys Xaa Xaa
            20                  25                  30

<210> 208
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 208
His Gly Glu Gly Thr Phe Thr Ser Asp Val Ser Ser Tyr Leu Glu Gly
1               5                   10                  15

Glu Ala Ala Lys Glu Phe Ile Ala Trp Leu Val Lys Xaa Xaa
            20                  25                  30

<210> 209
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 209
His Gly Glu Gly Thr Phe Thr Ser Asp Val Ser Ser Tyr Leu Glu Gly
1               5                   10                  15

Glu Ala Ala Lys Glu Phe Ile Ala Trp Leu Val Lys Xaa Xaa
            20                  25                  30

<210> 210
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 210
His Gly Glu Gly Thr Phe Thr Ser Asp Val Ser Ser Tyr Leu Glu Gly
1               5                   10                  15

Glu Ala Ala Arg Glu Phe Ile Ala Trp Leu Val Arg Xaa Xaa

20                  25                  30

<210> 211
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 211
His Gly Glu Gly Thr Phe Thr Ser Asp Val Ser Ser Tyr Leu Glu Gly
1               5                   10                  15

Glu Ala Ala Arg Glu Phe Ile Ala Trp Leu Val Arg Xaa Xaa
            20                  25                  30

<210> 212
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 212
His Gly Glu Gly Thr Phe Thr Ser Asp Val Ser Ser Tyr Leu Glu Gly
1               5                   10                  15

Glu Ala Ala Arg Glu Phe Ile Ala Trp Leu Val Arg Xaa Xaa
            20                  25                  30

<210> 213
<211> 30
<212> PRT

<213> Artificial Sequence

<220>
<223> Description of Artificial Sequence: Synthetic modified hGLP-1
      peptide


<220>
<221> MOD_RES
<222> (20)..(20)
<223> Lys(N-octanoyl)

<220>
<221> MOD_RES
<222> (24)..(24)
<223> Aib

<220>
<221> MOD_RES
<222> (29)..(29)
<223> Aib

<220>
<223> c-term amidation

<400> 213
His Gly Glu Gly Thr Phe Thr Ser Asp Val Ser Ser Tyr Leu Glu Gly
1               5                   10                  15


Gln Ala Ala Xaa Glu Phe Ile Xaa Trp Leu Val Arg Xaa Arg
            20                  25                  30


<210> 214
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (24)..(24)
<223> Aib

<220>
<221> MOD_RES
<222> (29)..(29)
<223> Aib



<220>
<223> c-term amidation

<400> 214
His Gly Glu Gly Thr Phe Thr Ser Asp Val Ser Ser Tyr Leu Glu Gly
1               5                   10                  15

Gln Ala Ala Xaa Glu Phe Ile Xaa Trp Leu Val Arg Xaa Arg
            20                  25                  30

<210> 215
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (24)..(24)
<223> Aib

<220>
<221> MOD_RES
<222> (29)..(29)
<223> Aib

<220>
<223> c-term amidation

<400> 215
His Gly Glu Gly Thr Phe Thr Ser Asp Val Ser Ser Tyr Leu Glu Gly
1               5                   10                  15

Gln Ala Ala Xaa Glu Phe Ile Xaa Trp Leu Val Arg Xaa Arg
            20                  25                  30

<210> 216
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 216

His Gly Glu Gly Thr Phe Thr Ser Asp Val Ser Ser Tyr Leu Glu Gly
1               5                   10                  15

Gln Ala Ala Arg Glu Phe Ile Xaa Trp Leu Val Xaa Xaa Arg
            20                  25                  30

<210> 217
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 217
His Gly Glu Gly Thr Phe Thr Ser Asp Val Ser Ser Tyr Leu Glu Gly
1               5                   10                  15

Gln Ala Ala Arg Glu Phe Ile Xaa Trp Leu Val Xaa Xaa Arg
            20                  25                  30

<210> 218
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 218
His Gly Glu Gly Thr Phe Thr Ser Asp Val Ser Ser Tyr Leu Glu Gly
1               5                   10                  15

Gln Ala Ala Arg Glu Phe Ile Xaa Trp Leu Val Xaa Xaa Arg
            20                  25                  30

<210> 219
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 219
His Gly Glu Gly Thr Phe Thr Ser Asp Val Ser Ser Tyr Leu Glu Gly
1               5                   10                  15

Gln Ala Ala Arg Glu Phe Ile Xaa Trp Leu Val Arg Xaa Xaa
            20                  25                  30

<210> 220
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 220
His Gly Glu Gly Thr Phe Thr Ser Asp Val Ser Ser Tyr Leu Glu Gly
1               5                   10                  15

Gln Ala Ala Arg Glu Phe Ile Xaa Trp Leu Val Arg Xaa Xaa
            20                  25                  30

<210> 221
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 221
His Gly Glu Gly Thr Phe Thr Ser Asp Val Ser Ser Tyr Leu Glu Gly
1               5                   10                  15

Glu Ala Ala Arg Glu Phe Ile Xaa Trp Leu Val Arg Xaa Xaa
            20                  25                  30

<210> 222
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 222
His Gly Glu Gly Thr Phe Thr Ser Asp Val Ser Ser Tyr Leu Glu Gly
1               5                   10                  15

Glu Ala Ala Lys Glu Phe Ile Ala Trp Xaa Val Lys Xaa Xaa
            20                  25                  30

<210> 223
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 223
His Gly Glu Gly Thr Phe Thr Ser Asp Val Ser Ser Tyr Leu Glu Gly
1               5                   10                  15

Glu Ala Ala Lys Glu Phe Ile Ala Trp Xaa Val Lys Xaa Xaa
            20                  25                  30

<210> 224
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> C-term amidation

<400> 224
His Gly Glu Gly Thr Phe Thr Ser Asp Val Ser Ser Tyr Leu Glu Gly
1               5                   10                  15

Glu Ala Ala Lys Glu Phe Ile Ala Trp Xaa Val Lys Xaa Xaa
            20                  25                  30

<210> 225
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> C-term amidation

<400> 225
His Gly Glu Gly Thr Phe Thr Ser Asp Val Ser Ser Tyr Leu Glu Gly
1               5                   10                  15

Glu Ala Ala Arg Glu Phe Ile Ala Trp Xaa Val Arg Xaa Xaa
            20                  25                  30

<210> 226
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 226
His Gly Glu Gly Thr Phe Thr Ser Asp Val Ser Ser Tyr Leu Glu Gly
1               5                   10                  15

Glu Ala Ala Arg Glu Phe Ile Ala Trp Xaa Val Arg Xaa Xaa
            20                  25                  30

<210> 227
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 227
His Gly Glu Gly Thr Phe Thr Ser Asp Val Ser Ser Tyr Leu Glu Gly
1               5                   10                  15

Glu Ala Ala Arg Glu Phe Ile Ala Trp Xaa Val Arg Xaa Xaa
            20                  25                  30

<210> 228
<211> 30
<212> PRT
<213> Artificial Sequence

<220>
<223> Description of Artificial Sequence: Synthetic modified hGLP-1
      peptide


<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Aib


<220>
<221> MOD_RES
<222> (30)..(30)
<223> Lys(N-octanoyl)


<220>
<223> c-term amidation

<400> 228
His Gly Glu Gly Thr Phe Thr Ser Asp Val Ser Ser Tyr Leu Glu Gly
1               5                   10                  15


Glu Xaa Ala Arg Glu Phe Ile Ala Trp Xaa Val Arg Xaa Xaa
            20                  25                  30


<210> 229
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 229
His Gly Glu Gly Thr Phe Thr Ser Asp Val Ser Ser Tyr Leu Glu Gly
1               5                   10                  15

Glu Xaa Ala Arg Glu Phe Ile Ala Trp Xaa Val Arg Xaa Xaa
            20                  25                  30

<210> 230
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 230
His Gly Glu Gly Thr Phe Thr Ser Asp Val Ser Ser Tyr Leu Glu Gly
1               5                   10                  15

Glu Xaa Ala Arg Glu Phe Ile Ala Trp Xaa Val Arg Xaa Xaa
            20                  25                  30

<210> 231
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (24)..(24)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 231
His Gly Glu Gly Thr Phe Thr Ser Asp Val Ser Ser Tyr Leu Glu Gly
1               5                   10                  15

Glu Xaa Ala Arg Glu Phe Ile Xaa Trp Xaa Val Arg Xaa Xaa
            20                  25                  30

<210> 232
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (24)..(24)
<223> Aib

<220>
<221> MOD_RES
<222> (26)..(26)
<223> A6c

<220>
<221> MOD_RES
<222> (29)..(29)

<223> Aib

<220>
<221> MOD_RES
<222> (30)..(30)
<223> Lys(N-tetradecanoyl)

<220>
<223> c-term amidation

<400> 232
His Gly Glu Gly Thr Phe Thr Ser Asp Val Ser Ser Tyr Leu Glu Gly
1               5                   10                  15

Glu Xaa Ala Arg Glu Phe Ile Xaa Trp Xaa Val Arg Xaa Xaa
            20                  25                  30

<210> 233
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (24)..(24)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 233
His Gly Glu Gly Thr Phe Thr Ser Asp Val Ser Ser Tyr Leu Glu Gly
1               5                   10                  15

Glu Xaa Ala Arg Glu Phe Ile Xaa Trp Xaa Val Arg Xaa Xaa
            20                  25                  30

<210> 234
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-HEPES-His

<220>
<221> MOD_RES
<222> (29)..(29)
<223> Beta-Ala

<220>
<223> c-term amidation

<400> 234
Xaa Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 235
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-HEPA-His

<220>
<221> MOD_RES
<222> (29)..(29)
<223> Beta-Ala

<220>
<223> c-term amidation

<400> 235
Xaa Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 236
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Aib

<220>
<223> c-term amidation

<400> 236
Xaa Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 237
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Beta-Ala

<220>
<223> c-term amidation

<400> 237
Xaa Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 238
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Aib

<220>
<223> c-term amidation

<400> 238
Xaa Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 239
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Beta-Ala

<220>
<223> c-term amidation

<400> 239
Xaa Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 240
<211> 30
<212> PRT
<213> Artificial Sequence

<220>
<223> Description of Artificial Sequence: Synthetic modified hGLP-1
      peptide

<220>

<221> MOD_RES
<222> (1)..(1)
<223> N-tetradecanoyl-His

<220>
<221> MOD_RES
<222> (29)..(29)
<223> Aib

<220>
<223> c-term amidation

<400> 240
Xaa Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg
            20                  25                  30

<210> 241
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Beta-Ala

<220>
<223> c-term amidation

<400> 241
Xaa Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg
            20                  25                  30

<210> 242
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanesulfonyl)

<220>
<221> MOD_RES
<222> (29)..(29)
<223> Aib

<220>
<223> c-term amidation

<400> 242
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 243
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-dodecanesulfonyl)

<220>
<221> MOD_RES
<222> (29)..(29)
<223> Aib

<220>
<223> c-term amidation

<400> 243
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 244
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanesulfonyl)

<220>
<221> MOD_RES
<222> (29)..(29)

<223> Aib

<220>
<223> c-term amidation

<400> 244
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 245
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanesulfonyl)

<220>
<221> MOD_RES
<222> (29)..(29)
<223> Aib

<220>
<223> c-term amidation

<400> 245
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 246
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-dodecanesulfonyl)

<220>
<221> MOD_RES
<222> (29)..(29)
<223> Aib

<220>

<223> c-term amidation

<400> 246
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 247
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanesulfonyl)

<220>
<221> MOD_RES
<222> (29)..(29)
<223> Aib

<220>
<223> c-term amidation

<400> 247
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 248
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanesulfonyl)

<220>
<223> c-term amidation

<400> 248
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 249
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanesulfonyl)

<220>
<223> c-term amidation

<400> 249
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 250
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-decylpiperazine))

<220>
<221> MOD_RES
<222> (29)..(29)
<223> Aib

<220>
<223> c-term amidation

<400> 250
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 251
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-dodecylpiperazine))

<220>
<221> MOD_RES
<222> (29)..(29)
<223> Aib

<220>
<223> c-term amidation

<400> 251
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 252
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-tetradecylpiperazine))

<220>
<221> MOD_RES
<222> (29)..(29)
<223> Aib

<220>
<223> c-term amidation

<400> 252
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 253
<211> 30
<212> PRT
<213> Artificial Sequence

<220>
<223> Description of Artificial Sequence: Synthetic modified hGLP-1
      peptide


<220>
<221> MOD_RES
<222> (20)..(20)
<223> Asp(1-(4-hexadecylpiperazine))

<220>
<221> MOD_RES
<222> (29)..(29)
<223> Aib

<220>
<223> c-term amidation

<400> 253
His Gly Glu Gly Thr Phe Thr Ser Asp Val Ser Ser Tyr Leu Glu Gly
1               5                   10                  15


Gln Ala Ala Xaa Glu Phe Ile Ala Trp Leu Val Arg Xaa Arg
            20                  25                  30


<210> 254
<211> 30
<212> PRT
<213> Artificial Sequence

<220>
<223> Description of Artificial Sequence: Synthetic modified hGLP-1
      peptide


<220>
<221> MOD_RES
<222> (28)..(28)
<223> Asp(1-(4-decylpiperazine))


<220>
<221> MOD_RES
<222> (29)..(29)
<223> Aib


<220>
<223> c-term amidation

<400> 254
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30

<210> 255
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-dodecylpiperazine))

<220>
<221> MOD_RES
<222> (29)..(29)
<223> Aib


<220>
<223> c-term amidation

<400> 255
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 256
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-tetradecylpiperazine))

<220>
<221> MOD_RES
<222> (29)..(29)
<223> Aib


<220>
<223> c-term amidation

<400> 256
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 257
<211> 30
<212> PRT

<213> Artificial Sequence

<220>
<223> Description of Artificial Sequence: Synthetic modified hGLP-1
      peptide

<220>
<221> MOD_RES
<222> (28)..(28)
<223> Asp(1-(4-hexadecylpiperazine))

<220>
<221> MOD_RES
<222> (29)..(29)
<223> Aib


<220>
<223> c-term amidation

<400> 257
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 258
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-decylpiperazine))


<220>
<223> c-term amidation

<400> 258
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Xaa
            20                  25                  30


<210> 259
<211> 30
<212> PRT
<213> Artificial Sequence

<220>
<223> Description of Artificial Sequence: Synthetic modified hGLP-1

peptide

<220>
<221> MOD_RES
<222> (29)..(29)
<223> Aib

<220>
<221> MOD_RES
<222> (30)..(30)
<223> Asp(1-(4-dodecylpiperazine)


<220>
<223> c-term amidation

<400> 259
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Xaa
            20                  25                  30


<210> 260
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<221> MOD_RES
<222> (30)..(30)
<223> Asp(1-(4-hexadecylpiperazine))


<220>
<223> c-term amidation

<400> 260
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Xaa
            20                  25                  30


<210> 261
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-decylpiperazine))

<220>
<223> c-term amidation

<400> 261
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 262
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-dodecylpiperazine))

<220>
<223> c-term amidation

<400> 262
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 263
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-tetradecylpiperazine))

<220>
<223> c-term amidation

<400> 263
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 264
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-hexadecylpiperazine))

<220>
<223> c-term amidation

<400> 264
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 265
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)

<223> Aib

<220>
<221> MOD_RES
<222> (32)..(32)
<223> Asp(1-(4-decylpiperazine))

<220>
<223> c-term amidation

<400> 265
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 266
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-dodecylpiperazine))

<220>
<223> c-term amidation

<400> 266
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 267
<211> 32
<212> PRT
<213> Artificial Sequence

<220>
<223> Description of Artificial Sequence: Synthetic modified hGLP-1
      peptide

<220>

<221> MOD_RES
<222> (29)..(29)
<223> Aib

<220>
<221> MOD_RES
<222> (31)..(31)
<223> Aib


<220>
<221> MOD_RES
<222> (32)..(32)
<223> Asp(1-(4-tetradecylpiperazine))

<220>
<223> c-term amidation

<400> 267
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Arg Xaa Xaa
            20                  25                  30


<210> 268
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib


<220>
<221> MOD_RES
<222> (32)..(32)
<223> Asp(1-(4-hexadecylpiperazine))

<220>
<223> c-term amidation

<400> 268
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Arg Xaa Xaa
            20                  25                  30


<210> 269
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-decylpiperazine))

<220>
<221> MOD_RES
<222> (29)..(29)
<223> Aib

<220>
<223> c-term amidation

<400> 269
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 270
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-dodecylpiperazine))

<220>
<221> MOD_RES
<222> (29)..(29)
<223> Aib

<220>
<223> c-term amidation

<400> 270
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 271
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-tetradecylpiperazine))

<220>
<221> MOD_RES
<222> (29)..(29)
<223> Aib


<220>
<223> c-term amidation

<400> 271
His Gly Glu Gly Thr Phe Thr Ser Asp Val Ser Ser Tyr Leu Glu Gly
1               5                   10                  15


Gln Ala Arg Xaa Glu Phe Ile Ala Trp Leu Val Arg Xaa Arg
            20                  25                  30


<210> 272
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-hexadecylpiperazine))

<220>
<221> MOD_RES
<222> (29)..(29)
<223> Aib


<220>
<223> c-term amidation

<400> 272
His Gly Glu Gly Thr Phe Thr Ser Asp Val Ser Ser Tyr Leu Glu Gly
1               5                   10                  15


Gln Ala Arg Xaa Glu Phe Ile Ala Trp Leu Val Arg Xaa Arg
            20                  25                  30


<210> 273
<211> 30
<212> PRT
<213> Artificial Sequence

<220>
<223> Description of Artificial Sequence: Synthetic modified hGLP-1
      peptide


<220>
<221> MOD_RES

<222> (28)..(28)
<223> Asp(1-(4-decylpiperazine))

<220>
<221> MOD_RES
<222> (29)..(29)
<223> Aib

<220>
<223> c-term amidation

<400> 273
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 274
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-dodecylpiperazine)

<220>
<221> MOD_RES
<222> (29)..(29)
<223> Aib

<220>
<223> c-term amidation

<400> 274
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 275
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-tetradecylpiperazine))

<220>
<221> MOD_RES
<222> (29)..(29)
<223> Aib


<220>
<223> c-term amidation

<400> 275
His Gly Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 276
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-hexadecylpiperazine))

<220>
<221> MOD_RES
<222> (29)..(29)
<223> Aib


<220>
<223> c-term amidation

<400> 276
His Gly Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 277
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib


<220>
<221> MOD_RES

<222> (30)..(30)
<223> Asp(1-(4-decylpiperazine))

<220>
<223> c-term amidation

<400> 277
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 278
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-dodecylpiperazine))

<220>
<223> c-term amidation

<400> 278
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 279
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-tetradecylpiperazine))

<220>

<223> c-term amidation

<400> 279
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 280
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-hexadecylpiperazine))

<220>
<223> c-term amidation

<400> 280
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 281
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-decylpiperazine))

<220>
<223> c-term amidation

<400> 281
His Gly Glu Gly Thr Phe Thr Ser Asp Val Ser Ser Tyr Leu Glu Gly

1                   5                        10                       15

Gln Ala Arg Arg Glu Phe Ile Ala Trp Leu Val Arg Xaa Arg Gly Xaa
                20                       25                       30

<210> 282
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-dodecylpiperazine))

<220>
<223> c-term amidation

<400> 282
His Gly Glu Gly Thr Phe Thr Ser Asp Val Ser Ser Tyr Leu Glu Gly
1                   5                        10                       15

Gln Ala Arg Arg Glu Phe Ile Ala Trp Leu Val Arg Xaa Arg Gly Xaa
                20                       25                       30

<210> 283
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-tetradecylpiperazine))

<220>
<223> c-term amidation

<400> 283
His Gly Glu Gly Thr Phe Thr Ser Asp Val Ser Ser Tyr Leu Glu Gly
1                   5                        10                       15

Gln Ala Arg Arg Glu Phe Ile Ala Trp Leu Val Arg Xaa Arg Gly Xaa

20                  25                  30

<210> 284
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-hexadecylpiperazine))

<220>
<223> c-term amidation

<400> 284
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 285
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-decylpiperazine))

<220>
<223> c-term amidation

<400> 285
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 286
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-dodecylpiperazine))

<220>
<223> c-term amidation

<400> 286
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 287
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-tetradecylpiperazine))

<220>
<223> c-term amidation

<400> 287

His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 288
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-hexadecylpiperazine))

<220>
<223> c-term amidation

<400> 288
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 289
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Glu(1-dodecylamino)

<220>
<223> c-term amidation

<400> 289
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 290
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Glu(1-dodecylamino)

<220>
<221> MOD_RES
<222> (29)..(29)
<223> Aib

<220>
<223> c-term amidation

<400> 290
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 291
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Glu(1-dodecylamino)

<220>
<221> MOD_RES
<222> (29)..(29)
<223> Aib

<220>
<223> c-term amidation

<400> 291
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 292
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Glu(1-dodecylamino)

<220>
<223> c-term amidation

<400> 292
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 293
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-decyl-1-piperazine)-acetyl))

<220>
<221> MOD_RES
<222> (29)..(29)
<223> Aib

<220>
<223> c-term amidation

<400> 293
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 294
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-dodecyl-1-piperazine)-acetyl))

<220>
<221> MOD_RES
<222> (29)..(29)
<223> Aib

<220>
<223> c-term amidation

<400> 294
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 295
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-tetradecyl-1-piperazine)-acetyl))

<220>
<221> MOD_RES
<222> (29)..(29)
<223> Aib

<220>
<223> c-term amidation

<400> 295
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg

20                  25                  30

<210> 296
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-hexadecyl-1-piperazine)-acetyl))

<220>
<221> MOD_RES
<222> (29)..(29)
<223> Aib

<220>
<223> c-term amidation

<400> 296
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 297
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-decyl-1-piperazine)-acetyl))

<220>
<221> MOD_RES
<222> (29)..(29)
<223> Aib

<220>
<223> c-term amidation

<400> 297
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 298

<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-dodecyl-1-piperazine)-acetyl))

<220>
<221> MOD_RES
<222> (29)..(29)
<223> Aib


<220>
<223> c-term amidation

<400> 298
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 299
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-tetradecyl-1-piperazine)-acetyl))

<220>
<221> MOD_RES
<222> (29)..(29)
<223> Aib


<220>
<223> c-term amidation

<400> 299
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 300
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-hexadecyl-1-piperazine)-acetyl))

<220>
<221> MOD_RES
<222> (29)..(29)
<223> Aib


<220>
<223> c-term amidation

<400> 300
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 301
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-decyl-1-piperazine)-acetyl))


<220>
<223> c-term amidation

<400> 301
His Gly Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Xaa
            20                  25                  30


<210> 302
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-dodecyl-1-piperazine)-acetyl))

<220>
<223> c-term amidation

<400> 302
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 303
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-hexadecyl-1-piperazine)-acetyl))

<220>
<223> c-term amidation

<400> 303
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 304
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-decyl-1-piperazine)-acetyl))

<220>
<223> c-term amidation

<400> 304
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 305
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-dodecyl-1-piperazine)-acetyl))

<220>
<223> c-term amidation

<400> 305
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 306
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-tetradecyl-1-piperazine)-acetyl))

<220>
<223> c-term amidation

<400> 306
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 307
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-hexadecyl-1-piperazine)-acetyl))

<220>
<223> c-term amidation

<400> 307
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 308
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)

<223> Lys(N-(2-(4-decyl-1-piperazine)-acetyl))

<220>
<223> c-term amidation

<400> 308
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 309
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-dodecyl-1-piperazine)-acetyl))

<220>
<223> c-term amidation

<400> 309
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 310
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)

<223> Aib

<220>
<221> MOD_RES
<222> (32)..(32)
<223> Lys(N-(2-(4-tetradecyl-1-piperazine)-acetyl))

<220>
<223> c-term amidation

<400> 310
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 311
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-hexadecyl-1-piperazine)-acetyl))

<220>
<223> c-term amidation

<400> 311
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 312
<211> 30
<212> PRT
<213> Artificial Sequence

<220>
<223> Description of Artificial Sequence: Synthetic modified hGLP-1
      peptide

<220>
<221> MOD_RES
<222> (20)..(20)

<223> Lys(N-(2-(4-decyl-1-piperazine)-acetyl))

<220>
<221> MOD_RES
<222> (29)..(29)
<223> Aib

<220>
<223> c-term amidation

<400> 312
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 313
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-dodecyl-1-piperazine)-acetyl))

<220>
<221> MOD_RES
<222> (29)..(29)
<223> Aib

<220>
<223> c-term amidation

<400> 313
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 314
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-tetradecyl-1-piperazine)-acetyl))

<220>
<221> MOD_RES
<222> (29)..(29)

<223> Aib

<220>
<223> c-term amidation

<400> 314
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 315
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-hexadecyl-1-piperazine)-acetyl))

<220>
<221> MOD_RES
<222> (29)..(29)
<223> Aib

<220>
<223> c-term amidation

<400> 315
His Gly Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 316
<211> 30
<212> PRT
<213> Artificial Sequence

<220>
<223> Description of Artificial Sequence: Synthetic modified hGLP-1

      peptide

<220>
<221> MOD_RES
<222> (28)..(28)
<223> Lys(N-(2-(4-decyl-1-piperazine)-acetyl))

<220>
<221> MOD_RES
<222> (29)..(29)
<223> Aib

<220>
<223> c-term amidation

<400> 316
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 317
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-dodecyl-1-piperazine)-acetyl))

<220>
<221> MOD_RES
<222> (29)..(29)
<223> Aib

<220>
<223> c-term amidation

<400> 317
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 318
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-tetradecyl-1-piperazine)-acetyl))

<220>
<221> MOD_RES
<222> (29)..(29)
<223> Aib

<220>
<223> c-term amidation

<400> 318
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 319
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-hexadecyl-1-piperazine)-acetyl))

<220>
<221> MOD_RES
<222> (29)..(29)
<223> Aib

<220>
<223> c-term amidation

<400> 319
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 320
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-decyl-1-piperazine)-acetyl))

<220>
<223> c-term amidation

<400> 320
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 321
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-dodecyl-1-piperazine)-acetyl))

<220>
<223> c-term amidation

<400> 321
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 322
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-tetradecyl-1-piperazine)-acetyl))

<220>
<223> c-term amidation

<400> 322
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 323
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-hexadecyl-1-piperazine)-acetyl))

<220>
<223> c-term amidation

<400> 323
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 324
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-decyl-1-piperazine)-acetyl))

<220>
<223> c-term amidation

<400> 324
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 325
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-dodecyl-1-piperazine)-acetyl))

<220>
<223> C-term amidation

<400> 325
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 326
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-tetradecyl-1-piperazine)-acetyl))

<220>
<223> C-term amidation

<400> 326
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 327
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-hexadecyl-1-piperazine)-acetyl))

<220>
<223> c-term amidation

<400> 327
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 328
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-decyl-1-piperazine)-acetyl))

<220>
<223> c-term amidation

<400> 328
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 329
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-dodecyl-1-piperazine)-acetyl))

<220>
<223> c-term amidation

<400> 329
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 330
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-tetradecyl-1-piperazine)-acetyl))

<220>
<223> c-term amidation

<400> 330
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 331
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-hexadecyl-1-piperazine)-acetyl))

<220>
<223> c-term amidation

<400> 331
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 332
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-decanoyl)

<400> 332
His Gly Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 333
<211> 30
<212> PRT
<213> Artificial Sequence

<220>
<223> Description of Artificial Sequence: Synthetic modified hGLP-1

peptide

<220>
<221> MOD_RES
<222> (29)..(29)
<223> Aib

<220>
<221> MOD_RES
<222> (30)..(30)
<223> Lys(N-decanoyl)

<400> 333
His Gly Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 334
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Ava

<220>
<221> MOD_RES
<222> (32)..(32)
<223> Ado

<220>
<223> c-term amidation

<400> 334
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 335
<211> 33
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Ava

<220>
<221> MOD_RES
<222> (33)..(33)
<223> Ado

<220>
<223> c-term amidation

<400> 335
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Asp Xaa
            20                  25                  30

Xaa

<210> 336
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aun

<220>
<223> c-term amidation

<400> 336
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 337
<211> 30
<212> PRT
<213> Artificial Sequence

<220>
<223> Description of Artificial Sequence: Synthetic modified hGLP-1
      peptide

<220>
<221> MOD_RES
<222> (11)..(11)
<223> Aib

<220>
<221> MOD_RES
<222> (29)..(29)
<223> Aib

<220>
<223> c-term amidation

<400> 337
His Gly Glu Gly Thr Phe Thr Ser Asp Val Xaa Ser Tyr Leu Glu Gly
1               5                   10                  15

Gln Ala Ala Lys Glu Phe Ile Ala Trp Leu Val Lys Xaa Arg
            20                  25                  30

<210> 338
<211> 33
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> D-Asp

<220>
<221> MOD_RES
<222> (32)..(32)
<223> Ava

<220>
<221> MOD_RES
<222> (33)..(33)
<223> Aun

<220>
<223> c-term amidation

<400> 338
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

Xaa

<210> 339
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 339
His Gly Glu Gly Thr Phe Thr Ser Asp Val Ser Ser Tyr Leu Glu Glu
1               5                   10                  15

Glu Ala Ala Lys Glu Phe Ile Ala Trp Leu Val Lys Xaa Arg
            20                  25                  30

<210> 340
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 340
His Gly Glu Gly Thr Phe Thr Ser Asp Val Ser Lys Tyr Leu Glu Gly
1               5                   10                  15

Gln Ala Ala Lys Glu Phe Ile Ala Trp Leu Val Lys Xaa Arg
            20                  25                  30

<210> 341
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 341
His Gly Glu Gly Thr Phe Thr Ser Asp Val Ser Ser Tyr Leu Glu Gly
1               5                   10                  15

Gln Ala Ala Lys Leu Phe Ile Ala Trp Leu Val Lys Xaa Arg
            20                  25                  30

<210> 342
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 342
His Gly Glu Gly Thr Phe Thr Ser Asp Val Ser Ser Tyr Leu Glu Gly
1               5                   10                  15

Gln Ala Ala Lys Glu Phe Ile Ala Trp Leu Lys Lys Xaa Arg
            20                  25                  30

<210> 343
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 343
His Gly Glu Gly Thr Phe Thr Ser Asp Val Ser Lys Tyr Leu Glu Gly
1               5                   10                  15

Gln Ala Ala Lys Leu Phe Ile Ala Trp Leu Val Lys Xaa Arg
            20                  25                  30

<210> 344
<211> 30
<212> PRT
<213> Artificial Sequence

<220>
<223> Description of Artificial Sequence: Synthetic modified hGLP-1
      peptide

<220>
<221> MOD_RES
<222> (30)..(30)
<223> D-Arg

<220>
<223> C-term amidation

<400> 344
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Xaa
            20                  25                  30

<210> 345
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> D-Arg

<220>
<223> C-term amidation

<400> 345
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg Xaa
            20                  25                  30

<210> 346
<211> 30
<212> PRT
<213> Artificial Sequence

<220>
<223> Description of Artificial Sequence: Synthetic modified hGLP-1
      peptide

<220>
<221> MOD_RES
<222> (21)..(21)
<223> Aib

<220>
<221> MOD_RES
<222> (29)..(29)
<223> Beta-Ala

<220>
<223> c-term amidation

<400> 346
His Gly Glu Gly Thr Phe Thr Ser Asp Val Ser Ser Tyr Leu Glu Gly
1               5                   10                  15

Gln Ala Ala Lys Xaa Phe Ile Ala Trp Leu Val Lys Xaa Arg
            20                  25                  30

<210> 347
<211> 32
<212> PRT
<213> Artificial Sequence

<220>
<223> Description of Artificial Sequence: Synthetic modified hGLP-1
      peptide

<220>
<221> MOD_RES
<222> (21)..(21)
<223> Aib

<220>
<221> MOD_RES
<222> (29)..(29)
<223> Beta-Ala

<220>
<221> MOD_RES
<222> (31)..(31)
<223> Beta-Ala

<220>
<223> c-term amidation

<400> 347
His Gly Glu Gly Thr Phe Thr Ser Asp Val Ser Ser Tyr Leu Glu Gly
1               5                   10                  15

Gln Ala Ala Lys Xaa Phe Ile Ala Trp Leu Val Lys Xaa Arg Xaa Arg
            20                  25                  30

<210> 348
<211> 33
<212> PRT

<213> Artificial Sequence

<220>
<223> Description of Artificial Sequence: Synthetic modified hGLP-1
      peptide

<220>
<221> MOD_RES
<222> (21)..(21)
<223> Aib

<220>
<221> MOD_RES
<222> (29)..(29)
<223> Beta-Ala

<220>
<221> MOD_RES
<222> (31)..(31)
<223> Beta-Ala

<220>
<223> c-term amidation

<400> 348
His Gly Glu Gly Thr Phe Thr Ser Asp Val Ser Ser Tyr Leu Glu Gly
1               5                   10                  15

Gln Ala Ala Lys Xaa Phe Ile Ala Trp Leu Val Lys Xaa Arg Xaa Arg
            20                  25                  30

Arg

<210> 349
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 349
His Gly Glu Gly Thr Phe Thr Ser Asp Val Ser Lys Tyr Leu Glu Gly
1               5                   10                  15

Gln Ala Ala Lys Lys Phe Ile Ala Trp Leu Val Lys Xaa Arg
            20                  25                  30

<210> 350
<211> 30
<212> PRT

<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 350
His Gly Glu Gly Thr Phe Thr Ser Asp Val Ser Ser Tyr Leu Glu Gly
1               5                   10                  15


Gln Ala Ala Lys Lys Phe Ile Ala Trp Leu Val Lys Xaa Arg
            20                  25                  30


<210> 351
<211> 32
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 351
His Gly Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Arg Gly Arg
            20                  25                  30


<210> 352
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>

<223> c-term amidation

<400> 352
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 353
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> D-Arg

<220>
<223> c-term amidation

<400> 353
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 354
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 354
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg Arg
            20                  25                  30

<210> 355
<211> 30

<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 355
His Gly Glu Gly Thr Phe Thr Ser Asp Val Ser Ser Tyr Leu Glu Gly
1               5                   10                  15


Gln Ala Ala Lys Glu Phe Ile Ala Phe Leu Val Lys Xaa Arg
            20                  25                  30


<210> 356
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<223> c-term amidation

<400> 356
His Gly Glu Gly Thr Phe Thr Ser Asp Val Ser Ser Tyr Leu Glu Gly
1               5                   10                  15


Gln Ala Ala Lys Glu Phe Ile Ala Phe Leu Val Lys Xaa Arg
            20                  25                  30


<210> 357
<211> 30
<212> PRT
<213> Artificial Sequence

<220>
<223> Description of Artificial Sequence: Synthetic modified hGLP-1
      peptide


<220>
<221> MOD_RES
<222> (25)..(25)
<223> 1-Nal or 2-Nal

<220>

<221> MOD_RES
<222> (29)..(29)
<223> Aib

<220>
<223> c-term amidation

<400> 357
His Gly Glu Gly Thr Phe Thr Ser Asp Val Ser Ser Tyr Leu Glu Gly
1               5                   10                  15

Gln Ala Ala Lys Glu Phe Ile Ala Xaa Leu Val Lys Xaa Arg
            20                  25                  30

<210> 358
<211> 30
<212> PRT
<213> Artificial Sequence

<220>
<223> Description of Artificial Sequence: Synthetic modified hGLP-1
      peptide

<220>
<221> MOD_RES
<222> (22)..(22)
<223> 1-Nal or 2-Nal

<220>
<221> MOD_RES
<222> (25)..(25)
<223> 1-Nal or 2-Nal

<220>
<221> MOD_RES
<222> (29)..(29)
<223> Aib

<220>
<223> c-term amidation

<400> 358
His Gly Glu Gly Thr Phe Thr Ser Asp Val Ser Ser Tyr Leu Glu Gly
1               5                   10                  15

Gln Ala Ala Lys Glu Xaa Ile Ala Xaa Leu Val Lys Xaa Arg
            20                  25                  30

<210> 359
<211> 30
<212> PRT
<213> Artificial Sequence

<220>
<223> Description of Artificial Sequence: Synthetic modified hGLP-1
      peptide

<220>
<221> MOD_RES
<222> (25)..(25)
<223> 1-Nal or 2-Nal

<220>
<221> MOD_RES
<222> (29)..(29)
<223> Aib

<220>
<223> c-term amidation

<400> 359
His Gly Glu Gly Thr Phe Thr Ser Asp Val Ser Ser Tyr Leu Glu Gly
1               5                   10                  15

Gln Ala Ala Arg Glu Phe Ile Ala Xaa Leu Val Arg Xaa Arg
            20                  25                  30

<210> 360
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 360
His Gly Glu Gly Thr Phe Thr Ser Asp Val Ser Ser Tyr Leu Glu Gly
1               5                   10                  15

Gln Ala Ala Arg Glu Phe Ile Ala Phe Leu Val Arg Xaa Arg
            20                  25                  30

<210> 361
<211> 30
<212> PRT
<213> Artificial Sequence

<220>
<223> Description of Artificial Sequence: Synthetic modified hGLP-1
      peptide

<220>
<221> MOD_RES
<222> (13)..(13)
<223> 1-Nal or 2-Nal

<220>
<221> MOD_RES
<222> (25)..(25)
<223> 1-Nal or 2-Nal

<220>
<221> MOD_RES
<222> (29)..(29)
<223> Aib

<220>
<223> c-term amidation

<400> 361
His Gly Glu Gly Thr Phe Thr Ser Asp Val Ser Ser Xaa Leu Glu Gly
1               5                   10                  15

Gln Ala Ala Lys Glu Phe Ile Ala Xaa Leu Val Lys Xaa Arg
            20                  25                  30

<210> 362
<211> 30
<212> PRT
<213> Artificial Sequence

<220>
<223> Description of Artificial Sequence: Synthetic modified hGLP-1
      peptide

<220>
<221> MOD_RES
<222> (6)..(6)
<223> 1-Nal or 2-Nal

<220>
<221> MOD_RES
<222> (25)..(25)
<223> 1-Nal or 2-Nal

<220>
<221> MOD_RES
<222> (29)..(29)
<223> Aib

<220>
<223> c-term amidation

<400> 362
His Gly Glu Gly Thr Xaa Thr Ser Asp Val Ser Ser Tyr Leu Glu Gly
1               5                   10                  15

Gln Ala Ala Lys Glu Phe Ile Ala Xaa Leu Val Lys Xaa Arg
            20                  25                  30

<210> 363
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)

<223> Lys(N-decanoyl)

<220>
<223> c-term amidation

<400> 363
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 364
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-decanoyl)

<220>
<221> MOD_RES
<222> (29)..(29)
<223> Aib

<220>
<223> c-term amidation

<400> 364
His Gly Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 365
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-dodecanoyl)

<220>
<223> c-term amidation

<400> 365
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 366
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> Ser(O-decanoyl)

<220>
<223> c-term amidation

<400> 366
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg Xaa
            20                  25                  30

<210> 367
<211> 33
<212> PRT
<213> Artificial Sequence

<220>
<223> Description of Artificial Sequence: Synthetic modified hGLP-1
      peptide

<220>
<221> MOD_RES
<222> (21)..(21)
<223> Aib

<220>
<221> MOD_RES
<222> (29)..(29)
<223> Beta-Ala

<220>
<221> MOD_RES

<222> (31)..(31)
<223> Beta-Ala

<220>
<221> MOD_RES
<222> (33)..(33)
<223> Lys(N-octanoyl)

<220>
<223> c-term amidation

<400> 367
His Gly Glu Gly Thr Phe Thr Ser Asp Val Ser Ser Tyr Leu Glu Gly
1               5                   10                  15

Gln Ala Ala Lys Xaa Phe Ile Ala Trp Leu Val Lys Xaa Arg Xaa Arg
            20                  25                  30

Xaa

<210> 368
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> Lys(N-octanoyl)

<220>
<223> c-term amidation

<400> 368
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 369
<211> 31
<212> PRT
<213> Artificial Sequence

<220>
<223> Description of Artificial Sequence: Synthetic modified hGLP-1
      peptide

<220>

<221> MOD_RES
<222> (29)..(29)
<223> Beta-Ala

<220>
<221> MOD_RES
<222> (31)..(31)
<223> Lys(N-decanoyl)

<220>
<223> c-term amidation

<400> 369
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 370
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> Lys(N-tetradecanoyl)

<220>
<223> c-term amidation

<400> 370
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 371
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> Lys(N-dodecanoyl)

<220>
<223> c-term amidation

<400> 371
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 372
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Beta-Ala

<220>
<221> MOD_RES
<222> (30)..(30)
<223> Lys(N-dodecanoyl)

<220>
<223> c-term amidation

<400> 372
Gly Glu Gly Thr Phe Thr Ser Asp Val Ser Ser Tyr Leu Glu Gly Gln
1               5                   10                  15

Ala Ala Arg Glu Phe Ile Ala Trp Leu Val Arg Xaa Arg Xaa
            20                  25                  30

<210> 373
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 373

His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Arg
            20                  25                  30

<210> 374
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 374
His Gly Glu Gly Thr Phe Thr Ser Asp Val Ser Ser Tyr Leu Glu Gly
1               5                   10                  15

Gln Ala Ala Arg Glu Phe Ile Ala Phe Leu Val Lys Xaa Arg
            20                  25                  30

<210> 375
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 375
His Gly Glu Gly Thr Phe Thr Ser Asp Val Ser Ser Tyr Leu Glu Gly
1               5                   10                  15

Gln Ala Ala Arg Glu Phe Ile Ala Phe Leu Val Lys Xaa Arg
            20                  25                  30

<210> 376
<211> 30
<212> PRT
<213> Artificial Sequence

<220>

<223> Description of Artificial Sequence: Synthetic modified hGLP-1
      peptide

<220>
<221> MOD_RES
<222> (29)..(29)
<223> Beta-Ala

<220>
<223> c-term amidation

<400> 376
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Arg
            20                  25                  30

<210> 377
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 377
His Gly Glu Gly Thr Phe Thr Ser Asp Val Ser Ser Tyr Leu Glu Gly
1               5                   10                  15

Gln Ala Ala Arg Glu Phe Ile Ala Phe Leu Val Arg Xaa Arg
            20                  25                  30

<210> 378
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 378
His Gly Glu Gly Thr Phe Thr Ser Asp Val Ser Ser Tyr Leu Glu Gly

1               5                   10                  15

Gln Ala Ala Arg Glu Phe Ile Ala Phe Leu Val Arg Xaa Arg
            20                  25                  30

<210> 379
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 379
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Arg Xaa Arg
            20                  25                  30

<210> 380
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 380
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Arg Xaa Arg
            20                  25                  30

<210> 381
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> C-term amidation

<400> 381
His Gly Glu Gly Thr Phe Thr Ser Asp Val Ser Ser Tyr Leu Glu Gly
1               5                   10                  15

Gln Ala Ala Lys Glu Phe Ile Ala Phe Leu Val Arg Xaa Arg
            20                  25                  30

<210> 382
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> C-term amidation

<400> 382
His Gly Glu Gly Thr Phe Thr Ser Asp Val Ser Ser Tyr Leu Glu Gly
1               5                   10                  15

Gln Ala Ala Lys Glu Phe Ile Ala Phe Leu Val Arg Xaa Arg
            20                  25                  30

<210> 383
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>

<221> MOD_RES
<222> (30)..(30)
<223> Lys(N-Aec-decanoyl)

<220>
<223> c-term amidation

<400> 383
His Gly Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 384
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> Ser(O-decanoyl)

<220>
<223> c-term amidation

<400> 384
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg Xaa
            20                  25                  30

<210> 385
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-Me-His

<220>
<221> MOD_RES
<222> (29)..(29)
<223> Aib

<220>

<223> c-term amidation

<400> 385
Xaa Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 386
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-Me-His

<220>
<221> MOD_RES
<222> (29)..(29)
<223> Beta-Ala

<220>
<223> c-term amidation

<400> 386
Xaa Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 387
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-Me-His

<220>
<221> MOD_RES
<222> (29)..(29)
<223> Aib

<220>
<223> c-term amidation

<400> 387
Xaa Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 388
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-Me-His

<220>
<221> MOD_RES
<222> (29)..(29)
<223> Beta-Ala

<220>
<223> c-term amidation

<400> 388
Xaa Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 389
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> A6c

<220>
<223> c-term amidation

<400> 389
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 390
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> A5c

<220>
<223> c-term amidation

<400> 390
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Arg
            20                  25                  30


<210> 391
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> D-Ala

<220>
<223> c-term amidation

<400> 391
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Arg
            20                  25                  30


<210> 392
<211> 30

<212> PRT
<213> Artificial Sequence

<220>
<223> Description of Artificial Sequence: Synthetic modified hGLP-1
      peptide


<220>
<221> MOD_RES
<222> (26)..(26)

<223> A6c

<220>
<221> MOD_RES
<222> (29)..(29)
<223> Aib

<220>
<223> c-term amidation

<400> 392
His Ser Glu Gly Thr Phe Thr Ser Asp Val Ser Ser Tyr Leu Glu Gly
1               5                   10                  15

Gln Ala Ala Lys Glu Phe Ile Ala Trp Xaa Val Lys Xaa Arg
            20                  25                  30

<210> 393
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A5c

<220>
<221> MOD_RES
<222> (29)..(29)
<223> Aib

<220>
<223> c-term amidation

<400> 393
His Ser Glu Gly Thr Phe Thr Ser Asp Val Ser Ser Tyr Leu Glu Gly
1               5                   10                  15

Gln Ala Ala Lys Glu Phe Ile Ala Trp Xaa Val Lys Xaa Arg
            20                  25                  30

<210> 394
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 394
His Ser Glu Gly Thr Phe Thr Ser Asp Val Ser Ser Tyr Leu Glu Gly
1               5                   10                  15

Glu Ala Ala Lys Glu Phe Ile Ala Trp Leu Val Lys Xaa Arg
            20                  25                  30

<210> 395
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Aib

<220>
<223> c-term amidation

<400> 395
His Ser Glu Gly Thr Phe Thr Ser Asp Val Ser Ser Tyr Leu Glu Gly
1               5                   10                  15

Gln Xaa Ala Lys Glu Phe Ile Ala Trp Leu Val Lys Xaa Arg
            20                  25                  30

<210> 396
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Aib

<220>
<223> c-term amidation

<400> 396
His Ser Glu Gly Thr Phe Thr Ser Asp Val Ser Ser Tyr Leu Glu Gly
1               5                   10                  15

Gln Ala Ala Lys Glu Phe Ile Xaa Trp Leu Val Lys Xaa Arg
            20                  25                  30


<210> 397
<211> 30
<212> PRT
<213> Artificial Sequence

<220>
<223> Description of Artificial Sequence: Synthetic modified hGLP-1
      peptide


<220>
<221> MOD_RES
<222> (19)..(19)
<223> Aib

<220>
<221> MOD_RES
<222> (29)..(29)
<223> Aib

<220>
<223> c-term amidation

<400> 397
His Ser Glu Gly Thr Phe Thr Ser Asp Val Ser Ser Tyr Leu Glu Gly
1               5                   10                  15


Gln Ala Xaa Lys Glu Phe Ile Ala Trp Leu Val Lys Xaa Arg
            20                  25                  30


<210> 398
<211> 30
<212> PRT
<213> Artificial Sequence

<220>
<223> Description of Artificial Sequence: Synthetic modified hGLP-1
      peptide


<220>
<221> MOD_RES
<222> (10)..(10)
<223> A6c

<220>
<221> MOD_RES
<222> (14)..(14)
<223> A6c

<220>
<221> MOD_RES
<222> (29)..(29)
<223> Aib

<220>
<223> c-term amidation

<400> 398
His Ser Glu Gly Thr Phe Thr Ser Asp Xaa Ser Ser Tyr Xaa Glu Gly

1                   5                   10                  15

Gln Ala Ala Lys Glu Phe Ile Ala Trp Leu Val Lys Xaa Arg
            20                  25                  30

<210> 399
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (23)..(23)
<223> A6c

<220>
<221> MOD_RES
<222> (26)..(26)
<223> Ac6

<220>
<221> MOD_RES
<222> (29)..(29)
<223> Aib

<220>
<223> c-term amidation

<400> 399
His Ser Glu Gly Thr Phe Thr Ser Asp Xaa Ser Ser Tyr Leu Glu Gly
1               5                   10                  15

Gln Ala Ala Lys Glu Phe Xaa Ala Trp Xaa Val Lys Xaa Arg
            20                  25                  30

<210> 400
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (26)..(26)

<223> A6c

<220>
<221> MOD_RES
<222> (29)..(29)
<223> Aib

<220>
<223> c-term amidation

<400> 400
His Ser Glu Gly Thr Phe Thr Ser Asp Val Ser Ser Tyr Xaa Glu Gly
1               5                   10                  15

Gln Ala Ala Lys Glu Phe Ile Ala Trp Xaa Val Lys Xaa Arg
            20                  25                  30

<210> 401
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (29)..(29)
<223> Aib

<220>
<223> c-term amidation

<400> 401
His Ser Glu Gly Thr Phe Thr Ser Asp Val Ser Ser Tyr Xaa Glu Gly
1               5                   10                  15

Gln Ala Ala Lys Glu Phe Ile Ala Trp Leu Val Lys Xaa Arg
            20                  25                  30

<210> 402
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 402
His Ser Glu Gly Thr Phe Thr Ser Asp Val Ser Ser Tyr Leu Glu Gly
1               5                   10                  15

Gln Ala Lys Lys Glu Phe Ile Ala Trp Leu Val Lys Xaa Arg
            20                  25                  30

<210> 403
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (18)..(18)
<223> Aib

<220>
<221> MOD_RES
<222> (29)..(29)
<223> Aib

<220>
<223> c-term amidation

<400> 403
His Ser Glu Gly Thr Phe Thr Ser Asp Val Ser Ser Tyr Xaa Glu Gly
1               5                   10                  15

Gln Xaa Ala Lys Glu Phe Ile Ala Trp Leu Val Lys Xaa Arg
            20                  25                  30

<210> 404
<211> 30
<212> PRT
<213> Artificial Sequence

<220>
<223> Description of Artificial Sequence: Synthetic modified hGLP-1
      peptide

<220>
<221> MOD_RES
<222> (23)..(23)
<223> A6c

<220>
<221> MOD_RES
<222> (26)..(26)
<223> A6c

<220>

<221> MOD_RES
<222> (29)..(29)
<223> Aib

<220>
<223> c-term amidation

<400> 404
His Ser Glu Gly Thr Phe Thr Ser Asp Val Ser Ser Tyr Leu Glu Gly
1               5                   10                  15

Gln Ala Ala Lys Glu Phe Xaa Ala Trp Xaa Val Lys Xaa Arg
            20                  25                  30

<210> 405
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Aib

<220>
<223> c-term amidation

<400> 405
His Ser Glu Gly Thr Phe Thr Ser Asp Val Ser Ser Tyr Leu Glu Gly
1               5                   10                  15

Gln Xaa Ala Lys Glu Phe Xaa Ala Trp Xaa Val Lys Xaa Arg
            20                  25                  30

<210> 406
<211> 30
<212> PRT
<213> Artificial Sequence

<220>
<223> Description of Artificial Sequence: Synthetic modified hGLP-1
      peptide

<220>
<221> MOD_RES
<222> (6)..(6)
<223> A6c

<220>
<221> MOD_RES
<222> (29)..(29)
<223> Aib

<220>
<223> c-term amidation

<400> 406
His Ser Glu Gly Thr Xaa Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 407
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> Cha

<220>
<221> MOD_RES
<222> (29)..(29)
<223> Aib

<220>
<223> c-term amidation

<400> 407
His Ser Glu Gly Thr Phe Thr Ser Asp Val Ser Ser Tyr Xaa Glu Gly
1               5                   10                  15

Gln Ala Ala Lys Glu Phe Ile Ala Trp Leu Val Lys Xaa Arg
            20                  25                  30

<210> 408
<211> 30
<212> PRT
<213> Artificial Sequence

<220>
<223> Description of Artificial Sequence: Synthetic modified hGLP-1
      peptide

<220>
<221> MOD_RES
<222> (27)..(27)

<223> A6c

<220>
<221> MOD_RES
<222> (29)..(29)
<223> Aib

<220>
<223> c-term amidation

<400> 408
His Ser Glu Gly Thr Phe Thr Ser Asp Val Ser Ser Tyr Leu Glu Gly
1               5                   10                  15

Gln Ala Ala Lys Glu Phe Ile Ala Trp Leu Xaa Lys Xaa Arg
            20                  25                  30

<210> 409
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (14)..(14)
<223> A6c

<220>
<221> MOD_RES
<222> (29)..(29)
<223> Beta-Ala

<220>
<223> c-term amidation

<400> 409
His Ser Glu Gly Thr Phe Thr Ser Asp Xaa Ser Ser Tyr Xaa Glu Gly
1               5                   10                  15

Gln Ala Ala Lys Glu Phe Ile Ala Trp Leu Val Lys Xaa Arg
            20                  25                  30

<210> 410
<211> 30
<212> PRT
<213> Artificial Sequence

<220>
<223> Description of Artificial Sequence: Synthetic modified hGLP-1
      peptide

<220>

<221> MOD_RES
<222> (16)..(16)
<223> Beta-Ala

<220>
<221> MOD_RES
<222> (29)..(29)
<223> Aib

<220>
<223> c-term amidation

<400> 410
His Ser Glu Gly Thr Phe Thr Ser Asp Val Ser Ser Tyr Leu Glu Xaa
1               5                   10                  15

Gln Ala Ala Lys Glu Phe Ile Ala Trp Leu Val Lys Xaa Arg
            20                  25                  30

<210> 411
<211> 30
<212> PRT
<213> Artificial Sequence

<220>
<223> Description of Artificial Sequence: Synthetic modified hGLP-1
      peptide

<220>
<221> MOD_RES
<222> (16)..(16)
<223> Aib

<220>
<221> MOD_RES
<222> (29)..(29)
<223> Aib

<220>
<223> c-term amidation

<400> 411
His Ser Glu Gly Thr Phe Thr Ser Asp Val Ser Ser Tyr Leu Glu Xaa
1               5                   10                  15

Gln Ala Ala Lys Glu Phe Ile Ala Trp Leu Val Lys Xaa Arg
            20                  25                  30

<210> 412
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<223> c-term amidation

<400> 412
His Ser Glu Gly Thr Phe Thr Ser Asp Val Ser Ser Tyr Leu Glu Gly
1               5                   10                  15

Glu Ala Ala Lys Glu Phe Ile Ala Trp Xaa Val Lys Xaa Arg
            20                  25                  30

<210> 413
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Aib

<220>
<223> c-term amidation

<400> 413
His Ser Glu Gly Thr Phe Thr Ser Asp Val Ser Ser Tyr Leu Glu Gly
1               5                   10                  15

Glu Xaa Ala Lys Glu Phe Ile Ala Trp Xaa Val Lys Xaa Arg
            20                  25                  30

<210> 414
<211> 30
<212> PRT
<213> Artificial Sequence

<220>
<223> Description of Artificial Sequence: Synthetic modified hGLP-1
      peptide

<220>
<221> MOD_RES
<222> (18)..(19)
<223> Aib

<220>
<221> MOD_RES
<222> (26)..(26)
<223> A6c

<220>
<221> MOD_RES
<222> (29)..(29)
<223> Aib

<220>
<223> C-term amidation

<400> 414
His Ser Glu Gly Thr Phe Thr Ser Asp Val Ser Ser Tyr Leu Glu Gly
1               5                   10                  15

Glu Xaa Xaa Lys Glu Phe Ile Ala Trp Xaa Val Lys Xaa Arg
            20                  25                  30

<210> 415
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (14)..(14)
<223> A6c

<220>
<221> MOD_RES
<222> (18)..(19)
<223> Aib

<220>
<221> MOD_RES
<222> (26)..(26)
<223> A6c

<220>
<221> MOD_RES
<222> (29)..(29)
<223> Aib

<220>
<223> C-term amidation

<400> 415y
His Ser Glu Gly Thr Phe Thr Ser Asp Xaa Ser Ser Tyr Xaa Glu Gly
1               5                   10                  15

Glu Xaa Xaa Lys Glu Phe Ile Ala Trp Xaa Val Lys Xaa Arg
            20                  25                  30

<210> 416
<211> 30
<212> PRT
<213> Artificial Sequence

<220>
<223> Description of Artificial Sequence: Synthetic modified hGLP-1
      peptide


<220>
<221> MOD_RES
<222> (26)..(26)
<223> A6c


<220>
<221> MOD_RES
<222> (29)..(29)
<223> Beta-Ala

<220>
<223> c-term amidation

<400> 416
His Ser Glu Gly Thr Phe Thr Ser Asp Val Ser Ser Tyr Leu Glu Gly
1               5                   10                  15


Gln Ala Ala Lys Glu Phe Ile Ala Trp Xaa Val Lys Xaa Arg
            20                  25                  30


<210> 417
<211> 30
<212> PRT
<213> Artificial Sequence

<220>
<223> Description of Artificial Sequence: Synthetic modified hGLP-1
      peptide


<220>
<221> MOD_RES
<222> (26)..(26)
<223> A5c

<220>
<221> MOD_RES
<222> (29)..(29)
<223> Beta-Ala

<220>
<223> c-term amidation

<400> 417
His Ser Glu Gly Thr Phe Thr Ser Asp Val Ser Ser Tyr Leu Glu Gly
1               5                   10                  15


Gln Ala Ala Lys Glu Phe Ile Ala Trp Xaa Val Lys Xaa Arg
            20                  25                  30


<210> 418

<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 418
His Ser Glu Gly Thr Phe Thr Ser Asp Val Ser Ser Tyr Leu Glu Gly
1               5                   10                  15


Glu Ala Ala Lys Glu Phe Ile Ala Trp Leu Val Lys Xaa Arg
            20                  25                  30


<210> 419
<211> 30
<212> PRT
<213> Artificial Sequence

<220>
<223> Description of Artificial Sequence: Synthetic modified hGLP-1
      peptide


<220>
<221> MOD_RES
<222> (18)..(18)
<223> Aib

<220>
<221> MOD_RES
<222> (29)..(29)
<223> Beta-Ala

<220>
<223> c-term amidation

<400> 419
His Ser Glu Gly Thr Phe Thr Ser Asp Val Ser Ser Tyr Leu Glu Gly
1               5                   10                  15


Gln Xaa Ala Lys Glu Phe Ile Ala Trp Leu Val Lys Xaa Arg
            20                  25                  30


<210> 420
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Beta-Ala

<220>
<223> c-term amidation

<400> 420
His Ser Glu Gly Thr Phe Thr Ser Asp Val Ser Ser Tyr Leu Glu Gly
1               5                   10                  15

Gln Ala Ala Lys Glu Phe Ile Xaa Trp Leu Val Lys Xaa Arg
            20                  25                  30

<210> 421
<211> 30
<212> PRT
<213> Artificial Sequence

<220>
<223> Description of Artificial Sequence: Synthetic modified hGLP-1
      peptide

<220>
<221> MOD_RES
<222> (19)..(19)
<223> Aib

<220>
<221> MOD_RES
<222> (29)..(29)
<223> Beta-Ala

<220>
<223> c-term amidation

<400> 421
His Ser Glu Gly Thr Phe Thr Ser Asp Val Ser Ser Tyr Leu Glu Gly
1               5                   10                  15

Gln Ala Xaa Lys Glu Phe Ile Ala Trp Leu Val Lys Xaa Arg
            20                  25                  30

<210> 422
<211> 30
<212> PRT
<213> Artificial Sequence

<220>
<223> Description of Artificial Sequence: Synthetic modified hGLP-1
      peptide

<220>
<221> MOD_RES
<222> (10)..(10)

<223> A6c

<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Beta-Ala

<220>
<223> c-term amidation

<400> 422
His Ser Glu Gly Thr Phe Thr Ser Asp Xaa Ser Ser Tyr Leu Glu Gly
1               5                   10                  15

Gln Ala Ala Lys Glu Phe Xaa Ala Trp Xaa Val Lys Xaa Arg
            20                  25                  30

<210> 423
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (26)..(26)
<223> A6c

<220>
<221> MOD_RES
<222> (29)..(29)
<223> Beta-Ala

<220>
<223> c-term amidation

<400> 423
His Ser Glu Gly Thr Phe Thr Ser Asp Val Ser Ser Tyr Xaa Glu Gly
1               5                   10                  15

Gln Ala Ala Lys Glu Phe Ile Ala Trp Xaa Val Lys Xaa Arg
            20                  25                  30

<210> 424
<211> 30

<212> PRT
<213> Artificial Sequence

<220>
<223> Description of Artificial Sequence: Synthetic modified hGLP-1
      peptide


<220>
<221> MOD_RES
<222> (14)..(14)
<223> A6c

<220>
<221> MOD_RES
<222> (29)..(29)
<223> Beta-Ala

<220>
<223> c-term amidation

<400> 424
His Ser Glu Gly Thr Phe Thr Ser Asp Val Ser Ser Tyr Xaa Glu Gly
1               5                   10                  15


Gln Ala Ala Lys Glu Phe Ile Ala Trp Leu Val Lys Xaa Arg
            20                  25                  30


<210> 425
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 425
His Ser Glu Gly Thr Phe Thr Ser Asp Val Ser Ser Tyr Leu Glu Gly
1               5                   10                  15



Gln Ala Lys Lys Glu Phe Ile Ala Trp Leu Val Lys Xaa Arg
            20                  25                  30


<210> 426
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> A6c

<220>
<221> MOD_RES
<222> (18)..(18)
<223> Aib

<220>
<221> MOD_RES
<222> (29)..(29)
<223> Beta-Ala

<220>
<223> c-term amidation

<400> 426
His Ser Glu Gly Thr Phe Thr Ser Asp Val Ser Ser Tyr Xaa Glu Gly
1               5                   10                  15

Gln Xaa Ala Lys Glu Phe Ile Ala Trp Leu Val Lys Xaa Arg
            20                  25                  30

<210> 427
<211> 30
<212> PRT
<213> Artificial Sequence

<220>
<223> Description of Artificial Sequence: Synthetic modified hGLP-1
      peptide

<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Beta-Ala

<220>
<223> c-term amidation

<400> 427
His Ser Glu Gly Thr Phe Thr Ser Asp Val Ser Ser Tyr Leu Glu Gly
1               5                   10                  15

Gln Ala Ala Lys Glu Phe Xaa Ala Trp Xaa Val Lys Xaa Arg
            20                  25                  30

<210> 428
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (23)..(23)
<223> A6c

<220>
<221> MOD_RES
<222> (26)..(26)
<223> A6c

<220>
<221> MOD_RES
<222> (29)..(29)
<223> Beta-Ala

<220>
<223> c-term amidation

<400> 428
His Ser Glu Gly Thr Phe Thr Ser Asp Val Ser Ser Tyr Leu Glu Gly
1               5                   10                  15

Gln Xaa Ala Lys Glu Phe Xaa Ala Trp Xaa Val Lys Xaa Arg
            20                  25                  30

<210> 429
<211> 30
<212> PRT
<213> Artificial Sequence

<220>
<223> Description of Artificial Sequence: Synthetic modified hGLP-1
      peptide

<220>
<221> MOD_RES
<222> (6)..(6)
<223> A6c

<220>
<221> MOD_RES
<222> (29)..(29)
<223> Beta-Ala

<220>
<223> c-term amidation

<400> 429
His Ser Glu Gly Thr Xaa Thr Ser Asp Val Ser Ser Tyr Leu Glu Gly
1               5                   10                  15

Gln Ala Ala Lys Glu Phe Ile Ala Trp Leu Val Lys Xaa Arg

20                  25                  30

<210> 430
<211> 30
<212> PRT
<213> Artificial Sequence

<220>
<223> Description of Artificial Sequence: Synthetic modified hGLP-1
      peptide

<220>
<221> MOD_RES
<222> (14)..(14)
<223> Cha

<220>
<221> MOD_RES
<222> (29)..(29)
<223> Beta-Ala

<220>
<223> c-term amidation

<400> 430
His Ser Glu Gly Thr Phe Thr Ser Asp Val Ser Ser Tyr Xaa Glu Gly
1               5                   10                  15

Gln Ala Ala Lys Glu Phe Ile Ala Trp Leu Val Lys Xaa Arg
            20                  25                  30

<210> 431
<211> 30
<212> PRT
<213> Artificial Sequence

<220>
<223> Description of Artificial Sequence: Synthetic modified hGLP-1
      peptide

<220>
<221> MOD_RES
<222> (27)..(27)
<223> A6c

<220>
<221> MOD_RES
<222> (29)..(29)
<223> Beta-Ala

<220>
<223> c-term amidation

<400> 431
His Ser Glu Gly Thr Phe Thr Ser Asp Val Ser Ser Tyr Leu Glu Gly
1               5                   10                  15

Gln Ala Ala Lys Glu Phe Ile Ala Trp Leu Xaa Lys Xaa Arg
            20                  25                  30

<210> 432
<211> 30
<212> PRT
<213> Artificial Sequence

<220>
<223> Description of Artificial Sequence: Synthetic modified hGLP-1
      peptide


<220>
<221> MOD_RES
<222> (16)..(16)
<223> Beta-Ala

<220>
<221> MOD_RES
<222> (29)..(29)
<223> Beta-Ala

<220>
<223> C-term amidation

<400> 432
His Ser Glu Gly Thr Phe Thr Ser Asp Val Ser Ser Tyr Leu Glu Xaa
1               5                   10                  15


Gln Ala Ala Lys Glu Phe Ile Ala Trp Leu Val Lys Xaa Arg
            20                  25                  30


<210> 433
<211> 30
<212> PRT
<213> Artificial Sequence

<220>
<223> Description of Artificial Sequence: Synthetic modified hGLP-1
      peptide


<220>
<221> MOD_RES
<222> (16)..(16)
<223> Aib

<220>
<221> MOD_RES
<222> (29)..(29)
<223> Beta-Ala

<220>
<223> C-term amidation

<400> 433
His Ser Glu Gly Thr Phe Thr Ser Asp Val Ser Ser Tyr Leu Glu Xaa
1               5                   10                  15


Gln Ala Ala Lys Glu Phe Ile Ala Trp Leu Val Lys Xaa Arg
            20                  25                  30


<210> 434
<211> 30
<212> PRT

<213> Artificial Sequence

<220>
<223> Description of Artificial Sequence: Synthetic modified hGLP-1
      peptide


<220>
<221> MOD_RES
<222> (26)..(26)
<223> A6c

<220>
<221> MOD_RES
<222> (29)..(29)
<223> Beta-Ala

<220>
<223> c-term amidation

<400> 434
His Ser Glu Gly Thr Phe Thr Ser Asp Val Ser Ser Tyr Leu Glu Gly
1               5                   10                  15


Glu Ala Ala Lys Glu Phe Ile Ala Trp Xaa Val Lys Xaa Arg
            20                  25                  30


<210> 435
<211> 30
<212> PRT
<213> Artificial Sequence

<220>
<223> Description of Artificial Sequence: Synthetic modified hGLP-1
      peptide


<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Beta-Ala

<220>
<223> c-term amidation

<400> 435
His Ser Glu Gly Thr Phe Thr Ser Asp Val Ser Ser Tyr Leu Glu Gly
1               5                   10                  15


Glu Xaa Ala Lys Glu Phe Ile Ala Trp Xaa Val Lys Xaa Arg
            20                  25                  30


<210> 436
<211> 30

<212> PRT
<213> Artificial Sequence

<220>
<223> Description of Artificial Sequence: Synthetic modified hGLP-1
      peptide


<220>
<221> MOD_RES
<222> (18)..(18)
<223> Aib

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 436
His Ser Glu Gly Thr Phe Thr Ser Asp Val Ser Ser Tyr Leu Glu Gly
1               5                   10                  15


Glu Xaa Ala Lys Glu Phe Ile Ala Trp Xaa Val Xaa Xaa Arg
            20                  25                  30


<210> 437
<211> 30
<212> PRT
<213> Artificial Sequence

<220>
<223> Description of Artificial Sequence: Synthetic modified hGLP-1
      peptide



<220>
<221> MOD_RES
<222> (18)..(19)
<223> Aib

<220>
<221> MOD_RES
<222> (26)..(26)
<223> A6c

<220>
<221> MOD_RES
<222> (29)..(29)
<223> Beta-Ala

<220>
<223> c-term amidation

<400> 437
His Ser Glu Gly Thr Phe Thr Ser Asp Val Ser Ser Tyr Leu Glu Gly
1               5                   10                  15

Glu Xaa Xaa Lys Glu Phe Ile Ala Trp Xaa Val Lys Xaa Arg
            20                  25                  30

<210> 438
<211> 30
<212> PRT
<213> Artificial Sequence

<220>
<223> Description of Artificial Sequence: Synthetic modified hGLP-1
      peptide

<220>
<221> MOD_RES
<222> (10)..(10)
<223> A6c

<220>
<221> MOD_RES
<222> (14)..(14)
<223> A6c

<220>
<221> MOD_RES
<222> (18)..(19)
<223> Aib

<220>
<221> MOD_RES
<222> (26)..(26)
<223> A6c

<220>
<221> MOD_RES
<222> (29)..(29)
<223> Beta-Ala

<220>
<223> C-term amidation

<400> 438
His Ser Glu Gly Thr Phe Thr Ser Asp Xaa Ser Ser Tyr Xaa Glu Gly
1               5                   10                  15

Glu Xaa Xaa Lys Glu Phe Ile Ala Trp Xaa Val Lys Xaa Arg
            20                  25                  30

<210> 439
<211> 30
<212> PRT
<213> Artificial Sequence

<220>
<223> Description of Artificial Sequence: Synthetic modified hGLP-1
      peptide

<220>

<221> MOD_RES
<222> (29)..(29)
<223> Aib

<220>
<221> MOD_RES
<222> (30)..(30)
<223> D-Arg

<220>
<223> c-term amidation

<400> 439
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 440
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> D-Lys

<220>
<223> c-term amidation

<400> 440
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 441
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>

<221> MOD_RES
<222> (30)..(30)
<223> D-Arg


<220>
<223> c-term amidation

<400> 441
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Xaa
            20                  25                  30


<210> 442
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> D-Lys


<220>
<223> c-term amidation

<400> 442
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Xaa
            20                  25                  30


<210> 443
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<223> c-term amidation

<400> 443

His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 444
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 444
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 445
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 445
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg
            20                  25                  30

<210> 446
<211> 30
<212> PRT
<213> Artificial Sequence

<220>

<223> Description of Artificial Sequence: Synthetic modified hGLP-1
      peptide

<220>
<221> MOD_RES
<222> (29)..(29)
<223> Beta-Ala

<220>
<223> c-term amidation

<400> 446
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg
            20                  25                  30

<210> 447
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<400> 447
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 448
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)

<223> Lys(N-tetradecanoyl)

<400> 448
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa Gly
            20                  25                  30

<210> 449
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<221> MOD_RES
<222> (31)..(31)
<223> Aib

<400> 449
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa Xaa
            20                  25                  30

<210> 450
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<221> MOD_RES
<222> (31)..(31)
<223> D-Ala

<400> 450
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa Xaa
            20                  25                  30

<210> 451
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-tetradecanoyl)

<400> 451
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 452
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Beta-Ala

<220>
<221> MOD_RES
<222> (32)..(32)
<223> Lys(N-tetradecanoyl)

<400> 452
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 453
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-tetradecanoyl)

<400> 453
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 454
<211> 31
<212> PRT
<213> Artificial Sequence

<220>
<223> Description of Artificial Sequence: Synthetic modified hGLP-1
      peptide

<220>
<221> MOD_RES
<222> (30)..(30)
<223> Lys(N-tetradecanoyl)

<220>
<221> MOD_RES
<222> (31)..(31)
<223> Beta-Ala

<400> 454
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Xaa Xaa
            20                  25                  30

<210> 455

<211> 31
<212> PRT
<213> Artificial Sequence

<220>
<223> Description of Artificial Sequence: Synthetic modified hGLP-1
      peptide

<220>
<221> MOD_RES
<222> (30)..(30)
<223> Lys(N-tetradecanoyl)

<220>
<221> MOD_RES
<222> (31)..(31)
<223> Aib

<400> 455
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Xaa Xaa
            20                  25                  30

<210> 456
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Ado

<400> 456
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 457
<211> 31
<212> PRT
<213> Artificial Sequence

<220>
<223> Description of Artificial Sequence: Synthetic modified hGLP-1
      peptide

<220>
<221> MOD_RES

<222> (29)..(29)
<223> Aib

<220>
<221> MOD_RES
<222> (31)..(31)
<223> Ado

<400> 457
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 458
<211> 31
<212> PRT
<213> Artificial Sequence

<220>
<223> Description of Artificial Sequence: Synthetic modified hGLP-1
      peptide

<220>
<221> MOD_RES
<222> (30)..(30)
<223> Lys(N-tetradecanoyl)

<220>
<221> MOD_RES
<222> (31)..(31)
<223> D-Ala

<400> 458
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Xaa Xaa
            20                  25                  30

<210> 459
<211> 32
<212> PRT
<213> Artificial Sequence

<220>
<223> Description of Artificial Sequence: Synthetic modified hGLP-1
      peptide

<220>
<221> MOD_RES
<222> (31)..(31)
<223> Aib

<220>
<221> MOD_RES
<222> (32)..(32)
<223> Lys(N-tetradecanoyl)

<400> 459
His Ser Glu Gly Thr Phe Thr Ser Asp Val Ser Ser Tyr Leu Glu Gly

1               5                   10                  15

Gln Ala Ala Arg Glu Phe Ile Ala Trp Leu Val Arg Gly Arg Xaa Xaa
            20                  25                  30

<210> 460
<211> 32
<212> PRT
<213> Artificial Sequence

<220>
<223> Description of Artificial Sequence: Synthetic modified hGLP-1
      peptide

<220>
<221> MOD_RES
<222> (31)..(31)
<223> Beta-Ala

<220>
<221> MOD_RES
<222> (32)..(32)
<223> Lys(N-tetradecanoyl)

<400> 460
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Gly Arg Xaa Xaa
            20                  25                  30

<210> 461
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 461
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Xaa Arg
            20                  25                  30

<210> 462
<211> 30
<212> PRT
<213> Artificial Sequence

<220>
<223> Description of Artificial Sequence: Synthetic modified hGLP-1
      peptide


<220>
<221> MOD_RES
<222> (20)..(20)
<223> Lys(N-tetradecanoyl)

<220>
<221> MOD_RES
<222> (29)..(29)
<223> Aib

<220>
<223> C-term amidation

<400> 462
His Ser Glu Gly Thr Phe Thr Ser Asp Val Ser Ser Tyr Leu Glu Gly
1               5                   10                  15


Gln Ala Ala Xaa Glu Phe Ile Ala Trp Leu Val Lys Xaa Arg
            20                  25                  30


<210> 463
<211> 30
<212> PRT
<213> Artificial Sequence

<220>
<223> Description of Artificial Sequence: Synthetic modified hGLP-1
      peptide


<220>
<221> MOD_RES
<222> (20)..(20)
<223> Lys(N-hexadecanoyl)

<220>
<221> MOD_RES
<222> (29)..(29)
<223> Aib

<220>
<223> C-term amidation

<400> 463
His Ser Glu Gly Thr Phe Thr Ser Asp Val Ser Ser Tyr Leu Glu Gly
1               5                   10                  15


Gln Ala Ala Xaa Glu Phe Ile Ala Trp Leu Val Lys Xaa Arg
            20                  25                  30


<210> 464
<211> 30
<212> PRT

<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Beta-Ala

<220>
<223> c-term amidation

<400> 464
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Xaa Arg
            20                  25                  30

<210> 465
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)
<223> Beta-Ala

<220>
<223> c-term amidation

<400> 465
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Xaa Arg
            20                  25                  30

<210> 466
<211> 30
<212> PRT
<213> Artificial Sequence

<220>
<223> Description of Artificial Sequence: Synthetic modified hGLP-1

peptide

<220>
<221> MOD_RES
<222> (20)..(20)
<223> Lys(N-hexadecanoyl)

<220>
<221> MOD_RES
<222> (29)..(29)
<223> Beta-Ala

<220>
<223> c-term amidation

<400> 466
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Lys Xaa Arg
            20                  25                  30

<210> 467
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 467
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 468
<211> 30
<212> PRT
<213> Artificial Sequence

<220>
<223> Description of Artificial Sequence: Synthetic modified hGLP-1
      peptide

<220>

<221> MOD_RES
<222> (20)..(20)
<223> Lys(N-tetradecanoyl)

<220>
<221> MOD_RES
<222> (29)..(29)
<223> Aib

<220>
<223> c-term amidation

<400> 468
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 469
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib

<220>
<223> c-term amidation

<400> 469
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 470
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-decanoyl)

<220>
<221> MOD_RES
<222> (29)..(29)
<223> Aib

<220>
<223> c-term amidation

<400> 470
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 471
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 471
His Ser Glu Gly Thr Phe Thr Ser Asp Val Ser Ser Tyr Leu Glu Gly
1               5                   10                  15

Gln Ala Lys Xaa Glu Phe Ile Ala Trp Leu Val Arg Xaa Arg
            20                  25                  30

<210> 472
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)

<223> Aib

<220>
<223> c-term amidation

<400> 472
His Ser Glu Gly Thr Phe Thr Ser Asp Val Ser Ser Tyr Leu Glu Gly
1               5                   10                  15

Gln Ala Lys Xaa Glu Phe Ile Ala Trp Leu Val Arg Xaa Arg
            20                  25                  30

<210> 473
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib

<220>
<223> c-term amidation

<400> 473
His Ser Glu Gly Thr Phe Thr Ser Asp Val Ser Ser Tyr Leu Glu Gly
1               5                   10                  15

Gln Ala Lys Xaa Glu Phe Ile Ala Trp Leu Val Arg Xaa Arg
            20                  25                  30

<210> 474
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 474
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 475
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)
<223> Aib

<220>
<223> c-term amidation

<400> 475
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 476
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib

<220>
<223> c-term amidation

<400> 476
His Ser Glu Gly Thr Phe Thr Ser Asp Val Ser Ser Tyr Leu Glu Gly

1               5                   10                  15

Gln Ala Arg Xaa Glu Phe Ile Ala Trp Leu Val Arg Xaa Arg
            20                  25                  30

<210> 477
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-decanoyl)

<220>
<221> MOD_RES
<222> (29)..(29)
<223> Aib

<220>
<223> c-term amidation

<400> 477
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 478
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Beta-Ala

<220>
<223> c-term amidation

<400> 478
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 479
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)
<223> Beta-Ala

<220>
<223> c-term amidation

<400> 479
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 480
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Beta-Ala

<220>
<223> c-term amidation

<400> 480
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 481
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-decanoyl)

<220>
<221> MOD_RES
<222> (29)..(29)
<223> Beta-Ala


<220>
<223> c-term amidation

<400> 481
His Ser Glu Gly Thr Phe Thr Ser Asp Val Ser Ser Tyr Leu Glu Gly
1               5                   10                  15


Gln Ala Ala Xaa Glu Phe Ile Ala Trp Leu Val Arg Xaa Arg
            20                  25                  30


<210> 482
<211> 30
<212> PRT
<213> Artificial Sequence

<220>
<223> Description of Artificial Sequence: Synthetic modified hGLP-1
      peptide



<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 482
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Xaa Xaa Arg
            20                  25                  30


<210> 483
<211> 30
<212> PRT

<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 483
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Xaa Xaa Arg
            20                  25                  30

<210> 484
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 484
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Xaa Xaa Arg
            20                  25                  30

<210> 485
<211> 30
<212> PRT
<213> Artificial Sequence

<220>

<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Aib


<220>
<223> c-term amidation

<400> 485
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 486
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib


<220>
<223> c-term amidation

<400> 486
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 487
<211> 30
<212> PRT
<213> Artificial Sequence

<220>
<223> Description of Artificial Sequence: Synthetic modified hGLP-1
      peptide

<220>

<221> MOD_RES
<222> (28)..(28)
<223> Lys(N-decanoyl)

<220>
<221> MOD_RES
<222> (29)..(29)
<223> Aib


<220>
<223> c-term amidation

<400> 487
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 488
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Aib


<220>
<223> c-term amidation

<400> 488
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 489
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Aib


<220>
<223> c-term amidation

<400> 489
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30



<210> 490
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib


<220>
<223> c-term amidation

<400> 490
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 491
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-decanoyl)

<220>
<221> MOD_RES
<222> (29)..(29)

<223> Aib

<220>
<223> c-term amidation

<400> 491
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 492
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 492
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 493
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 493
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 494
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 494
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 495
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 495
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 496
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> C-term amidation

<400> 496
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 497
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> C-term amidation

<400> 497
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 498
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 498
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 499
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 499
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 500
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 500
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 501
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 501
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 502
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 502
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 503
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-octanoyl)

<220>
<223> c-term amidation

<400> 503
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 504
<211> 32

<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-decanoyl)

<220>
<223> c-term amidation

<400> 504
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 505
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-tetradecanoyl)

<220>
<223> c-term amidation

<400> 505
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 506
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-hexadecanoyl)

<220>
<223> C-term amidation

<400> 506
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 507
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-octanoyl)

<220>
<223> C-term amidation

<400> 507
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 508
<211> 32
<212> PRT

<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-decanoyl)

<220>
<223> c-term amidation

<400> 508
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 509
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-tetradecanoyl)

<220>
<223> c-term amidation

<400> 509
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 510
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-hexadecanoyl)

<220>
<223> c-term amidation

<400> 510
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Arg Xaa Arg Xaa Xaa
            20                  25                  30


<210> 511
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-octanoyl))

<220>
<223> c-term amidation

<400> 511
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 512
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-decanoyl)

<220>
<223> c-term amidation

<400> 512
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 513
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-hexadecanoyl)

<220>
<223> c-term amidation

<400> 513
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 514
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 514
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 515
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 515
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 516
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 516
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 517
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 517
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 518

<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 518
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 519
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 519
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 520
<211> 30
<212> PRT
<213> Artificial Sequence

<220>
<223> Description of Artificial Sequence: Synthetic modified hGLP-1

peptide

<220>
<221> MOD_RES
<222> (29)..(29)
<223> Aib

<220>
<221> MOD_RES
<222> (30)..(30)
<223> Lys(N-decanoyl)

<220>
<223> c-term amidation

<400> 520
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 521
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 521
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Xaa Xaa Arg
            20                  25                  30

<210> 522
<211> 30
<212> PRT
<213> Artificial Sequence

<220>
<223> Description of Artificial Sequence: Synthetic modified hGLP-1
      peptide

<220>
<221> MOD_RES

<222> (28)..(28)
<223> Lys(N-tetradecanoyl)

<220>
<221> MOD_RES
<222> (29)..(29)
<223> Beta-Ala


<220>
<223> c-term amidation

<400> 522
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Xaa Xaa Arg
            20                  25                  30


<210> 523
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Beta-Ala


<220>
<223> c-term amidation

<400> 523
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Xaa Xaa Arg
            20                  25                  30


<210> 524
<211> 30
<212> PRT
<213> Artificial Sequence

<220>
<223> Description of Artificial Sequence: Synthetic modified hGLP-1
      peptide


<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 524
His Ser Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 525
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 525
His Ser Glu Gly Thr Phe Thr Ser Asp Val Ser Ser Tyr Leu Glu Gly
1               5                   10                  15

Glu Ala Ala Lys Glu Phe Ile Ala Trp Leu Val Xaa Xaa Arg
            20                  25                  30

<210> 526
<211> 30
<212> PRT
<213> Artificial Sequence

<220>
<223> Description of Artificial Sequence: Synthetic modified hGLP-1
      peptide

<220>
<221> MOD_RES
<222> (26)..(26)

<223> A6c

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 526
His Ser Glu Gly Thr Phe Thr Ser Asp Val Ser Ser Tyr Leu Glu Gly
1               5                   10                  15

Glu Ala Ala Lys Glu Phe Ile Ala Trp Xaa Val Xaa Xaa Arg
            20                  25                  30

<210> 527
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 527
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 528
<211> 30
<212> PRT
<213> Artificial Sequence

<220>
<223> Description of Artificial Sequence: Synthetic modified hGLP-1
      peptide

<220>
<221> MOD_RES
<222> (28)..(28)

<223> Lys(N-tetradecanoyl)

<220>
<221> MOD_RES
<222> (29)..(29)
<223> Beta-Ala


<220>
<223> c-term amidation

<400> 528
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 529
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Beta-Ala


<220>
<223> c-term amidation

<400> 529
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 530
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-decanoyl)

<220>
<221> MOD_RES

<222> (29)..(29)
<223> Beta-Ala


<220>
<223> c-term amidation

<400> 530
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 531
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Beta-Ala


<220>
<223> c-term amidation

<400> 531
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 532
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Beta-Ala

<220>
<223> c-term amidation

<400> 532
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 533
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Beta-Ala

<220>
<223> c-term amidation

<400> 533
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 534
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-decanoyl)

<220>
<221> MOD_RES
<222> (29)..(29)
<223> Beta-Ala

<220>
<223> c-term amidation

<400> 534

His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 535
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Beta-Ala

<220>
<223> c-term amidation

<400> 535
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 536
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Beta-Ala

<220>
<223> c-term amidation

<400> 536
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 537
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Beta-Ala

<220>
<223> c-term amidation

<400> 537
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Xaa Xaa Arg
            20                  25                  30

<210> 538
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 538
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 539
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> C-term amidation

<400> 539
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 540
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-hexadecanoyl)

<220>
<223> C-term amidation

<400> 540
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 541
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> C-term amidation

<400> 541
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 542
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> C-term amidation

<400> 542
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 543
<211> 30
<212> PRT
<213> Artificial Sequence

<220>
<223> Description of Artificial Sequence: Synthetic modified hGLP-1
      peptide

<220>

<221> MOD_RES
<222> (29)..(29)
<223> Beta-Ala

<220>
<221> MOD_RES
<222> (30)..(30)
<223> Lys(N-hexadecanoyl)

<220>
<223> c-term amidation

<400> 543
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Xaa
            20                  25                  30

<210> 544
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 544
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 545
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES

<222> (30)..(30)
<223> Lys(N-tetradecanoyl)

<220>
<223> c-term amidation

<400> 545
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 546
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-hexadecanoyl)

<220>
<223> c-term amidation

<400> 546
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 547
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-decanoyl)

<220>
<223> c-term amidation

<400> 547
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 548
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 548
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 549
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> c-term amidation

<400> 549
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa

20                  25                  30

<210> 550
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-hexadecanoyl)

<220>
<223> c-term amidation

<400> 550
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 551
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-octanoyl)

<220>
<223> c-term amidation

<400> 551
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 552
<211> 30
<212> PRT

<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-tetradecanoyl)

<220>
<223> c-term amidation

<400> 552
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Arg Xaa Xaa
            20                  25                  30


<210> 553
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-hexadecanoyl)

<220>
<223> c-term amidation

<400> 553
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Arg Xaa Xaa
            20                  25                  30


<210> 554
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (30)..(30)
<223> Lys(N-decanoyl)

<220>
<223> c-term amidation

<400> 554
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 555
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (26)..(26)
<223> A6c

<220>
<221> MOD_RES
<222> (29)..(29)
<223> Aib

<220>
<223> c-term amidation

<400> 555
His Ser Glu Gly Thr Phe Thr Ser Asp Val Ser Ser Tyr Leu Glu Gly
1               5                   10                  15

Gln Ala Ala Xaa Glu Phe Ile Ala Trp Xaa Val Arg Xaa Arg
            20                  25                  30

<210> 556
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (26)..(26)
<223> A6c

<220>
<221> MOD_RES
<222> (29)..(29)
<223> Aib


<220>
<223> c-term amidation

<400> 556
His Ser Glu Gly Thr Phe Thr Ser Asp Val Ser Ser Tyr Leu Glu Gly
1               5                   10                  15


Gln Ala Ala Xaa Glu Phe Ile Ala Trp Xaa Val Arg Xaa Arg
            20                  25                  30


<210> 557
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (26)..(26)
<223> A6c

<220>
<221> MOD_RES
<222> (29)..(29)
<223> Aib


<220>
<223> c-term amidation

<400> 557
His Ser Glu Gly Thr Phe Thr Ser Asp Val Ser Ser Tyr Leu Glu Gly
1               5                   10                  15


Gln Ala Ala Xaa Glu Phe Ile Ala Trp Xaa Val Arg Xaa Arg
            20                  25                  30


<210> 558
<211> 30

<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 558
His Ser Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 559
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 559
His Ser Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 560
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 560
His Ser Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 561
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 561
His Ser Glu Gly Thr Phe Thr Ser Asp Val Ser Ser Tyr Leu Glu Gly
1               5                   10                  15

Gln Ala Ala Lys Glu Phe Ile Ala Trp Xaa Val Xaa Xaa Arg
            20                  25                  30

<210> 562
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 562
His Ser Glu Gly Thr Phe Thr Ser Asp Val Ser Ser Tyr Leu Glu Gly
1               5                   10                  15

Gln Ala Ala Arg Glu Phe Ile Ala Trp Xaa Val Xaa Xaa Arg
            20                  25                  30

<210> 563
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)


<220>
<223> c-term amidation

<400> 563
His Ser Glu Gly Thr Phe Thr Ser Asp Val Ser Ser Tyr Leu Glu Gly
1               5                   10                  15


Gln Ala Ala Lys Glu Phe Ile Ala Trp Xaa Val Lys Xaa Xaa
            20                  25                  30


<210> 564
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib


<220>
<221> MOD_RES
<222> (30)..(30)
<223> Lys(N-tetradecanoyl)

<220>
<223> c-term amidation

<400> 564
His Ser Glu Gly Thr Phe Thr Ser Asp Val Ser Ser Tyr Leu Glu Gly
1               5                   10                  15


Gln Ala Ala Lys Glu Phe Ile Ala Trp Xaa Val Lys Xaa Xaa
            20                  25                  30


<210> 565
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib


<220>
<221> MOD_RES
<222> (30)..(30)
<223> Lys(N-hexadecanoyl)

<220>
<223> c-term amidation

<400> 565
His Ser Glu Gly Thr Phe Thr Ser Asp Val Ser Ser Tyr Leu Glu Gly
1               5                   10                  15


Gln Ala Ala Lys Glu Phe Ile Ala Trp Xaa Val Lys Xaa Xaa
            20                  25                  30


<210> 566
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib


<220>
<221> MOD_RES
<222> (30)..(30)
<223> Lys(N-octanoyl)

<220>
<223> c-term amidation

<400> 566
His Ser Glu Gly Thr Phe Thr Ser Asp Val Ser Ser Tyr Leu Glu Gly
1               5                   10                  15


Gln Ala Ala Arg Glu Phe Ile Ala Trp Xaa Val Lys Xaa Xaa
            20                  25                  30


<210> 567
<211> 30
<212> PRT
<213> Artificial Sequence

<220>
<223> Description of Artificial Sequence: Synthetic modified hGLP-1

peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 567
His Ser Glu Gly Thr Phe Thr Ser Asp Val Ser Ser Tyr Leu Glu Gly
1               5                   10                  15

Gln Ala Ala Arg Glu Phe Ile Ala Trp Xaa Val Lys Xaa Xaa
            20                  25                  30

<210> 568
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 568
His Ser Glu Gly Thr Phe Thr Ser Asp Val Ser Ser Tyr Leu Glu Gly
1               5                   10                  15

Gln Ala Ala Arg Glu Phe Ile Ala Trp Xaa Val Lys Xaa Xaa
            20                  25                  30

<210> 569
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib


<220>
<221> MOD_RES
<222> (30)..(30)
<223> Lys(N-octanoyl)

<220>
<223> c-term amidation

<400> 569
His Ser Glu Gly Thr Phe Thr Ser Asp Val Ser Ser Tyr Leu Glu Gly
1               5                   10                  15


Gln Ala Ala Arg Glu Phe Ile Ala Trp Xaa Val Arg Xaa Xaa
            20                  25                  30


<210> 570
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib


<220>
<221> MOD_RES
<222> (30)..(30)
<223> Lys(N-decanoyl)

<220>
<223> c-term amidation

<400> 570
His Ser Glu Gly Thr Phe Thr Ser Asp Val Ser Ser Tyr Leu Glu Gly
1               5                   10                  15

Gln Ala Ala Arg Glu Phe Ile Ala Trp Xaa Val Arg Xaa Xaa
            20                  25                  30

<210> 571
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> C-term amidation

<400> 571
His Ser Glu Gly Thr Phe Thr Ser Asp Val Ser Ser Tyr Leu Glu Gly
1               5                   10                  15

Gln Ala Ala Arg Glu Phe Ile Ala Trp Xaa Val Arg Xaa Xaa
            20                  25                  30

<210> 572
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 572
His Ser Glu Gly Thr Phe Thr Ser Asp Val Ser Ser Tyr Leu Glu Gly
1               5                   10                  15

Gln Ala Ala Arg Glu Phe Ile Ala Trp Xaa Val Arg Xaa Xaa
            20                  25                  30

<210> 573
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 573
His Ser Glu Gly Thr Phe Thr Ser Asp Val Ser Ser Tyr Leu Glu Gly
1               5                   10                  15

Gln Xaa Ala Xaa Glu Phe Ile Ala Trp Leu Val Arg Xaa Arg
            20                  25                  30

<210> 574
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (20)..(20)
<223> Lys(N-tetradecanoyl)

<220>
<221> MOD_RES
<222> (29)..(29)
<223> Aib


<220>
<223> c-term amidation

<400> 574
His Ser Glu Gly Thr Phe Thr Ser Asp Val Ser Ser Tyr Leu Glu Gly
1               5                   10                  15


Gln Xaa Ala Xaa Glu Phe Ile Ala Trp Leu Val Arg Xaa Arg
            20                  25                  30


<210> 575
<211> 30
<212> PRT
<213> Artificial Sequence

<220>
<223> Description of Artificial Sequence: Synthetic modified hGLP-1
      peptide


<220>
<221> MOD_RES
<222> (18)..(18)
<223> Aib

<220>
<221> MOD_RES
<222> (20)..(20)
<223> Lys(N-hexadecanoyl)

<220>
<221> MOD_RES
<222> (29)..(29)
<223> Aib


<220>
<223> c-term amidation

<400> 575
His Ser Glu Gly Thr Phe Thr Ser Asp Val Ser Ser Tyr Leu Glu Gly
1               5                   10                  15


Gln Xaa Ala Xaa Glu Phe Ile Ala Trp Leu Val Arg Xaa Arg
            20                  25                  30


<210> 576
<211> 30
<212> PRT
<213> Artificial Sequence

<220>
<223> Description of Artificial Sequence: Synthetic modified hGLP-1
      peptide


<220>

<221> MOD_RES
<222> (18)..(18)
<223> Aib

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 576
His Ser Glu Gly Thr Phe Thr Ser Asp Val Ser Ser Tyr Leu Glu Gly
1               5                   10                  15

Gln Xaa Ala Arg Glu Phe Ile Ala Trp Leu Val Xaa Xaa Arg
            20                  25                  30

<210> 577
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 577
His Ser Glu Gly Thr Phe Thr Ser Asp Val Ser Ser Tyr Leu Glu Gly
1               5                   10                  15

Gln Xaa Ala Arg Glu Phe Ile Ala Trp Leu Val Xaa Xaa Arg
            20                  25                  30

<210> 578
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 578
His Ser Glu Gly Thr Phe Thr Ser Asp Val Ser Ser Tyr Leu Glu Gly
1               5                   10                  15

Gln Xaa Ala Arg Glu Phe Ile Ala Trp Leu Val Xaa Xaa Arg
            20                  25                  30

<210> 579
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 579
His Ser Glu Gly Thr Phe Thr Ser Asp Val Ser Ser Tyr Leu Glu Gly
1               5                   10                  15

Gln Xaa Ala Arg Glu Phe Ile Ala Trp Leu Val Arg Xaa Xaa
            20                  25                  30

<210> 580
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 580
His Ser Glu Gly Thr Phe Thr Ser Asp Val Ser Ser Tyr Leu Glu Gly
1               5                   10                  15

Gln Xaa Ala Arg Glu Phe Ile Ala Trp Leu Val Arg Xaa Xaa
            20                  25                  30

<210> 581
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 581
His Ser Glu Gly Thr Phe Thr Ser Asp Val Ser Ser Tyr Leu Glu Gly
1               5                   10                  15

Gln Xaa Ala Arg Glu Phe Ile Ala Trp Leu Val Arg Xaa Xaa
            20                  25                  30

<210> 582
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 582
His Ser Glu Gly Thr Phe Thr Ser Asp Val Ser Ser Tyr Leu Glu Gly
1               5                   10                  15

Glu Xaa Ala Lys Glu Phe Ile Ala Trp Xaa Val Xaa Xaa Arg
            20                  25                  30

<210> 583
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (29)..(29)
<223> Aib

<220>
<223> c-term amidation

<400> 583
His Ser Glu Gly Thr Phe Thr Ser Asp Val Ser Ser Tyr Leu Glu Gly
1               5                   10                  15

Glu Ala Ala Xaa Glu Phe Ile Ala Trp Leu Val Arg Xaa Arg
            20                  25                  30

<210> 584
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (29)..(29)
<223> Aib

<220>
<223> c-term amidation

<400> 584
His Ser Glu Gly Thr Phe Thr Ser Asp Val Ser Ser Tyr Leu Glu Gly
1               5                   10                  15

Glu Ala Ala Xaa Glu Phe Ile Ala Trp Leu Val Arg Xaa Arg
            20                  25                  30

<210> 585
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (29)..(29)
<223> Aib

<220>
<223> c-term amidation

<400> 585
His Ser Glu Gly Thr Phe Thr Ser Asp Val Ser Ser Tyr Leu Glu Gly
1               5                   10                  15

Glu Ala Ala Xaa Glu Phe Ile Ala Trp Leu Val Arg Xaa Arg
            20                  25                  30

<210> 586
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 586
His Ser Glu Gly Thr Phe Thr Ser Asp Val Ser Ser Tyr Leu Glu Gly
1               5                   10                  15

Glu Ala Ala Lys Glu Phe Ile Ala Trp Leu Val Xaa Xaa Arg
            20                  25                  30

<210> 587
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (28)..(28)
<223> Lys(N-octanoyl)

<220>
<221> MOD_RES
<222> (29)..(29)

<223> Aib

<220>
<223> c-term amidation

<400> 587
His Ser Glu Gly Thr Phe Thr Ser Asp Val Ser Ser Tyr Leu Glu Gly
1               5                   10                  15

Glu Ala Ala Lys Glu Phe Ile Ala Trp Xaa Val Xaa Xaa Arg
            20                  25                  30

<210> 588
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 588
His Ser Glu Gly Thr Phe Thr Ser Asp Val Ser Ser Tyr Leu Glu Gly
1               5                   10                  15

Glu Ala Ala Arg Glu Phe Ile Ala Trp Leu Val Xaa Xaa Arg
            20                  25                  30

<210> 589
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 589
His Ser Glu Gly Thr Phe Thr Ser Asp Val Ser Ser Tyr Leu Glu Gly
1               5                   10                  15

Glu Ala Ala Arg Glu Phe Ile Ala Trp Leu Val Xaa Xaa Arg
            20                  25                  30

<210> 590
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 590
His Ser Glu Gly Thr Phe Thr Ser Asp Val Ser Ser Tyr Leu Glu Gly
1               5                   10                  15

Glu Ala Ala Arg Glu Phe Ile Ala Trp Leu Val Xaa Xaa Arg
            20                  25                  30

<210> 591
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 591
His Ser Glu Gly Thr Phe Thr Ser Asp Val Ser Ser Tyr Leu Glu Gly

1               5                   10                  15

Glu Ala Ala Lys Glu Phe Ile Ala Trp Leu Val Lys Xaa Xaa
            20                  25                  30

<210> 592
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 592
His Ser Glu Gly Thr Phe Thr Ser Asp Val Ser Ser Tyr Leu Glu Gly
1               5                   10                  15

Glu Ala Ala Lys Glu Phe Ile Ala Trp Leu Val Lys Xaa Xaa
            20                  25                  30

<210> 593
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 593
His Ser Glu Gly Thr Phe Thr Ser Asp Val Ser Ser Tyr Leu Glu Gly
1               5                   10                  15

Glu Ala Ala Lys Glu Phe Ile Ala Trp Leu Val Lys Xaa Xaa
            20                  25                  30

<210> 594
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 594
His Ser Glu Gly Thr Phe Thr Ser Asp Val Ser Ser Tyr Leu Glu Gly
1               5                   10                  15

Glu Ala Ala Arg Glu Phe Ile Ala Trp Leu Val Arg Xaa Xaa
            20                  25                  30

<210> 595
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 595
His Ser Glu Gly Thr Phe Thr Ser Asp Val Ser Ser Tyr Leu Glu Gly
1               5                   10                  15

Glu Ala Ala Arg Glu Phe Ile Ala Trp Leu Val Arg Xaa Xaa
            20                  25                  30

<210> 596
<211> 30
<212> PRT

<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 596
His Ser Glu Gly Thr Phe Thr Ser Asp Val Ser Ser Tyr Leu Glu Gly
1               5                   10                  15

Glu Ala Ala Arg Glu Phe Ile Ala Trp Leu Val Arg Xaa Xaa
            20                  25                  30

<210> 597
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanoyl)

<220>
<221> MOD_RES
<222> (24)..(24)
<223> Aib

<220>
<221> MOD_RES
<222> (29)..(29)
<223> Aib

<220>
<223> c-term amidation

<400> 597
His Ser Glu Gly Thr Phe Thr Ser Asp Val Ser Ser Tyr Leu Glu Gly
1               5                   10                  15

Gln Ala Ala Xaa Glu Phe Ile Xaa Trp Leu Val Arg Xaa Arg
            20                  25                  30

<210> 598
<211> 30

<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-tetradecanoyl)

<220>
<221> MOD_RES
<222> (24)..(24)
<223> Aib

<220>
<221> MOD_RES
<222> (29)..(29)
<223> Aib


<220>
<223> c-term amidation

<400> 598
His Ser Glu Gly Thr Phe Thr Ser Asp Val Ser Ser Tyr Leu Glu Gly
1               5                   10                  15


Gln Ala Ala Xaa Glu Phe Ile Xaa Trp Leu Val Arg Xaa Arg
            20                  25                  30


<210> 599
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanoyl)

<220>
<221> MOD_RES
<222> (24)..(24)
<223> Aib

<220>
<221> MOD_RES
<222> (29)..(29)
<223> Aib

<220>
<223> c-term amidation

<400> 599
His Ser Glu Gly Thr Phe Thr Ser Asp Val Ser Ser Tyr Leu Glu Gly
1               5                   10                  15


Gln Ala Ala Xaa Glu Phe Ile Xaa Trp Leu Val Arg Xaa Arg

20                  25                  30

<210> 600
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (28)..(28)
<223> Lys(N-octanoyl)

<220>
<221> MOD_RES
<222> (29)..(29)
<223> Aib

<220>
<223> c-term amidation

<400> 600
His Ser Glu Gly Thr Phe Thr Ser Asp Val Ser Ser Tyr Leu Glu Gly
1               5                   10                  15

Gln Ala Ala Arg Glu Phe Ile Xaa Trp Leu Val Xaa Xaa Arg
            20                  25                  30

<210> 601
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (28)..(28)
<223> Lys(N-tetradecanoyl)

<220>
<221> MOD_RES
<222> (29)..(29)
<223> Aib

<220>
<223> c-term amidation

<400> 601
His Ser Glu Gly Thr Phe Thr Ser Asp Val Ser Ser Tyr Leu Glu Gly
1               5                   10                  15

Gln Ala Ala Arg Glu Phe Ile Xaa Trp Leu Val Xaa Xaa Arg
            20                  25                  30

<210> 602
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (28)..(28)
<223> Lys(N-hexadecanoyl)

<220>
<221> MOD_RES
<222> (29)..(29)
<223> Aib

<220>
<223> c-term amidation

<400> 602
His Ser Glu Gly Thr Phe Thr Ser Asp Val Ser Ser Tyr Leu Glu Gly
1               5                   10                  15

Gln Ala Ala Arg Glu Phe Ile Xaa Trp Leu Val Xaa Xaa Arg
            20                  25                  30

<210> 603
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Aib

<220>
<221> MOD_RES

<222> (30)..(30)
<223> Lys(N-octanoyl)

<220>
<223> c-term amidation

<400> 603
His Ser Glu Gly Thr Phe Thr Ser Asp Val Ser Ser Tyr Leu Glu Gly
1               5                   10                  15

Gln Ala Ala Arg Glu Phe Ile Xaa Trp Leu Val Arg Xaa Xaa
            20                  25                  30

<210> 604
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 604
His Ser Glu Gly Thr Phe Thr Ser Asp Val Ser Ser Tyr Leu Glu Gly
1               5                   10                  15

Gln Ala Ala Arg Glu Phe Ile Xaa Trp Leu Val Arg Xaa Xaa
            20                  25                  30

<210> 605
<211> 30
<212> PRT
<213> Artificial Sequence

<220>
<223> Description of Artificial Sequence: Synthetic modified hGLP-1
      peptide

<220>
<221> MOD_RES
<222> (24)..(24)
<223> Aib

<220>
<221> MOD_RES
<222> (29)..(29)

<223> Aib

<220>
<221> MOD_RES
<222> (30)..(30)
<223> Lys(N-hexadecanoyl)

<220>
<223> c-term amidation

<400> 605
His Ser Glu Gly Thr Phe Thr Ser Asp Val Ser Ser Tyr Leu Glu Gly
1               5                   10                  15

Gln Ala Ala Arg Glu Phe Ile Xaa Trp Leu Val Arg Xaa Xaa
            20                  25                  30

<210> 606
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 606
His Ser Glu Gly Thr Phe Thr Ser Asp Val Ser Ser Tyr Leu Glu Gly
1               5                   10                  15

Glu Ala Ala Lys Glu Phe Ile Ala Trp Xaa Val Lys Xaa Xaa
            20                  25                  30

<210> 607
<211> 30
<212> PRT
<213> Artificial Sequence

<220>
<223> Description of Artificial Sequence: Synthetic modified hGLP-1
      peptide

<220>
<221> MOD_RES
<222> (26)..(26)

<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 607
His Ser Glu Gly Thr Phe Thr Ser Asp Val Ser Ser Tyr Leu Glu Gly
1               5                   10                  15

Glu Ala Ala Lys Glu Phe Ile Ala Trp Xaa Val Lys Xaa Xaa
            20                  25                  30

<210> 608
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 608
His Ser Glu Gly Thr Phe Thr Ser Asp Val Ser Ser Tyr Leu Glu Gly
1               5                   10                  15

Glu Ala Ala Lys Glu Phe Ile Ala Trp Xaa Val Lys Xaa Xaa
            20                  25                  30

<210> 609
<211> 30
<212> PRT
<213> Artificial Sequence

<220>
<223> Description of Artificial Sequence: Synthetic modified hGLP-1

peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 609
His Ser Glu Gly Thr Phe Thr Ser Asp Val Ser Ser Tyr Leu Glu Gly
1               5                   10                  15

Glu Ala Ala Arg Glu Phe Ile Ala Trp Xaa Val Arg Xaa Xaa
            20                  25                  30

<210> 610
<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 610
His Ser Glu Gly Thr Phe Thr Ser Asp Val Ser Ser Tyr Leu Glu Gly
1               5                   10                  15

Glu Ala Ala Arg Glu Phe Ile Ala Trp Xaa Val Arg Xaa Xaa
            20                  25                  30

<210> 611

<211> 30
<212> PRT
<213> Artificial Sequence

<220>
<223> Description of Artificial Sequence: Synthetic modified hGLP-1
      peptide

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 611
His Ser Glu Gly Thr Phe Thr Ser Asp Val Ser Ser Tyr Leu Glu Gly
1               5                   10                  15

Glu Ala Ala Arg Glu Phe Ile Ala Trp Xaa Val Arg Xaa Xaa
            20                  25                  30

<210> 612
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 612

His Ser Glu Gly Thr Phe Thr Ser Asp Val Ser Ser Tyr Leu Glu Gly
1               5                   10                  15

Glu Xaa Ala Arg Glu Phe Ile Ala Trp Xaa Val Arg Xaa Xaa
            20                  25                  30

<210> 613
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> c-term amidation

<400> 613
His Ser Glu Gly Thr Phe Thr Ser Asp Val Ser Ser Tyr Leu Glu Gly
1               5                   10                  15

Glu Xaa Ala Arg Glu Phe Ile Ala Trp Xaa Val Arg Xaa Xaa
            20                  25                  30

<210> 614
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 614
His Ser Glu Gly Thr Phe Thr Ser Asp Val Ser Ser Tyr Leu Glu Gly
1               5                   10                  15

Glu Xaa Ala Arg Glu Phe Ile Ala Trp Xaa Val Arg Xaa Xaa
            20                  25                  30

<210> 615
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (24)..(24)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-octanoyl)

<220>
<223> c-term amidation

<400> 615
His Ser Glu Gly Thr Phe Thr Ser Asp Val Ser Ser Tyr Leu Glu Gly
1               5                   10                  15

Glu Xaa Ala Arg Glu Phe Ile Xaa Trp Xaa Val Arg Xaa Xaa
            20                  25                  30

<210> 616
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (24)..(24)
<223> Aib

<220>
<221> MOD_RES
<222> (26)..(26)
<223> A6c

<220>
<221> MOD_RES
<222> (29)..(29)
<223> Aib

<220>
<221> MOD_RES
<222> (30)..(30)
<223> Lys(N-tetradecanoyl)

<220>
<223> C-term amidation

<400> 616
His Ser Glu Gly Thr Phe Thr Ser Asp Val Ser Ser Tyr Leu Glu Gly
1               5                   10                  15


Glu Xaa Ala Arg Glu Phe Ile Xaa Trp Xaa Val Arg Xaa Xaa
            20                  25                  30


<210> 617
<211> 30
<212> PRT
<213> Artificial Sequence

<220>
<223> Description of Artificial Sequence: Synthetic modified hGLP-1
      peptide

<220>
<221> MOD_RES
<222> (18)..(18)
<223> Aib

<220>
<221> MOD_RES
<222> (24)..(24)
<223> Aib

<220>
<221> MOD_RES

<222> (26)..(26)
<223> A6c

<220>
<221> MOD_RES
<222> (29)..(29)
<223> Aib

<220>
<221> MOD_RES
<222> (30)..(30)
<223> Lys(N-hexadecanoyl)

<220>
<223> c-term amidation

<400> 617
His Ser Glu Gly Thr Phe Thr Ser Asp Val Ser Ser Tyr Leu Glu Gly
1               5                   10                  15

Glu Xaa Ala Arg Glu Phe Ile Xaa Trp Xaa Val Arg Xaa Xaa
            20                  25                  30

<210> 618
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-HEPES-His

<220>
<221> MOD_RES
<222> (29)..(29)
<223> Beta-Ala

<220>
<223> c-term amidation

<400> 618
Xaa Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 619
<211> 30
<212> PRT
<213> Artificial Sequence

<220>
<223> Description of Artificial Sequence: Synthetic modified hGLP-1
      peptide

<220>

<221> MOD_RES
<222> (1)..(1)
<223> N-HEPA-His

<220>
<221> MOD_RES
<222> (29)..(29)
<223> Beta-Ala

<220>
<223> c-term amidation

<400> 619
Xaa Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 620
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Aib

<220>
<223> c-term amidation

<400> 620
Xaa Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 621
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Beta-Ala

<220>
<223> c-term amidation

<400> 621
Xaa Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 622
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Aib

<220>
<223> c-term amidation

<400> 622
Xaa Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 623
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Beta-Ala

<220>
<223> c-term amidation

<400> 623
Xaa Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 624
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Aib

<220>
<223> c-term amidation

<400> 624
Xaa Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg
            20                  25                  30

<210> 625
<211> 30
<212> PRT
<213> Artificial Sequence

<220>
<223> Description of Artificial Sequence: Synthetic modified hGLP-1
      peptide

<220>
<221> MOD_RES
<222> (1)..(1)
<223> N-tetradecanoyl-His

<220>
<221> MOD_RES
<222> (29)..(29)
<223> Beta-Ala

<220>
<223> c-term amidation

<400> 625
Xaa Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg
            20                  25                  30

<210> 626
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-octanesulfonyl)

<220>
<221> MOD_RES
<222> (29)..(29)
<223> Aib

<220>
<223> c-term amidation

<400> 626
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 627
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-dodecanesulfonyl)

<220>
<221> MOD_RES
<222> (29)..(29)
<223> Aib

<220>
<223> c-term amidation

<400> 627
His Ser Glu Gly Thr Phe Thr Ser Asp Val Ser Ser Tyr Leu Glu Gly

1                   5                   10                  15

Gln Ala Ala Xaa Glu Phe Ile Ala Trp Leu Val Arg Xaa Arg
            20                  25                  30

<210> 628
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-hexadecanesulfonyl)

<220>
<221> MOD_RES
<222> (29)..(29)
<223> Aib

<220>
<223> c-term amidation

<400> 628
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 629
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-octanesulfonyl)

<220>
<221> MOD_RES
<222> (29)..(29)
<223> Aib

<220>
<223> c-term amidation

<400> 629
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg

20                  25                  30


<210> 630
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-dodecanesulfonyl)

<220>
<221> MOD_RES
<222> (29)..(29)
<223> Aib


<220>
<223> c-term amidation

<400> 630
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 631
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-hexadecanesulfonyl)

<220>
<221> MOD_RES
<222> (29)..(29)
<223> Aib


<220>
<223> c-term amidation

<400> 631
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 632

<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-octanesulfonyl)

<220>
<223> c-term amidation

<400> 632
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 633
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-hexadecanesulfonyl)

<220>
<223> c-term amidation

<400> 633
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 634
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-decylpiperazine))

<220>
<221> MOD_RES
<222> (29)..(29)
<223> Aib

<220>
<223> c-term amidation

<400> 634
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 635
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-dodecylpiperazine))

<220>
<221> MOD_RES
<222> (29)..(29)
<223> Aib

<220>
<223> c-term amidation

<400> 635
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 636
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-tetradecylpiperazine))

<220>
<221> MOD_RES
<222> (29)..(29)
<223> Aib


<220>
<223> c-term amidation

<400> 636
His Ser Glu Gly Thr Phe Thr Ser Asp Val Ser Ser Tyr Leu Glu Gly
1               5                   10                  15


Gln Ala Ala Xaa Glu Phe Ile Ala Trp Leu Val Arg Xaa Arg
            20                  25                  30


<210> 637
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-hexadecylpiperazine))

<220>
<221> MOD_RES
<222> (29)..(29)
<223> Aib


<220>
<223> c-term amidation

<400> 637
His Ser Glu Gly Thr Phe Thr Ser Asp Val Ser Ser Tyr Leu Glu Gly
1               5                   10                  15



Gln Ala Ala Xaa Glu Phe Ile Ala Trp Leu Val Arg Xaa Arg
            20                  25                  30


<210> 638
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-decylpiperazine))

<220>
<221> MOD_RES
<222> (29)..(29)
<223> Aib

<220>
<223> c-term amidation

<400> 638
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 639
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-dodecylpiperazine))

<220>
<221> MOD_RES
<222> (29)..(29)
<223> Aib

<220>
<223> c-term amidation

<400> 639
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 640
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-tetradecylpiperazine))

<220>
<221> MOD_RES
<222> (29)..(29)
<223> Aib


<220>
<223> c-term amidation

<400> 640
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30




<210> 641
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-hexadecylpiperazine))

<220>
<221> MOD_RES
<222> (29)..(29)
<223> Aib


<220>
<223> c-term amidation

<400> 641
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Xaa Xaa Arg
            20                  25                  30


<210> 642
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<221> MOD_RES

<222> (30)..(30)
<223> Asp(1-(4-decylpiperazine))


<220>
<223> c-term amidation

<400> 642
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Xaa
            20                  25                  30


<210> 643
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-dodecylpiperazine))

<220>
<223> c-term amidation

<400> 643
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Xaa
            20                  25                  30


<210> 644
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-hexadecylpiperazine))


<220>

<223> c-term amidation

<400> 644
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 645
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-decylpiperazine))

<220>
<223> c-term amidation

<400> 645
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 646
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-dodecylpiperazine))

<220>
<223> c-term amidation

<400> 646
His Ser Glu Gly Thr Phe Thr Ser Asp Val Ser Ser Tyr Leu Glu Gly

1               5                   10                  15

Gln Ala Ala Arg Glu Phe Ile Ala Trp Leu Val Arg Xaa Arg Gly Xaa
            20                  25                  30

<210> 647
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-tetradecylpiperazine))

<220>
<223> c-term amidation

<400> 647
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 648
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-hexadecylpiperazine))

<220>
<223> c-term amidation

<400> 648
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 649
<211> 32
<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-decylpiperazine)

<220>
<223> c-term amidation

<400> 649
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Arg Xaa Xaa
            20                  25                  30


<210> 650
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-dodecylpiperazine))

<220>
<223> c-term amidation

<400> 650
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 651
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-tetradecylpiperazine))

<220>
<223> c-term amidation

<400> 651
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 652
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-hexadecylpiperazine))

<220>
<223> c-term amidation

<400> 652
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 653
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-decylpiperazine))

<220>
<221> MOD_RES
<222> (29)..(29)
<223> Aib

<220>
<223> c-term amidation

<400> 653
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 654
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-dodecylpiperazine))

<220>
<221> MOD_RES
<222> (29)..(29)
<223> Aib

<220>
<223> c-term amidation

<400> 654

His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 655
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-tetradecylpiperazine))

<220>
<221> MOD_RES
<222> (29)..(29)
<223> Aib

<220>
<223> c-term amidation

<400> 655
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 656
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Asp(1-(4-hexadecylpiperazine))

<220>
<221> MOD_RES
<222> (29)..(29)
<223> Aib

<220>
<223> c-term amidation

<400> 656
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 657
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-decylpiperazine))

<220>
<221> MOD_RES
<222> (29)..(29)
<223> Aib

<220>
<223> c-term amidation

<400> 657
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 658
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-dodecylpiperazine)

<220>
<221> MOD_RES
<222> (29)..(29)
<223> Aib

<220>
<223> c-term amidation

<400> 658
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 659
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-tetradecylpiperazine))

<220>
<221> MOD_RES
<222> (29)..(29)
<223> Aib


<220>
<223> c-term amidation

<400> 659
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 660
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Asp(1-(4-hexadecylpiperazine))

<220>
<221> MOD_RES
<222> (29)..(29)
<223> Aib


<220>
<223> c-term amidation

<400> 660
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 661
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-decylpiperazine))

<220>
<223> c-term amidation

<400> 661
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 662
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-dodecylpiperazine))

<220>
<223> c-term amidation

<400> 662
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 663
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-tetradecylpiperazine))

<220>
<223> c-term amidation

<400> 663
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 664
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Asp(1-(4-hexadecylpiperazine))

<220>
<223> c-term amidation

<400> 664
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 665
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-decylpiperazine))

<220>
<223> c-term amidation

<400> 665
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 666
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-dodecylpiperazine))

<220>
<223> c-term amidation

<400> 666
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 667
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-tetradecylpiperazine))

<220>
<223> c-term amidation

<400> 667
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 668
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Asp(1-(4-hexadecylpiperazine))

<220>
<223> c-term amidation

<400> 668
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 669
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-decylpiperazine))

<220>
<223> c-term amidation

<400> 669
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 670
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-dodecylpiperazine))

<220>
<223> c-term amidation

<400> 670
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 671
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-tetradecylpiperazine))

<220>
<223> c-term amidation

<400> 671
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 672
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Asp(1-(4-hexadecylpiperazine))

<220>
<223> c-term amidation

<400> 672
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 673
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Glu(1-dodecylamino)

<220>
<223> c-term amidation

<400> 673
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 674
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Glu(1-dodecylamino)

<220>
<221> MOD_RES
<222> (29)..(29)
<223> Aib

<220>
<223> c-term amidation

<400> 674
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 675
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Glu(1-dodecylamino)

<220>
<221> MOD_RES
<222> (29)..(29)

<223> Aib

<220>
<223> c-term amidation

<400> 675
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 676
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Glu(1-dodecylamino)

<220>
<223> c-term amidation

<400> 676
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 677
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-decyl-1-piperazine)-acetyl))

<220>
<221> MOD_RES

<222> (29)..(29)
<223> Aib

<220>
<223> c-term amidation

<400> 677
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 678
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-dodecyl-1-piperazine)-acetyl))

<220>
<221> MOD_RES
<222> (29)..(29)
<223> Aib

<220>
<223> c-term amidation

<400> 678
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 679
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-tetradecyl-1-piperazine)-acetyl))

<220>
<221> MOD_RES
<222> (29)..(29)
<223> Aib

<220>
<223> c-term amidation

<400> 679
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 680
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-hexadecyl-1-piperazine)-acetyl))

<220>
<221> MOD_RES
<222> (29)..(29)
<223> Aib

<220>
<223> c-term amidation

<400> 680
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 681
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-decyl-1-piperazine)-acetyl))

<220>
<221> MOD_RES
<222> (29)..(29)
<223> Aib

<220>
<223> c-term amidation

<400> 681
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 682
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-dodecyl-1-piperazine)-acetyl))

<220>
<221> MOD_RES
<222> (29)..(29)
<223> Aib

<220>
<223> c-term amidation

<400> 682
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 683
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-tetradecyl-1-piperazine)-acetyl))

<220>
<221> MOD_RES
<222> (29)..(29)
<223> Aib

<220>
<223> c-term amidation

<400> 683
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 684
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-hexadecyl-1-piperazine)-acetyl))

<220>
<221> MOD_RES
<222> (29)..(29)
<223> Aib

<220>
<223> c-term amidation

<400> 684
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Xaa Xaa Arg
            20                  25                  30

<210> 685
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-decyl-1-piperazine)-acetyl))

<220>
<223> c-term amidation

<400> 685
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 686
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-dodecyl-1-piperazine)-acetyl))

<220>
<223> c-term amidation

<400> 686
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 687
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-hexadecyl-1-piperazine)-acetyl))

<220>
<223> c-term amidation

<400> 687
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 688
<211> 32
<212> PRT

<213> Artificial Sequence

<220>
<223> Description of Artificial Sequence: Synthetic modified hGLP-1
      peptide


<220>
<221> MOD_RES
<222> (29)..(29)
<223> Aib

<220>
<221> MOD_RES
<222> (32)..(32)
<223> Lys(N-(2-(4-decyl-1-piperazine)-acetyl))

<220>
<223> c-term amidation

<400> 688
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Arg Gly Xaa
            20                  25                  30


<210> 689
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-dodecyl-1-piperazine)-acetyl))

<220>
<223> c-term amidation

<400> 689
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Arg Gly Xaa
            20                  25                  30


<210> 690
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-tetradecyl-1-piperazine)-acetyl))

<220>
<223> c-term amidation

<400> 690
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 691
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-hexadecyl-1-piperazine)-acetyl))

<220>
<223> c-term amidation

<400> 691
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Gly Xaa
            20                  25                  30

<210> 692
<211> 32
<212> PRT
<213> Artificial Sequence

<220>
<223> Description of Artificial Sequence: Synthetic modified hGLP-1
      peptide

<220>
<221> MOD_RES
<222> (29)..(29)

<223> Aib

<220>
<221> MOD_RES
<222> (31)..(31)
<223> Aib

<220>
<221> MOD_RES
<222> (32)..(32)
<223> Lys(N-(2-(4-decyl-1-piperazine)-acetyl))

<220>
<223> c-term amidation

<400> 692
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 693
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-dodecyl-1-piperazine)-acetyl))

<220>
<223> c-term amidation

<400> 693
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 694
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-tetradecyl-1-piperazine)-acetyl))

<220>
<223> c-term amidation

<400> 694
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 695
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-hexadecyl-1-piperazine)-acetyl))

<220>
<223> c-term amidation

<400> 695
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 696
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-decyl-1-piperazine)-acetyl))

<220>
<221> MOD_RES
<222> (29)..(29)
<223> Aib

<220>
<223> c-term amidation

<400> 696
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 697
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-dodecyl-1-piperazine)-acetyl))

<220>
<221> MOD_RES
<222> (29)..(29)
<223> Aib

<220>
<223> c-term amidation

<400> 697
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 698
<211> 30
<212> PRT
<213> Artificial Sequence

<220>
<223> Description of Artificial Sequence: Synthetic modified hGLP-1

peptide

<220>
<221> MOD_RES
<222> (20)..(20)
<223> Lys(N-(2-(4-tetradecyl-1-piperazine)-acetyl))

<220>
<221> MOD_RES
<222> (29)..(29)
<223> Aib

<220>
<223> c-term amidation

<400> 698
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 699
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-(2-(4-hexadecyl-1-piperazine)-acetyl))

<220>
<221> MOD_RES
<222> (29)..(29)
<223> Aib

<220>
<223> c-term amidation

<400> 699
His Ser Glu Gly Thr Phe Thr Ser Asp Val Ser Ser Tyr Leu Glu Gly
1               5                   10                  15

Gln Ala Arg Xaa Glu Phe Ile Ala Trp Leu Val Arg Xaa Arg
            20                  25                  30

<210> 700
<211> 30
<212> PRT
<213> Artificial Sequence

<220>
<223> Description of Artificial Sequence: Synthetic modified hGLP-1
      peptide

<220>

<221> MOD_RES
<222> (28)..(28)
<223> Lys(N-(2-(4-decyl-1-piperazine)-acetyl))

<220>
<221> MOD_RES
<222> (29)..(29)
<223> Aib

<220>
<223> c-term amidation

<400> 700
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 701
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-dodecyl-1-piperazine)-acetyl))

<220>
<221> MOD_RES
<222> (29)..(29)
<223> Aib

<220>
<223> c-term amidation

<400> 701
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Xaa Xaa Arg
            20                  25                  30

<210> 702
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-tetradecyl-1-piperazine)-acetyl))

<220>

<221> MOD_RES
<222> (29)..(29)
<223> Aib


<220>
<223> c-term amidation

<400> 702
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 703
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Lys(N-(2-(4-hexadecyl-1-piperazine)-acetyl))

<220>
<221> MOD_RES
<222> (29)..(29)
<223> Aib


<220>
<223> c-term amidation

<400> 703
His Ser Glu Gly Thr Phe Thr Ser Asp Val Ser Ser Tyr Leu Glu Gly
1               5                   10                  15


Gln Ala Arg Arg Glu Phe Ile Ala Trp Leu Val Xaa Xaa Arg
            20                  25                  30


<210> 704
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<221> MOD_RES
<222> (30)..(30)
<223> Lys(N-(2-(4-decyl-1-piperazine)-acetyl))

<220>
<223> c-term amidation

<400> 704
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 705
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-dodecyl-1-piperazine)-acetyl))

<220>
<223> c-term amidation

<400> 705
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 706
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-tetradecyl-1-piperazine)-acetyl))

<220>
<223> c-term amidation

<400> 706
His Ser Glu Gly Thr Phe Thr Ser Asp Val Ser Ser Tyr Leu Glu Gly

1                   5                   10                  15

Gln Ala Arg Arg Glu Phe Ile Ala Trp Leu Val Arg Xaa Xaa
            20                  25                  30

<210> 707
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-(2-(4-hexadecyl-1-piperazine)-acetyl))

<220>
<223> c-term amidation

<400> 707
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Xaa
            20                  25                  30

<210> 708
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-decyl-1-piperazine)-acetyl)))

<220>
<223> c-term amidation

<400> 708
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 709
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-dodecyl-1-piperazine)-acetyl))

<220>
<223> c-term amidation

<400> 709
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 710
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-tetradecyl-1-piperazine)-acetyl))

<220>
<223> c-term amidation

<400> 710
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 711
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Lys(N-(2-(4-hexadecyl-1-piperazine)-acetyl))

<220>
<223> c-term amidation

<400> 711
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Gly Xaa
            20                  25                  30

<210> 712
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-decyl-1-piperazine)-acetyl))

<220>
<223> c-term amidation

<400> 712
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 713
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-dodecyl-1-piperazine)-acetyl))

<220>
<223> c-term amidation

<400> 713
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 714
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-tetradecyl-1-piperazine)-acetyl))

<220>
<223> c-term amidation

<400> 714
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 715
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aib

<220>
<221> MOD_RES
<222> (32)..(32)
<223> Lys(N-(2-(4-hexadecyl-1-piperazine)-acetyl))

<220>
<223> c-term amidation

<400> 715
His Ser Glu Gly Thr Phe Thr Ser Asp Val Ser Ser Tyr Leu Glu Gly
1               5                   10                  15

Gln Ala Arg Arg Glu Phe Ile Ala Trp Leu Val Arg Xaa Arg Xaa Xaa
            20                  25                  30

<210> 716
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-decanoyl)

<400> 716
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 717
<211> 30
<212> PRT

<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-decanoyl)

<400> 717
His Ser Glu Gly Thr Phe Thr Ser Asp Val Ser Ser Tyr Leu Glu Gly
1               5                   10                  15

Gln Ala Lys Arg Glu Phe Ile Ala Trp Leu Val Arg Xaa Xaa
            20                  25                  30

<210> 718
<211> 32
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Ava

<220>
<221> MOD_RES
<222> (32)..(32)
<223> Ado

<220>
<223> c-term amidation

<400> 718
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

<210> 719
<211> 33
<212> PRT
<213> Artificial Sequence

<220>

<223> Description of Artificial Sequence: Synthetic modified hGLP-1
      peptide

<220>
<221> MOD_RES
<222> (29)..(29)
<223> Aib

<220>
<221> MOD_RES
<222> (32)..(32)
<223> Ava

<220>
<221> MOD_RES
<222> (33)..(33)
<223> Ado

<220>
<223> C-term amidation

<400> 719
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Asp Xaa
            20                  25                  30

Xaa

<210> 720
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (31)..(31)
<223> Aun

<220>
<223> C-term amidation

<400> 720
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 721
<211> 30
<212> PRT
<213> Artificial Sequence

<220>
<223> Description of Artificial Sequence: Synthetic modified hGLP-1
      peptide


<220>
<221> MOD_RES
<222> (11)..(11)
<223> Aib

<220>
<221> MOD_RES
<222> (29)..(29)
<223> Aib

<220>
<223> c-term amidation

<400> 721
His Ser Glu Gly Thr Phe Thr Ser Asp Val Xaa Ser Tyr Leu Glu Gly
1               5                   10                  15


Gln Ala Ala Lys Glu Phe Ile Ala Trp Leu Val Lys Xaa Arg
            20                  25                  30


<210> 722
<211> 33
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<221> MOD_RES
<222> (31)..(31)
<223> D-Asp

<220>
<221> MOD_RES
<222> (32)..(32)
<223> Ava

<220>
<221> MOD_RES
<222> (33)..(33)
<223> Aun

<220>
<223> c-term amidation

<400> 722
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa Xaa
            20                  25                  30

Xaa

<210> 723
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 723
His Ser Glu Gly Thr Phe Thr Ser Asp Val Ser Ser Tyr Leu Glu Glu
1               5                   10                  15

Glu Ala Ala Lys Glu Phe Ile Ala Trp Leu Val Lys Xaa Arg
            20                  25                  30

<210> 724
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 724
His Ser Glu Gly Thr Phe Thr Ser Asp Val Ser Lys Tyr Leu Glu Gly
1               5                   10                  15

Gln Ala Ala Lys Glu Phe Ile Ala Trp Leu Val Lys Xaa Arg
            20                  25                  30

<210> 725
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 725
His Ser Glu Gly Thr Phe Thr Ser Asp Val Ser Ser Tyr Leu Glu Gly
1               5                   10                  15

Gln Ala Ala Lys Leu Phe Ile Ala Trp Leu Val Lys Xaa Arg
            20                  25                  30

<210> 726
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 726
His Ser Glu Gly Thr Phe Thr Ser Asp Val Ser Ser Tyr Leu Glu Gly
1               5                   10                  15

Gln Ala Ala Lys Glu Phe Ile Ala Trp Leu Lys Lys Xaa Arg
            20                  25                  30

<210> 727
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 727

His Ser Glu Gly Thr Phe Thr Ser Asp Val Ser Lys Tyr Leu Glu Gly
1               5                   10                  15

Gln Ala Ala Lys Leu Phe Ile Ala Trp Leu Val Lys Xaa Arg
            20                  25                  30

<210> 728
<211> 30
<212> PRT
<213> Artificial Sequence

<220>
<223> Description of Artificial Sequence: Synthetic modified hGLP-1
      peptide

<220>
<221> MOD_RES
<222> (30)..(30)
<223> D-Arg

<220>
<223> c-term amidation

<400> 728
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Xaa
            20                  25                  30

<210> 729
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> D-Arg

<220>
<223> c-term amidation

<400> 729
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg Xaa
            20                  25                  30

<210> 730

<211> 30
<212> PRT
<213> Artificial Sequence

<220>
<223> Description of Artificial Sequence: Synthetic modified hGLP-1
      peptide


<220>
<221> MOD_RES
<222> (21)..(21)
<223> Aib

<220>
<221> MOD_RES
<222> (29)..(29)
<223> Beta-Ala

<220>
<223> c-term amidation

<400> 730
His Ser Glu Gly Thr Phe Thr Ser Asp Val Ser Ser Tyr Leu Glu Gly
1               5                   10                  15


Gln Ala Ala Lys Xaa Phe Ile Ala Trp Leu Val Lys Xaa Arg
            20                  25                  30


<210> 731
<211> 32
<212> PRT
<213> Artificial Sequence

<220>
<223> Description of Artificial Sequence: Synthetic modified hGLP-1
      peptide


<220>
<221> MOD_RES
<222> (21)..(21)
<223> Aib

<220>
<221> MOD_RES
<222> (29)..(29)
<223> Beta-Ala

<220>
<221> MOD_RES
<222> (31)..(31)
<223> Beta-Ala

<220>
<223> c-term amidation

<400> 731
His Ser Glu Gly Thr Phe Thr Ser Asp Val Ser Ser Tyr Leu Glu Gly
1               5                   10                  15


Gln Ala Ala Lys Xaa Phe Ile Ala Trp Leu Val Lys Xaa Arg Xaa Arg
            20                  25                  30

<210> 732
<211> 33
<212> PRT
<213> Artificial Sequence

<220>
<223> Description of Artificial Sequence: Synthetic modified hGLP-1
      peptide


<220>
<221> MOD_RES
<222> (21)..(21)
<223> Aib

<220>
<221> MOD_RES
<222> (29)..(29)
<223> Beta-Ala

<220>
<221> MOD_RES
<222> (31)..(31)
<223> Beta-Ala

<220>
<223> c-term amidation

<400> 732
His Ser Glu Gly Thr Phe Thr Ser Asp Val Ser Ser Tyr Leu Glu Gly
1               5                   10                  15


Gln Ala Ala Lys Xaa Phe Ile Ala Trp Leu Val Lys Xaa Arg Xaa Arg
            20                  25                  30


Arg




<210> 733
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 733
His Ser Glu Gly Thr Phe Thr Ser Asp Val Ser Lys Tyr Leu Glu Gly
1               5                   10                  15


Gln Ala Ala Lys Lys Phe Ile Ala Trp Leu Val Lys Xaa Arg
            20                  25                  30

<210> 734
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 734
His Ser Glu Gly Thr Phe Thr Ser Asp Val Ser Ser Tyr Leu Glu Gly
1               5                   10                  15


Gln Ala Ala Lys Lys Phe Ile Ala Trp Leu Val Lys Xaa Arg
            20                  25                  30


<210> 735
<211> 32
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 735
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Arg Gly Arg
            20                  25                  30


<210> 736
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 736
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg
            20                  25                  30

<210> 737
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> D-Arg

<220>
<223> c-term amidation

<400> 737
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 738
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 738
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg Arg
            20                  25                  30

<210> 739

<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<223> c-term amidation

<400> 739
His Ser Glu Gly Thr Phe Thr Ser Asp Val Ser Ser Tyr Leu Glu Gly
1               5                   10                  15


Gln Ala Ala Lys Glu Phe Ile Ala Phe Leu Val Lys Xaa Arg
            20                  25                  30


<210> 740
<211> 30
<212> PRT
<213> Artificial Sequence

<220>
<223> Description of Artificial Sequence: Synthetic modified hGLP-1
      peptide


<220>
<221> MOD_RES
<222> (29)..(29)
<223> Aib

<220>
<223> c-term amidation

<400> 740
His Ser Glu Gly Thr Phe Thr Ser Asp Val Ser Ser Tyr Leu Glu Gly
1               5                   10                  15


Gln Ala Ala Lys Glu Phe Ile Ala Phe Leu Val Lys Xaa Arg
            20                  25                  30


<210> 741
<211> 30
<212> PRT
<213> Artificial Sequence

<220>
<223> Description of Artificial Sequence: Synthetic modified hGLP-1
      peptide


<220>
<221> MOD_RES
<222> (25)..(25)
<223> 1-Nal or 2-Nal

<220>
<221> MOD_RES
<222> (29)..(29)
<223> Aib

<220>
<223> c-term amidation

<400> 741
His Ser Glu Gly Thr Phe Thr Ser Asp Val Ser Ser Tyr Leu Glu Gly
1               5                   10                  15

Gln Ala Ala Lys Glu Phe Ile Ala Xaa Leu Val Lys Xaa Arg
            20                  25                  30

<210> 742
<211> 30
<212> PRT
<213> Artificial Sequence

<220>
<223> Description of Artificial Sequence: Synthetic modified hGLP-1
      peptide

<220>
<221> MOD_RES
<222> (22)..(22)
<223> 1-Nal or 2-Nal

<220>
<221> MOD_RES
<222> (25)..(25)
<223> 1-Nal or 2-Nal

<220>
<221> MOD_RES
<222> (29)..(29)
<223> Aib

<220>
<223> c-term amidation

<400> 742
His Ser Glu Gly Thr Phe Thr Ser Asp Val Ser Ser Tyr Leu Glu Gly
1               5                   10                  15

Gln Ala Ala Lys Glu Xaa Ile Ala Xaa Leu Val Lys Xaa Arg
            20                  25                  30

<210> 743
<211> 30
<212> PRT
<213> Artificial Sequence

<220>
<223> Description of Artificial Sequence: Synthetic modified hGLP-1
      peptide

<220>
<221> MOD_RES
<222> (25)..(25)

<223> 1-Nal or 2-Nal

<220>
<221> MOD_RES
<222> (29)..(29)
<223> Aib

<220>
<223> c-term amidation

<400> 743
His Ser Glu Gly Thr Phe Thr Ser Asp Val Ser Ser Tyr Leu Glu Gly
1               5                   10                  15

Gln Ala Ala Arg Glu Phe Ile Ala Xaa Leu Val Arg Xaa Arg
            20                  25                  30

<210> 744
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 744
His Ser Glu Gly Thr Phe Thr Ser Asp Val Ser Ser Tyr Leu Glu Gly
1               5                   10                  15

Gln Ala Ala Arg Glu Phe Ile Ala Phe Leu Val Arg Xaa Arg
            20                  25                  30

<210> 745
<211> 30
<212> PRT
<213> Artificial Sequence

<220>
<223> Description of Artificial Sequence: Synthetic modified hGLP-1
      peptide

<220>
<221> MOD_RES
<222> (13)..(13)
<223> 1-Nal or 2-Nal

<220>
<221> MOD_RES
<222> (25)..(25)
<223> 1-Nal or 2-Nal

<220>
<221> MOD_RES

<222> (29)..(29)
<223> Aib

<220>
<223> c-term amidation

<400> 745
His Ser Glu Gly Thr Phe Thr Ser Asp Val Ser Ser Xaa Leu Glu Gly
1               5                   10                  15

Gln Ala Ala Lys Glu Phe Ile Ala Xaa Leu Val Lys Xaa Arg
            20                  25                  30

<210> 746
<211> 30
<212> PRT
<213> Artificial Sequence

<220>
<223> Description of Artificial Sequence: Synthetic modified hGLP-1
      peptide

<220>
<221> MOD_RES
<222> (6)..(6)
<223> 1-Nal or 2-Nal

<220>
<221> MOD_RES
<222> (25)..(25)
<223> 1-Nal or 2-Nal

<220>
<221> MOD_RES
<222> (29)..(29)
<223> Aib

<220>
<223> c-term amidation

<400> 746
His Ser Glu Gly Thr Xaa Thr Ser Asp Val Ser Ser Tyr Leu Glu Gly
1               5                   10                  15

Gln Ala Ala Lys Glu Phe Ile Ala Xaa Leu Val Lys Xaa Arg
            20                  25                  30

<210> 747
<211> 30
<212> PRT
<213> Artificial Sequence

<220>
<223> Description of Artificial Sequence: Synthetic modified hGLP-1
      peptide

<220>
<221> MOD_RES

<222> (29)..(29)
<223> Aib

<220>
<221> MOD_RES
<222> (30)..(30)
<223> Lys(N-decanoyl)

<220>
<223> c-term amidation

<400> 747
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Xaa
            20                  25                  30

<210> 748
<211> 30
<212> PRT
<213> Artificial Sequence

<220>
<223> Description of Artificial Sequence: Synthetic modified hGLP-1
      peptide

<220>
<221> MOD_RES
<222> (20)..(20)
<223> Lys(N-decanoyl)

<220>
<221> MOD_RES
<222> (29)..(29)
<223> Aib

<220>
<223> c-term amidation

<400> 748
His Ser Glu Gly Thr Phe Thr Ser Asp Val Ser Ser Tyr Leu Glu Gly
1               5                   10                  15

Gln Ala Ala Xaa Glu Phe Ile Ala Trp Leu Val Arg Xaa Arg
            20                  25                  30

<210> 749
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<221> MOD_RES
<222> (30)..(30)
<223> Lys(N-dodecanoyl)


<220>
<223> c-term amidation

<400> 749
His Ser Glu Gly Thr Phe Thr Ser Asp Val Ser Ser Tyr Leu Glu Gly
1               5                   10                  15


Gln Ala Ala Arg Glu Phe Ile Ala Trp Leu Val Arg Xaa Xaa
            20                  25                  30


<210> 750
<211> 31
<212> PRT
<213> Artificial Sequence

<220>
<223> Description of Artificial Sequence: Synthetic modified hGLP-1
      peptide


<220>
<221> MOD_RES
<222> (29)..(29)
<223> Beta-Ala

<220>
<221> MOD_RES
<222> (31)..(31)
<223> Ser(O-decanoyl)

<220>
<223> c-term amidation

<400> 750
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Xaa Arg Xaa
            20                  25                  30



<210> 751
<211> 33
<212> PRT
<213> Artificial Sequence

<220>
<223> Description of Artificial Sequence: Synthetic modified hGLP-1
      peptide


<220>
<221> MOD_RES
<222> (21)..(21)
<223> Aib

<220>
<221> MOD_RES

<222> (29)..(29)
<223> Beta-Ala

<220>
<221> MOD_RES
<222> (31)..(31)
<223> Beta-Ala

<220>
<221> MOD_RES
<222> (33)..(33)
<223> Lys(N-octanoyl)

<220>
<223> c-term amidation

<400> 751
His Ser Glu Gly Thr Phe Thr Ser Asp Val Ser Ser Tyr Leu Glu Gly
1               5                   10                  15

Gln Ala Ala Lys Xaa Phe Ile Ala Trp Leu Val Lys Xaa Arg Xaa Arg
            20                  25                  30

Xaa

<210> 752
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> Lys(N-octanoyl)

<220>
<223> c-term amidation

<400> 752
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 753
<211> 31
<212> PRT
<213> Artificial Sequence

<220>
<223> Description of Artificial Sequence: Synthetic modified hGLP-1

peptide

<220>
<221> MOD_RES
<222> (29)..(29)
<223> Beta-Ala

<220>
<221> MOD_RES
<222> (31)..(31)
<223> Lys(N-decanoyl)

<220>
<223> c-term amidation

<400> 753
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 754
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> Lys(N-tetradecanoyl)

<220>
<223> c-term amidation

<400> 754
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 755
<211> 31
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<221> MOD_RES
<222> (31)..(31)
<223> Lys(N-dodecanoyl)

<220>
<223> c-term amidation

<400> 755
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Arg Xaa
            20                  25                  30

<210> 756
<211> 30
<212> PRT
<213> Artificial Sequence

<220>
<223> Description of Artificial Sequence: Synthetic modified hGLP-1
      peptide

<220>
<221> MOD_RES
<222> (28)..(28)
<223> Beta-Ala

<220>
<221> MOD_RES
<222> (30)..(30)
<223> Lys(N-dodecanoyl)

<220>
<223> c-term amidation

<400> 756
Ser Glu Gly Thr Phe Thr Ser Asp Val Ser Ser Tyr Leu Glu Gly Gln
1               5                   10                  15

Ala Ala Arg Glu Phe Ile Ala Trp Leu Val Arg Xaa Arg Xaa
            20                  25                  30

<210> 757
<211> 30
<212> PRT
<213> Artificial Sequence

<220>
<223> Description of Artificial Sequence: Synthetic modified hGLP-1
      peptide

<220>
<221> MOD_RES
<222> (29)..(29)

<223> Beta-Ala

<220>
<221> MOD_RES
<222> (30)..(30)
<223> Lys(N-Aec-decanoyl)

<220>
<223> c-term amidation

<400> 757
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Arg Xaa Xaa
            20                  25                  30

<210> 758
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 758
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Arg
            20                  25                  30

<210> 759
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 759
His Ser Glu Gly Thr Phe Thr Ser Asp Val Ser Ser Tyr Leu Glu Gly
1               5                   10                  15

Gln Ala Ala Arg Glu Phe Ile Ala Phe Leu Val Lys Xaa Arg
            20                  25                  30

<210> 760
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 760
His Ser Glu Gly Thr Phe Thr Ser Asp Val Ser Ser Tyr Leu Glu Gly
1               5                   10                  15

Gln Ala Ala Arg Glu Phe Ile Ala Phe Leu Val Lys Xaa Arg
            20                  25                  30

<210> 761
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 761
His Ser Glu Gly Thr Phe Thr Ser Asp Val Ser Ser Tyr Leu Glu Gly
1               5                   10                  15

Gln Ala Ala Arg Glu Phe Ile Ala Trp Leu Val Lys Xaa Arg
            20                  25                  30

<210> 762
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 762
His Ser Glu Gly Thr Phe Thr Ser Asp Val Ser Ser Tyr Leu Glu Gly
1               5                   10                  15

Gln Ala Ala Arg Glu Phe Ile Ala Phe Leu Val Arg Xaa Arg
            20                  25                  30

<210> 763
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 763
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Arg Xaa Arg
            20                  25                  30

<210> 764
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 764
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Arg Xaa Arg
            20                  25                  30

<210> 765
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 765
His Ser Glu Gly Thr Phe Thr Ser Asp Val Ser Ser Tyr Leu Glu Gly
1               5                   10                  15

Gln Ala Ala Lys Glu Phe Ile Ala Phe Leu Val Arg Xaa Arg
            20                  25                  30

<210> 766
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 766
His Ser Glu Gly Thr Phe Thr Ser Asp Val Ser Ser Tyr Leu Glu Gly
1               5                   10                  15

Gln Ala Ala Lys Glu Phe Ile Ala Phe Leu Val Arg Xaa Arg
            20                  25                  30

<210> 767
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 767
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 768
<211> 30
<212> PRT
<213> Artificial Sequence

<220>
<223> Description of Artificial Sequence: Synthetic modified hGLP-1
      peptide

<220>
<221> MOD_RES
<222> (2)..(2)
<223> Abu

<220>
<221> MOD_RES
<222> (29)..(29)
<223> Beta-Ala

<220>
<223> c-term amidation

<400> 768
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 769
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Beta-Ala

<220>
<223> c-term amidation

<400> 769
His Val Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 770
<211> 30
<212> PRT
<213> Artificial Sequence

<220>
<223> Description of Artificial Sequence: Synthetic modified hGLP-1
      peptide

<220>
<221> MOD_RES
<222> (2)..(2)
<223> Beta-Ala

<220>
<221> MOD_RES
<222> (29)..(29)
<223> Beta-Ala

<220>
<223> c-term amidation

<400> 770
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 771
<211> 30
<212> PRT
<213> Artificial Sequence

<220>
<223> Description of Artificial Sequence: Synthetic modified hGLP-1
      peptide

<220>
<221> MOD_RES
<222> (2)..(2)
<223> Abu

<220>
<221> MOD_RES
<222> (29)..(29)
<223> Aib

<220>
<223> c-term amidation

<400> 771
HIs Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 772
<211> 30
<212> PRT
<213> Artificial Sequence

<220>
<223> Description of Artificial Sequence: Synthetic modified hGLP-1
      peptide

<220>
<221> MOD_RES
<222> (29)..(29)
<223> Aib

<220>
<223> c-term amidation

<400> 772
His Val Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 773
<211> 30
<212> PRT
<213> Artificial Sequence

<220>
<223> Description of Artificial Sequence: Synthetic modified hGLP-1
      peptide

<220>
<221> MOD_RES
<222> (2)..(2)
<223> Beta-Ala

<220>
<221> MOD_RES
<222> (29)..(29)
<223> Aib

<220>
<223> c-term amidation

<400> 773
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 774
<211> 30
<212> PRT

<213> Artificial Sequence

<220>
<223> Description of Artificial Sequence: Illustrative synthetic
      modified hGLP-1 peptide

<220>
<221> MOD_RES
<222> (2)..(2)
<223> A5c

<220>
<223> c-term amidation

<400> 774
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 775
<211> 30
<212> PRT
<213> Homo sapiens

<220>
<223> Illustrative hGLP-1(7-36)

<220>
<223> c-term may or may not be amidated

<400> 775
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 776
<211> 31
<212> PRT
<213> Homo sapiens

<220>
<223> Illustrative hGLP-1(7-37)

<220>
<223> c-term may or may not be amidated

<400> 776
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210> 777
<211> 32
<212> PRT
<213> Homo sapiens

<220>
<223> Illustrative hGLP-1(7-38)

<220>
<223> c-term may or may not be amidated

<400> 777
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

<210> 778
<211> 30
<212> PRT
<213> Artificial Sequence

<220>
<223> Description of Artificial Sequence: Synthetic modified
      hGLP-1 peptide

<220>
<223> c-term amidation

<220>
<221> MOD_RES
<222> (1)..(1)
<223> Tma-His

<400> 778
Xaa Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 779
<211> 30
<212> PRT
<213> Artificial Sequence

<220>
<223> Description of Artificial Sequence: Synthetic modified
      hGLP-1 peptide

<220>
<223> c-term amidation

<220>
<221> MOD_RES
<222> (29)..(29)
<223> Beta-Ala

<400> 779
His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 780
<211> 30
<212> PRT
<213> Artificial Sequence

<220>
<223> Description of Artificial Sequence: Synthetic modified
      hGLP-1 peptide

<220>
<223> c-term amidation

<220>
<221> MOD_RES
<222> (29)..(29)
<223> Aib

<400> 780
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210> 781
<211> 30
<212> PRT
<213> Artificial Sequence

<220>
<223> Description of Artificial Sequence: Synthetic modified
      hGLP-1 peptide

<220>
<223> c-term amidation

<220>
<221> MOD_RES
<222> (29)..(29)
<223> Beta-Ala

<400> 781
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30